# EUROPEAN PATENT APPLICATION

(11) **EP 4 644 419 A1**
(43) Date of publication of application: **05.11.2025**
(21) Application number: 23909961.7
(22) Date of filing: 04.12.2023
(51) Int. Cl.: C07K 16/28, C12N 15/13, C12N 5/10, A61K 39/395, A61P 35/00

(54) **ANTIBODY TARGETING CD112R OR ANTIGEN-BINDING FRAGMENT THEREOF AND USE THEREOF**

(30) Priority: 30.12.2022 CN 202211743931; 08.05.2023 CN 202310508581
(71) Applicant: Hangzhou Zhongmeihuadong Pharmaceutical Co., Ltd., GongShu District HangZhou, Zhejiang 310011 (CN)
(72) Inventor: SHU, Qingyu, Hangzhou, Zhejiang 310011 (CN); LIN, Chen, Hangzhou, Zhejiang 310011 (CN); FANG, Xuefei, Hangzhou, Zhejiang 310011 (CN); CHEN, Min, Hangzhou, Zhejiang 310011 (CN)
(74) Representative: Nederlandsch Octrooibureau
(86) International application number: PCT/CN2023/136100
(87) International publication number: WO 2024/140031

(57) **Abstract**

The present application relates to an anti-CD112R antibody or an antigen-binding fragment thereof, and a preparation method therefor and the use thereof. The present invention further relates to the therapeutic and diagnostic uses of the antibodies and antibody fragments.

## Description

### Technical Field

The present application relates to an antibody or an antigen-binding fragment thereof that specifically recognizes CD 112R, and a preparation method and use thereof.

### Background Art

CD112R, also known as Poliovirus Receptor Related Immunoglobulin Domaincontaining Protein, Q6DKI7 or C7orf15, is a transmembrane domain protein of 326 amino acids in length, having a signal peptide (spanning amino acids 1 to 40), an ectodomain (spanning amino acids 41 to 171), a transmembrane domain (spanning amino acids 172 to 190), and a cytoplasmic domain (spanning amino acids 191 to 326). CD112R binds to Poliovirus Receptor Related Protein 2 (PVLR2, also known as nectin2, CD112 or herpesvirus entry mediator B (HVEB), human plasma membrane glycoprotein), i.e., the binding partner of CD112R.

Administration of anti-CD112R immunotherapy provides an opportunity to increase, enhance and maintain an immune response. CD112R is an inhibitory receptor mainly expressed by T cells and NK cells, and competes with the activating receptor CD226 for binding to CD112. The interaction between CD112 and CD112R has a higher affinity than the interaction with CD226, thereby effectively regulating CD226-mediated cell activation. Anti CD112R antibodies blocking the interaction with CD112 directly limit downstream of CD112R inhibitory signaling, while promote greater immune cell activation by increasing the interaction between CD226 with CD112. Treatment with anti-CD112R antibodies provides an opportunity to down-regulate inhibitory signals that may occur when CD112R-expressing immune cells bind to CD112 on tumor cells and/or myeloid cells within tumor microenvironments, and may enhance, increase and maintain anti-tumor immune responses.

Although the prior art has disclosed anti-CD112R antibodies, there is still an urgent need to develop high quality anti-CD112R antibodies.

### Summary

In a first aspect, the present disclosure provides an antibody targeting human CD112R, which has the following advantages:
(1) binding human/cynomolgus monkey CD112R protein with high affinity;
(2) inhibiting or blocking the binding of human CD112R protein to human CD112 or cells expressing human CD112;
(3) After blocking the binding of CD112R protein to CD112, CD8 T cells was stimulated to kill tumor cells.

In one embodiment, the invention provides an anti-CD112R antibody that specifically binds to CD112R and an antigen-binding fragment thereof, comprising:
1) three heavy chain CDRs (HCDR1, HCDR2, HCDR3) comprised in the heavy chain variable region as set forth in SEQ ID NO: 430 and three light chain CDRs (LCDR1, LCDR2, LCDR3) comprised in the light chain variable region as set forth in SEQ ID NO: 431;
2) three heavy chain CDRs (HCDR1, HCDR2, HCDR3) comprised in the heavy chain variable region as set forth in SEQ ID NO: 432 and three light chain CDRs (LCDR1, LCDR2, LCDR3) comprised in the light chain variable region as set forth in SEQ ID NO: 433;
3) three heavy chain CDRs (HCDR1, HCDR2, HCDR3) comprised in the heavy chain variable region as set forth in SEQ ID NO: 434 and three light chain CDRs (LCDR1, LCDR2, LCDR3) comprised in the light chain variable region as set forth in SEQ ID NO: 435;
4) three heavy chain CDRs (HCDR1, HCDR2, HCDR3) comprised in the heavy chain variable region as set forth in SEQ ID NO: 436 and three light chain CDRs (LCDR1, LCDR2, LCDR3) comprised in the light chain variable region as set forth in SEQ ID NO: 437;
5) three heavy chain CDRs (HCDR1, HCDR2, HCDR3) comprised in the heavy chain variable region as set forth in SEQ ID NO: 438 and three light chain CDRs (LCDR1, LCDR2, LCDR3) comprised in the light chain variable region as set forth in SEQ ID NO: 439;
6) three heavy chain CDRs (HCDR1, HCDR2, HCDR3) comprised in the heavy chain variable region as set forth in SEQ ID NO: 438 and three light chain CDRs (LCDR1, LCDR2, LCDR3) comprised in the light chain variable region as set forth in SEQ ID NO: 440;
7) three heavy chain CDRs (HCDR1, HCDR2, HCDR3) comprised in the heavy chain variable region as set forth in SEQ ID NO: 441 and three light chain CDRs (LCDR1, LCDR2, LCDR3) comprised in the light chain variable region as set forth in SEQ ID NO: 442;
8) three heavy chain CDRs (HCDR1, HCDR2, HCDR3) comprised in the heavy chain variable region as set forth in SEQ ID NO: 443 and three light chain CDRs (LCDR1, LCDR2, LCDR3) comprised in the light chain variable region as set forth in SEQ ID NO: 444;
9) three heavy chain CDRs (HCDR1, HCDR2, HCDR3) comprised in the heavy chain variable region as set forth in SEQ ID NO: 445 and three light chain CDRs (LCDR1, LCDR2, LCDR3) comprised in the light chain variable region as set forth in SEQ ID NO: 446;
10) three heavy chain CDRs (HCDR1, HCDR2, HCDR3) comprised in the heavy chain variable region as set forth in SEQ ID NO: 447 and three light chain CDRs (LCDR1, LCDR2, LCDR3) comprised in the light chain variable region as set forth in SEQ ID NO: 448;
11) three heavy chain CDRs (HCDR1, HCDR2, HCDR3) comprised in the heavy chain variable region as set forth in SEQ ID NO: 449 and three light chain CDRs (LCDR1, LCDR2, LCDR3) comprised in the light chain variable region as set forth in SEQ ID NO: 450;
12) three heavy chain CDRs (HCDR1, HCDR2, HCDR3) comprised in the heavy chain variable region as set forth in SEQ ID NO: 451 and three light chain CDRs (LCDR1, LCDR2, LCDR3) comprised in the light chain variable region as set forth in SEQ ID NO: 452;
13) three heavy chain CDRs (HCDR1, HCDR2, HCDR3) comprised in the heavy chain variable region as set forth in SEQ ID NO: 453 and three light chain CDRs (LCDR1, LCDR2, LCDR3) comprised in the light chain variable region as set forth in SEQ ID NO: 454;
14) three heavy chain CDRs (HCDR1, HCDR2, HCDR3) comprised in the heavy chain variable region as set forth in SEQ ID NO: 455 and three light chain CDRs (LCDR1, LCDR2, LCDR3) comprised in the light chain variable region as set forth in SEQ ID NO: 456;
15) three heavy chain CDRs (HCDR1, HCDR2, HCDR3) comprised in the heavy chain variable region as set forth in SEQ ID NO: 457 and three light chain CDRs (LCDR1, LCDR2, LCDR3) comprised in the light chain variable region as set forth in SEQ ID NO: 458;
16) three heavy chain CDRs (HCDR1, HCDR2, HCDR3) comprised in the heavy chain variable region as set forth in SEQ ID NO: 459 and three light chain CDRs (LCDR1, LCDR2, LCDR3) comprised in the light chain variable region as set forth in SEQ ID NO: 460;
17) three heavy chain CDRs (HCDR1, HCDR2, HCDR3) comprised in the heavy chain variable region as set forth in SEQ ID NO: 461 and three light chain CDRs (LCDR1, LCDR2, LCDR3) comprised in the light chain variable region as set forth in SEQ ID NO: 462;
18) three heavy chain CDRs (HCDR1, HCDR2, HCDR3) comprised in the heavy chain variable region as set forth in SEQ ID NO: 463 and three light chain CDRs (LCDR1, LCDR2, LCDR3) comprised in the light chain variable region as set forth in SEQ ID NO: 464;
19) three heavy chain CDRs (HCDR1, HCDR2, HCDR3) comprised in the heavy chain variable region as set forth in SEQ ID NO: 465 and three light chain CDRs (LCDR1, LCDR2, LCDR3) comprised in the light chain variable region as set forth in SEQ ID NO: 466;
20) three heavy chain CDRs (HCDR1, HCDR2, HCDR3) comprised in the heavy chain variable region as set forth in SEQ ID NO: 467 and three light chain CDRs (LCDR1, LCDR2, LCDR3) comprised in the light chain variable region as set forth in SEQ ID NO: 468;
21) three heavy chain CDRs (HCDR1, HCDR2, HCDR3) comprised in the heavy chain variable region as set forth in SEQ ID NO: 469 and three light chain CDRs (LCDR1, LCDR2, LCDR3) comprised in the light chain variable region as set forth in SEQ ID NO: 470;
22) three heavy chain CDRs (HCDR1, HCDR2, HCDR3) comprised in the heavy chain variable region as set forth in SEQ ID NO: 471 and three light chain CDRs (LCDR1, LCDR2, LCDR3) comprised in the light chain variable region as set forth in SEQ ID NO: 472;
23) three heavy chain CDRs (HCDR1, HCDR2, HCDR3) comprised in the heavy chain variable region as set forth in SEQ ID NO: 473 and three light chain CDRs (LCDR1, LCDR2, LCDR3) comprised in the light chain variable region as set forth in SEQ ID NO: 474;
24) three heavy chain CDRs (HCDR1, HCDR2, HCDR3) comprised in the heavy chain variable region as set forth in SEQ ID NO: 475 and three light chain CDRs (LCDR1, LCDR2, LCDR3) comprised in the light chain variable region as set forth in SEQ ID NO: 476;
25) three heavy chain CDRs (HCDR1, HCDR2, HCDR3) comprised in the heavy chain variable region as set forth in SEQ ID NO: 477 and three light chain CDRs (LCDR1, LCDR2, LCDR3) comprised in the light chain variable region as set forth in SEQ ID NO: 478;
26) three heavy chain CDRs (HCDR1, HCDR2, HCDR3) comprised in the heavy chain variable region as set forth in SEQ ID NO: 479 and three light chain CDRs (LCDR1, LCDR2, LCDR3) comprised in the light chain variable region as set forth in SEQ ID NO: 480;
27) three heavy chain CDRs (HCDR1, HCDR2, HCDR3) comprised in the heavy chain variable region as set forth in SEQ ID NO: 481 and three light chain CDRs (LCDR1, LCDR2, LCDR3) comprised in the light chain variable region as set forth in SEQ ID NO: 482;
28) three heavy chain CDRs (HCDR1, HCDR2, HCDR3) comprised in the heavy chain variable region as set forth in SEQ ID NO: 483 and three light chain CDRs (LCDR1, LCDR2, LCDR3) comprised in the light chain variable region as set forth in SEQ ID NO: 484;
29) three heavy chain CDRs (HCDR1, HCDR2, HCDR3) comprised in the heavy chain variable region as set forth in SEQ ID NO: 485 and three light chain CDRs (LCDR1, LCDR2, LCDR3) comprised in the light chain variable region as set forth in SEQ ID NO: 486;
30) three heavy chain CDRs (HCDR1, HCDR2, HCDR3) comprised in the heavy chain variable region as set forth in SEQ ID NO: 487 and three light chain CDRs (LCDR1, LCDR2, LCDR3) comprised in the light chain variable region as set forth in SEQ ID NO: 456;
31) three heavy chain CDRs (HCDR1, HCDR2, HCDR3) comprised in the heavy chain variable region as set forth in SEQ ID NO: 488 and three light chain CDRs (LCDR1, LCDR2, LCDR3) comprised in the light chain variable region as set forth in SEQ ID NO: 489;
32) three heavy chain CDRs (HCDR1, HCDR2, HCDR3) comprised in the heavy chain variable region as set forth in SEQ ID NO: 490 and three light chain CDRs (LCDR1, LCDR2, LCDR3) comprised in the light chain variable region as set forth in SEQ ID NO: 491;
33) three heavy chain CDRs (HCDR1, HCDR2, HCDR3) comprised in the heavy chain variable region as set forth in SEQ ID NO: 492 and three light chain CDRs (LCDR1, LCDR2, LCDR3) comprised in the light chain variable region as set forth in SEQ ID NO: 489;
34) three heavy chain CDRs (HCDR1, HCDR2, HCDR3) comprised in the heavy chain variable region as set forth in SEQ ID NO: 493 and three light chain CDRs (LCDR1, LCDR2, LCDR3) comprised in the light chain variable region as set forth in SEQ ID NO: 494;
35) three heavy chain CDRs (HCDR1, HCDR2, HCDR3) comprised in the heavy chain variable region as set forth in SEQ ID NO: 493 and three light chain CDRs (LCDR1, LCDR2, LCDR3) comprised in the light chain variable region as set forth in SEQ ID NO: 495;
36) three heavy chain CDRs (HCDR1, HCDR2, HCDR3) comprised in the heavy chain variable region as set forth in SEQ ID NO: 496 and three light chain CDRs (LCDR1, LCDR2, LCDR3) comprised in the light chain variable region as set forth in SEQ ID NO: 497;
37) three heavy chain CDRs (HCDR1, HCDR2, HCDR3) comprised in the heavy chain variable region as set forth in SEQ ID NO: 498 and three light chain CDRs (LCDR1, LCDR2, LCDR3) comprised in the light chain variable region as set forth in SEQ ID NO: 497;
38) three heavy chain CDRs (HCDR1, HCDR2, HCDR3) comprised in the heavy chain variable region as set forth in SEQ ID NO: 499 and three light chain CDRs (LCDR1, LCDR2, LCDR3) comprised in the light chain variable region as set forth in SEQ ID NO: 500;
39) three heavy chain CDRs (HCDR1, HCDR2, HCDR3) comprised in the heavy chain variable region as set forth in SEQ ID NO: 501 and three light chain CDRs (LCDR1, LCDR2, LCDR3) comprised in the light chain variable region as set forth in SEQ ID NO: 503;
40) three heavy chain CDRs (HCDR1, HCDR2, HCDR3) comprised in the heavy chain variable region as set forth in SEQ ID NO: 502 and three light chain CDRs (LCDR1, LCDR2, LCDR3) comprised in the light chain variable region as set forth in SEQ ID NO: 503;
41) three heavy chain CDRs (HCDR1, HCDR2, HCDR3) comprised in the heavy chain variable region as set forth in SEQ ID NO: 504 and three light chain CDRs (LCDR1, LCDR2, LCDR3) comprised in the light chain variable region as set forth in SEQ ID NO: 505;
42) three heavy chain CDRs (HCDR1, HCDR2, HCDR3) comprised in the heavy chain variable region as set forth in SEQ ID NO: 499 and three light chain CDRs (LCDR1, LCDR2, LCDR3) comprised in the light chain variable region as set forth in SEQ ID NO: 506;
43) three heavy chain CDRs (HCDR1, HCDR2, HCDR3) comprised in the heavy chain variable region as set forth in SEQ ID NO: 507 and three light chain CDRs (LCDR1, LCDR2, LCDR3) comprised in the light chain variable region as set forth in SEQ ID NO: 503;
44) three heavy chain CDRs (HCDR1, HCDR2, HCDR3) comprised in the heavy chain variable region as set forth in SEQ ID NO: 508 and three light chain CDRs (LCDR1, LCDR2, LCDR3) comprised in the light chain variable region as set forth in SEQ ID NO: 509;
45) three heavy chain CDRs (HCDR1, HCDR2, HCDR3) comprised in the heavy chain variable region as set forth in SEQ ID NO: 510 and three light chain CDRs (LCDR1, LCDR2, LCDR3) comprised in the light chain variable region as set forth in SEQ ID NO: 511;
46) three heavy chain CDRs (HCDR1, HCDR2, HCDR3) comprised in the heavy chain variable region as set forth in SEQ ID NO: 512 and three light chain CDRs (LCDR1, LCDR2, LCDR3) comprised in the light chain variable region as set forth in SEQ ID NO: 513;
47) three heavy chain CDRs (HCDR1, HCDR2, HCDR3) comprised in the heavy chain variable region as set forth in SEQ ID NO: 514 and three light chain CDRs (LCDR1, LCDR2, LCDR3) comprised in the light chain variable region as set forth in SEQ ID NO: 515;
48) three heavy chain CDRs (HCDR1, HCDR2, HCDR3) comprised in the heavy chain variable region as set forth in SEQ ID NO: 501 and three light chain CDRs (LCDR1, LCDR2, LCDR3) comprised in the light chain variable region as set forth in SEQ ID NO: 516;
49) three heavy chain CDRs (HCDR1, HCDR2, HCDR3) comprised in the heavy chain variable region as set forth in SEQ ID NO: 517 and three light chain CDRs (LCDR1, LCDR2, LCDR3) comprised in the light chain variable region as set forth in SEQ ID NO: 518;
50) three heavy chain CDRs (HCDR1, HCDR2, HCDR3) comprised in the heavy chain variable region as set forth in SEQ ID NO: 519 and three light chain CDRs (LCDR1, LCDR2, LCDR3) comprised in the light chain variable region as set forth in SEQ ID NO: 520;
51) three heavy chain CDRs (HCDR1, HCDR2, HCDR3) comprised in the heavy chain variable region as set forth in SEQ ID NO: 521 and three light chain CDRs (LCDR1, LCDR2, LCDR3) comprised in the light chain variable region as set forth in SEQ ID NO: 522;
52) three heavy chain CDRs (HCDR1, HCDR2, HCDR3) comprised in the heavy chain variable region as set forth in SEQ ID NO: 523 and three light chain CDRs (LCDR1, LCDR2, LCDR3) comprised in the light chain variable region as set forth in SEQ ID NO: 524;
53) three heavy chain CDRs (HCDR1, HCDR2, HCDR3) comprised in the heavy chain variable region as set forth in SEQ ID NO: 525 and three light chain CDRs (LCDR1, LCDR2, LCDR3) comprised in the light chain variable region as set forth in SEQ ID NO: 486;
54) three heavy chain CDRs (HCDR1, HCDR2, HCDR3) comprised in the heavy chain variable region as set forth in SEQ ID NO: 526 and three light chain CDRs (LCDR1, LCDR2, LCDR3) comprised in the light chain variable region as set forth in SEQ ID NO: 527;
55) three heavy chain CDRs (HCDR1, HCDR2, HCDR3) comprised in the heavy chain variable region as set forth in SEQ ID NO: 528 and three light chain CDRs (LCDR1, LCDR2, LCDR3) comprised in the light chain variable region as set forth in SEQ ID NO: 529;
56) three heavy chain CDRs (HCDR1, HCDR2, HCDR3) comprised in the heavy chain variable region as set forth in SEQ ID NO: 530 and three light chain CDRs (LCDR1, LCDR2, LCDR3) comprised in the light chain variable region as set forth in SEQ ID NO: 531;
57) three heavy chain CDRs (HCDR1, HCDR2, HCDR3) comprised in the heavy chain variable region as set forth in SEQ ID NO: 530 and three light chain CDRs (LCDR1, LCDR2, LCDR3) comprised in the light chain variable region as set forth in SEQ ID NO: 533;
58) three heavy chain CDRs (HCDR1, HCDR2, HCDR3) comprised in the heavy chain variable region as set forth in SEQ ID NO: 532 and three light chain CDRs (LCDR1, LCDR2, LCDR3) comprised in the light chain variable region as set forth in SEQ ID NO: 531;
59) three heavy chain CDRs (HCDR1, HCDR2, HCDR3) comprised in the heavy chain variable region as set forth in SEQ ID NO: 532 and three light chain CDRs (LCDR1, LCDR2, LCDR3) comprised in the light chain variable region as set forth in SEQ ID NO: 533;
60) three heavy chain CDRs (HCDR1, HCDR2, HCDR3) comprised in the heavy chain variable region as set forth in SEQ ID NO: 534 and three light chain CDRs (LCDR1, LCDR2, LCDR3) comprised in the light chain variable region as set forth in SEQ ID NO: 535;
61) three heavy chain CDRs (HCDR1, HCDR2, HCDR3) comprised in the heavy chain variable region as set forth in SEQ ID NO: 536 and three light chain CDRs (LCDR1, LCDR2, LCDR3) comprised in the light chain variable region as set forth in SEQ ID NO: 537;
62) three heavy chain CDRs (HCDR1, HCDR2, HCDR3) comprised in the heavy chain variable region as set forth in SEQ ID NO: 538 and three light chain CDRs (LCDR1, LCDR2, LCDR3) comprised in the light chain variable region as set forth in SEQ ID NO: 539;
63) three heavy chain CDRs (HCDR1, HCDR2, HCDR3) comprised in the heavy chain variable region as set forth in SEQ ID NO: 540 and three light chain CDRs (LCDR1, LCDR2, LCDR3) comprised in the light chain variable region as set forth in SEQ ID NO: 541;
64) three heavy chain CDRs (HCDR1, HCDR2, HCDR3) comprised in the heavy chain variable region as set forth in SEQ ID NO: 540 and three light chain CDRs (LCDR1, LCDR2, LCDR3) comprised in the light chain variable region as set forth in SEQ ID NO: 542;
65) three heavy chain CDRs (HCDR1, HCDR2, HCDR3) comprised in the heavy chain variable region as set forth in SEQ ID NO: 543 and three light chain CDRs (LCDR1, LCDR2, LCDR3) comprised in the light chain variable region as set forth in SEQ ID NO: 544;
66) three heavy chain CDRs (HCDR1, HCDR2, HCDR3) comprised in the heavy chain variable region as set forth in SEQ ID NO: 545 and three light chain CDRs (LCDR1, LCDR2, LCDR3) comprised in the light chain variable region as set forth in SEQ ID NO: 546;
67) three heavy chain CDRs (HCDR1, HCDR2, HCDR3) comprised in the heavy chain variable region as set forth in SEQ ID NO: 547 and three light chain CDRs (LCDR1, LCDR2, LCDR3) comprised in the light chain variable region as set forth in SEQ ID NO: 548;
68) three heavy chain CDRs (HCDR1, HCDR2, HCDR3) comprised in the heavy chain variable region as set forth in SEQ ID NO: 549 and three light chain CDRs (LCDR1, LCDR2, LCDR3) comprised in the light chain variable region as set forth in SEQ ID NO: 550;
69) three heavy chain CDRs (HCDR1, HCDR2, HCDR3) comprised in the heavy chain variable region as set forth in SEQ ID NO: 551 and three light chain CDRs (LCDR1, LCDR2, LCDR3) comprised in the light chain variable region as set forth in SEQ ID NO: 552;
70) three heavy chain CDRs (HCDR1, HCDR2, HCDR3) comprised in the heavy chain variable region as set forth in SEQ ID NO: 553 and three light chain CDRs (LCDR1, LCDR2, LCDR3) comprised in the light chain variable region as set forth in SEQ ID NO: 554;
71) three heavy chain CDRs (HCDR1, HCDR2, HCDR3) comprised in the heavy chain variable region as set forth in SEQ ID NO: 555 and three light chain CDRs (LCDR1, LCDR2, LCDR3) comprised in the light chain variable region as set forth in SEQ ID NO: 556;
72) three heavy chain CDRs (HCDR1, HCDR2, HCDR3) comprised in the heavy chain variable region as set forth in SEQ ID NO: 557 and three light chain CDRs (LCDR1, LCDR2, LCDR3) comprised in the light chain variable region as set forth in SEQ ID NO: 558;
73) three heavy chain CDRs (HCDR1, HCDR2, HCDR3) comprised in the heavy chain variable region as set forth in SEQ ID NO: 559 and three light chain CDRs (LCDR1, LCDR2, LCDR3) comprised in the light chain variable region as set forth in SEQ ID NO: 560;
74) three heavy chain CDRs (HCDR1, HCDR2, HCDR3) comprised in the heavy chain variable region as set forth in SEQ ID NO: 561 and three light chain CDRs (LCDR1, LCDR2, LCDR3) comprised in the light chain variable region as set forth in SEQ ID NO: 554;
75) three heavy chain CDRs (HCDR1, HCDR2, HCDR3) comprised in the heavy chain variable region as set forth in SEQ ID NO: 562 and three light chain CDRs (LCDR1, LCDR2, LCDR3) comprised in the light chain variable region as set forth in SEQ ID NO: 563;
76) three heavy chain CDRs (HCDR1, HCDR2, HCDR3) comprised in the heavy chain variable region as set forth in SEQ ID NO: 564 and three light chain CDRs (LCDR1, LCDR2, LCDR3) comprised in the light chain variable region as set forth in SEQ ID NO: 565;
77) three heavy chain CDRs (HCDR1, HCDR2, HCDR3) comprised in the heavy chain variable region as set forth in SEQ ID NO: 566 and three light chain CDRs (LCDR1, LCDR2, LCDR3) comprised in the light chain variable region as set forth in SEQ ID NO: 567;
78) three heavy chain CDRs (HCDR1, HCDR2, HCDR3) comprised in the heavy chain variable region as set forth in SEQ ID NO: 568 and three light chain CDRs (LCDR1, LCDR2, LCDR3) comprised in the light chain variable region as set forth in SEQ ID NO: 569;
79) three heavy chain CDRs (HCDR1, HCDR2, HCDR3) comprised in the heavy chain variable region as set forth in SEQ ID NO: 570 and three light chain CDRs (LCDR1, LCDR2, LCDR3) comprised in the light chain variable region as set forth in SEQ ID NO: 571;
80) three heavy chain CDRs (HCDR1, HCDR2, HCDR3) comprised in the heavy chain variable region as set forth in SEQ ID NO: 572 and three light chain CDRs (LCDR1, LCDR2, LCDR3) comprised in the light chain variable region as set forth in SEQ ID NO: 573;
81) three heavy chain CDRs (HCDR1, HCDR2, HCDR3) comprised in the heavy chain variable region as set forth in SEQ ID NO: 574 and three light chain CDRs (LCDR1, LCDR2, LCDR3) comprised in the light chain variable region as set forth in SEQ ID NO: 575;
82) three heavy chain CDRs (HCDR1, HCDR2, HCDR3) comprised in the heavy chain variable region as set forth in SEQ ID NO: 576 and three light chain CDRs (LCDR1, LCDR2, LCDR3) comprised in the light chain variable region as set forth in SEQ ID NO: 578;
83) three heavy chain CDRs (HCDR1, HCDR2, HCDR3) comprised in the heavy chain variable region as set forth in SEQ ID NO: 577 and three light chain CDRs (LCDR1, LCDR2, LCDR3) comprised in the light chain variable region as set forth in SEQ ID NO: 578;
84) three heavy chain CDRs (HCDR1, HCDR2, HCDR3) comprised in the heavy chain variable region as set forth in SEQ ID NO: 579 and three light chain CDRs (LCDR1, LCDR2, LCDR3) comprised in the light chain variable region as set forth in SEQ ID NO: 580;
85) three heavy chain CDRs (HCDR1, HCDR2, HCDR3) comprised in the heavy chain variable region as set forth in SEQ ID NO: 581 and three light chain CDRs (LCDR1, LCDR2, LCDR3) comprised in the light chain variable region as set forth in SEQ ID NO: 582;
86) three heavy chain CDRs (HCDR1, HCDR2, HCDR3) comprised in the heavy chain variable region as set forth in SEQ ID NO: 583 and three light chain CDRs (LCDR1, LCDR2, LCDR3) comprised in the light chain variable region as set forth in SEQ ID NO: 584;
87) three heavy chain CDRs (HCDR1, HCDR2, HCDR3) comprised in the heavy chain variable region as set forth in SEQ ID NO: 585 and three light chain CDRs (LCDR1, LCDR2, LCDR3) comprised in the light chain variable region as set forth in SEQ ID NO: 586;
88) three heavy chain CDRs (HCDR1, HCDR2, HCDR3) comprised in the heavy chain variable region as set forth in SEQ ID NO: 587 and three light chain CDRs (LCDR1, LCDR2, LCDR3) comprised in the light chain variable region as set forth in SEQ ID NO: 588;
89) three heavy chain CDRs (HCDR1, HCDR2, HCDR3) comprised in the heavy chain variable region as set forth in SEQ ID NO: 587 and three light chain CDRs (LCDR1, LCDR2, LCDR3) comprised in the light chain variable region as set forth in SEQ ID NO: 589;
90) three heavy chain CDRs (HCDR1, HCDR2, HCDR3) comprised in the heavy chain variable region as set forth in SEQ ID NO: 590 and three light chain CDRs (LCDR1, LCDR2, LCDR3) comprised in the light chain variable region as set forth in SEQ ID NO: 591;
91) three heavy chain CDRs (HCDR1, HCDR2, HCDR3) comprised in the heavy chain variable region as set forth in SEQ ID NO: 592 and three light chain CDRs (LCDR1, LCDR2, LCDR3) comprised in the light chain variable region as set forth in SEQ ID NO: 593;
92) three heavy chain CDRs (HCDR1, HCDR2, HCDR3) comprised in the heavy chain variable region as set forth in SEQ ID NO: 592 and three light chain CDRs (LCDR1, LCDR2, LCDR3) comprised in the light chain variable region as set forth in SEQ ID NO: 594;
93) three heavy chain CDRs (HCDR1, HCDR2, HCDR3) comprised in the heavy chain variable region as set forth in SEQ ID NO: 595 and three light chain CDRs (LCDR1, LCDR2, LCDR3) comprised in the light chain variable region as set forth in SEQ ID NO: 596;
94) three heavy chain CDRs (HCDR1, HCDR2, HCDR3) comprised in the heavy chain variable region as set forth in SEQ ID NO: 595 and three light chain CDRs (LCDR1, LCDR2, LCDR3) comprised in the light chain variable region as set forth in SEQ ID NO: 594;
95) three heavy chain CDRs (HCDR1, HCDR2, HCDR3) comprised in the heavy chain variable region as set forth in SEQ ID NO: 597 and three light chain CDRs (LCDR1, LCDR2, LCDR3) comprised in the light chain variable region as set forth in SEQ ID NO: 598;
96) three heavy chain CDRs (HCDR1, HCDR2, HCDR3) comprised in the heavy chain variable region as set forth in SEQ ID NO: 597 and three light chain CDRs (LCDR1, LCDR2, LCDR3) comprised in the light chain variable region as set forth in SEQ ID NO: 594;
97) three heavy chain CDRs (HCDR1, HCDR2, HCDR3) comprised in the heavy chain variable region as set forth in SEQ ID NO: 599 and three light chain CDRs (LCDR1, LCDR2, LCDR3) comprised in the light chain variable region as set forth in SEQ ID NO: 600;
98) three heavy chain CDRs (HCDR1, HCDR2, HCDR3) comprised in the heavy chain variable region as set forth in SEQ ID NO: 601 and three light chain CDRs (LCDR1, LCDR2, LCDR3) comprised in the light chain variable region as set forth in SEQ ID NO: 602;
99) three heavy chain CDRs (HCDR1, HCDR2, HCDR3) comprised in the heavy chain variable region as set forth in SEQ ID NO: 603 and three light chain CDRs (LCDR1, LCDR2, LCDR3) comprised in the light chain variable region as set forth in SEQ ID NO: 604;
100) three heavy chain CDRs (HCDR1, HCDR2, HCDR3) comprised in the heavy chain variable region as set forth in SEQ ID NO: 605 and three light chain CDRs (LCDR1, LCDR2, LCDR3) comprised in the light chain variable region as set forth in SEQ ID NO: 606;
101) three heavy chain CDRs (HCDR1, HCDR2, HCDR3) comprised in the heavy chain variable region as set forth in SEQ ID NO: 607 and three light chain CDRs (LCDR1, LCDR2, LCDR3) comprised in the light chain variable region as set forth in SEQ ID NO: 608;
102) three heavy chain CDRs (HCDR1, HCDR2, HCDR3) comprised in the heavy chain variable region as set forth in SEQ ID NO: 609 and three light chain CDRs (LCDR1, LCDR2, LCDR3) comprised in the light chain variable region as set forth in SEQ ID NO: 610;
103) three heavy chain CDRs (HCDR1, HCDR2, HCDR3) comprised in the heavy chain variable region as set forth in SEQ ID NO: 611 and three light chain CDRs (LCDR1, LCDR2, LCDR3) comprised in the light chain variable region as set forth in SEQ ID NO: 612;
104) three heavy chain CDRs (HCDR1, HCDR2, HCDR3) comprised in the heavy chain variable region as set forth in SEQ ID NO: 613 and three light chain CDRs (LCDR1, LCDR2, LCDR3) comprised in the light chain variable region as set forth in SEQ ID NO: 614;
105) three heavy chain CDRs (HCDR1, HCDR2, HCDR3) comprised in the heavy chain variable region as set forth in SEQ ID NO: 615 and three light chain CDRs (LCDR1, LCDR2, LCDR3) comprised in the light chain variable region as set forth in SEQ ID NO: 616;
106) three heavy chain CDRs (HCDR1, HCDR2, HCDR3) comprised in the heavy chain variable region as set forth in SEQ ID NO: 617 and three light chain CDRs (LCDR1, LCDR2, LCDR3) comprised in the light chain variable region as set forth in SEQ ID NO: 618;
107) three heavy chain CDRs (HCDR1, HCDR2, HCDR3) comprised in the heavy chain variable region as set forth in SEQ ID NO: 619 and three light chain CDRs (LCDR1, LCDR2, LCDR3) comprised in the light chain variable region as set forth in SEQ ID NO: 620;
108) three heavy chain CDRs (HCDR1, HCDR2, HCDR3) comprised in the heavy chain variable region as set forth in SEQ ID NO: 621 and three light chain CDRs (LCDR1, LCDR2, LCDR3) comprised in the light chain variable region as set forth in SEQ ID NO: 622;
109) three heavy chain CDRs (HCDR1, HCDR2, HCDR3) comprised in the heavy chain variable region as set forth in SEQ ID NO: 623 and three light chain CDRs (LCDR1, LCDR2, LCDR3) comprised in the light chain variable region as set forth in SEQ ID NO: 624;
110) three heavy chain CDRs (HCDR1, HCDR2, HCDR3) comprised in the heavy chain variable region as set forth in SEQ ID NO: 625 and three light chain CDRs (LCDR1, LCDR2, LCDR3) comprised in the light chain variable region as set forth in SEQ ID NO: 626;
111) three heavy chain CDRs (HCDR1, HCDR2, HCDR3) comprised in the heavy chain variable region as set forth in SEQ ID NO: 627 and three light chain CDRs (LCDR1, LCDR2, LCDR3) comprised in the light chain variable region as set forth in SEQ ID NO: 628;
112) three heavy chain CDRs (HCDR1, HCDR2, HCDR3) comprised in the heavy chain variable region as set forth in SEQ ID NO: 629 and three light chain CDRs (LCDR1, LCDR2, LCDR3) comprised in the light chain variable region as set forth in SEQ ID NO: 630;
113) three heavy chain CDRs (HCDR1, HCDR2, HCDR3) comprised in the heavy chain variable region as set forth in SEQ ID NO: 631 and three light chain CDRs (LCDR1, LCDR2, LCDR3) comprised in the light chain variable region as set forth in SEQ ID NO: 632;
114) three heavy chain CDRs (HCDR1, HCDR2, HCDR3) comprised in the heavy chain variable region as set forth in SEQ ID NO: 617 and three light chain CDRs (LCDR1, LCDR2, LCDR3) comprised in the light chain variable region as set forth in SEQ ID NO: 633;
115) three heavy chain CDRs (HCDR1, HCDR2, HCDR3) comprised in the heavy chain variable region as set forth in SEQ ID NO: 634 and three light chain CDRs (LCDR1, LCDR2, LCDR3) comprised in the light chain variable region as set forth in SEQ ID NO: 635;
116) three heavy chain CDRs (HCDR1, HCDR2, HCDR3) comprised in the heavy chain variable region as set forth in SEQ ID NO: 636 and three light chain CDRs (LCDR1, LCDR2, LCDR3) comprised in the light chain variable region as set forth in SEQ ID NO: 637;
117) three heavy chain CDRs (HCDR1, HCDR2, HCDR3) comprised in the heavy chain variable region as set forth in SEQ ID NO: 638 and three light chain CDRs (LCDR1, LCDR2, LCDR3) comprised in the light chain variable region as set forth in SEQ ID NO: 639;
118) three heavy chain CDRs (HCDR1, HCDR2, HCDR3) comprised in the heavy chain variable region as set forth in SEQ ID NO: 640 and three light chain CDRs (LCDR1, LCDR2, LCDR3) comprised in the light chain variable region as set forth in SEQ ID NO: 641;
119) three heavy chain CDRs (HCDR1, HCDR2, HCDR3) comprised in the heavy chain variable region as set forth in SEQ ID NO: 642 and three light chain CDRs (LCDR1, LCDR2, LCDR3) comprised in the light chain variable region as set forth in SEQ ID NO: 643;
120) three heavy chain CDRs (HCDR1, HCDR2, HCDR3) comprised in the heavy chain variable region as set forth in SEQ ID NO: 644 and three light chain CDRs (LCDR1, LCDR2, LCDR3) comprised in the light chain variable region as set forth in SEQ ID NO: 645;
121) three heavy chain CDRs (HCDR1, HCDR2, HCDR3) comprised in the heavy chain variable region as set forth in SEQ ID NO: 646 and three light chain CDRs (LCDR1, LCDR2, LCDR3) comprised in the light chain variable region as set forth in SEQ ID NO: 647;
122) three heavy chain CDRs (HCDR1, HCDR2, HCDR3) comprised in the heavy chain variable region as set forth in SEQ ID NO: 648 and three light chain CDRs (LCDR1, LCDR2, LCDR3) comprised in the light chain variable region as set forth in SEQ ID NO: 649;
123) three heavy chain CDRs (HCDR1, HCDR2, HCDR3) comprised in the heavy chain variable region as set forth in SEQ ID NO: 650 and three light chain CDRs (LCDR1, LCDR2, LCDR3) comprised in the light chain variable region as set forth in SEQ ID NO: 651;
124) three heavy chain CDRs (HCDR1, HCDR2, HCDR3) comprised in the heavy chain variable region as set forth in SEQ ID NO: 652 and three light chain CDRs (LCDR1, LCDR2, LCDR3) comprised in the light chain variable region as set forth in SEQ ID NO: 653;
125) three heavy chain CDRs (HCDR1, HCDR2, HCDR3) comprised in the heavy chain variable region as set forth in SEQ ID NO: 654 and three light chain CDRs (LCDR1, LCDR2, LCDR3) comprised in the light chain variable region as set forth in SEQ ID NO: 655;
126) 3 heavy chain CDRs (HCDR1, HCDR2, HCDR3) included in the heavy chain variable region shown in SEQ ID NO: 656 and three light chain CDRs (LCDR1, LCDR2, LCDR3) included in the light chain variable region shown in SEQ ID NO: 653.

In one embodiment, the invention provides an anti-CD112R antibody that specifically bind to CD112R and an antigen-binding fragment thereof, comprising:
1) HCDR1, HCDR2, HCDR3 comprising the sequence as set forth in SEQ ID NOs: 1, 64 and 162, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NOs: 239, 307 and 357, or a sequence containing an amino acid substitution (e.g., conservative substitution), deletion or insertion of one or more and no more than 3 amino acids relative to the sequence;
2) HCDR1, HCDR2, HCDR3 comprising the sequence as set forth in SEQ ID NOs: 2, 65 and 162, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NOs: 239, 308, and 358, or a sequence containing an amino acid substitution (e.g., conservative substitution), deletion or insertion of one or more and no more than 3 amino acids relative to the sequence;
3) HCDR1, HCDR2, HCDR3 comprising the sequence as set forth in SEQ ID NO: 3, 66, 163, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 240, 309, 359, or a sequence containing an amino acid substitution (e.g., conservative substitution), deletion or insertion of one or more and no more than 3 amino acids relative to the sequence;
4) HCDR1, HCDR2, HCDR3 comprising the sequence as set forth in SEQ ID NO: 4, 67, 164, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 239, 310, 360, or a sequence containing an amino acid substitution (e.g., conservative substitution), deletion or insertion of one or more and no more than 3 amino acids relative to the sequence;
5) HCDR1, HCDR2, HCDR3 comprising the sequence as set forth in SEQ ID NO: 5, 68, 165, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 361, or a sequence containing an amino acid substitution (e.g., conservative substitution), deletion or insertion of one or more and no more than 3 amino acids relative to the sequence;
6) HCDR1, HCDR2, HCDR3 comprising the sequence as set forth in SEQ ID NO: 5, 68, 165, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 242, 312, 362, or a sequence containing an amino acid substitution (e.g., conservative substitution), deletion or insertion of one or more and no more than 3 amino acids relative to the sequence;
7) HCDR1, HCDR2, HCDR3 comprising the sequence as set forth in SEQ ID NO: 6, 69, 166, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 243, 313, 363, or a sequence containing an amino acid substitution (e.g., conservative substitution), deletion or insertion of one or more and no more than 3 amino acids relative to the sequence;
8) HCDR1, HCDR2, HCDR3 comprising the sequence as set forth in SEQ ID NO: 7, 70, 167, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 244, 314, 364, or a sequence containing an amino acid substitution (e.g., conservative substitution), deletion or insertion of one or more and no more than 3 amino acids relative to the sequence;
9) HCDR1, HCDR2, HCDR3 comprising the sequence as set forth in SEQ ID NO: 8, 71, 168, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 245, 313, 365, or a sequence containing an amino acid substitution (e.g., conservative substitution), deletion or insertion of one or more and no more than 3 amino acids relative to the sequence;
10) HCDR1, HCDR2, HCDR3 comprising the sequence as set forth in SEQ ID NO: 3, 72, 163, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 359, or a sequence containing an amino acid substitution (e.g., conservative substitution), deletion or insertion of one or more and no more than 3 amino acids relative to the sequence;
11) HCDR1, HCDR2, HCDR3 comprising the sequence as set forth in SEQ ID NO: 9, 73, 168, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 245, 313, 363, or a sequence containing an amino acid substitution (e.g., conservative substitution), deletion or insertion of one or more and no more than 3 amino acids relative to the sequence;
12) HCDR1, HCDR2, HCDR3 comprising the sequence as set forth in SEQ ID NO: 3, 74, 163, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 246, 311, 359, or a sequence containing an amino acid substitution (e.g., conservative substitution), deletion or insertion of one or more and no more than 3 amino acids relative to the sequence;
13) HCDR1, HCDR2, HCDR3 comprising the sequence as set forth in SEQ ID NO: 10, 75, 169, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 247, 315, 366, or a sequence containing an amino acid substitution (e.g., conservative substitution), deletion or insertion of one or more and no more than 3 amino acids relative to the sequence;
14) HCDR1, HCDR2, HCDR3 comprising the sequence as set forth in SEQ ID NO: 10, 75, 170, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 247, 315, 366, or a sequence containing an amino acid substitution (e.g., conservative substitution), deletion or insertion of one or more and no more than 3 amino acids relative to the sequence;
15) HCDR1, HCDR2, HCDR3 comprising the sequence as set forth in SEQ ID NO: 9, 76, 168, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 243, 313, 363, or a sequence containing an amino acid substitution (e.g., conservative substitution), deletion or insertion of one or more and no more than 3 amino acids relative to the sequence;
16) HCDR1, HCDR2, HCDR3 comprising the sequence as set forth in SEQ ID NO: 9, 77, 168, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 248, 313, 363, or a sequence containing an amino acid substitution (e.g., conservative substitution), deletion or insertion of one or more and no more than 3 amino acids relative to the sequence;
17) HCDR1, HCDR2, HCDR3 comprising the sequence as set forth in SEQ ID NO: 8, 77, 168, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 243, 313, 365, or a sequence containing an amino acid substitution (e.g., conservative substitution), deletion or insertion of one or more and no more than 3 amino acids relative to the sequence;
18) HCDR1, HCDR2, HCDR3 comprising the sequence as set forth in SEQ ID NO: 7, 78, 167, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 244, 314, 364, or a sequence containing an amino acid substitution (e.g., conservative substitution), deletion or insertion of one or more and no more than 3 amino acids relative to the sequence;
19) HCDR1, HCDR2, HCDR3 comprising the sequence as set forth in SEQ ID NO: 11, 79, 171, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 249, 316, 367, or a sequence containing an amino acid substitution (e.g., conservative substitution), deletion or insertion of one or more and no more than 3 amino acids relative to the sequence;
20) HCDR1, HCDR2, HCDR3 comprising the sequence as set forth in SEQ ID NO: 2, 80, 162, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 239, 317, 368, or a sequence containing an amino acid substitution (e.g., conservative substitution), deletion or insertion of one or more and no more than 3 amino acids relative to the sequence;
21) HCDR1, HCDR2, HCDR3 comprising the sequence as set forth in SEQ ID NO: 12, 81, 172, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 250, 315, 369, or a sequence containing an amino acid substitution (e.g., conservative substitution), deletion or insertion of one or more and no more than 3 amino acids relative to the sequence;
22) HCDR1, HCDR2, HCDR3 comprising the sequence as set forth in SEQ ID NO: 10, 82, 169, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 247, 315, 370, or a sequence containing an amino acid substitution (e.g., conservative substitution), deletion or insertion of one or more and no more than 3 amino acids relative to the sequence;
23) HCDR1, HCDR2, HCDR3 comprising the sequence as set forth in SEQ ID NO: 2, 83, 173, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 242, 312, 371, or a sequence containing an amino acid substitution (e.g., conservative substitution), deletion or insertion of one or more and no more than 3 amino acids relative to the sequence;
24) HCDR1, HCDR2, HCDR3 comprising the sequence as set forth in SEQ ID NO: 13, 84, 173, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 242, 312, 372, or a sequence containing an amino acid substitution (e.g., conservative substitution), deletion or insertion of one or more and no more than 3 amino acids relative to the sequence;
25) HCDR1, HCDR2, HCDR3 comprising the sequence as set forth in SEQ ID NO: 2, 85, 174, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 251, 318, 373, or a sequence containing an amino acid substitution (e.g., conservative substitution), deletion or insertion of one or more and no more than 3 amino acids relative to the sequence;
26) HCDR1, HCDR2, HCDR3 comprising the sequence as set forth in SEQ ID NO: 14, 86, 175, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 252, 319, 374, or a sequence containing an amino acid substitution (e.g., conservative substitution), deletion or insertion of one or more and no more than 3 amino acids relative to the sequence;
27) HCDR1, HCDR2, HCDR3 comprising the sequence as set forth in SEQ ID NO: 15, 87, 176, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 253, 320, 375, or a sequence containing an amino acid substitution (e.g., conservative substitution), deletion or insertion of one or more and no more than 3 amino acids relative to the sequence;
28) HCDR1, HCDR2, HCDR3 comprising the sequence as set forth in SEQ ID NO: 16, 88, 177, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 254, 321, 376, or a sequence containing an amino acid substitution (e.g., conservative substitution), deletion or insertion of one or more and no more than 3 amino acids relative to the sequence;
29) HCDR1, HCDR2, HCDR3 comprising the sequence as set forth in SEQ ID NO: 2, 89, 178, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 377, or a sequence containing an amino acid substitution (e.g., conservative substitution), deletion or insertion of one or more and no more than 3 amino acids relative to the sequence;
30) HCDR1, HCDR2, HCDR3 comprising the sequence as set forth in SEQ ID NO: 17, 75, 169, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 247, 315, 366, or a sequence containing an amino acid substitution (e.g., conservative substitution), deletion or insertion of one or more and no more than 3 amino acids relative to the sequence;
31) HCDR1, HCDR2, HCDR3 comprising the sequence as set forth in SEQ ID NO: 18, 85, 162, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 239, 322, 378, or a sequence containing an amino acid substitution (e.g., conservative substitution), deletion or insertion of one or more and no more than 3 amino acids relative to the sequence;
32) HCDR1, HCDR2, HCDR3 comprising the sequence as set forth in SEQ ID NO: 2, 90, 162, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 255, 308, 368, or a sequence containing an amino acid substitution (e.g., conservative substitution), deletion or insertion of one or more and no more than 3 amino acids relative to the sequence;
33) HCDR1, HCDR2, HCDR3 comprising the sequence as set forth in SEQ ID NO: 19, 91, 179, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 239, 322, 378, or a sequence containing an amino acid substitution (e.g., conservative substitution), deletion or insertion of one or more and no more than 3 amino acids relative to the sequence;
34) HCDR1, HCDR2, HCDR3 comprising the sequence as set forth in SEQ ID NO: 2, 85, 180, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 256, 323, 379, or a sequence containing an amino acid substitution (e.g., conservative substitution), deletion or insertion of one or more and no more than 3 amino acids relative to the sequence;
35) HCDR1, HCDR2, HCDR3 comprising the sequence as set forth in SEQ ID NO: 2, 85, 180, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 239, 308, 380, or a sequence containing an amino acid substitution (e.g., conservative substitution), deletion or insertion of one or more and no more than 3 amino acids relative to the sequence;
36) HCDR1, HCDR2, HCDR3 comprising the sequence as set forth in SEQ ID NO: 20, 92, 181, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 257, 324, 381, or a sequence containing an amino acid substitution (e.g., conservative substitution), deletion or insertion of one or more and no more than 3 amino acids relative to the sequence;
37) HCDR1, HCDR2, HCDR3 comprising the sequence as set forth in SEQ ID NO: 21, 93, 182, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 258, 325, 382, or a sequence containing an amino acid substitution (e.g., conservative substitution), deletion or insertion of one or more and no more than 3 amino acids relative to the sequence;
38) HCDR1, HCDR2, HCDR3 comprising the sequence as set forth in SEQ ID NO: 22, 94, 183, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 259, 323, 383, or a sequence containing an amino acid substitution (e.g., conservative substitution), deletion or insertion of one or more and no more than 3 amino acids relative to the sequence;
39) HCDR1, HCDR2, HCDR3 comprising the sequence as set forth in SEQ ID NO: 23, 94, 183, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 259, 323, 383, or a sequence containing an amino acid substitution (e.g., conservative substitution), deletion or insertion of one or more and no more than 3 amino acids relative to the sequence;
40) HCDR1, HCDR2, HCDR3 comprising the sequence as set forth in SEQ ID NO: 24, 95, 184, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 247, 326, 384, or a sequence containing an amino acid substitution (e.g., conservative substitution), deletion or insertion of one or more and no more than 3 amino acids relative to the sequence;
41) HCDR1, HCDR2, HCDR3 comprising the sequence as set forth in SEQ ID NO: 21, 93, 182, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 247, 326, 385, or a sequence containing an amino acid substitution (e.g., conservative substitution), deletion or insertion of one or more and no more than 3 amino acids relative to the sequence;
42) HCDR1, HCDR2, HCDR3 comprising the sequence as set forth in SEQ ID NO: 22, 94, 183, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 259, 323, 383, or a sequence containing an amino acid substitution (e.g., conservative substitution), deletion or insertion of one or more and no more than 3 amino acids relative to the sequence;
43) HCDR1, HCDR2, HCDR3 comprising the sequence as set forth in SEQ ID NO: 25, 96, 185, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 260, 327, 386, or a sequence containing an amino acid substitution (e.g., conservative substitution), deletion or insertion of one or more and no more than 3 amino acids relative to the sequence;
44) HCDR1, HCDR2, HCDR3 comprising the sequence as set forth in SEQ ID NO: 26, 97, 186, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 261, 328, 387, or a sequence containing an amino acid substitution (e.g., conservative substitution), deletion or insertion of one or more and no more than 3 amino acids relative to the sequence;
45) HCDR1, HCDR2, HCDR3 comprising the sequence as set forth in SEQ ID NO: 27, 98, 187, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 262, 328, 388, or a sequence containing an amino acid substitution (e.g., conservative substitution), deletion or insertion of one or more and no more than 3 amino acids relative to the sequence;
46) HCDR1, HCDR2, HCDR3 comprising the sequence as set forth in SEQ ID NO: 28, 99, 188, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 263, 328, 388, or a sequence containing an amino acid substitution (e.g., conservative substitution), deletion or insertion of one or more and no more than 3 amino acids relative to the sequence;
47) HCDR1, HCDR2, HCDR3 comprising the sequence as set forth in SEQ ID NO: 22, 94, 183, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 239, 308, 389, or a sequence containing an amino acid substitution (e.g., conservative substitution), deletion or insertion of one or more and no more than 3 amino acids relative to the sequence;
48) HCDR1, HCDR2, HCDR3 comprising the sequence as set forth in SEQ ID NO: 5, 100, 189, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 264, 329, 390, or a sequence containing an amino acid substitution (e.g., conservative substitution), deletion or insertion of one or more and no more than 3 amino acids relative to the sequence;
49) HCDR1, HCDR2, HCDR3 comprising the sequence as set forth in SEQ ID NO: 24, 101, 190, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 247, 326, 385, or a sequence containing an amino acid substitution (e.g., conservative substitution), deletion or insertion of one or more and no more than 3 amino acids relative to the sequence;
50) HCDR1, HCDR2, HCDR3 comprising the sequence as set forth in SEQ ID NO: 28, 102, 191, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 265, 328, 391, or a sequence containing an amino acid substitution (e.g., conservative substitution), deletion or insertion of one or more and no more than 3 amino acids relative to the sequence;
51) HCDR1, HCDR2, HCDR3 comprising the sequence as set forth in SEQ ID NO: 28, 103, 192, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 266, 328, 388, or a sequence containing an amino acid substitution (e.g., conservative substitution), deletion or insertion of one or more and no more than 3 amino acids relative to the sequence;
52) HCDR1, HCDR2, HCDR3 comprising the sequence as set forth in SEQ ID NO: 29, 104, 163, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 377, or a sequence containing an amino acid substitution (e.g., conservative substitution), deletion or insertion of one or more and no more than 3 amino acids relative to the sequence;
53) HCDR1, HCDR2, HCDR3 comprising the sequence as set forth in SEQ ID NO: 30, 105, 193, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 267, 330, 392, or a sequence containing an amino acid substitution (e.g., conservative substitution), deletion or insertion of one or more and no more than 3 amino acids relative to the sequence;
54) HCDR1, HCDR2, HCDR3 comprising the sequence as set forth in SEQ ID NO: 5, 106, 194, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 268, 331, 393, or a sequence containing an amino acid substitution (e.g., conservative substitution), deletion or insertion of one or more and no more than 3 amino acids relative to the sequence;
55) HCDR1, HCDR2, HCDR3 comprising the sequence as set forth in SEQ ID NO: 31, 107, 195, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 256, 318, 392, or a sequence containing an amino acid substitution (e.g., conservative substitution), deletion or insertion of one or more and no more than 3 amino acids relative to the sequence;
56) HCDR1, HCDR2, HCDR3 comprising the sequence as set forth in SEQ ID NO: 31, 107, 195, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 269, 332, 394, or a sequence containing an amino acid substitution (e.g., conservative substitution), deletion or insertion of one or more and no more than 3 amino acids relative to the sequence;
57) HCDR1, HCDR2, HCDR3 comprising the sequence as set forth in SEQ ID NO: 32, 108, 196, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 256, 318, 392, or a sequence containing an amino acid substitution (e.g., conservative substitution), deletion or insertion of one or more and no more than 3 amino acids relative to the sequence;
58) HCDR1, HCDR2, HCDR3 comprising the sequence as set forth in SEQ ID NO: 32, 108, 196, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 269, 332, 394, or a sequence containing an amino acid substitution (e.g., conservative substitution), deletion or insertion of one or more and no more than 3 amino acids relative to the sequence;
59) HCDR1, HCDR2, HCDR3 comprising the sequence as set forth in SEQ ID NO: 29, 109, 197, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 395, or a sequence containing an amino acid substitution (e.g., conservative substitution), deletion or insertion of one or more and no more than 3 amino acids relative to the sequence;
60) HCDR1, HCDR2, HCDR3 comprising the sequence as set forth in SEQ ID NO: 33, 110, 198, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 270, 333, 396, or a sequence containing an amino acid substitution (e.g., conservative substitution), deletion or insertion of one or more and no more than 3 amino acids relative to the sequence;
61) HCDR1, HCDR2, HCDR3 comprising the sequence as set forth in SEQ ID NO: 5, 111, 199, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 256, 318, 397, or a sequence containing an amino acid substitution (e.g., conservative substitution), deletion or insertion of one or more and no more than 3 amino acids relative to the sequence;
62) HCDR1, HCDR2, HCDR3 comprising the sequence as set forth in SEQ ID NO: 34, 112, 200, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 269, 334, 398, or a sequence containing an amino acid substitution (e.g., conservative substitution), deletion or insertion of one or more and no more than 3 amino acids relative to the sequence;
63) HCDR1, HCDR2, HCDR3 comprising the sequence as set forth in SEQ ID NO: 34, 112, 200, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 271, 335, 399, or a sequence containing an amino acid substitution (e.g., conservative substitution), deletion or insertion of one or more and no more than 3 amino acids relative to the sequence;
64) HCDR1, HCDR2, HCDR3 comprising the sequence as set forth in SEQ ID NO: 35, 113, 201, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 400, or a sequence containing an amino acid substitution (e.g., conservative substitution), deletion or insertion of one or more and no more than 3 amino acids relative to the sequence;
65) HCDR1, HCDR2, HCDR3 comprising the sequence as set forth in SEQ ID NO: 36, 114, 202, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 272, 336, 401, or a sequence containing an amino acid substitution (e.g., conservative substitution), deletion or insertion of one or more and no more than 3 amino acids relative to the sequence;
66) HCDR1, HCDR2, HCDR3 comprising the sequence as set forth in SEQ ID NO: 29, 115, 203, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 402, or a sequence containing an amino acid substitution (e.g., conservative substitution), deletion or insertion of one or more and no more than 3 amino acids relative to the sequence;
67) HCDR1, HCDR2, HCDR3 comprising the sequence as set forth in SEQ ID NO: 37, 116, 204, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 273, 337, 403, or a sequence containing an amino acid substitution (e.g., conservative substitution), deletion or insertion of one or more and no more than 3 amino acids relative to the sequence;
68) HCDR1, HCDR2, HCDR3 comprising the sequence as set forth in SEQ ID NO: 5, 117, 205, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 274, 338, 404, or a sequence containing an amino acid substitution (e.g., conservative substitution), deletion or insertion of one or more and no more than 3 amino acids relative to the sequence;
69) HCDR1, HCDR2, HCDR3 comprising the sequence as set forth in SEQ ID NO: 38, 110, 198, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 270, 333, 405, or a sequence containing an amino acid substitution (e.g., conservative substitution), deletion or insertion of one or more and no more than 3 amino acids relative to the sequence;
70) HCDR1, HCDR2, HCDR3 comprising the sequence as set forth in SEQ ID NO: 39, 118, 204, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 275, 337, 406, or a sequence containing an amino acid substitution (e.g., conservative substitution), deletion or insertion of one or more and no more than 3 amino acids relative to the sequence;
71) HCDR1, HCDR2, HCDR3 comprising the sequence as set forth in SEQ ID NO: 5, 119, 206, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 276, 338, 407, or a sequence containing an amino acid substitution (e.g., conservative substitution), deletion or insertion of one or more and no more than 3 amino acids relative to the sequence;
72) HCDR1, HCDR2, HCDR3 comprising the sequence as set forth in SEQ ID NO: 29, 109, 197, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 395, or a sequence containing an amino acid substitution (e.g., conservative substitution), deletion or insertion of one or more and no more than 3 amino acids relative to the sequence;
73) HCDR1, HCDR2, HCDR3 comprising the sequence as set forth in SEQ ID NO: 38, 110, 198, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 270, 333, 405, or a sequence containing an amino acid substitution (e.g., conservative substitution), deletion or insertion of one or more and no more than 3 amino acids relative to the sequence;
74) HCDR1, HCDR2, HCDR3 comprising the sequence as set forth in SEQ ID NO: 5, 120, 205, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 277, 338, 407, or a sequence containing an amino acid substitution (e.g., conservative substitution), deletion or insertion of one or more and no more than 3 amino acids relative to the sequence;
75) HCDR1, HCDR2, HCDR3 comprising the sequence as set forth in SEQ ID NO: 5, 121, 207, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 278, 338, 404, or a sequence containing an amino acid substitution (e.g., conservative substitution), deletion or insertion of one or more and no more than 3 amino acids relative to the sequence;
76) HCDR1, HCDR2, HCDR3 comprising the sequence as set forth in SEQ ID NO: 35, 122, 201, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 279, 311, 408, or a sequence containing an amino acid substitution (e.g., conservative substitution), deletion or insertion of one or more and no more than 3 amino acids relative to the sequence;
77) HCDR1, HCDR2, HCDR3 comprising the sequence as set forth in SEQ ID NO: 35, 123, 208, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 280, 311, 400, or a sequence containing an amino acid substitution (e.g., conservative substitution), deletion or insertion of one or more and no more than 3 amino acids relative to the sequence;
78) HCDR1, HCDR2, HCDR3 comprising the sequence as set forth in SEQ ID NO: 5, 124, 209, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 281, 338, 407, or a sequence containing an amino acid substitution (e.g., conservative substitution), deletion or insertion of one or more and no more than 3 amino acids relative to the sequence;
79) HCDR1, HCDR2, HCDR3 comprising the sequence as set forth in SEQ ID NO: 40, 125, 210, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 282, 339, 409, or a sequence containing an amino acid substitution (e.g., conservative substitution), deletion or insertion of one or more and no more than 3 amino acids relative to the sequence;
80) HCDR1, HCDR2, HCDR3 comprising the sequence as set forth in SEQ ID NO: 41, 126, 211, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 245, 333, 410, or a sequence containing an amino acid substitution (e.g., conservative substitution), deletion or insertion of one or more and no more than 3 amino acids relative to the sequence;
81) HCDR1, HCDR2, HCDR3 comprising the sequence as set forth in SEQ ID NO: 10, 127, 212, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 239, 308, 411, or a sequence containing an amino acid substitution (e.g., conservative substitution), deletion or insertion of one or more and no more than 3 amino acids relative to the sequence;
82) HCDR1, HCDR2, HCDR3 comprising the sequence as set forth in SEQ ID NO: 40, 125, 213, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 239, 308, 411, or a sequence containing an amino acid substitution (e.g., conservative substitution), deletion or insertion of one or more and no more than 3 amino acids relative to the sequence;
83) HCDR1, HCDR2, HCDR3 comprising the sequence as set forth in SEQ ID NO: 42, 128, 214, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 283, 324, 412, or a sequence containing an amino acid substitution (e.g., conservative substitution), deletion or insertion of one or more and no more than 3 amino acids relative to the sequence;
84) HCDR1, HCDR2, HCDR3 comprising the sequence as set forth in SEQ ID NO: 43, 129, 215, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 284, 339, 409, or a sequence containing an amino acid substitution (e.g., conservative substitution), deletion or insertion of one or more and no more than 3 amino acids relative to the sequence;
85) HCDR1, HCDR2, HCDR3 comprising the sequence as set forth in SEQ ID NO: 42, 130, 216, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 285, 340, 409, or a sequence containing an amino acid substitution (e.g., conservative substitution), deletion or insertion of one or more and no more than 3 amino acids relative to the sequence;
86) HCDR1, HCDR2, HCDR3 comprising the sequence as set forth in SEQ ID NO: 44, 131, 217, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 286, 341, 413, or a sequence containing an amino acid substitution (e.g., conservative substitution), deletion or insertion of one or more and no more than 3 amino acids relative to the sequence;
87) HCDR1, HCDR2, HCDR3 comprising the sequence as set forth in SEQ ID NO: 44, 132, 218, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 282, 341, 414, or a sequence containing an amino acid substitution (e.g., conservative substitution), deletion or insertion of one or more and no more than 3 amino acids relative to the sequence;
88) HCDR1, HCDR2, HCDR3 comprising the sequence as set forth in SEQ ID NO: 44, 132, 218, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 287, 342, 415, or a sequence containing an amino acid substitution (e.g., conservative substitution), deletion or insertion of one or more and no more than 3 amino acids relative to the sequence;
89) HCDR1, HCDR2, HCDR3 comprising the sequence as set forth in SEQ ID NO: 45, 133, 219, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 288, 343, 416, or a sequence containing an amino acid substitution (e.g., conservative substitution), deletion or insertion of one or more and no more than 3 amino acids relative to the sequence;
90) HCDR1, HCDR2, HCDR3 comprising the sequence as set forth in SEQ ID NO: 46, 134, 220, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 289, 324, 417, or a sequence containing an amino acid substitution (e.g., conservative substitution), deletion or insertion of one or more and no more than 3 amino acids relative to the sequence;
91) HCDR1, HCDR2, HCDR3 comprising the sequence as set forth in SEQ ID NO: 46, 134, 220, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 290, 344, 418, or a sequence containing an amino acid substitution (e.g., conservative substitution), deletion or insertion of one or more and no more than 3 amino acids relative to the sequence;
92) HCDR1, HCDR2, HCDR3 comprising the sequence as set forth in SEQ ID NO: 47, 135, 221, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 291, 345, 407, or a sequence containing an amino acid substitution (e.g., conservative substitution), deletion or insertion of one or more and no more than 3 amino acids relative to the sequence;
93) HCDR1, HCDR2, HCDR3 comprising the sequence as set forth in SEQ ID NO: 47, 135, 221, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 290, 344, 418, or a sequence containing an amino acid substitution (e.g., conservative substitution), deletion or insertion of one or more and no more than 3 amino acids relative to the sequence;
94) HCDR1, HCDR2, HCDR3 comprising the sequence as set forth in SEQ ID NO: 48, 136, 222, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 292, 346, 409, or a sequence containing an amino acid substitution (e.g., conservative substitution), deletion or insertion of one or more and no more than 3 amino acids relative to the sequence;
95) HCDR1, HCDR2, HCDR3 comprising the sequence as set forth in SEQ ID NO: 48, 136, 222, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 290, 344, 418, or a sequence containing an amino acid substitution (e.g., conservative substitution), deletion or insertion of one or more and no more than 3 amino acids relative to the sequence;
96) HCDR1, HCDR2, HCDR3 comprising the sequence as set forth in SEQ ID NO: 49, 137, 163, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 240, 347, 419, or a sequence containing an amino acid substitution (e.g., conservative substitution), deletion or insertion of one or more and no more than 3 amino acids relative to the sequence;
97) HCDR1, HCDR2, HCDR3 comprising the sequence as set forth in SEQ ID NO: 45, 138, 223, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 288, 343, 416, or a sequence containing an amino acid substitution (e.g., conservative substitution), deletion or insertion of one or more and no more than 3 amino acids relative to the sequence;
98) HCDR1, HCDR2, HCDR3 comprising the sequence as set forth in SEQ ID NO: 50, 139, 224, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 249, 348, 420, or a sequence containing an amino acid substitution (e.g., conservative substitution), deletion or insertion of one or more and no more than 3 amino acids relative to the sequence;
99) HCDR1, HCDR2, HCDR3 comprising the sequence as set forth in SEQ ID NO: 51, 140, 225, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 256, 349, 421, or a sequence containing an amino acid substitution (e.g., conservative substitution), deletion or insertion of one or more and no more than 3 amino acids relative to the sequence;
100) HCDR1, HCDR2, HCDR3 comprising the sequence as set forth in SEQ ID NO: 47, 141, 221, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 293, 345, 407, or a sequence containing an amino acid substitution (e.g., conservative substitution), deletion or insertion of one or more and no more than 3 amino acids relative to the sequence;
101) HCDR1, HCDR2, HCDR3 comprising the sequence as set forth in SEQ ID NO: 52, 142, 226, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 294, 350, 422, or a sequence containing an amino acid substitution (e.g., conservative substitution), deletion or insertion of one or more and no more than 3 amino acids relative to the sequence;
102) HCDR1, HCDR2, HCDR3 comprising the sequence as set forth in SEQ ID NO: 53, 143, 178, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 295, 311, 423, or a sequence containing an amino acid substitution (e.g., conservative substitution), deletion or insertion of one or more and no more than 3 amino acids relative to the sequence;
103) HCDR1, HCDR2, HCDR3 comprising the sequence as set forth in SEQ ID NO: 53, 144, 178, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 361, or a sequence containing an amino acid substitution (e.g., conservative substitution), deletion or insertion of one or more and no more than 3 amino acids relative to the sequence;
104) HCDR1, HCDR2, HCDR3 comprising the sequence as set forth in SEQ ID NO: 53, 145, 227, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 361, or a sequence containing an amino acid substitution (e.g., conservative substitution), deletion or insertion of one or more and no more than 3 amino acids relative to the sequence;
105) HCDR1, HCDR2, HCDR3 comprising the sequence as set forth in SEQ ID NO: 54, 146, 228, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 296, 328, 391, or a sequence containing an amino acid substitution (e.g., conservative substitution), deletion or insertion of one or more and no more than 3 amino acids relative to the sequence;
106) HCDR1, HCDR2, HCDR3 comprising the sequence as set forth in SEQ ID NO: 55, 147, 228, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 266, 328, 391, or a sequence containing an amino acid substitution (e.g., conservative substitution), deletion or insertion of one or more and no more than 3 amino acids relative to the sequence;
107) HCDR1, HCDR2, HCDR3 comprising the sequence as set forth in SEQ ID NO: 50, 148, 229, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 297, 351, 424, or a sequence containing an amino acid substitution (e.g., conservative substitution), deletion or insertion of one or more and no more than 3 amino acids relative to the sequence;
108) HCDR1, HCDR2, HCDR3 comprising the sequence as set forth in SEQ ID NO: 56, 149, 230, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 298, 323, 425, or a sequence containing an amino acid substitution (e.g., conservative substitution), deletion or insertion of one or more and no more than 3 amino acids relative to the sequence;
109) HCDR1, HCDR2, HCDR3 comprising the sequence as set forth in SEQ ID NO: 57, 150, 231, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 299, 338, 407, or a sequence containing an amino acid substitution (e.g., conservative substitution), deletion or insertion of one or more and no more than 3 amino acids relative to the sequence;
110) HCDR1, HCDR2, HCDR3 comprising the sequence as set forth in SEQ ID NO: 58, 151, 232, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 300, 352, 426, or a sequence containing an amino acid substitution (e.g., conservative substitution), deletion or insertion of one or more and no more than 3 amino acids relative to the sequence;
111) HCDR1, HCDR2, HCDR3 comprising the sequence as set forth in SEQ ID NO: 59, 152, 233, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 301, 328, 388, or a sequence containing an amino acid substitution (e.g., conservative substitution), deletion or insertion of one or more and no more than 3 amino acids relative to the sequence;
112) HCDR1, HCDR2, HCDR3 comprising the sequence as set forth in SEQ ID NO: 60, 153, 234, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 239, 308, 360, or a sequence containing an amino acid substitution (e.g., conservative substitution), deletion or insertion of one or more and no more than 3 amino acids relative to the sequence;
113) HCDR1, HCDR2, HCDR3 comprising the sequence as set forth in SEQ ID NO: 54, 146, 228, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 302, 328, 391, or a sequence containing an amino acid substitution (e.g., conservative substitution), deletion or insertion of one or more and no more than 3 amino acids relative to the sequence;
114) HCDR1, HCDR2, HCDR3 comprising the sequence as set forth in SEQ ID NO: 61, 154, 235, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 241, 311, 361, or a sequence containing an amino acid substitution (e.g., conservative substitution), deletion or insertion of one or more and no more than 3 amino acids relative to the sequence;
115) HCDR1, HCDR2, HCDR3 comprising the sequence as set forth in SEQ ID NO: 40, 125, 210, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 282, 353, 409, or a sequence containing an amino acid substitution (e.g., conservative substitution), deletion or insertion of one or more and no more than 3 amino acids relative to the sequence;
116) HCDR1, HCDR2, HCDR3 comprising the sequence as set forth in SEQ ID NO: 44, 155, 236, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 303, 324, 409, or a sequence containing an amino acid substitution (e.g., conservative substitution), deletion or insertion of one or more and no more than 3 amino acids relative to the sequence;
117) HCDR1, HCDR2, HCDR3 comprising the sequence as set forth in SEQ ID NO: 44, 156, 217, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 282, 341, 414, or a sequence containing an amino acid substitution (e.g., conservative substitution), deletion or insertion of one or more and no more than 3 amino acids relative to the sequence;
118) HCDR1, HCDR2, HCDR3 comprising the sequence as set forth in SEQ ID NO: 62, 157, 237, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 304, 332, 427, or a sequence containing an amino acid substitution (e.g., conservative substitution), deletion or insertion of one or more and no more than 3 amino acids relative to the sequence;
119) HCDR1, HCDR2, HCDR3 comprising the sequence as set forth in SEQ ID NO: 24, 158, 184, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 247, 326, 428, or a sequence containing an amino acid substitution (e.g., conservative substitution), deletion or insertion of one or more and no more than 3 amino acids relative to the sequence;
120) HCDR1, HCDR2, HCDR3 comprising the sequence as set forth in SEQ ID NO: 3, 159, 189, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 249, 354, 382, or a sequence containing an amino acid substitution (e.g., conservative substitution), deletion or insertion of one or more and no more than 3 amino acids relative to the sequence;
121) HCDR1, HCDR2, HCDR3 comprising the sequence as set forth in SEQ ID NO: 63, 160, 238, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 305, 355, 429, or a sequence containing an amino acid substitution (e.g., conservative substitution), deletion or insertion of one or more and no more than 3 amino acids relative to the sequence; or
122) HCDR1, HCDR2, HCDR3 comprising the sequence as set forth in SEQ ID NO: 42, 161, 214, or a sequence containing an amino acid substitution (e.g., conservative substitution), deletion or insertion of one or more and no more than 3 amino acids relative to the sequence; and LCDR1, LCDR2, LCDR3 comprising the sequence as set forth in SEQ ID NO: 306, 356, 409, or a sequence containing an amino acid substitution (e.g., conservative substitution), deletion or insertion of one or more and no more than 3 amino acids relative to the sequence.

In one embodiment, the invention provides an anti-CD112R antibody that specifically binds CD112R and an antigen-binding fragment thereof, comprising a heavy chain variable region, wherein:
1) the heavy chain variable region comprises the amino acid sequence as set forth in SEQ ID NO: 430, or an amino acid sequence having at least 90%, 91%, 92%, 93%, 94%, 95%, 96%, 97%, 98% or 99% identity with the amino acid sequence of SEQ ID NO: 430, or consists of SEQ ID NO: 430;
2) the heavy chain variable region comprises the amino acid sequence as set forth in SEQ ID NO: 432, or an amino acid sequence having at least 90%, 91%, 92%, 93%, 94%, 95%, 96%, 97%, 98% or 99% identity with the amino acid sequence of SEQ ID NO: 432, or consists of SEQ ID NO: 432;
3) the heavy chain variable region comprises the amino acid sequence as set forth in SEQ ID NO: 434, or an amino acid sequence having at least 90%, 91%, 92%, 93%, 94%, 95%, 96%, 97%, 98% or 99% identity with the amino acid sequence of SEQ ID NO: 434, or consists of SEQ ID NO: 434;
4) the heavy chain variable region comprises the amino acid sequence as set forth in SEQ ID NO: 436, or an amino acid sequence having at least 90%, 91%, 92%, 93%, 94%, 95%, 96%, 97%, 98% or 99% identity with the amino acid sequence of SEQ ID NO: 436, or consists of SEQ ID NO: 436;
5) the heavy chain variable region comprises the amino acid sequence as set forth in SEQ ID NO: 438, or an amino acid sequence having at least 90%, 91%, 92%, 93%, 94%, 95%, 96%, 97%, 98% or 99% identity with the amino acid sequence of SEQ ID NO: 438, or consists of SEQ ID NO: 438;
6) the heavy chain variable region comprises the amino acid sequence as set forth in SEQ ID NO: 441, or an amino acid sequence having at least 90%, 91%, 92%, 93%, 94%, 95%, 96%, 97%, 98% or 99% identity with the amino acid sequence of SEQ ID NO: 441, or consists of SEQ ID NO: 441;
7) the heavy chain variable region comprises the amino acid sequence as set forth in SEQ ID NO: 443, or an amino acid sequence having at least 90%, 91%, 92%, 93%, 94%, 95%, 96%, 97%, 98% or 99% identity with the amino acid sequence of SEQ ID NO: 443, or consists of SEQ ID NO: 443;
8) the heavy chain variable region comprises the amino acid sequence as set forth in SEQ ID NO: 445, or an amino acid sequence having at least 90%, 91%, 92%, 93%, 94%, 95%, 96%, 97%, 98% or 99% identity with the amino acid sequence of SEQ ID NO: 445, or consists of SEQ ID NO: 445;
9) the heavy chain variable region comprises the amino acid sequence as set forth in SEQ ID NO: 447, or an amino acid sequence having at least 90%, 91%, 92%, 93%, 94%, 95%, 96%, 97%, 98% or 99% identity with the amino acid sequence of SEQ ID NO: 447, or consists of SEQ ID NO: 447;
10) the heavy chain variable region comprises the amino acid sequence as set forth in SEQ ID NO: 449, or an amino acid sequence having at least 90%, 91%, 92%, 93%, 94%, 95%, 96%, 97%, 98% or 99% identity with the amino acid sequence of SEQ ID NO: 449, or consists of SEQ ID NO: 449;
11) the heavy chain variable region comprises the amino acid sequence as set forth in SEQ ID NO: 451, or an amino acid sequence having at least 90%, 91%, 92%, 93%, 94%, 95%, 96%, 97%, 98% or 99% identity with the amino acid sequence of SEQ ID NO: 451, or consists of SEQ ID NO: 451;
12) the heavy chain variable region comprises the amino acid sequence as set forth in SEQ ID NO: 453, or an amino acid sequence having at least 90%, 91%, 92%, 93%, 94%, 95%, 96%, 97%, 98% or 99% identity with the amino acid sequence of SEQ ID NO: 453, or consists of SEQ ID NO: 453;
13) the heavy chain variable region comprises the amino acid sequence as set forth in SEQ ID NO: 455, or an amino acid sequence having at least 90%, 91%, 92%, 93%, 94%, 95%, 96%, 97%, 98% or 99% identity with the amino acid sequence of SEQ ID NO: 455, or consists of SEQ ID NO: 455;
14) the heavy chain variable region comprises the amino acid sequence as set forth in SEQ ID NO: 457, or an amino acid sequence having at least 90%, 91%, 92%, 93%, 94%, 95%, 96%, 97%, 98% or 99% identity with the amino acid sequence of SEQ ID NO: 457, or consists of SEQ ID NO: 457;
15) the heavy chain variable region comprises the amino acid sequence as set forth in SEQ ID NO: 459, or an amino acid sequence having at least 90%, 91%, 92%, 93%, 94%, 95%, 96%, 97%, 98% or 99% identity with the amino acid sequence of SEQ ID NO: 459, or consists of SEQ ID NO: 459;
16) the heavy chain variable region comprises the amino acid sequence as set forth in SEQ ID NO: 461, or an amino acid sequence having at least 90%, 91%, 92%, 93%, 94%, 95%, 96%, 97%, 98% or 99% identity with the amino acid sequence of SEQ ID NO: 461, or consists of SEQ ID NO: 461;
17) the heavy chain variable region comprises the amino acid sequence as set forth in SEQ ID NO: 463, or an amino acid sequence having at least 90%, 91%, 92%, 93%, 94%, 95%, 96%, 97%, 98% or 99% identity with the amino acid sequence of SEQ ID NO: 463, or consists of SEQ ID NO: 463;
18) the heavy chain variable region comprises the amino acid sequence as set forth in SEQ ID NO: 465, or an amino acid sequence having at least 90%, 91%, 92%, 93%, 94%, 95%, 96%, 97%, 98% or 99% identity with the amino acid sequence of SEQ ID NO: 465, or consists of SEQ ID NO: 465;
19) the heavy chain variable region comprises the amino acid sequence as set forth in SEQ ID NO: 467, or an amino acid sequence having at least 90%, 91%, 92%, 93%, 94%, 95%, 96%, 97%, 98% or 99% identity with the amino acid sequence of SEQ ID NO: 467, or consists of SEQ ID NO: 467;
20) the heavy chain variable region comprises the amino acid sequence as set forth in SEQ ID NO: 469, or an amino acid sequence having at least 90%, 91%, 92%, 93%, 94%, 95%, 96%, 97%, 98% or 99% identity with the amino acid sequence of SEQ ID NO: 469, or consists of SEQ ID NO: 469;
21) the heavy chain variable region comprises the amino acid sequence as set forth in SEQ ID NO: 471, or an amino acid sequence having at least 90%, 91%, 92%, 93%, 94%, 95%, 96%, 97%, 98% or 99% identity with the amino acid sequence of SEQ ID NO: 471, or consists of SEQ ID NO: 471;
22) the heavy chain variable region comprises the amino acid sequence as set forth in SEQ ID NO: 473, or an amino acid sequence having at least 90%, 91%, 92%, 93%, 94%, 95%, 96%, 97%, 98% or 99% identity with the amino acid sequence of SEQ ID NO: 473, or consists of SEQ ID NO: 473;
23) the heavy chain variable region comprises the amino acid sequence as set forth in SEQ ID NO: 475, or an amino acid sequence having at least 90%, 91%, 92%, 93%, 94%, 95%, 96%, 97%, 98% or 99% identity with the amino acid sequence of SEQ ID NO: 475, or consists of SEQ ID NO: 475;
24) the heavy chain variable region comprises the amino acid sequence as set forth in SEQ ID NO: 477, or an amino acid sequence having at least 90%, 91%, 92%, 93%, 94%, 95%, 96%, 97%, 98% or 99% identity with the amino acid sequence of SEQ ID NO: 477, or consists of SEQ ID NO: 477;
25) the heavy chain variable region comprises the amino acid sequence as set forth in SEQ ID NO: 479, or an amino acid sequence having at least 90%, 91%, 92%, 93%, 94%, 95%, 96%, 97%, 98% or 99% identity with the amino acid sequence of SEQ ID NO: 479, or consists of SEQ ID NO: 479;
26) the heavy chain variable region comprises the amino acid sequence as set forth in SEQ ID NO: 481, or an amino acid sequence having at least 90%, 91%, 92%, 93%, 94%, 95%, 96%, 97%, 98% or 99% identity with the amino acid sequence of SEQ ID NO: 481, or consists of SEQ ID NO: 481;
27) the heavy chain variable region comprises the amino acid sequence as set forth in SEQ ID NO: 483, or an amino acid sequence having at least 90%, 91%, 92%, 93%, 94%, 95%, 96%, 97%, 98% or 99% identity with the amino acid sequence of SEQ ID NO: 483, or consists of SEQ ID NO: 483;
28) the heavy chain variable region comprises the amino acid sequence as set forth in SEQ ID NO: 485, or an amino acid sequence having at least 90%, 91%, 92%, 93%, 94%, 95%, 96%, 97%, 98% or 99% identity with the amino acid sequence of SEQ ID NO: 485, or consists of SEQ ID NO: 485;
29) the heavy chain variable region comprises the amino acid sequence as set forth in SEQ ID NO: 487, or an amino acid sequence having at least 90%, 91%, 92%, 93%, 94%, 95%, 96%, 97%, 98% or 99% identity with the amino acid sequence of SEQ ID NO: 487, or consists of SEQ ID NO: 487;
30) the heavy chain variable region comprises the amino acid sequence as set forth in SEQ ID NO: 488, or an amino acid sequence having at least 90%, 91%, 92%, 93%, 94%, 95%, 96%, 97%, 98% or 99% identity with the amino acid sequence of SEQ ID NO: 488, or consists of SEQ ID NO: 488;
31) the heavy chain variable region comprises the amino acid sequence as set forth in SEQ ID NO: 490, or an amino acid sequence having at least 90%, 91%, 92%, 93%, 94%, 95%, 96%, 97%, 98% or 99% identity with the amino acid sequence of SEQ ID NO: 490, or consists of SEQ ID NO: 490;
32) the heavy chain variable region comprises the amino acid sequence as set forth in SEQ ID NO: 492, or an amino acid sequence having at least 90%, 91%, 92%, 93%, 94%, 95%, 96%, 97%, 98% or 99% identity with the amino acid sequence of SEQ ID NO: 492, or consists of SEQ ID NO: 492;
33) the heavy chain variable region comprises the amino acid sequence as set forth in SEQ ID NO: 493, or an amino acid sequence having at least 90%, 91%, 92%, 93%, 94%, 95%, 96%, 97%, 98% or 99% identity with the amino acid sequence of SEQ ID NO: 493, or consists of SEQ ID NO: 493;
34) the heavy chain variable region comprises the amino acid sequence as set forth in SEQ ID NO: 496, or an amino acid sequence having at least 90%, 91%, 92%, 93%, 94%, 95%, 96%, 97%, 98% or 99% identity with the amino acid sequence of SEQ ID NO: 496, or consists of SEQ ID NO: 496;
35) the heavy chain variable region comprises the amino acid sequence as set forth in SEQ ID NO: 498, or an amino acid sequence having at least 90%, 91%, 92%, 93%, 94%, 95%, 96%, 97%, 98% or 99% identity with the amino acid sequence of SEQ ID NO: 498, or consists of SEQ ID NO: 498;
36) the heavy chain variable region comprises the amino acid sequence as set forth in SEQ ID NO: 499, or an amino acid sequence having at least 90%, 91%, 92%, 93%, 94%, 95%, 96%, 97%, 98% or 99% identity with the amino acid sequence of SEQ ID NO: 499, or consists of SEQ ID NO: 499;
37) the heavy chain variable region comprises the amino acid sequence as set forth in SEQ ID NO: 501, or an amino acid sequence having at least 90%, 91%, 92%, 93%, 94%, 95%, 96%, 97%, 98% or 99% identity with the amino acid sequence of SEQ ID NO: 501, or consists of SEQ ID NO: 501;
38) the heavy chain variable region comprises the amino acid sequence as set forth in SEQ ID NO: 502, or an amino acid sequence having at least 90%, 91%, 92%, 93%, 94%, 95%, 96%, 97%, 98% or 99% identity with the amino acid sequence of SEQ ID NO: 502, or consists of SEQ ID NO: 502;
39) the heavy chain variable region comprises the amino acid sequence as set forth in SEQ ID NO: 504, or an amino acid sequence having at least 90%, 91%, 92%, 93%, 94%, 95%, 96%, 97%, 98% or 99% identity with the amino acid sequence of SEQ ID NO: 504, or consists of SEQ ID NO: 504;
40) the heavy chain variable region comprises the amino acid sequence as set forth in SEQ ID NO: 507, or an amino acid sequence having at least 90%, 91%, 92%, 93%, 94%, 95%, 96%, 97%, 98% or 99% identity with the amino acid sequence of SEQ ID NO: 507, or consists of SEQ ID NO: 507;
41) the heavy chain variable region comprises the amino acid sequence as set forth in SEQ ID NO: 508, or an amino acid sequence having at least 90%, 91%, 92%, 93%, 94%, 95%, 96%, 97%, 98% or 99% identity with the amino acid sequence of SEQ ID NO: 508, or consists of SEQ ID NO: 508;
42) the heavy chain variable region comprises the amino acid sequence as set forth in SEQ ID NO: 510, or an amino acid sequence having at least 90%, 91%, 92%, 93%, 94%, 95%, 96%, 97%, 98% or 99% identity with the amino acid sequence of SEQ ID NO: 510, or consists of SEQ ID NO: 510;
43) the heavy chain variable region comprises the amino acid sequence as set forth in SEQ ID NO: 512, or an amino acid sequence having at least 90%, 91%, 92%, 93%, 94%, 95%, 96%, 97%, 98% or 99% identity with the amino acid sequence of SEQ ID NO: 512, or consists of SEQ ID NO: 512;
44) the heavy chain variable region comprises the amino acid sequence as set forth in SEQ ID NO: 514, or an amino acid sequence having at least 90%, 91%, 92%, 93%, 94%, 95%, 96%, 97%, 98% or 99% identity with the amino acid sequence of SEQ ID NO: 514, or consists of SEQ ID NO: 514;
45) the heavy chain variable region comprises the amino acid sequence as set forth in SEQ ID NO: 517, or an amino acid sequence having at least 90%, 91%, 92%, 93%, 94%, 95%, 96%, 97%, 98% or 99% identity with the amino acid sequence of SEQ ID NO: 517, or consists of SEQ ID NO: 517;
46) the heavy chain variable region comprises the amino acid sequence as set forth in SEQ ID NO: 519, or an amino acid sequence having at least 90%, 91%, 92%, 93%, 94%, 95%, 96%, 97%, 98% or 99% identity with the amino acid sequence of SEQ ID NO: 519, or consists of SEQ ID NO: 519;
47) the heavy chain variable region comprises the amino acid sequence as set forth in SEQ ID NO: 521, or an amino acid sequence having at least 90%, 91%, 92%, 93%, 94%, 95%, 96%, 97%, 98% or 99% identity with the amino acid sequence of SEQ ID NO: 521, or consists of SEQ ID NO: 521;
48) the heavy chain variable region comprises the amino acid sequence as set forth in SEQ ID NO: 523, or an amino acid sequence having at least 90%, 91%, 92%, 93%, 94%, 95%, 96%, 97%, 98% or 99% identity with the amino acid sequence of SEQ ID NO: 523, or consists of SEQ ID NO: 523;
49) the heavy chain variable region comprises the amino acid sequence as set forth in SEQ ID NO: 525, or an amino acid sequence having at least 90%, 91%, 92%, 93%, 94%, 95%, 96%, 97%, 98% or 99% identity with the amino acid sequence of SEQ ID NO: 525, or consists of SEQ ID NO: 525;
50) the heavy chain variable region comprises the amino acid sequence as set forth in SEQ ID NO: 526, or an amino acid sequence having at least 90%, 91%, 92%, 93%, 94%, 95%, 96%, 97%, 98% or 99% identity with the amino acid sequence of SEQ ID NO: 526, or consists of SEQ ID NO: 526;
51) the heavy chain variable region comprises the amino acid sequence as set forth in SEQ ID NO: 528, or an amino acid sequence having at least 90%, 91%, 92%, 93%, 94%, 95%, 96%, 97%, 98% or 99% identity with the amino acid sequence of SEQ ID NO: 528, or consists of SEQ ID NO: 528;
52) the heavy chain variable region comprises the amino acid sequence as set forth in SEQ ID NO: 530, or an amino acid sequence having at least 90%, 91%, 92%, 93%, 94%, 95%, 96%, 97%, 98% or 99% identity with the amino acid sequence of SEQ ID NO: 530, or consists of SEQ ID NO: 530;
53) the heavy chain variable region comprises the amino acid sequence as set forth in SEQ ID NO: 532, or an amino acid sequence having at least 90%, 91%, 92%, 93%, 94%, 95%, 96%, 97%, 98% or 99% identity with the amino acid sequence of SEQ ID NO: 532, or consists of SEQ ID NO: 532;
54) the heavy chain variable region comprises the amino acid sequence as set forth in SEQ ID NO: 534, or an amino acid sequence having at least 90%, 91%, 92%, 93%, 94%, 95%, 96%, 97%, 98% or 99% identity with the amino acid sequence of SEQ ID NO: 534, or consists of SEQ ID NO: 534;
55) the heavy chain variable region comprises the amino acid sequence as set forth in SEQ ID NO: 536, or an amino acid sequence having at least 90%, 91%, 92%, 93%, 94%, 95%, 96%, 97%, 98% or 99% identity with the amino acid sequence of SEQ ID NO: 536, or consists of SEQ ID NO: 536;
56) the heavy chain variable region comprises the amino acid sequence as set forth in SEQ ID NO: 538, or an amino acid sequence having at least 90%, 91%, 92%, 93%, 94%, 95%, 96%, 97%, 98% or 99% identity with the amino acid sequence of SEQ ID NO: 538, or consists of SEQ ID NO: 538;
57) the heavy chain variable region comprises the amino acid sequence as set forth in SEQ ID NO: 540, or an amino acid sequence having at least 90%, 91%, 92%, 93%, 94%, 95%, 96%, 97%, 98% or 99% identity with the amino acid sequence of SEQ ID NO: 540, or consists of SEQ ID NO: 540;
58) the heavy chain variable region comprises the amino acid sequence as set forth in SEQ ID NO: 543, or an amino acid sequence having at least 90%, 91%, 92%, 93%, 94%, 95%, 96%, 97%, 98% or 99% identity with the amino acid sequence of SEQ ID NO: 543, or consists of SEQ ID NO: 543;
59) the heavy chain variable region comprises the amino acid sequence as set forth in SEQ ID NO: 545, or an amino acid sequence having at least 90%, 91%, 92%, 93%, 94%, 95%, 96%, 97%, 98% or 99% identity with the amino acid sequence of SEQ ID NO: 545, or consists of SEQ ID NO: 545;
60) the heavy chain variable region comprises the amino acid sequence as set forth in SEQ ID NO: 547, or an amino acid sequence having at least 90%, 91%, 92%, 93%, 94%, 95%, 96%, 97%, 98% or 99% identity with the amino acid sequence of SEQ ID NO: 547, or consists of SEQ ID NO: 547;
61) the heavy chain variable region comprises the amino acid sequence as set forth in SEQ ID NO: 549, or an amino acid sequence having at least 90%, 91%, 92%, 93%, 94%, 95%, 96%, 97%, 98% or 99% identity with the amino acid sequence of SEQ ID NO: 549, or consists of SEQ ID NO: 549;
62) the heavy chain variable region comprises the amino acid sequence as set forth in SEQ ID NO: 551, or an amino acid sequence having at least 90%, 91%, 92%, 93%, 94%, 95%, 96%, 97%, 98% or 99% identity with the amino acid sequence of SEQ ID NO: 551, or consists of SEQ ID NO: 551;
63) the heavy chain variable region comprises the amino acid sequence as set forth in SEQ ID NO: 553, or an amino acid sequence having at least 90%, 91%, 92%, 93%, 94%, 95%, 96%, 97%, 98% or 99% identity with the amino acid sequence of SEQ ID NO: 553, or consists of SEQ ID NO: 553;
64) the heavy chain variable region comprises the amino acid sequence as set forth in SEQ ID NO: 555, or an amino acid sequence having at least 90%, 91%, 92%, 93%, 94%, 95%, 96%, 97%, 98% or 99% identity with the amino acid sequence of SEQ ID NO: 555, or consists of SEQ ID NO: 555;
65) the heavy chain variable region comprises the amino acid sequence as set forth in SEQ ID NO: 557, or an amino acid sequence having at least 90%, 91%, 92%, 93%, 94%, 95%, 96%, 97%, 98% or 99% identity with the amino acid sequence of SEQ ID NO: 557, or consists of SEQ ID NO: 557;
66) the heavy chain variable region comprises the amino acid sequence as set forth in SEQ ID NO: 559, or an amino acid sequence having at least 90%, 91%, 92%, 93%, 94%, 95%, 96%, 97%, 98% or 99% identity with the amino acid sequence of SEQ ID NO: 559, or consists of SEQ ID NO: 559;
67) the heavy chain variable region comprises the amino acid sequence as set forth in SEQ ID NO: 561, or an amino acid sequence having at least 90%, 91%, 92%, 93%, 94%, 95%, 96%, 97%, 98% or 99% identity with the amino acid sequence of SEQ ID NO: 561, or consists of SEQ ID NO: 561;
68) the heavy chain variable region comprises the amino acid sequence as set forth in SEQ ID NO: 562, or an amino acid sequence having at least 90%, 91%, 92%, 93%, 94%, 95%, 96%, 97%, 98% or 99% identity with the amino acid sequence of SEQ ID NO: 562, or consists of SEQ ID NO: 562;
69) the heavy chain variable region comprises the amino acid sequence as set forth in SEQ ID NO: 564, or an amino acid sequence having at least 90%, 91%, 92%, 93%, 94%, 95%, 96%, 97%, 98% or 99% identity with the amino acid sequence of SEQ ID NO: 564, or consists of SEQ ID NO: 564;
70) the heavy chain variable region comprises the amino acid sequence as set forth in SEQ ID NO: 566, or an amino acid sequence having at least 90%, 91%, 92%, 93%, 94%, 95%, 96%, 97%, 98% or 99% identity with the amino acid sequence of SEQ ID NO: 566, or consists of SEQ ID NO: 566;
71) the heavy chain variable region comprises the amino acid sequence as set forth in SEQ ID NO: 568, or an amino acid sequence having at least 90%, 91%, 92%, 93%, 94%, 95%, 96%, 97%, 98% or 99% identity with the amino acid sequence of SEQ ID NO: 568, or consists of SEQ ID NO: 568;
72) the heavy chain variable region comprises the amino acid sequence as set forth in SEQ ID NO: 570, or an amino acid sequence having at least 90%, 91%, 92%, 93%, 94%, 95%, 96%, 97%, 98% or 99% identity with the amino acid sequence of SEQ ID NO: 570, or consists of SEQ ID NO: 570;
73) the heavy chain variable region comprises the amino acid sequence as set forth in SEQ ID NO: 572, or an amino acid sequence having at least 90%, 91%, 92%, 93%, 94%, 95%, 96%, 97%, 98% or 99% identity with the amino acid sequence of SEQ ID NO: 572, or consists of SEQ ID NO: 572;
74) the heavy chain variable region comprises the amino acid sequence as set forth in SEQ ID NO: 574, or an amino acid sequence having at least 90%, 91%, 92%, 93%, 94%, 95%, 96%, 97%, 98% or 99% identity with the amino acid sequence of SEQ ID NO: 574, or consists of SEQ ID NO: 574;
75) the heavy chain variable region comprises the amino acid sequence as set forth in SEQ ID NO: 576, or an amino acid sequence having at least 90%, 91%, 92%, 93%, 94%, 95%, 96%, 97%, 98% or 99% identity with the amino acid sequence of SEQ ID NO: 576, or consists of SEQ ID NO: 576;
76) the heavy chain variable region comprises the amino acid sequence as set forth in SEQ ID NO: 577, or an amino acid sequence having at least 90%, 91%, 92%, 93%, 94%, 95%, 96%, 97%, 98% or 99% identity with the amino acid sequence of SEQ ID NO: 577, or consists of SEQ ID NO: 577;
77) the heavy chain variable region comprises the amino acid sequence as set forth in SEQ ID NO: 579, or an amino acid sequence having at least 90%, 91%, 92%, 93%, 94%, 95%, 96%, 97%, 98% or 99% identity with the amino acid sequence of SEQ ID NO: 579, or consists of SEQ ID NO: 579;
78) the heavy chain variable region comprises the amino acid sequence as set forth in SEQ ID NO: 581, or an amino acid sequence having at least 90%, 91%, 92%, 93%, 94%, 95%, 96%, 97%, 98% or 99% identity with the amino acid sequence of SEQ ID NO: 581, or consists of SEQ ID NO: 581;
79) the heavy chain variable region comprises the amino acid sequence as set forth in SEQ ID NO: 583, or an amino acid sequence having at least 90%, 91%, 92%, 93%, 94%, 95%, 96%, 97%, 98% or 99% identity with the amino acid sequence of SEQ ID NO: 583, or consists of SEQ ID NO: 583;
80) the heavy chain variable region comprises the amino acid sequence as set forth in SEQ ID NO: 585, or an amino acid sequence having at least 90%, 91%, 92%, 93%, 94%, 95%, 96%, 97%, 98% or 99% identity with the amino acid sequence of SEQ ID NO: 585, or consists of SEQ ID NO: 585;
81) the heavy chain variable region comprises the amino acid sequence as set forth in SEQ ID NO: 587, or an amino acid sequence having at least 90%, 91%, 92%, 93%, 94%, 95%, 96%, 97%, 98% or 99% identity with the amino acid sequence of SEQ ID NO: 587, or consists of SEQ ID NO: 587;
82) the heavy chain variable region comprises the amino acid sequence as set forth in SEQ ID NO: 590, or an amino acid sequence having at least 90%, 91%, 92%, 93%, 94%, 95%, 96%, 97%, 98% or 99% identity with the amino acid sequence of SEQ ID NO: 590, or consists of SEQ ID NO: 590;
83) the heavy chain variable region comprises the amino acid sequence as set forth in SEQ ID NO: 592, or an amino acid sequence having at least 90%, 91%, 92%, 93%, 94%, 95%, 96%, 97%, 98% or 99% identity with the amino acid sequence of SEQ ID NO: 592, or consists of SEQ ID NO: 592;
84) the heavy chain variable region comprises the amino acid sequence as set forth in SEQ ID NO: 595, or an amino acid sequence having at least 90%, 91%, 92%, 93%, 94%, 95%, 96%, 97%, 98% or 99% identity with the amino acid sequence of SEQ ID NO: 595, or consists of SEQ ID NO: 595;
85) the heavy chain variable region comprises the amino acid sequence as set forth in SEQ ID NO: 597, or an amino acid sequence having at least 90%, 91%, 92%, 93%, 94%, 95%, 96%, 97%, 98% or 99% identity with the amino acid sequence of SEQ ID NO: 597, or consists of SEQ ID NO: 597;
86) the heavy chain variable region comprises the amino acid sequence as set forth in SEQ ID NO: 599, or an amino acid sequence having at least 90%, 91%, 92%, 93%, 94%, 95%, 96%, 97%, 98% or 99% identity with the amino acid sequence of SEQ ID NO: 599, or consists of SEQ ID NO: 599;
87) the heavy chain variable region comprises the amino acid sequence as set forth in SEQ ID NO: 601, or an amino acid sequence having at least 90%, 91%, 92%, 93%, 94%, 95%, 96%, 97%, 98% or 99% identity with the amino acid sequence of SEQ ID NO: 601, or consists of SEQ ID NO: 601;
88) the heavy chain variable region comprises the amino acid sequence as set forth in SEQ ID NO: 603, or an amino acid sequence having at least 90%, 91%, 92%, 93%, 94%, 95%, 96%, 97%, 98% or 99% identity with the amino acid sequence of SEQ ID NO: 603, or consists of SEQ ID NO: 603;
89) the heavy chain variable region comprises the amino acid sequence as set forth in SEQ ID NO: 605, or an amino acid sequence having at least 90%, 91%, 92%, 93%, 94%, 95%, 96%, 97%, 98% or 99% identity with the amino acid sequence of SEQ ID NO: 605, or consists of SEQ ID NO: 605;
90) the heavy chain variable region comprises the amino acid sequence as set forth in SEQ ID NO: 607, or an amino acid sequence having at least 90%, 91%, 92%, 93%, 94%, 95%, 96%, 97%, 98% or 99% identity with the amino acid sequence of SEQ ID NO: 607, or consists of SEQ ID NO: 607;
91) the heavy chain variable region comprises the amino acid sequence as set forth in SEQ ID NO: 609, or an amino acid sequence having at least 90%, 91%, 92%, 93%, 94%, 95%, 96%, 97%, 98% or 99% identity with the amino acid sequence of SEQ ID NO: 609, or consists of SEQ ID NO: 609;
92) the heavy chain variable region comprises the amino acid sequence as set forth in SEQ ID NO: 611, or an amino acid sequence having at least 90%, 91%, 92%, 93%, 94%, 95%, 96%, 97%, 98% or 99% identity with the amino acid sequence of SEQ ID NO: 611, or consists of SEQ ID NO: 611;
93) the heavy chain variable region comprises the amino acid sequence as set forth in SEQ ID NO: 613, or an amino acid sequence having at least 90%, 91%, 92%, 93%, 94%, 95%, 96%, 97%, 98% or 99% identity with the amino acid sequence of SEQ ID NO: 613, or consists of SEQ ID NO: 613;
94) the heavy chain variable region comprises the amino acid sequence as set forth in SEQ ID NO: 615, or an amino acid sequence having at least 90%, 91%, 92%, 93%, 94%, 95%, 96%, 97%, 98% or 99% identity with the amino acid sequence of SEQ ID NO: 615, or consists of SEQ ID NO: 615;
95) the heavy chain variable region comprises the amino acid sequence as set forth in SEQ ID NO: 617, or an amino acid sequence having at least 90%, 91%, 92%, 93%, 94%, 95%, 96%, 97%, 98% or 99% identity with the amino acid sequence of SEQ ID NO: 617, or consists of SEQ ID NO: 617;
96) the heavy chain variable region comprises the amino acid sequence as set forth in SEQ ID NO: 619, or an amino acid sequence having at least 90%, 91%, 92%, 93%, 94%, 95%, 96%, 97%, 98% or 99% identity with the amino acid sequence of SEQ ID NO: 619, or consists of SEQ ID NO: 619;
97) the heavy chain variable region comprises the amino acid sequence as set forth in SEQ ID NO: 621, or an amino acid sequence having at least 90%, 91%, 92%, 93%, 94%, 95%, 96%, 97%, 98% or 99% identity with the amino acid sequence of SEQ ID NO: 621, or consists of SEQ ID NO: 621;
98) the heavy chain variable region comprises the amino acid sequence as set forth in SEQ ID NO: 623, or an amino acid sequence having at least 90%, 91%, 92%, 93%, 94%, 95%, 96%, 97%, 98% or 99% identity with the amino acid sequence of SEQ ID NO: 623, or consists of SEQ ID NO: 623;
99) the heavy chain variable region comprises the amino acid sequence as set forth in SEQ ID NO: 625, or an amino acid sequence having at least 90%, 91%, 92%, 93%, 94%, 95%, 96%, 97%, 98% or 99% identity with the amino acid sequence of SEQ ID NO: 625, or consists of SEQ ID NO: 625;
100) the heavy chain variable region comprises the amino acid sequence as set forth in SEQ ID NO: 627, or an amino acid sequence having at least 90%, 91%, 92%, 93%, 94%, 95%, 96%, 97%, 98% or 99% identity with the amino acid sequence of SEQ ID NO: 627, or consists of SEQ ID NO: 627;
101) the heavy chain variable region comprises the amino acid sequence as set forth in SEQ ID NO: 629, or an amino acid sequence having at least 90%, 91%, 92%, 93%, 94%, 95%, 96%, 97%, 98% or 99% identity with the amino acid sequence of SEQ ID NO: 629, or consists of SEQ ID NO: 629;
102) the heavy chain variable region comprises the amino acid sequence as set forth in SEQ ID NO: 631, or an amino acid sequence having at least 90%, 91%, 92%, 93%, 94%, 95%, 96%, 97%, 98% or 99% identity with the amino acid sequence of SEQ ID NO: 631, or consists of SEQ ID NO: 631;
103) the heavy chain variable region comprises the amino acid sequence as set forth in SEQ ID NO: 634, or an amino acid sequence having at least 90%, 91%, 92%, 93%, 94%, 95%, 96%, 97%, 98% or 99% identity with the amino acid sequence of SEQ ID NO: 634, or consists of SEQ ID NO: 634;
104) the heavy chain variable region comprises the amino acid sequence as set forth in SEQ ID NO: 636, or an amino acid sequence having at least 90%, 91%, 92%, 93%, 94%, 95%, 96%, 97%, 98% or 99% identity with the amino acid sequence of SEQ ID NO: 636, or consists of SEQ ID NO: 636;
105) the heavy chain variable region comprises the amino acid sequence as set forth in SEQ ID NO: 638, or an amino acid sequence having at least 90%, 91%, 92%, 93%, 94%, 95%, 96%, 97%, 98% or 99% identity with the amino acid sequence of SEQ ID NO: 638, or consists of SEQ ID NO: 638;
106) the heavy chain variable region comprises the amino acid sequence as set forth in SEQ ID NO: 640, or an amino acid sequence having at least 90%, 91%, 92%, 93%, 94%, 95%, 96%, 97%, 98% or 99% identity with the amino acid sequence of SEQ ID NO: 640, or consists of SEQ ID NO: 640;
107) the heavy chain variable region comprises the amino acid sequence as set forth in SEQ ID NO: 642, or an amino acid sequence having at least 90%, 91%, 92%, 93%, 94%, 95%, 96%, 97%, 98% or 99% identity with the amino acid sequence of SEQ ID NO: 642, or consists of SEQ ID NO: 642;
108) the heavy chain variable region comprises the amino acid sequence as set forth in SEQ ID NO: 644, or an amino acid sequence having at least 90%, 91%, 92%, 93%, 94%, 95%, 96%, 97%, 98% or 99% identity with the amino acid sequence of SEQ ID NO: 644, or consists of SEQ ID NO: 644;
109) the heavy chain variable region comprises the amino acid sequence as set forth in SEQ ID NO: 646, or an amino acid sequence having at least 90%, 91%, 92%, 93%, 94%, 95%, 96%, 97%, 98% or 99% identity with the amino acid sequence of SEQ ID NO: 646, or consists of SEQ ID NO: 646;
110) the heavy chain variable region comprises the amino acid sequence as set forth in SEQ ID NO: 648, or an amino acid sequence having at least 90%, 91%, 92%, 93%, 94%, 95%, 96%, 97%, 98% or 99% identity with the amino acid sequence of SEQ ID NO: 648, or consists of SEQ ID NO: 648;
111) the heavy chain variable region comprises the amino acid sequence as set forth in SEQ ID NO: 650, or an amino acid sequence having at least 90%, 91%, 92%, 93%, 94%, 95%, 96%, 97%, 98% or 99% identity with the amino acid sequence of SEQ ID NO: 650, or consists of SEQ ID NO: 650;
112) the heavy chain variable region comprises the amino acid sequence as set forth in SEQ ID NO: 652, or an amino acid sequence having at least 90%, 91%, 92%, 93%, 94%, 95%, 96%, 97%, 98% or 99% identity with the amino acid sequence of SEQ ID NO: 652, or consists of SEQ ID NO: 652;
113) the heavy chain variable region comprises the amino acid sequence as set forth in SEQ ID NO: 654, or an amino acid sequence having at least 90%, 91%, 92%, 93%, 94%, 95%, 96%, 97%, 98% or 99% identity with the amino acid sequence of SEQ ID NO: 654, or consists of SEQ ID NO: 654; or
114) The heavy chain variable region comprises the amino acid sequence as set forth in SEQ ID NO: 656, or an amino acid sequence having at least 90%, 91%, 92%, 93%, 94%, 95%, 96%, 97%, 98% or 99% identity to the amino acid sequence of SEQ ID NO: 656, or consists of SEQ ID NO: 656.

In one embodiment, the invention provides an anti-CD112R antibody that specifically binds CD 112R and an antigen-binding fragment thereof, comprising a light chain variable region, wherein:
1) the light chain variable region comprises the amino acid sequence as set forth in SEQ ID NO: 431, or an amino acid sequence having at least 90%, 91%, 92%, 93%, 94%, 95%, 96%, 97%, 98% or 99% identity with the amino acid sequence of SEQ ID NO: 431, or consists of SEQ ID NO: 431;
2) the light chain variable region comprises the amino acid sequence as set forth in SEQ ID NO: 433, or an amino acid sequence having at least 90%, 91%, 92%, 93%, 94%, 95%, 96%, 97%, 98% or 99% identity with the amino acid sequence of SEQ ID NO: 433, or consists of SEQ ID NO: 433;
3) the light chain variable region comprises the amino acid sequence as set forth in SEQ ID NO: 435, or an amino acid sequence having at least 90%, 91%, 92%, 93%, 94%, 95%, 96%, 97%, 98% or 99% identity with the amino acid sequence of SEQ ID NO: 435, or consists of SEQ ID NO: 435;
4) the light chain variable region comprises the amino acid sequence as set forth in SEQ ID NO: 437, or an amino acid sequence having at least 90%, 91%, 92%, 93%, 94%, 95%, 96%, 97%, 98% or 99% identity with the amino acid sequence of SEQ ID NO: 437, or consists of SEQ ID NO: 437;
5) the light chain variable region comprises the amino acid sequence as set forth in SEQ ID NO: 439, or an amino acid sequence having at least 90%, 91%, 92%, 93%, 94%, 95%, 96%, 97%, 98% or 99% identity with the amino acid sequence of SEQ ID NO: 439, or consists of SEQ ID NO: 439;
6) the light chain variable region comprises the amino acid sequence as set forth in SEQ ID NO: 440, or an amino acid sequence having at least 90%, 91%, 92%, 93%, 94%, 95%, 96%, 97%, 98% or 99% identity with the amino acid sequence of SEQ ID NO: 440, or consists of SEQ ID NO: 440;
7) the light chain variable region comprises the amino acid sequence as set forth in SEQ ID NO: 442, or an amino acid sequence having at least 90%, 91%, 92%, 93%, 94%, 95%, 96%, 97%, 98% or 99% identity with the amino acid sequence of SEQ ID NO: 442, or consists of SEQ ID NO: 442;
8) the light chain variable region comprises the amino acid sequence as set forth in SEQ ID NO: 444, or an amino acid sequence having at least 90%, 91%, 92%, 93%, 94%, 95%, 96%, 97%, 98% or 99% identity with the amino acid sequence of SEQ ID NO: 444, or consists of SEQ ID NO: 444;
9) the light chain variable region comprises the amino acid sequence as set forth in SEQ ID NO: 446, or an amino acid sequence having at least 90%, 91%, 92%, 93%, 94%, 95%, 96%, 97%, 98% or 99% identity with the amino acid sequence of SEQ ID NO: 446, or consists of SEQ ID NO: 446;
10) the light chain variable region comprises the amino acid sequence as set forth in SEQ ID NO: 448, or an amino acid sequence having at least 90%, 91%, 92%, 93%, 94%, 95%, 96%, 97%, 98% or 99% identity with the amino acid sequence of SEQ ID NO: 448, or consists of SEQ ID NO: 448;
11) the light chain variable region comprises the amino acid sequence as set forth in SEQ ID NO: 450, or an amino acid sequence having at least 90%, 91%, 92%, 93%, 94%, 95%, 96%, 97%, 98% or 99% identity with the amino acid sequence of SEQ ID NO: 450, or consists of SEQ ID NO: 450;
12) the light chain variable region comprises the amino acid sequence as set forth in SEQ ID NO: 452, or an amino acid sequence having at least 90%, 91%, 92%, 93%, 94%, 95%, 96%, 97%, 98% or 99% identity with the amino acid sequence of SEQ ID NO: 452, or consists of SEQ ID NO: 452;
13) the light chain variable region comprises the amino acid sequence as set forth in SEQ ID NO: 454, or an amino acid sequence having at least 90%, 91%, 92%, 93%, 94%, 95%, 96%, 97%, 98% or 99% identity with the amino acid sequence of SEQ ID NO: 454, or consists of SEQ ID NO: 454;
14) the light chain variable region comprises the amino acid sequence as set forth in SEQ ID NO: 456, or an amino acid sequence having at least 90%, 91%, 92%, 93%, 94%, 95%, 96%, 97%, 98% or 99% identity with the amino acid sequence of SEQ ID NO: 456, or consists of SEQ ID NO: 456;
15) the light chain variable region comprises the amino acid sequence as set forth in SEQ ID NO: 458, or an amino acid sequence having at least 90%, 91%, 92%, 93%, 94%, 95%, 96%, 97%, 98% or 99% identity with the amino acid sequence of SEQ ID NO: 458, or consists of SEQ ID NO: 458;
16) the light chain variable region comprises the amino acid sequence as set forth in SEQ ID NO: 460, or an amino acid sequence having at least 90%, 91%, 92%, 93%, 94%, 95%, 96%, 97%, 98% or 99% identity with the amino acid sequence of SEQ ID NO: 460, or consists of SEQ ID NO: 460;
17) the light chain variable region comprises the amino acid sequence as set forth in SEQ ID NO: 462, or an amino acid sequence having at least 90%, 91%, 92%, 93%, 94%, 95%, 96%, 97%, 98% or 99% identity with the amino acid sequence of SEQ ID NO: 462, or consists of SEQ ID NO: 462;
18) the light chain variable region comprises the amino acid sequence as set forth in SEQ ID NO: 464, or an amino acid sequence having at least 90%, 91%, 92%, 93%, 94%, 95%, 96%, 97%, 98% or 99% identity with the amino acid sequence of SEQ ID NO: 464, or consists of SEQ ID NO: 464;
19) the light chain variable region comprises the amino acid sequence as set forth in SEQ ID NO: 466, or an amino acid sequence having at least 90%, 91%, 92%, 93%, 94%, 95%, 96%, 97%, 98% or 99% identity with the amino acid sequence of SEQ ID NO: 466, or consists of SEQ ID NO: 466;
20) the light chain variable region comprises the amino acid sequence as set forth in SEQ ID NO: 468, or an amino acid sequence having at least 90%, 91%, 92%, 93%, 94%, 95%, 96%, 97%, 98% or 99% identity with the amino acid sequence of SEQ ID NO: 468, or consists of SEQ ID NO: 468;
21) the light chain variable region comprises the amino acid sequence as set forth in SEQ ID NO: 470, or an amino acid sequence having at least 90%, 91%, 92%, 93%, 94%, 95%, 96%, 97%, 98% or 99% identity with the amino acid sequence of SEQ ID NO: 470, or consists of SEQ ID NO: 470;
22) the light chain variable region comprises the amino acid sequence as set forth in SEQ ID NO: 472, or an amino acid sequence having at least 90%, 91%, 92%, 93%, 94%, 95%, 96%, 97%, 98% or 99% identity with the amino acid sequence of SEQ ID NO: 472, or consists of SEQ ID NO: 472;
23) the light chain variable region comprises the amino acid sequence as set forth in SEQ ID NO: 474, or an amino acid sequence having at least 90%, 91%, 92%, 93%, 94%, 95%, 96%, 97%, 98% or 99% identity with the amino acid sequence of SEQ ID NO: 474, or consists of SEQ ID NO: 474;
24) the light chain variable region comprises the amino acid sequence as set forth in SEQ ID NO: 476, or an amino acid sequence having at least 90%, 91%, 92%, 93%, 94%, 95%, 96%, 97%, 98% or 99% identity with the amino acid sequence of SEQ ID NO: 476, or consists of SEQ ID NO: 476;
25) the light chain variable region comprises the amino acid sequence as set forth in SEQ ID NO: 478, or an amino acid sequence having at least 90%, 91%, 92%, 93%, 94%, 95%, 96%, 97%, 98% or 99% identity with the amino acid sequence of SEQ ID NO: 478, or consists of SEQ ID NO: 478;
26) the light chain variable region comprises the amino acid sequence as set forth in SEQ ID NO: 480, or an amino acid sequence having at least 90%, 91%, 92%, 93%, 94%, 95%, 96%, 97%, 98% or 99% identity with the amino acid sequence of SEQ ID NO: 480, or consists of SEQ ID NO: 480;
27) the light chain variable region comprises the amino acid sequence as set forth in SEQ ID NO: 482, or an amino acid sequence having at least 90%, 91%, 92%, 93%, 94%, 95%, 96%, 97%, 98% or 99% identity with the amino acid sequence of SEQ ID NO: 482, or consists of SEQ ID NO: 482;
28) the light chain variable region comprises the amino acid sequence as set forth in SEQ ID NO: 484, or an amino acid sequence having at least 90%, 91%, 92%, 93%, 94%, 95%, 96%, 97%, 98% or 99% identity with the amino acid sequence of SEQ ID NO: 484, or consists of SEQ ID NO: 484;
29) the light chain variable region comprises the amino acid sequence as set forth in SEQ ID NO: 486, or an amino acid sequence having at least 90%, 91%, 92%, 93%, 94%, 95%, 96%, 97%, 98% or 99% identity with the amino acid sequence of SEQ ID NO: 486, or consists of SEQ ID NO: 486;
30) the light chain variable region comprises the amino acid sequence as set forth in SEQ ID NO: 489, or an amino acid sequence having at least 90%, 91%, 92%, 93%, 94%, 95%, 96%, 97%, 98% or 99% identity with the amino acid sequence of SEQ ID NO: 489, or consists of SEQ ID NO: 489;
31) the light chain variable region comprises the amino acid sequence as set forth in SEQ ID NO: 491, or an amino acid sequence having at least 90%, 91%, 92%, 93%, 94%, 95%, 96%, 97%, 98% or 99% identity with the amino acid sequence of SEQ ID NO: 491, or consists of SEQ ID NO: 491;
32) the light chain variable region comprises the amino acid sequence as set forth in SEQ ID NO: 494, or an amino acid sequence having at least 90%, 91%, 92%, 93%, 94%, 95%, 96%, 97%, 98% or 99% identity with the amino acid sequence of SEQ ID NO: 494, or consists of SEQ ID NO: 494;
33) the light chain variable region comprises the amino acid sequence as set forth in SEQ ID NO: 495, or an amino acid sequence having at least 90%, 91%, 92%, 93%, 94%, 95%, 96%, 97%, 98% or 99% identity with the amino acid sequence of SEQ ID NO: 495, or consists of SEQ ID NO: 495;
34) the light chain variable region comprises the amino acid sequence as set forth in SEQ ID NO: 497, or an amino acid sequence having at least 90%, 91%, 92%, 93%, 94%, 95%, 96%, 97%, 98% or 99% identity with the amino acid sequence of SEQ ID NO: 497, or consists of SEQ ID NO: 497;
35) the light chain variable region comprises the amino acid sequence as set forth in SEQ ID NO: 500, or an amino acid sequence having at least 90%, 91%, 92%, 93%, 94%, 95%, 96%, 97%, 98% or 99% identity with the amino acid sequence of SEQ ID NO: 500, or consists of SEQ ID NO: 500;
36) the light chain variable region comprises the amino acid sequence as set forth in SEQ ID NO: 503, or an amino acid sequence having at least 90%, 91%, 92%, 93%, 94%, 95%, 96%, 97%, 98% or 99% identity with the amino acid sequence of SEQ ID NO: 503, or consists of SEQ ID NO: 503;
37) the light chain variable region comprises the amino acid sequence as set forth in SEQ ID NO: 505, or an amino acid sequence having at least 90%, 91%, 92%, 93%, 94%, 95%, 96%, 97%, 98% or 99% identity with the amino acid sequence of SEQ ID NO: 505, or consists of SEQ ID NO: 505;
38) the light chain variable region comprises the amino acid sequence as set forth in SEQ ID NO: 506, or an amino acid sequence having at least 90%, 91%, 92%, 93%, 94%, 95%, 96%, 97%, 98% or 99% identity with the amino acid sequence of SEQ ID NO: 506, or consists of SEQ ID NO: 506;
39) the light chain variable region comprises the amino acid sequence as set forth in SEQ ID NO: 509, or an amino acid sequence having at least 90%, 91%, 92%, 93%, 94%, 95%, 96%, 97%, 98% or 99% identity with the amino acid sequence of SEQ ID NO: 509, or consists of SEQ ID NO: 509;
40) the light chain variable region comprises the amino acid sequence as set forth in SEQ ID NO: 511, or an amino acid sequence having at least 90%, 91%, 92%, 93%, 94%, 95%, 96%, 97%, 98% or 99% identity with the amino acid sequence of SEQ ID NO: 511, or consists of SEQ ID NO: 511;
41) the light chain variable region comprises the amino acid sequence as set forth in SEQ ID NO: 513, or an amino acid sequence having at least 90%, 91%, 92%, 93%, 94%, 95%, 96%, 97%, 98% or 99% identity with the amino acid sequence of SEQ ID NO: 513, or consists of SEQ ID NO: 513;
42) the light chain variable region comprises the amino acid sequence as set forth in SEQ ID NO: 515, or an amino acid sequence having at least 90%, 91%, 92%, 93%, 94%, 95%, 96%, 97%, 98% or 99% identity with the amino acid sequence of SEQ ID NO: 515, or consists of SEQ ID NO: 515;
43) the light chain variable region comprises the amino acid sequence as set forth in SEQ ID NO: 516, or an amino acid sequence having at least 90%, 91%, 92%, 93%, 94%, 95%, 96%, 97%, 98% or 99% identity with the amino acid sequence of SEQ ID NO: 516, or consists of SEQ ID NO: 516;
44) the light chain variable region comprises the amino acid sequence as set forth in SEQ ID NO: 518, or an amino acid sequence having at least 90%, 91%, 92%, 93%, 94%, 95%, 96%, 97%, 98% or 99% identity with the amino acid sequence of SEQ ID NO: 518, or consists of SEQ ID NO: 518;
45) the light chain variable region comprises the amino acid sequence as set forth in SEQ ID NO: 520, or an amino acid sequence having at least 90%, 91%, 92%, 93%, 94%, 95%, 96%, 97%, 98% or 99% identity with the amino acid sequence of SEQ ID NO: 520, or consists of SEQ ID NO: 520;
46) the light chain variable region comprises the amino acid sequence as set forth in SEQ ID NO: 522, or an amino acid sequence having at least 90%, 91%, 92%, 93%, 94%, 95%, 96%, 97%, 98% or 99% identity with the amino acid sequence of SEQ ID NO: 522, or consists of SEQ ID NO: 522;
47) the light chain variable region comprises the amino acid sequence as set forth in SEQ ID NO: 524, or an amino acid sequence having at least 90%, 91%, 92%, 93%, 94%, 95%, 96%, 97%, 98% or 99% identity with the amino acid sequence of SEQ ID NO: 524, or consists of SEQ ID NO: 524;
48) the light chain variable region comprises the amino acid sequence as set forth in SEQ ID NO: 527, or an amino acid sequence having at least 90%, 91%, 92%, 93%, 94%, 95%, 96%, 97%, 98% or 99% identity with the amino acid sequence of SEQ ID NO: 527, or consists of SEQ ID NO: 527;
49) the light chain variable region comprises the amino acid sequence as set forth in SEQ ID NO: 529, or an amino acid sequence having at least 90%, 91%, 92%, 93%, 94%, 95%, 96%, 97%, 98% or 99% identity with the amino acid sequence of SEQ ID NO: 529, or consists of SEQ ID NO: 529;
50) the light chain variable region comprises the amino acid sequence as set forth in SEQ ID NO: 531, or an amino acid sequence having at least 90%, 91%, 92%, 93%, 94%, 95%, 96%, 97%, 98% or 99% identity with the amino acid sequence of SEQ ID NO: 531, or consists of SEQ ID NO: 531;
51) the light chain variable region comprises the amino acid sequence as set forth in SEQ ID NO: 533, or an amino acid sequence having at least 90%, 91%, 92%, 93%, 94%, 95%, 96%, 97%, 98% or 99% identity with the amino acid sequence of SEQ ID NO: 533, or consists of SEQ ID NO: 533;
52) the light chain variable region comprises the amino acid sequence as set forth in SEQ ID NO: 535, or an amino acid sequence having at least 90%, 91%, 92%, 93%, 94%, 95%, 96%, 97%, 98% or 99% identity with the amino acid sequence of SEQ ID NO: 535, or consists of SEQ ID NO: 535;
53) the light chain variable region comprises the amino acid sequence as set forth in SEQ ID NO: 537, or an amino acid sequence having at least 90%, 91%, 92%, 93%, 94%, 95%, 96%, 97%, 98% or 99% identity with the amino acid sequence of SEQ ID NO: 537, or consists of SEQ ID NO: 537;
54) the light chain variable region comprises the amino acid sequence as set forth in SEQ ID NO: 539, or an amino acid sequence having at least 90%, 91%, 92%, 93%, 94%, 95%, 96%, 97%, 98% or 99% identity with the amino acid sequence of SEQ ID NO: 539, or consists of SEQ ID NO: 539;
55) the light chain variable region comprises the amino acid sequence as set forth in SEQ ID NO: 541, or an amino acid sequence having at least 90%, 91%, 92%, 93%, 94%, 95%, 96%, 97%, 98% or 99% identity with the amino acid sequence of SEQ ID NO: 541, or consists of SEQ ID NO: 541;
56) the light chain variable region comprises the amino acid sequence as set forth in SEQ ID NO: 542, or an amino acid sequence having at least 90%, 91%, 92%, 93%, 94%, 95%, 96%, 97%, 98% or 99% identity with the amino acid sequence of SEQ ID NO: 542, or consists of SEQ ID NO: 542;
57) the light chain variable region comprises the amino acid sequence as set forth in SEQ ID NO: 544, or an amino acid sequence having at least 90%, 91%, 92%, 93%, 94%, 95%, 96%, 97%, 98% or 99% identity with the amino acid sequence of SEQ ID NO: 544, or consists of SEQ ID NO: 544;
58) the light chain variable region comprises the amino acid sequence as set forth in SEQ ID NO: 546, or an amino acid sequence having at least 90%, 91%, 92%, 93%, 94%, 95%, 96%, 97%, 98% or 99% identity with the amino acid sequence of SEQ ID NO: 546, or consists of SEQ ID NO: 546;
59) the light chain variable region comprises the amino acid sequence as set forth in SEQ ID NO: 548, or an amino acid sequence having at least 90%, 91%, 92%, 93%, 94%, 95%, 96%, 97%, 98% or 99% identity with the amino acid sequence of SEQ ID NO: 548, or consists of SEQ ID NO: 548;
60) the light chain variable region comprises the amino acid sequence as set forth in SEQ ID NO: 550, or an amino acid sequence having at least 90%, 91%, 92%, 93%, 94%, 95%, 96%, 97%, 98% or 99% identity with the amino acid sequence of SEQ ID NO: 550, or consists of SEQ ID NO: 550;
61) the light chain variable region comprises the amino acid sequence as set forth in SEQ ID NO: 552, or an amino acid sequence having at least 90%, 91%, 92%, 93%, 94%, 95%, 96%, 97%, 98% or 99% identity with the amino acid sequence of SEQ ID NO: 552, or consists of SEQ ID NO: 552;
62) the light chain variable region comprises the amino acid sequence as set forth in SEQ ID NO: 554, or an amino acid sequence having at least 90%, 91%, 92%, 93%, 94%, 95%, 96%, 97%, 98% or 99% identity with the amino acid sequence of SEQ ID NO: 554, or consists of SEQ ID NO: 554;
63) the light chain variable region comprises the amino acid sequence as set forth in SEQ ID NO: 556, or an amino acid sequence having at least 90%, 91%, 92%, 93%, 94%, 95%, 96%, 97%, 98% or 99% identity with the amino acid sequence of SEQ ID NO: 556, or consists of SEQ ID NO: 556;
64) the light chain variable region comprises the amino acid sequence as set forth in SEQ ID NO: 558, or an amino acid sequence having at least 90%, 91%, 92%, 93%, 94%, 95%, 96%, 97%, 98% or 99% identity with the amino acid sequence of SEQ ID NO: 558, or consists of SEQ ID NO: 558;
65) the light chain variable region comprises the amino acid sequence as set forth in SEQ ID NO: 560, or an amino acid sequence having at least 90%, 91%, 92%, 93%, 94%, 95%, 96%, 97%, 98% or 99% identity with the amino acid sequence of SEQ ID NO: 560, or consists of SEQ ID NO: 560;
66) the light chain variable region comprises the amino acid sequence as set forth in SEQ ID NO: 563, or an amino acid sequence having at least 90%, 91%, 92%, 93%, 94%, 95%, 96%, 97%, 98% or 99% identity with the amino acid sequence of SEQ ID NO: 563, or consists of SEQ ID NO: 563;
67) the light chain variable region comprises the amino acid sequence as set forth in SEQ ID NO: 565, or an amino acid sequence having at least 90%, 91%, 92%, 93%, 94%, 95%, 96%, 97%, 98% or 99% identity with the amino acid sequence of SEQ ID NO: 565, or consists of SEQ ID NO: 565;
68) the light chain variable region comprises the amino acid sequence as set forth in SEQ ID NO: 567, or an amino acid sequence having at least 90%, 91%, 92%, 93%, 94%, 95%, 96%, 97%, 98% or 99% identity with the amino acid sequence of SEQ ID NO: 567, or consists of SEQ ID NO: 567;
69) the light chain variable region comprises the amino acid sequence as set forth in SEQ ID NO: 569, or an amino acid sequence having at least 90%, 91%, 92%, 93%, 94%, 95%, 96%, 97%, 98% or 99% identity with the amino acid sequence of SEQ ID NO: 569, or consists of SEQ ID NO: 569;
70) the light chain variable region comprises the amino acid sequence as set forth in SEQ ID NO: 571, or an amino acid sequence having at least 90%, 91%, 92%, 93%, 94%, 95%, 96%, 97%, 98% or 99% identity with the amino acid sequence of SEQ ID NO: 571, or consists of SEQ ID NO: 571;
71) the light chain variable region comprises the amino acid sequence as set forth in SEQ ID NO: 573, or an amino acid sequence having at least 90%, 91%, 92%, 93%, 94%, 95%, 96%, 97%, 98% or 99% identity with the amino acid sequence of SEQ ID NO: 573, or consists of SEQ ID NO: 573;
72) the light chain variable region comprises the amino acid sequence as set forth in SEQ ID NO: 575, or an amino acid sequence having at least 90%, 91%, 92%, 93%, 94%, 95%, 96%, 97%, 98% or 99% identity with the amino acid sequence of SEQ ID NO: 575, or consists of SEQ ID NO: 575;
73) the light chain variable region comprises the amino acid sequence as set forth in SEQ ID NO: 578, or an amino acid sequence having at least 90%, 91%, 92%, 93%, 94%, 95%, 96%, 97%, 98% or 99% identity with the amino acid sequence of SEQ ID NO: 578, or consists of SEQ ID NO: 578;
74) the light chain variable region comprises the amino acid sequence as set forth in SEQ ID NO: 580, or an amino acid sequence having at least 90%, 91%, 92%, 93%, 94%, 95%, 96%, 97%, 98% or 99% identity with the amino acid sequence of SEQ ID NO: 580, or consists of SEQ ID NO: 580;
75) the light chain variable region comprises the amino acid sequence as set forth in SEQ ID NO: 582, or an amino acid sequence having at least 90%, 91%, 92%, 93%, 94%, 95%, 96%, 97%, 98% or 99% identity with the amino acid sequence of SEQ ID NO: 582, or consists of SEQ ID NO: 582;
76) the light chain variable region comprises the amino acid sequence as set forth in SEQ ID NO: 584, or an amino acid sequence having at least 90%, 91%, 92%, 93%, 94%, 95%, 96%, 97%, 98% or 99% identity with the amino acid sequence of SEQ ID NO: 584, or consists of SEQ ID NO: 584;
77) the light chain variable region comprises the amino acid sequence as set forth in SEQ ID NO: 586, or an amino acid sequence having at least 90%, 91%, 92%, 93%, 94%, 95%, 96%, 97%, 98% or 99% identity with the amino acid sequence of SEQ ID NO: 586, or consists of SEQ ID NO: 586;
78) the light chain variable region comprises the amino acid sequence as set forth in SEQ ID NO: 588, or an amino acid sequence having at least 90%, 91%, 92%, 93%, 94%, 95%, 96%, 97%, 98% or 99% identity with the amino acid sequence of SEQ ID NO: 588, or consists of SEQ ID NO: 588;
79) the light chain variable region comprises the amino acid sequence as set forth in SEQ ID NO: 589, or an amino acid sequence having at least 90%, 91%, 92%, 93%, 94%, 95%, 96%, 97%, 98% or 99% identity with the amino acid sequence of SEQ ID NO: 589, or consists of SEQ ID NO: 589;
80) the light chain variable region comprises the amino acid sequence as set forth in SEQ ID NO: 591, or an amino acid sequence having at least 90%, 91%, 92%, 93%, 94%, 95%, 96%, 97%, 98% or 99% identity with the amino acid sequence of SEQ ID NO: 591, or consists of SEQ ID NO: 591;
81) the light chain variable region comprises the amino acid sequence as set forth in SEQ ID NO: 593, or an amino acid sequence having at least 90%, 91%, 92%, 93%, 94%, 95%, 96%, 97%, 98% or 99% identity with the amino acid sequence of SEQ ID NO: 593, or consists of SEQ ID NO: 593;
82) the light chain variable region comprises the amino acid sequence as set forth in SEQ ID NO: 594, or an amino acid sequence having at least 90%, 91%, 92%, 93%, 94%, 95%, 96%, 97%, 98% or 99% identity with the amino acid sequence of SEQ ID NO: 594, or consists of SEQ ID NO: 594;
83) the light chain variable region comprises the amino acid sequence as set forth in SEQ ID NO: 596, or an amino acid sequence having at least 90%, 91%, 92%, 93%, 94%, 95%, 96%, 97%, 98% or 99% identity with the amino acid sequence of SEQ ID NO: 596, or consists of SEQ ID NO: 596;
84) the light chain variable region comprises the amino acid sequence as set forth in SEQ ID NO: 598, or an amino acid sequence having at least 90%, 91%, 92%, 93%, 94%, 95%, 96%, 97%, 98% or 99% identity with the amino acid sequence of SEQ ID NO: 598, or consists of SEQ ID NO: 598;
85) the light chain variable region comprises the amino acid sequence as set forth in SEQ ID NO: 600, or an amino acid sequence having at least 90%, 91%, 92%, 93%, 94%, 95%, 96%, 97%, 98% or 99% identity with the amino acid sequence of SEQ ID NO: 600, or consists of SEQ ID NO: 600;
86) the light chain variable region comprises the amino acid sequence as set forth in SEQ ID NO: 602, or an amino acid sequence having at least 90%, 91%, 92%, 93%, 94%, 95%, 96%, 97%, 98% or 99% identity with the amino acid sequence of SEQ ID NO: 602, or consists of SEQ ID NO: 602;
87) the light chain variable region comprises the amino acid sequence as set forth in SEQ ID NO: 604, or an amino acid sequence having at least 90%, 91%, 92%, 93%, 94%, 95%, 96%, 97%, 98% or 99% identity with the amino acid sequence of SEQ ID NO: 604, or consists of SEQ ID NO: 604;
88) the light chain variable region comprises the amino acid sequence as set forth in SEQ ID NO: 606, or an amino acid sequence having at least 90%, 91%, 92%, 93%, 94%, 95%, 96%, 97%, 98% or 99% identity with the amino acid sequence of SEQ ID NO: 606, or consists of SEQ ID NO: 606;
89) the light chain variable region comprises the amino acid sequence as set forth in SEQ ID NO: 608, or an amino acid sequence having at least 90%, 91%, 92%, 93%, 94%, 95%, 96%, 97%, 98% or 99% identity with the amino acid sequence of SEQ ID NO: 608, or consists of SEQ ID NO: 608;
90) the light chain variable region comprises the amino acid sequence as set forth in SEQ ID NO: 610, or an amino acid sequence having at least 90%, 91%, 92%, 93%, 94%, 95%, 96%, 97%, 98% or 99% identity with the amino acid sequence of SEQ ID NO: 610, or consists of SEQ ID NO: 610;
91) the light chain variable region comprises the amino acid sequence as set forth in SEQ ID NO: 612, or an amino acid sequence having at least 90%, 91%, 92%, 93%, 94%, 95%, 96%, 97%, 98% or 99% identity with the amino acid sequence of SEQ ID NO: 612, or consists of SEQ ID NO: 612;
92) the light chain variable region comprises the amino acid sequence as set forth in SEQ ID NO: 614, or an amino acid sequence having at least 90%, 91%, 92%, 93%, 94%, 95%, 96%, 97%, 98% or 99% identity with the amino acid sequence of SEQ ID NO: 614, or consists of SEQ ID NO: 614;
93) the light chain variable region comprises the amino acid sequence as set forth in SEQ ID NO: 616, or an amino acid sequence having at least 90%, 91%, 92%, 93%, 94%, 95%, 96%, 97%, 98% or 99% identity with the amino acid sequence of SEQ ID NO: 616, or consists of SEQ ID NO: 616;
94) the light chain variable region comprises the amino acid sequence as set forth in SEQ ID NO: 618, or an amino acid sequence having at least 90%, 91%, 92%, 93%, 94%, 95%, 96%, 97%, 98% or 99% identity with the amino acid sequence of SEQ ID NO: 618, or consists of SEQ ID NO: 618;
95) the light chain variable region comprises the amino acid sequence as set forth in SEQ ID NO: 620, or an amino acid sequence having at least 90%, 91%, 92%, 93%, 94%, 95%, 96%, 97%, 98% or 99% identity with the amino acid sequence of SEQ ID NO: 620, or consists of SEQ ID NO: 620;
96) the light chain variable region comprises the amino acid sequence as set forth in SEQ ID NO: 622, or an amino acid sequence having at least 90%, 91%, 92%, 93%, 94%, 95%, 96%, 97%, 98% or 99% identity with the amino acid sequence of SEQ ID NO: 622, or consists of SEQ ID NO: 622;
97) the light chain variable region comprises the amino acid sequence as set forth in SEQ ID NO: 624, or an amino acid sequence having at least 90%, 91%, 92%, 93%, 94%, 95%, 96%, 97%, 98% or 99% identity with the amino acid sequence of SEQ ID NO: 624, or consists of SEQ ID NO: 624;
98) the light chain variable region comprises the amino acid sequence as set forth in SEQ ID NO: 626, or an amino acid sequence having at least 90%, 91%, 92%, 93%, 94%, 95%, 96%, 97%, 98% or 99% identity with the amino acid sequence of SEQ ID NO: 626, or consists of SEQ ID NO: 626;
99) the light chain variable region comprises the amino acid sequence as set forth in SEQ ID NO: 628, or an amino acid sequence having at least 90%, 91%, 92%, 93%, 94%, 95%, 96%, 97%, 98% or 99% identity with the amino acid sequence of SEQ ID NO: 628, or consists of SEQ ID NO: 628;
100) the light chain variable region comprises the amino acid sequence as set forth in SEQ ID NO: 630, or an amino acid sequence having at least 90%, 91%, 92%, 93%, 94%, 95%, 96%, 97%, 98% or 99% identity with the amino acid sequence of SEQ ID NO: 630, or consists of SEQ ID NO: 630;
101) the light chain variable region comprises the amino acid sequence as set forth in SEQ ID NO: 632, or an amino acid sequence having at least 90%, 91%, 92%, 93%, 94%, 95%, 96%, 97%, 98% or 99% identity with the amino acid sequence of SEQ ID NO: 632, or consists of SEQ ID NO: 632;
102) the light chain variable region comprises the amino acid sequence as set forth in SEQ ID NO: 633, or an amino acid sequence having at least 90%, 91%, 92%, 93%, 94%, 95%, 96%, 97%, 98% or 99% identity with the amino acid sequence of SEQ ID NO: 633, or consists of SEQ ID NO: 633;
103) the light chain variable region comprises the amino acid sequence as set forth in SEQ ID NO: 635, or an amino acid sequence having at least 90%, 91%, 92%, 93%, 94%, 95%, 96%, 97%, 98% or 99% identity with the amino acid sequence of SEQ ID NO: 635, or consists of SEQ ID NO: 635;
104) the light chain variable region comprises the amino acid sequence as set forth in SEQ ID NO: 637, or an amino acid sequence having at least 90%, 91%, 92%, 93%, 94%, 95%, 96%, 97%, 98% or 99% identity with the amino acid sequence of SEQ ID NO: 637, or consists of SEQ ID NO: 637;
105) the light chain variable region comprises the amino acid sequence as set forth in SEQ ID NO: 639, or an amino acid sequence having at least 90%, 91%, 92%, 93%, 94%, 95%, 96%, 97%, 98% or 99% identity with the amino acid sequence of SEQ ID NO: 639, or consists of SEQ ID NO: 639;
106) the light chain variable region comprises the amino acid sequence as set forth in SEQ ID NO: 641, or an amino acid sequence having at least 90%, 91%, 92%, 93%, 94%, 95%, 96%, 97%, 98% or 99% identity with the amino acid sequence of SEQ ID NO: 641, or consists of SEQ ID NO: 641;
107) the light chain variable region comprises the amino acid sequence as set forth in SEQ ID NO: 643, or an amino acid sequence having at least 90%, 91%, 92%, 93%, 94%, 95%, 96%, 97%, 98% or 99% identity with the amino acid sequence of SEQ ID NO: 643, or consists of SEQ ID NO: 643;
108) the light chain variable region comprises the amino acid sequence as set forth in SEQ ID NO: 645, or an amino acid sequence having at least 90%, 91%, 92%, 93%, 94%, 95%, 96%, 97%, 98% or 99% identity with the amino acid sequence of SEQ ID NO: 645, or consists of SEQ ID NO: 645;
109) the light chain variable region comprises the amino acid sequence as set forth in SEQ ID NO: 647, or an amino acid sequence having at least 90%, 91%, 92%, 93%, 94%, 95%, 96%, 97%, 98% or 99% identity with the amino acid sequence of SEQ ID NO: 647, or consists of SEQ ID NO: 647;
110) the light chain variable region comprises the amino acid sequence as set forth in SEQ ID NO: 649, or an amino acid sequence having at least 90%, 91%, 92%, 93%, 94%, 95%, 96%, 97%, 98% or 99% identity with the amino acid sequence of SEQ ID NO: 649, or consists of SEQ ID NO: 649;
111) the light chain variable region comprises the amino acid sequence as set forth in SEQ ID NO: 651, or an amino acid sequence having at least 90%, 91%, 92%, 93%, 94%, 95%, 96%, 97%, 98% or 99% identity with the amino acid sequence of SEQ ID NO: 651, or consists of SEQ ID NO: 651;
112) the light chain variable region comprises the amino acid sequence as set forth in SEQ ID NO: 653, or an amino acid sequence having at least 90%, 91%, 92%, 93%, 94%, 95%, 96%, 97%, 98% or 99% identity to the amino acid sequence of SEQ ID NO: 653, or consists of SEQ ID NO: 653; or
113) The light chain variable region comprises the amino acid sequence as set forth in SEQ ID NO: 655, or an amino acid sequence having at least 90%, 91%, 92%, 93%, 94%, 95%, 96%, 97%, 98% or 99% identity to the amino acid sequence of SEQ ID NO: 655, or consists of SEQ ID NO: 655.

In another embodiment, the invention provides an anti-CD112R antibody that specifically binds CD112R and an antigen-binding fragment thereof, comprising a heavy chain variable region and a light chain variable region, wherein:
1) the heavy chain variable region comprises the amino acid sequence as set forth in SEQ ID NO: 430, or an amino acid sequence having at least 90%, 91%, 92%, 93%, 94%, 95%, 96%, 97%, 98% or 99% identity with the amino acid sequence of SEQ ID NO: 430, or consists of SEQ ID NO: 430, and the light chain variable region comprises the amino acid sequence as set forth in SEQ ID NO: 431, or an amino acid sequence having at least 90%, 91%, 92%, 93%, 94%, 95%, 96%, 97%, 98% or 99% identity with the amino acid sequence of SEQ ID NO: 431, or consists of SEQ ID NO: 431;
2) the heavy chain variable region comprises the amino acid sequence as set forth in SEQ ID NO: 432, or an amino acid sequence having at least 90%, 91%, 92%, 93%, 94%, 95%, 96%, 97%, 98% or 99% identity with the amino acid sequence of SEQ ID NO: 432, or consists of SEQ ID NO: 432, and the light chain variable region comprises the amino acid sequence as set forth in SEQ ID NO: 433, or an amino acid sequence having at least 90%, 91%, 92%, 93%, 94%, 95%, 96%, 97%, 98% or 99% identity with the amino acid sequence of SEQ ID NO: 433, or consists of SEQ ID NO: 433;
3) the heavy chain variable region comprises the amino acid sequence as set forth in SEQ ID NO: 434, or an amino acid sequence having at least 90%, 91%, 92%, 93%, 94%, 95%, 96%, 97%, 98% or 99% identity with the amino acid sequence of SEQ ID NO: 434, or consists of SEQ ID NO: 434, and the light chain variable region comprises the amino acid sequence as set forth in SEQ ID NO: 435, or an amino acid sequence having at least 90%, 91%, 92%, 93%, 94%, 95%, 96%, 97%, 98% or 99% identity with the amino acid sequence of SEQ ID NO: 435, or consists of SEQ ID NO: 435;
4) the heavy chain variable region comprises the amino acid sequence as set forth in SEQ ID NO: 436, or an amino acid sequence having at least 90%, 91%, 92%, 93%, 94%, 95%, 96%, 97%, 98% or 99% identity with the amino acid sequence of SEQ ID NO: 436, or consists of SEQ ID NO: 436, and the light chain variable region comprises the amino acid sequence as set forth in SEQ ID NO: 437, or an amino acid sequence having at least 90%, 91%, 92%, 93%, 94%, 95%, 96%, 97%, 98% or 99% identity with the amino acid sequence of SEQ ID NO: 437, or consists of SEQ ID NO: 437;
5) the heavy chain variable region comprises the amino acid sequence as set forth in SEQ ID NO: 438, or an amino acid sequence having at least 90%, 91%, 92%, 93%, 94%, 95%, 96%, 97%, 98% or 99% identity with the amino acid sequence of SEQ ID NO: 438, or consists of SEQ ID NO: 438, and the light chain variable region comprises the amino acid sequence as set forth in SEQ ID NO: 439, or an amino acid sequence having at least 90%, 91%, 92%, 93%, 94%, 95%, 96%, 97%, 98% or 99% identity with the amino acid sequence of SEQ ID NO: 439, or consists of SEQ ID NO: 439;
6) the heavy chain variable region comprises the amino acid sequence as set forth in SEQ ID NO: 438, or an amino acid sequence having at least 90%, 91%, 92%, 93%, 94%, 95%, 96%, 97%, 98% or 99% identity with the amino acid sequence of SEQ ID NO: 438, or consists of SEQ ID NO: 438, and the light chain variable region comprises the amino acid sequence as set forth in SEQ ID NO: 440, or an amino acid sequence having at least 90%, 91%, 92%, 93%, 94%, 95%, 96%, 97%, 98% or 99% identity with the amino acid sequence of SEQ ID NO: 440, or consists of SEQ ID NO: 440;
7) the heavy chain variable region comprises the amino acid sequence as set forth in SEQ ID NO: 441, or an amino acid sequence having at least 90%, 91%, 92%, 93%, 94%, 95%, 96%, 97%, 98% or 99% identity with the amino acid sequence of SEQ ID NO: 441, or consists of SEQ ID NO: 441, and the light chain variable region comprises the amino acid sequence as set forth in SEQ ID NO: 442, or an amino acid sequence having at least 90%, 91%, 92%, 93%, 94%, 95%, 96%, 97%, 98% or 99% identity with the amino acid sequence of SEQ ID NO: 442, or consists of SEQ ID NO: 442;
8) the heavy chain variable region comprises the amino acid sequence as set forth in SEQ ID NO: 443, or an amino acid sequence having at least 90%, 91%, 92%, 93%, 94%, 95%, 96%, 97%, 98% or 99% identity with the amino acid sequence of SEQ ID NO: 443, or consists of SEQ ID NO: 443, and the light chain variable region comprises the amino acid sequence as set forth in SEQ ID NO: 444, or an amino acid sequence having at least 90%, 91%, 92%, 93%, 94%, 95%, 96%, 97%, 98% or 99% identity with the amino acid sequence of SEQ ID NO: 444, or consists of SEQ ID NO: 444;
9) the heavy chain variable region comprises the amino acid sequence as set forth in SEQ ID NO: 445, or an amino acid sequence having at least 90%, 91%, 92%, 93%, 94%, 95%, 96%, 97%, 98% or 99% identity with the amino acid sequence of SEQ ID NO: 445, or consists of SEQ ID NO: 445, and the light chain variable region comprises the amino acid sequence as set forth in SEQ ID NO: 446, or an amino acid sequence having at least 90%, 91%, 92%, 93%, 94%, 95%, 96%, 97%, 98% or 99% identity with the amino acid sequence of SEQ ID NO: 446, or consists of SEQ ID NO: 446;
10) the heavy chain variable region comprises the amino acid sequence as set forth in SEQ ID NO: 447, or an amino acid sequence having at least 90%, 91%, 92%, 93%, 94%, 95%, 96%, 97%, 98% or 99% identity with the amino acid sequence of SEQ ID NO: 447, or consists of SEQ ID NO: 447, and the light chain variable region comprises the amino acid sequence as set forth in SEQ ID NO: 448, or an amino acid sequence having at least 90%, 91%, 92%, 93%, 94%, 95%, 96%, 97%, 98% or 99% identity with the amino acid sequence of SEQ ID NO: 448, or consists of SEQ ID NO: 448;
11) the heavy chain variable region comprises the amino acid sequence as set forth in SEQ ID NO: 449, or an amino acid sequence having at least 90%, 91%, 92%, 93%, 94%, 95%, 96%, 97%, 98% or 99% identity with the amino acid sequence of SEQ ID NO: 449, or consists of SEQ ID NO: 449, and the light chain variable region comprises the amino acid sequence as set forth in SEQ ID NO: 450, or an amino acid sequence having at least 90%, 91%, 92%, 93%, 94%, 95%, 96%, 97%, 98% or 99% identity with the amino acid sequence of SEQ ID NO: 450, or consists of SEQ ID NO: 450;
12) the heavy chain variable region comprises the amino acid sequence as set forth in SEQ ID NO: 451, or an amino acid sequence having at least 90%, 91%, 92%, 93%, 94%, 95%, 96%, 97%, 98% or 99% identity with the amino acid sequence of SEQ ID NO: 451, or consists of SEQ ID NO: 451, and the light chain variable region comprises the amino acid sequence as set forth in SEQ ID NO: 452, or an amino acid sequence having at least 90%, 91%, 92%, 93%, 94%, 95%, 96%, 97%, 98% or 99% identity with the amino acid sequence of SEQ ID NO: 452, or consists of SEQ ID NO: 452;
13) the heavy chain variable region comprises the amino acid sequence as set forth in SEQ ID NO: 453, or an amino acid sequence having at least 90%, 91%, 92%, 93%, 94%, 95%, 96%, 97%, 98% or 99% identity with the amino acid sequence of SEQ ID NO: 453, or consists of SEQ ID NO: 453, and the light chain variable region comprises the amino acid sequence as set forth in SEQ ID NO: 454, or an amino acid sequence having at least 90%, 91%, 92%, 93%, 94%, 95%, 96%, 97%, 98% or 99% identity with the amino acid sequence of SEQ ID NO: 454, or consists of SEQ ID NO: 454;
14) the heavy chain variable region comprises the amino acid sequence as set forth in SEQ ID NO: 455, or an amino acid sequence having at least 90%, 91%, 92%, 93%, 94%, 95%, 96%, 97%, 98% or 99% identity with the amino acid sequence of SEQ ID NO: 455, or consists of SEQ ID NO: 455, and the light chain variable region comprises the amino acid sequence as set forth in SEQ ID NO: 456, or an amino acid sequence having at least 90%, 91%, 92%, 93%, 94%, 95%, 96%, 97%, 98% or 99% identity with the amino acid sequence of SEQ ID NO: 456, or consists of SEQ ID NO: 456;
15) the heavy chain variable region comprises the amino acid sequence as set forth in SEQ ID NO: 457, or an amino acid sequence having at least 90%, 91%, 92%, 93%, 94%, 95%, 96%, 97%, 98% or 99% identity with the amino acid sequence of SEQ ID NO: 457, or consists of SEQ ID NO: 457, and the light chain variable region comprises the amino acid sequence as set forth in SEQ ID NO: 458, or an amino acid sequence having at least 90%, 91%, 92%, 93%, 94%, 95%, 96%, 97%, 98% or 99% identity with the amino acid sequence of SEQ ID NO: 458, or consists of SEQ ID NO: 458;
16) the heavy chain variable region comprises the amino acid sequence as set forth in SEQ ID NO: 459, or an amino acid sequence having at least 90%, 91%, 92%, 93%, 94%, 95%, 96%, 97%, 98% or 99% identity with the amino acid sequence of SEQ ID NO: 459, or consists of SEQ ID NO: 459, and the light chain variable region comprises the amino acid sequence as set forth in SEQ ID NO: 460, or an amino acid sequence having at least 90%, 91%, 92%, 93%, 94%, 95%, 96%, 97%, 98% or 99% identity with the amino acid sequence of SEQ ID NO: 460, or consists of SEQ ID NO: 460;
17) the heavy chain variable region comprises the amino acid sequence as set forth in SEQ ID NO: 461, or an amino acid sequence having at least 90%, 91%, 92%, 93%, 94%, 95%, 96%, 97%, 98% or 99% identity with the amino acid sequence of SEQ ID NO: 461, or consists of SEQ ID NO: 461, and the light chain variable region comprises the amino acid sequence as set forth in SEQ ID NO: 462, or an amino acid sequence having at least 90%, 91%, 92%, 93%, 94%, 95%, 96%, 97%, 98% or 99% identity with the amino acid sequence of SEQ ID NO: 462, or consists of SEQ ID NO: 462;
18) the heavy chain variable region comprises the amino acid sequence as set forth in SEQ ID NO: 463, or an amino acid sequence having at least 90%, 91%, 92%, 93%, 94%, 95%, 96%, 97%, 98% or 99% identity with the amino acid sequence of SEQ ID NO: 463, or consists of SEQ ID NO: 463, and the light chain variable region comprises the amino acid sequence as set forth in SEQ ID NO: 464, or an amino acid sequence having at least 90%, 91%, 92%, 93%, 94%, 95%, 96%, 97%, 98% or 99% identity with the amino acid sequence of SEQ ID NO: 464, or consists of SEQ ID NO: 464;
19) the heavy chain variable region comprises the amino acid sequence as set forth in SEQ ID NO: 465, or an amino acid sequence having at least 90%, 91%, 92%, 93%, 94%, 95%, 96%, 97%, 98% or 99% identity with the amino acid sequence of SEQ ID NO: 465, or consists of SEQ ID NO: 465, and the light chain variable region comprises the amino acid sequence as set forth in SEQ ID NO: 466, or an amino acid sequence having at least 90%, 91%, 92%, 93%, 94%, 95%, 96%, 97%, 98% or 99% identity with the amino acid sequence of SEQ ID NO: 466, or consists of SEQ ID NO: 466;
20) the heavy chain variable region comprises the amino acid sequence as set forth in SEQ ID NO: 467, or an amino acid sequence having at least 90%, 91%, 92%, 93%, 94%, 95%, 96%, 97%, 98% or 99% identity with the amino acid sequence of SEQ ID NO: 467, or consists of SEQ ID NO: 467, and the light chain variable region comprises the amino acid sequence as set forth in SEQ ID NO: 468, or an amino acid sequence having at least 90%, 91%, 92%, 93%, 94%, 95%, 96%, 97%, 98% or 99% identity with the amino acid sequence of SEQ ID NO: 468, or consists of SEQ ID NO: 468;
21) the heavy chain variable region comprises the amino acid sequence as set forth in SEQ ID NO: 469, or an amino acid sequence having at least 90%, 91%, 92%, 93%, 94%, 95%, 96%, 97%, 98% or 99% identity with the amino acid sequence of SEQ ID NO: 469, or consists of SEQ ID NO: 469, and the light chain variable region comprises the amino acid sequence as set forth in SEQ ID NO: 470, or an amino acid sequence having at least 90%, 91%, 92%, 93%, 94%, 95%, 96%, 97%, 98% or 99% identity with the amino acid sequence of SEQ ID NO: 470, or consists of SEQ ID NO: 470;
22) the heavy chain variable region comprises the amino acid sequence as set forth in SEQ ID NO: 471, or an amino acid sequence having at least 90%, 91%, 92%, 93%, 94%, 95%, 96%, 97%, 98% or 99% identity with the amino acid sequence of SEQ ID NO: 471, or consists of SEQ ID NO: 471, and the light chain variable region comprises the amino acid sequence as set forth in SEQ ID NO: 472, or an amino acid sequence having at least 90%, 91%, 92%, 93%, 94%, 95%, 96%, 97%, 98% or 99% identity with the amino acid sequence of SEQ ID NO: 472, or consists of SEQ ID NO: 472;
23) the heavy chain variable region comprises the amino acid sequence as set forth in SEQ ID NO: 473, or an amino acid sequence having at least 90%, 91%, 92%, 93%, 94%, 95%, 96%, 97%, 98% or 99% identity with the amino acid sequence of SEQ ID NO: 473, or consists of SEQ ID NO: 473, and the light chain variable region comprises the amino acid sequence as set forth in SEQ ID NO: 474, or an amino acid sequence having at least 90%, 91%, 92%, 93%, 94%, 95%, 96%, 97%, 98% or 99% identity with the amino acid sequence of SEQ ID NO: 474, or consists of SEQ ID NO: 474;
24) the heavy chain variable region comprises the amino acid sequence as set forth in SEQ ID NO: 475, or an amino acid sequence having at least 90%, 91%, 92%, 93%, 94%, 95%, 96%, 97%, 98% or 99% identity with the amino acid sequence of SEQ ID NO: 475, or consists of SEQ ID NO: 475, and the light chain variable region comprises the amino acid sequence as set forth in SEQ ID NO: 476, or an amino acid sequence having at least 90%, 91%, 92%, 93%, 94%, 95%, 96%, 97%, 98% or 99% identity with the amino acid sequence of SEQ ID NO: 476, or consists of SEQ ID NO: 476;
25) the heavy chain variable region comprises the amino acid sequence as set forth in SEQ ID NO: 477, or an amino acid sequence having at least 90%, 91%, 92%, 93%, 94%, 95%, 96%, 97%, 98% or 99% identity with the amino acid sequence of SEQ ID NO: 477, or consists of SEQ ID NO: 477, and the light chain variable region comprises the amino acid sequence as set forth in SEQ ID NO: 478, or an amino acid sequence having at least 90%, 91%, 92%, 93%, 94%, 95%, 96%, 97%, 98% or 99% identity with the amino acid sequence of SEQ ID NO: 478, or consists of SEQ ID NO: 478;
26) the heavy chain variable region comprises the amino acid sequence as set forth in SEQ ID NO: 479, or an amino acid sequence having at least 90%, 91%, 92%, 93%, 94%, 95%, 96%, 97%, 98% or 99% identity to the amino acid sequence of SEQ ID NO: 479, or consists of SEQ ID NO: 479, and the light chain variable region comprises the amino acid sequence as set forth in SEQ ID NO: 480, or an amino acid sequence having at least 90%, 91%, 92%, 93%, 94%, 95%, 96%, 97%, 98% or 99% identity to the amino acid sequence of SEQ ID NO: 480, or consists of SEQ ID NO: 480; or
27) The heavy chain variable region comprises the amino acid sequence as set forth in SEQ ID NO: 481, or an amino acid sequence having at least 90%, 91%, 92%, 93%, 94%, 95%, 96%, 97%, 98% or 99% identity to the amino acid sequence of SEQ ID NO: 481, or consists of SEQ ID NO: 481, and the light chain variable region comprises the amino acid sequence as set forth in SEQ ID NO: 482, or an amino acid sequence having at least 90%, 91%, 92%, 93%, 94%, 95%, 96%, 97%, 98% or 99% identity to the amino acid sequence of SEQ ID NO: 482, or consists of SEQ ID NO: 482.
28) the heavy chain variable region comprises the amino acid sequence as set forth in SEQ ID NO: 483, or an amino acid sequence having at least 90%, 91%, 92%, 93%, 94%, 95%, 96%, 97%, 98% or 99% identity with the amino acid sequence of SEQ ID NO: 483, or consists of SEQ ID NO: 483, and the light chain variable region comprises the amino acid sequence as set forth in SEQ ID NO: 484, or an amino acid sequence having at least 90%, 91%, 92%, 93%, 94%, 95%, 96%, 97%, 98% or 99% identity with the amino acid sequence of SEQ ID NO: 484, or consists of SEQ ID NO: 484;
29) the heavy chain variable region comprises the amino acid sequence as set forth in SEQ ID NO: 485, or an amino acid sequence having at least 90%, 91%, 92%, 93%, 94%, 95%, 96%, 97%, 98% or 99% identity with the amino acid sequence of SEQ ID NO: 485, or consists of SEQ ID NO: 485, and the light chain variable region comprises the amino acid sequence as set forth in SEQ ID NO: 486, or an amino acid sequence having at least 90%, 91%, 92%, 93%, 94%, 95%, 96%, 97%, 98% or 99% identity with the amino acid sequence of SEQ ID NO: 486, or consists of SEQ ID NO: 486;
30) the heavy chain variable region comprises the amino acid sequence as set forth in SEQ ID NO: 487, or an amino acid sequence having at least 90%, 91%, 92%, 93%, 94%, 95%, 96%, 97%, 98% or 99% identity with the amino acid sequence of SEQ ID NO: 487, or consists of SEQ ID NO: 487, and the light chain variable region comprises the amino acid sequence as set forth in SEQ ID NO: 456, or an amino acid sequence having at least 90%, 91%, 92%, 93%, 94%, 95%, 96%, 97%, 98% or 99% identity with the amino acid sequence of SEQ ID NO: 456, or consists of SEQ ID NO: 456;
31) the heavy chain variable region comprises the amino acid sequence as set forth in SEQ ID NO: 488, or an amino acid sequence having at least 90%, 91%, 92%, 93%, 94%, 95%, 96%, 97%, 98% or 99% identity with the amino acid sequence of SEQ ID NO: 488, or consists of SEQ ID NO: 488, and the light chain variable region comprises the amino acid sequence as set forth in SEQ ID NO: 489, or an amino acid sequence having at least 90%, 91%, 92%, 93%, 94%, 95%, 96%, 97%, 98% or 99% identity with the amino acid sequence of SEQ ID NO: 489, or consists of SEQ ID NO: 489;
32) the heavy chain variable region comprises the amino acid sequence as set forth in SEQ ID NO: 490, or an amino acid sequence having at least 90%, 91%, 92%, 93%, 94%, 95%, 96%, 97%, 98% or 99% identity with the amino acid sequence of SEQ ID NO: 490, or consists of SEQ ID NO: 490, and the light chain variable region comprises the amino acid sequence as set forth in SEQ ID NO: 491, or an amino acid sequence having at least 90%, 91%, 92%, 93%, 94%, 95%, 96%, 97%, 98% or 99% identity with the amino acid sequence of SEQ ID NO: 491, or consists of SEQ ID NO: 491;
33) the heavy chain variable region comprises the amino acid sequence as set forth in SEQ ID NO: 492, or an amino acid sequence having at least 90%, 91%, 92%, 93%, 94%, 95%, 96%, 97%, 98% or 99% identity with the amino acid sequence of SEQ ID NO: 492, or consists of SEQ ID NO: 492, and the light chain variable region comprises the amino acid sequence as set forth in SEQ ID NO: 489, or an amino acid sequence having at least 90%, 91%, 92%, 93%, 94%, 95%, 96%, 97%, 98% or 99% identity with the amino acid sequence of SEQ ID NO: 489, or consists of SEQ ID NO: 489;
34) the heavy chain variable region comprises the amino acid sequence as set forth in SEQ ID NO: 493, or an amino acid sequence having at least 90%, 91%, 92%, 93%, 94%, 95%, 96%, 97%, 98% or 99% identity with the amino acid sequence of SEQ ID NO: 493, or consists of SEQ ID NO: 493, and the light chain variable region comprises the amino acid sequence as set forth in SEQ ID NO: 494, or an amino acid sequence having at least 90%, 91%, 92%, 93%, 94%, 95%, 96%, 97%, 98% or 99% identity with the amino acid sequence of SEQ ID NO: 494, or consists of SEQ ID NO: 494;
35) the heavy chain variable region comprises the amino acid sequence as set forth in SEQ ID NO: 493, or an amino acid sequence having at least 90%, 91%, 92%, 93%, 94%, 95%, 96%, 97%, 98% or 99% identity with the amino acid sequence of SEQ ID NO: 493, or consists of SEQ ID NO: 493, and the light chain variable region comprises the amino acid sequence as set forth in SEQ ID NO: 495, or an amino acid sequence having at least 90%, 91%, 92%, 93%, 94%, 95%, 96%, 97%, 98% or 99% identity with the amino acid sequence of SEQ ID NO: 495, or consists of SEQ ID NO: 495;
36) the heavy chain variable region comprises the amino acid sequence as set forth in SEQ ID NO: 496, or an amino acid sequence having at least 90%, 91%, 92%, 93%, 94%, 95%, 96%, 97%, 98% or 99% identity with the amino acid sequence of SEQ ID NO: 496, or consists of SEQ ID NO: 496, and the light chain variable region comprises the amino acid sequence as set forth in SEQ ID NO: 497, or an amino acid sequence having at least 90%, 91%, 92%, 93%, 94%, 95%, 96%, 97%, 98% or 99% identity with the amino acid sequence of SEQ ID NO: 497, or consists of SEQ ID NO: 497;
37) the heavy chain variable region comprises the amino acid sequence as set forth in SEQ ID NO: 498, or an amino acid sequence having at least 90%, 91%, 92%, 93%, 94%, 95%, 96%, 97%, 98% or 99% identity with the amino acid sequence of SEQ ID NO: 498, or consists of SEQ ID NO: 498, and the light chain variable region comprises the amino acid sequence as set forth in SEQ ID NO: 497, or an amino acid sequence having at least 90%, 91%, 92%, 93%, 94%, 95%, 96%, 97%, 98% or 99% identity with the amino acid sequence of SEQ ID NO: 497, or consists of SEQ ID NO: 497;
38) the heavy chain variable region comprises the amino acid sequence as set forth in SEQ ID NO: 499, or an amino acid sequence having at least 90%, 91%, 92%, 93%, 94%, 95%, 96%, 97%, 98% or 99% identity with the amino acid sequence of SEQ ID NO: 499, or consists of SEQ ID NO: 499, and the light chain variable region comprises the amino acid sequence as set forth in SEQ ID NO: 500, or an amino acid sequence having at least 90%, 91%, 92%, 93%, 94%, 95%, 96%, 97%, 98% or 99% identity with the amino acid sequence of SEQ ID NO: 500, or consists of SEQ ID NO: 500;
39) the heavy chain variable region comprises the amino acid sequence as set forth in SEQ ID NO: 501, or an amino acid sequence having at least 90%, 91%, 92%, 93%, 94%, 95%, 96%, 97%, 98% or 99% identity with the amino acid sequence of SEQ ID NO: 501, or consists of SEQ ID NO: 501, and the light chain variable region comprises the amino acid sequence as set forth in SEQ ID NO: 503, or an amino acid sequence having at least 90%, 91%, 92%, 93%, 94%, 95%, 96%, 97%, 98% or 99% identity with the amino acid sequence of SEQ ID NO: 503, or consists of SEQ ID NO: 503;
40) the heavy chain variable region comprises the amino acid sequence as set forth in SEQ ID NO: 502, or an amino acid sequence having at least 90%, 91%, 92%, 93%, 94%, 95%, 96%, 97%, 98% or 99% identity with the amino acid sequence of SEQ ID NO: 502, or consists of SEQ ID NO: 502, and the light chain variable region comprises the amino acid sequence as set forth in SEQ ID NO: 503, or an amino acid sequence having at least 90%, 91%, 92%, 93%, 94%, 95%, 96%, 97%, 98% or 99% identity with the amino acid sequence of SEQ ID NO: 503, or consists of SEQ ID NO: 503;
41) the heavy chain variable region comprises the amino acid sequence as set forth in SEQ ID NO: 504, or an amino acid sequence having at least 90%, 91%, 92%, 93%, 94%, 95%, 96%, 97%, 98% or 99% identity with the amino acid sequence of SEQ ID NO: 504, or consists of SEQ ID NO: 504, and the light chain variable region comprises the amino acid sequence as set forth in SEQ ID NO: 505, or an amino acid sequence having at least 90%, 91%, 92%, 93%, 94%, 95%, 96%, 97%, 98% or 99% identity with the amino acid sequence of SEQ ID NO: 505, or consists of SEQ ID NO: 505;
42) the heavy chain variable region comprises the amino acid sequence as set forth in SEQ ID NO: 499, or an amino acid sequence having at least 90%, 91%, 92%, 93%, 94%, 95%, 96%, 97%, 98% or 99% identity with the amino acid sequence of SEQ ID NO: 499, or consists of SEQ ID NO: 499, and the light chain variable region comprises the amino acid sequence as set forth in SEQ ID NO: 506, or an amino acid sequence having at least 90%, 91%, 92%, 93%, 94%, 95%, 96%, 97%, 98% or 99% identity with the amino acid sequence of SEQ ID NO: 506, or consists of SEQ ID NO: 506;
43) the heavy chain variable region comprises the amino acid sequence as set forth in SEQ ID NO: 507, or an amino acid sequence having at least 90%, 91%, 92%, 93%, 94%, 95%, 96%, 97%, 98% or 99% identity with the amino acid sequence of SEQ ID NO: 507, or consists of SEQ ID NO: 507, and the light chain variable region comprises the amino acid sequence as set forth in SEQ ID NO: 503, or an amino acid sequence having at least 90%, 91%, 92%, 93%, 94%, 95%, 96%, 97%, 98% or 99% identity with the amino acid sequence of SEQ ID NO: 503, or consists of SEQ ID NO: 503;
44) the heavy chain variable region comprises the amino acid sequence as set forth in SEQ ID NO: 508, or an amino acid sequence having at least 90%, 91%, 92%, 93%, 94%, 95%, 96%, 97%, 98% or 99% identity with the amino acid sequence of SEQ ID NO: 508, or consists of SEQ ID NO: 508, and the light chain variable region comprises the amino acid sequence as set forth in SEQ ID NO: 509, or an amino acid sequence having at least 90%, 91%, 92%, 93%, 94%, 95%, 96%, 97%, 98% or 99% identity with the amino acid sequence of SEQ ID NO: 509, or consists of SEQ ID NO: 509;
45) the heavy chain variable region comprises the amino acid sequence as set forth in SEQ ID NO: 510, or an amino acid sequence having at least 90%, 91%, 92%, 93%, 94%, 95%, 96%, 97%, 98% or 99% identity with the amino acid sequence of SEQ ID NO: 510, or consists of SEQ ID NO: 510, and the light chain variable region comprises the amino acid sequence as set forth in SEQ ID NO: 511, or an amino acid sequence having at least 90%, 91%, 92%, 93%, 94%, 95%, 96%, 97%, 98% or 99% identity with the amino acid sequence of SEQ ID NO: 511, or consists of SEQ ID NO: 511;
46) the heavy chain variable region comprises the amino acid sequence as set forth in SEQ ID NO: 512, or an amino acid sequence having at least 90%, 91%, 92%, 93%, 94%, 95%, 96%, 97%, 98% or 99% identity to the amino acid sequence of SEQ ID NO: 512, or consists of SEQ ID NO: 512, and the light chain variable region comprises the amino acid sequence as set forth in SEQ ID NO: 513, or an amino acid sequence having at least 90%, 91%, 92%, 93%, 94%, 95%, 96%, 97%, 98% or 99% identity to the amino acid sequence of SEQ ID NO: 513, or consists of SEQ ID NO: 513; or
47) The heavy chain variable region comprises the amino acid sequence as set forth in SEQ ID NO: 514, or an amino acid sequence having at least 90%, 91%, 92%, 93%, 94%, 95%, 96%, 97%, 98% or 99% identity to the amino acid sequence of SEQ ID NO: 514, or consists of SEQ ID NO: 514, and the light chain variable region comprises the amino acid sequence as set forth in SEQ ID NO: 515, or an amino acid sequence having at least 90%, 91%, 92%, 93%, 94%, 95%, 96%, 97%, 98% or 99% identity to the amino acid sequence of SEQ ID NO: 515, or consists of SEQ ID NO: 515.
48) the heavy chain variable region comprises the amino acid sequence as set forth in SEQ ID NO: 501, or an amino acid sequence having at least 90%, 91%, 92%, 93%, 94%, 95%, 96%, 97%, 98% or 99% identity with the amino acid sequence of SEQ ID NO: 501, or consists of SEQ ID NO: 501, and the light chain variable region comprises the amino acid sequence as set forth in SEQ ID NO: 516, or an amino acid sequence having at least 90%, 91%, 92%, 93%, 94%, 95%, 96%, 97%, 98% or 99% identity with the amino acid sequence of SEQ ID NO: 516, or consists of SEQ ID NO: 516;
49) the heavy chain variable region comprises the amino acid sequence as set forth in SEQ ID NO: 517, or an amino acid sequence having at least 90%, 91%, 92%, 93%, 94%, 95%, 96%, 97%, 98% or 99% identity with the amino acid sequence of SEQ ID NO: 517, or consists of SEQ ID NO: 517, and the light chain variable region comprises the amino acid sequence as set forth in SEQ ID NO: 518, or an amino acid sequence having at least 90%, 91%, 92%, 93%, 94%, 95%, 96%, 97%, 98% or 99% identity with the amino acid sequence of SEQ ID NO: 518, or consists of SEQ ID NO: 518;
50) the heavy chain variable region comprises the amino acid sequence as set forth in SEQ ID NO: 519, or an amino acid sequence having at least 90%, 91%, 92%, 93%, 94%, 95%, 96%, 97%, 98% or 99% identity with the amino acid sequence of SEQ ID NO: 519, or consists of SEQ ID NO: 519, and the light chain variable region comprises the amino acid sequence as set forth in SEQ ID NO: 520, or an amino acid sequence having at least 90%, 91%, 92%, 93%, 94%, 95%, 96%, 97%, 98% or 99% identity with the amino acid sequence of SEQ ID NO: 520, or consists of SEQ ID NO: 520;
51) the heavy chain variable region comprises the amino acid sequence as set forth in SEQ ID NO: 521, or an amino acid sequence having at least 90%, 91%, 92%, 93%, 94%, 95%, 96%, 97%, 98% or 99% identity with the amino acid sequence of SEQ ID NO: 521, or consists of SEQ ID NO: 521, and the light chain variable region comprises the amino acid sequence as set forth in SEQ ID NO: 522, or an amino acid sequence having at least 90%, 91%, 92%, 93%, 94%, 95%, 96%, 97%, 98% or 99% identity with the amino acid sequence of SEQ ID NO: 522, or consists of SEQ ID NO: 522;
52) the heavy chain variable region comprises the amino acid sequence as set forth in SEQ ID NO: 523, or an amino acid sequence having at least 90%, 91%, 92%, 93%, 94%, 95%, 96%, 97%, 98% or 99% identity with the amino acid sequence of SEQ ID NO: 523, or consists of SEQ ID NO: 523, and the light chain variable region comprises the amino acid sequence as set forth in SEQ ID NO: 524, or an amino acid sequence having at least 90%, 91%, 92%, 93%, 94%, 95%, 96%, 97%, 98% or 99% identity with the amino acid sequence of SEQ ID NO: 524, or consists of SEQ ID NO: 524;
53) the heavy chain variable region comprises the amino acid sequence as set forth in SEQ ID NO: 525, or an amino acid sequence having at least 90%, 91%, 92%, 93%, 94%, 95%, 96%, 97%, 98% or 99% identity with the amino acid sequence of SEQ ID NO: 525, or consists of SEQ ID NO: 525, and the light chain variable region comprises the amino acid sequence as set forth in SEQ ID NO: 486, or an amino acid sequence having at least 90%, 91%, 92%, 93%, 94%, 95%, 96%, 97%, 98% or 99% identity with the amino acid sequence of SEQ ID NO: 486, or consists of SEQ ID NO: 486;
54) the heavy chain variable region comprises the amino acid sequence as set forth in SEQ ID NO: 526, or an amino acid sequence having at least 90%, 91%, 92%, 93%, 94%, 95%, 96%, 97%, 98% or 99% identity with the amino acid sequence of SEQ ID NO: 526, or consists of SEQ ID NO: 526, and the light chain variable region comprises the amino acid sequence as set forth in SEQ ID NO: 527, or an amino acid sequence having at least 90%, 91%, 92%, 93%, 94%, 95%, 96%, 97%, 98% or 99% identity with the amino acid sequence of SEQ ID NO: 527, or consists of SEQ ID NO: 527;
55) the heavy chain variable region comprises the amino acid sequence as set forth in SEQ ID NO: 528, or an amino acid sequence having at least 90%, 91%, 92%, 93%, 94%, 95%, 96%, 97%, 98% or 99% identity with the amino acid sequence of SEQ ID NO: 528, or consists of SEQ ID NO: 528, and the light chain variable region comprises the amino acid sequence as set forth in SEQ ID NO: 529, or an amino acid sequence having at least 90%, 91%, 92%, 93%, 94%, 95%, 96%, 97%, 98% or 99% identity with the amino acid sequence of SEQ ID NO: 529, or consists of SEQ ID NO: 529;
56) the heavy chain variable region comprises the amino acid sequence as set forth in SEQ ID NO: 530, or an amino acid sequence having at least 90%, 91%, 92%, 93%, 94%, 95%, 96%, 97%, 98% or 99% identity with the amino acid sequence of SEQ ID NO: 530, or consists of SEQ ID NO: 530, and the light chain variable region comprises the amino acid sequence as set forth in SEQ ID NO: 531, or an amino acid sequence having at least 90%, 91%, 92%, 93%, 94%, 95%, 96%, 97%, 98% or 99% identity with the amino acid sequence of SEQ ID NO: 531, or consists of SEQ ID NO: 531;
57) the heavy chain variable region comprises the amino acid sequence as set forth in SEQ ID NO: 530, or an amino acid sequence having at least 90%, 91%, 92%, 93%, 94%, 95%, 96%, 97%, 98% or 99% identity with the amino acid sequence of SEQ ID NO: 530, or consists of SEQ ID NO: 530, and the light chain variable region comprises the amino acid sequence as set forth in SEQ ID NO: 533, or an amino acid sequence having at least 90%, 91%, 92%, 93%, 94%, 95%, 96%, 97%, 98% or 99% identity with the amino acid sequence of SEQ ID NO: 533, or consists of SEQ ID NO: 533;
58) the heavy chain variable region comprises the amino acid sequence as set forth in SEQ ID NO: 532, or an amino acid sequence having at least 90%, 91%, 92%, 93%, 94%, 95%, 96%, 97%, 98% or 99% identity with the amino acid sequence of SEQ ID NO: 532, or consists of SEQ ID NO: 532, and the light chain variable region comprises the amino acid sequence as set forth in SEQ ID NO: 531, or an amino acid sequence having at least 90%, 91%, 92%, 93%, 94%, 95%, 96%, 97%, 98% or 99% identity with the amino acid sequence of SEQ ID NO: 531, or consists of SEQ ID NO: 531;
59) the heavy chain variable region comprises the amino acid sequence as set forth in SEQ ID NO: 532, or an amino acid sequence having at least 90%, 91%, 92%, 93%, 94%, 95%, 96%, 97%, 98% or 99% identity with the amino acid sequence of SEQ ID NO: 532, or consists of SEQ ID NO: 532, and the light chain variable region comprises the amino acid sequence as set forth in SEQ ID NO: 533, or an amino acid sequence having at least 90%, 91%, 92%, 93%, 94%, 95%, 96%, 97%, 98% or 99% identity with the amino acid sequence of SEQ ID NO: 533, or consists of SEQ ID NO: 533;
60) the heavy chain variable region comprises the amino acid sequence as set forth in SEQ ID NO: 534, or an amino acid sequence having at least 90%, 91%, 92%, 93%, 94%, 95%, 96%, 97%, 98% or 99% identity with the amino acid sequence of SEQ ID NO: 534, or consists of SEQ ID NO: 534, and the light chain variable region comprises the amino acid sequence as set forth in SEQ ID NO: 535, or an amino acid sequence having at least 90%, 91%, 92%, 93%, 94%, 95%, 96%, 97%, 98% or 99% identity with the amino acid sequence of SEQ ID NO: 535, or consists of SEQ ID NO: 535;
61) the heavy chain variable region comprises the amino acid sequence as set forth in SEQ ID NO: 536, or an amino acid sequence having at least 90%, 91%, 92%, 93%, 94%, 95%, 96%, 97%, 98% or 99% identity with the amino acid sequence of SEQ ID NO: 536, or consists of SEQ ID NO: 536, and the light chain variable region comprises the amino acid sequence as set forth in SEQ ID NO: 537, or an amino acid sequence having at least 90%, 91%, 92%, 93%, 94%, 95%, 96%, 97%, 98% or 99% identity with the amino acid sequence of SEQ ID NO: 537, or consists of SEQ ID NO: 537;
62) the heavy chain variable region comprises the amino acid sequence as set forth in SEQ ID NO: 538, or an amino acid sequence having at least 90%, 91%, 92%, 93%, 94%, 95%, 96%, 97%, 98% or 99% identity with the amino acid sequence of SEQ ID NO: 538, or consists of SEQ ID NO: 538, and the light chain variable region comprises the amino acid sequence as set forth in SEQ ID NO: 539, or an amino acid sequence having at least 90%, 91%, 92%, 93%, 94%, 95%, 96%, 97%, 98% or 99% identity with the amino acid sequence of SEQ ID NO: 539, or consists of SEQ ID NO: 539;
63) the heavy chain variable region comprises the amino acid sequence as set forth in SEQ ID NO: 540, or an amino acid sequence having at least 90%, 91%, 92%, 93%, 94%, 95%, 96%, 97%, 98% or 99% identity with the amino acid sequence of SEQ ID NO: 540, or consists of SEQ ID NO: 540, and the light chain variable region comprises the amino acid sequence as set forth in SEQ ID NO: 541, or an amino acid sequence having at least 90%, 91%, 92%, 93%, 94%, 95%, 96%, 97%, 98% or 99% identity with the amino acid sequence of SEQ ID NO: 541, or consists of SEQ ID NO: 541;
64) the heavy chain variable region comprises the amino acid sequence as set forth in SEQ ID NO: 540, or an amino acid sequence having at least 90%, 91%, 92%, 93%, 94%, 95%, 96%, 97%, 98% or 99% identity with the amino acid sequence of SEQ ID NO: 540, or consists of SEQ ID NO: 540, and the light chain variable region comprises the amino acid sequence as set forth in SEQ ID NO: 542, or an amino acid sequence having at least 90%, 91%, 92%, 93%, 94%, 95%, 96%, 97%, 98% or 99% identity with the amino acid sequence of SEQ ID NO: 542, or consists of SEQ ID NO: 542;
65) the heavy chain variable region comprises the amino acid sequence as set forth in SEQ ID NO: 543, or an amino acid sequence having at least 90%, 91%, 92%, 93%, 94%, 95%, 96%, 97%, 98% or 99% identity with the amino acid sequence of SEQ ID NO: 543, or consists of SEQ ID NO: 543, and the light chain variable region comprises the amino acid sequence as set forth in SEQ ID NO: 544, or an amino acid sequence having at least 90%, 91%, 92%, 93%, 94%, 95%, 96%, 97%, 98% or 99% identity with the amino acid sequence of SEQ ID NO: 544, or consists of SEQ ID NO: 544;
66) the heavy chain variable region comprises the amino acid sequence as set forth in SEQ ID NO: 545, or an amino acid sequence having at least 90%, 91%, 92%, 93%, 94%, 95%, 96%, 97%, 98% or 99% identity to the amino acid sequence of SEQ ID NO: 545, or consists of SEQ ID NO: 545, and the light chain variable region comprises the amino acid sequence as set forth in SEQ ID NO: 546, or an amino acid sequence having at least 90%, 91%, 92%, 93%, 94%, 95%, 96%, 97%, 98% or 99% identity to the amino acid sequence of SEQ ID NO: 546, or consists of SEQ ID NO: 546; or
67) The heavy chain variable region comprises the amino acid sequence as set forth in SEQ ID NO: 547, or an amino acid sequence having at least 90%, 91%, 92%, 93%, 94%, 95%, 96%, 97%, 98% or 99% identity to the amino acid sequence of SEQ ID NO: 547, or consists of SEQ ID NO: 547, and the light chain variable region comprises the amino acid sequence as set forth in SEQ ID NO: 548, or an amino acid sequence having at least 90%, 91%, 92%, 93%, 94%, 95%, 96%, 97%, 98% or 99% identity to the amino acid sequence of SEQ ID NO: 548, or consists of SEQ ID NO: 548.
68) the heavy chain variable region comprises the amino acid sequence as set forth in SEQ ID NO: 549, or an amino acid sequence having at least 90%, 91%, 92%, 93%, 94%, 95%, 96%, 97%, 98% or 99% identity with the amino acid sequence of SEQ ID NO: 549, or consists of SEQ ID NO: 549, and the light chain variable region comprises the amino acid sequence as set forth in SEQ ID NO: 550, or an amino acid sequence having at least 90%, 91%, 92%, 93%, 94%, 95%, 96%, 97%, 98% or 99% identity with the amino acid sequence of SEQ ID NO: 550, or consists of SEQ ID NO: 550;
69) the heavy chain variable region comprises the amino acid sequence as set forth in SEQ ID NO: 551, or an amino acid sequence having at least 90%, 91%, 92%, 93%, 94%, 95%, 96%, 97%, 98% or 99% identity with the amino acid sequence of SEQ ID NO: 551, or consists of SEQ ID NO: 551, and the light chain variable region comprises the amino acid sequence as set forth in SEQ ID NO: 552, or an amino acid sequence having at least 90%, 91%, 92%, 93%, 94%, 95%, 96%, 97%, 98% or 99% identity with the amino acid sequence of SEQ ID NO: 552, or consists of SEQ ID NO: 552;
70) the heavy chain variable region comprises the amino acid sequence as set forth in SEQ ID NO: 553, or an amino acid sequence having at least 90%, 91%, 92%, 93%, 94%, 95%, 96%, 97%, 98% or 99% identity with the amino acid sequence of SEQ ID NO: 553, or consists of SEQ ID NO: 553, and the light chain variable region comprises the amino acid sequence as set forth in SEQ ID NO: 554, or an amino acid sequence having at least 90%, 91%, 92%, 93%, 94%, 95%, 96%, 97%, 98% or 99% identity with the amino acid sequence of SEQ ID NO: 554, or consists of SEQ ID NO: 554;
71) the heavy chain variable region comprises the amino acid sequence as set forth in SEQ ID NO: 555, or an amino acid sequence having at least 90%, 91%, 92%, 93%, 94%, 95%, 96%, 97%, 98% or 99% identity with the amino acid sequence of SEQ ID NO: 555, or consists of SEQ ID NO: 555, and the light chain variable region comprises the amino acid sequence as set forth in SEQ ID NO: 556, or an amino acid sequence having at least 90%, 91%, 92%, 93%, 94%, 95%, 96%, 97%, 98% or 99% identity with the amino acid sequence of SEQ ID NO: 556, or consists of SEQ ID NO: 556;
72) the heavy chain variable region comprises the amino acid sequence as set forth in SEQ ID NO: 557, or an amino acid sequence having at least 90%, 91%, 92%, 93%, 94%, 95%, 96%, 97%, 98% or 99% identity with the amino acid sequence of SEQ ID NO: 557, or consists of SEQ ID NO: 557, and the light chain variable region comprises the amino acid sequence as set forth in SEQ ID NO: 558, or an amino acid sequence having at least 90%, 91%, 92%, 93%, 94%, 95%, 96%, 97%, 98% or 99% identity with the amino acid sequence of SEQ ID NO: 558, or consists of SEQ ID NO: 558;
73) the heavy chain variable region comprises the amino acid sequence as set forth in SEQ ID NO: 559, or an amino acid sequence having at least 90%, 91%, 92%, 93%, 94%, 95%, 96%, 97%, 98% or 99% identity with the amino acid sequence of SEQ ID NO: 559, or consists of SEQ ID NO: 559, and the light chain variable region comprises the amino acid sequence as set forth in SEQ ID NO: 560, or an amino acid sequence having at least 90%, 91%, 92%, 93%, 94%, 95%, 96%, 97%, 98% or 99% identity with the amino acid sequence of SEQ ID NO: 560, or consists of SEQ ID NO: 560;
74) the heavy chain variable region comprises the amino acid sequence as set forth in SEQ ID NO: 561, or an amino acid sequence having at least 90%, 91%, 92%, 93%, 94%, 95%, 96%, 97%, 98% or 99% identity with the amino acid sequence of SEQ ID NO: 561, or consists of SEQ ID NO: 561, and the light chain variable region comprises the amino acid sequence as set forth in SEQ ID NO: 554, or an amino acid sequence having at least 90%, 91%, 92%, 93%, 94%, 95%, 96%, 97%, 98% or 99% identity with the amino acid sequence of SEQ ID NO: 554, or consists of SEQ ID NO: 554;
75) the heavy chain variable region comprises the amino acid sequence as set forth in SEQ ID NO: 562, or an amino acid sequence having at least 90%, 91%, 92%, 93%, 94%, 95%, 96%, 97%, 98% or 99% identity with the amino acid sequence of SEQ ID NO: 562, or consists of SEQ ID NO: 562, and the light chain variable region comprises the amino acid sequence as set forth in SEQ ID NO: 563, or an amino acid sequence having at least 90%, 91%, 92%, 93%, 94%, 95%, 96%, 97%, 98% or 99% identity with the amino acid sequence of SEQ ID NO: 563, or consists of SEQ ID NO: 563;
76) the heavy chain variable region comprises the amino acid sequence as set forth in SEQ ID NO: 564, or an amino acid sequence having at least 90%, 91%, 92%, 93%, 94%, 95%, 96%, 97%, 98% or 99% identity with the amino acid sequence of SEQ ID NO: 564, or consists of SEQ ID NO: 564, and the light chain variable region comprises the amino acid sequence as set forth in SEQ ID NO: 565, or an amino acid sequence having at least 90%, 91%, 92%, 93%, 94%, 95%, 96%, 97%, 98% or 99% identity with the amino acid sequence of SEQ ID NO: 565, or consists of SEQ ID NO: 565;
77) the heavy chain variable region comprises the amino acid sequence as set forth in SEQ ID NO: 566, or an amino acid sequence having at least 90%, 91%, 92%, 93%, 94%, 95%, 96%, 97%, 98% or 99% identity with the amino acid sequence of SEQ ID NO: 566, or consists of SEQ ID NO: 566, and the light chain variable region comprises the amino acid sequence as set forth in SEQ ID NO: 567, or an amino acid sequence having at least 90%, 91%, 92%, 93%, 94%, 95%, 96%, 97%, 98% or 99% identity with the amino acid sequence of SEQ ID NO: 567, or consists of SEQ ID NO: 567;
78) the heavy chain variable region comprises the amino acid sequence as set forth in SEQ ID NO: 568, or an amino acid sequence having at least 90%, 91%, 92%, 93%, 94%, 95%, 96%, 97%, 98% or 99% identity with the amino acid sequence of SEQ ID NO: 568, or consists of SEQ ID NO: 568, and the light chain variable region comprises the amino acid sequence as set forth in SEQ ID NO: 569, or an amino acid sequence having at least 90%, 91%, 92%, 93%, 94%, 95%, 96%, 97%, 98% or 99% identity with the amino acid sequence of SEQ ID NO: 569, or consists of SEQ ID NO: 569;
79) the heavy chain variable region comprises the amino acid sequence as set forth in SEQ ID NO: 570, or an amino acid sequence having at least 90%, 91%, 92%, 93%, 94%, 95%, 96%, 97%, 98% or 99% identity with the amino acid sequence of SEQ ID NO: 570, or consists of SEQ ID NO: 570, and the light chain variable region comprises the amino acid sequence as set forth in SEQ ID NO: 571, or an amino acid sequence having at least 90%, 91%, 92%, 93%, 94%, 95%, 96%, 97%, 98% or 99% identity with the amino acid sequence of SEQ ID NO: 571, or consists of SEQ ID NO: 571;
80) the heavy chain variable region comprises the amino acid sequence as set forth in SEQ ID NO: 572, or an amino acid sequence having at least 90%, 91%, 92%, 93%, 94%, 95%, 96%, 97%, 98% or 99% identity with the amino acid sequence of SEQ ID NO: 572, or consists of SEQ ID NO: 572, and the light chain variable region comprises the amino acid sequence as set forth in SEQ ID NO: 573, or an amino acid sequence having at least 90%, 91%, 92%, 93%, 94%, 95%, 96%, 97%, 98% or 99% identity with the amino acid sequence of SEQ ID NO: 573, or consists of SEQ ID NO: 573;
81) the heavy chain variable region comprises the amino acid sequence as set forth in SEQ ID NO: 574, or an amino acid sequence having at least 90%, 91%, 92%, 93%, 94%, 95%, 96%, 97%, 98% or 99% identity with the amino acid sequence of SEQ ID NO: 574, or consists of SEQ ID NO: 574, and the light chain variable region comprises the amino acid sequence as set forth in SEQ ID NO: 575, or an amino acid sequence having at least 90%, 91%, 92%, 93%, 94%, 95%, 96%, 97%, 98% or 99% identity with the amino acid sequence of SEQ ID NO: 575, or consists of SEQ ID NO: 575;
82) the heavy chain variable region comprises the amino acid sequence as set forth in SEQ ID NO: 576, or an amino acid sequence having at least 90%, 91%, 92%, 93%, 94%, 95%, 96%, 97%, 98% or 99% identity with the amino acid sequence of SEQ ID NO: 576, or consists of SEQ ID NO: 576, and the light chain variable region comprises the amino acid sequence as set forth in SEQ ID NO: 578, or an amino acid sequence having at least 90%, 91%, 92%, 93%, 94%, 95%, 96%, 97%, 98% or 99% identity with the amino acid sequence of SEQ ID NO: 578, or consists of SEQ ID NO: 578;
83) the heavy chain variable region comprises the amino acid sequence as set forth in SEQ ID NO: 577, or an amino acid sequence having at least 90%, 91%, 92%, 93%, 94%, 95%, 96%, 97%, 98% or 99% identity with the amino acid sequence of SEQ ID NO: 577, or consists of SEQ ID NO: 577, and the light chain variable region comprises the amino acid sequence as set forth in SEQ ID NO: 578, or an amino acid sequence having at least 90%, 91%, 92%, 93%, 94%, 95%, 96%, 97%, 98% or 99% identity with the amino acid sequence of SEQ ID NO: 578, or consists of SEQ ID NO: 578;
84) the heavy chain variable region comprises the amino acid sequence as set forth in SEQ ID NO: 579, or an amino acid sequence having at least 90%, 91%, 92%, 93%, 94%, 95%, 96%, 97%, 98% or 99% identity with the amino acid sequence of SEQ ID NO: 579, or consists of SEQ ID NO: 579, and the light chain variable region comprises the amino acid sequence as set forth in SEQ ID NO: 580, or an amino acid sequence having at least 90%, 91%, 92%, 93%, 94%, 95%, 96%, 97%, 98% or 99% identity with the amino acid sequence of SEQ ID NO: 580, or consists of SEQ ID NO: 580;
85) the heavy chain variable region comprises the amino acid sequence as set forth in SEQ ID NO: 581, or an amino acid sequence having at least 90%, 91%, 92%, 93%, 94%, 95%, 96%, 97%, 98% or 99% identity with the amino acid sequence of SEQ ID NO: 581, or consists of SEQ ID NO: 581, and the light chain variable region comprises the amino acid sequence as set forth in SEQ ID NO: 582, or an amino acid sequence having at least 90%, 91%, 92%, 93%, 94%, 95%, 96%, 97%, 98% or 99% identity with the amino acid sequence of SEQ ID NO: 582, or consists of SEQ ID NO: 582;
86) the heavy chain variable region comprises the amino acid sequence as set forth in SEQ ID NO: 583, or an amino acid sequence having at least 90%, 91%, 92%, 93%, 94%, 95%, 96%, 97%, 98% or 99% identity to the amino acid sequence of SEQ ID NO: 583, or consists of SEQ ID NO: 583, and the light chain variable region comprises the amino acid sequence as set forth in SEQ ID NO: 584, or an amino acid sequence having at least 90%, 91%, 92%, 93%, 94%, 95%, 96%, 97%, 98% or 99% identity to the amino acid sequence of SEQ ID NO: 584, or consists of SEQ ID NO: 584; or
87) The heavy chain variable region comprises the amino acid sequence as set forth in SEQ ID NO: 585, or an amino acid sequence having at least 90%, 91%, 92%, 93%, 94%, 95%, 96%, 97%, 98% or 99% identity to the amino acid sequence of SEQ ID NO: 585, or consists of SEQ ID NO: 585, and the light chain variable region comprises the amino acid sequence as set forth in SEQ ID NO: 586, or an amino acid sequence having at least 90%, 91%, 92%, 93%, 94%, 95%, 96%, 97%, 98% or 99% identity to the amino acid sequence of SEQ ID NO: 586, or consists of SEQ ID NO: 586.
88) the heavy chain variable region comprises the amino acid sequence as set forth in SEQ ID NO: 587, or an amino acid sequence having at least 90%, 91%, 92%, 93%, 94%, 95%, 96%, 97%, 98% or 99% identity with the amino acid sequence of SEQ ID NO: 587, or consists of SEQ ID NO: 587, and the light chain variable region comprises the amino acid sequence as set forth in SEQ ID NO: 588, or an amino acid sequence having at least 90%, 91%, 92%, 93%, 94%, 95%, 96%, 97%, 98% or 99% identity with the amino acid sequence of SEQ ID NO: 588, or consists of SEQ ID NO: 588;
89) the heavy chain variable region comprises the amino acid sequence as set forth in SEQ ID NO: 587, or an amino acid sequence having at least 90%, 91%, 92%, 93%, 94%, 95%, 96%, 97%, 98% or 99% identity with the amino acid sequence of SEQ ID NO: 587, or consists of SEQ ID NO: 587, and the light chain variable region comprises the amino acid sequence as set forth in SEQ ID NO: 589, or an amino acid sequence having at least 90%, 91%, 92%, 93%, 94%, 95%, 96%, 97%, 98% or 99% identity with the amino acid sequence of SEQ ID NO: 589, or consists of SEQ ID NO: 589;
90) the heavy chain variable region comprises the amino acid sequence as set forth in SEQ ID NO: 590, or an amino acid sequence having at least 90%, 91%, 92%, 93%, 94%, 95%, 96%, 97%, 98% or 99% identity with the amino acid sequence of SEQ ID NO: 590, or consists of SEQ ID NO: 590, and the light chain variable region comprises the amino acid sequence as set forth in SEQ ID NO: 591, or an amino acid sequence having at least 90%, 91%, 92%, 93%, 94%, 95%, 96%, 97%, 98% or 99% identity with the amino acid sequence of SEQ ID NO: 591, or consists of SEQ ID NO: 591;
91) the heavy chain variable region comprises the amino acid sequence as set forth in SEQ ID NO: 592, or an amino acid sequence having at least 90%, 91%, 92%, 93%, 94%, 95%, 96%, 97%, 98% or 99% identity with the amino acid sequence of SEQ ID NO: 592, or consists of SEQ ID NO: 592, and the light chain variable region comprises the amino acid sequence as set forth in SEQ ID NO: 593, or an amino acid sequence having at least 90%, 91%, 92%, 93%, 94%, 95%, 96%, 97%, 98% or 99% identity with the amino acid sequence of SEQ ID NO: 593, or consists of SEQ ID NO: 593;
92) the heavy chain variable region comprises the amino acid sequence as set forth in SEQ ID NO: 592, or an amino acid sequence having at least 90%, 91%, 92%, 93%, 94%, 95%, 96%, 97%, 98% or 99% identity with the amino acid sequence of SEQ ID NO: 592, or consists of SEQ ID NO: 592, and the light chain variable region comprises the amino acid sequence as set forth in SEQ ID NO: 594, or an amino acid sequence having at least 90%, 91%, 92%, 93%, 94%, 95%, 96%, 97%, 98% or 99% identity with the amino acid sequence of SEQ ID NO: 594, or consists of SEQ ID NO: 594;
93) the heavy chain variable region comprises the amino acid sequence as set forth in SEQ ID NO: 595, or an amino acid sequence having at least 90%, 91%, 92%, 93%, 94%, 95%, 96%, 97%, 98% or 99% identity with the amino acid sequence of SEQ ID NO: 595, or consists of SEQ ID NO: 595, and the light chain variable region comprises the amino acid sequence as set forth in SEQ ID NO: 596, or an amino acid sequence having at least 90%, 91%, 92%, 93%, 94%, 95%, 96%, 97%, 98% or 99% identity with the amino acid sequence of SEQ ID NO: 596, or consists of SEQ ID NO: 596;
94) the heavy chain variable region comprises the amino acid sequence as set forth in SEQ ID NO: 595, or an amino acid sequence having at least 90%, 91%, 92%, 93%, 94%, 95%, 96%, 97%, 98% or 99% identity with the amino acid sequence of SEQ ID NO: 595, or consists of SEQ ID NO: 595, and the light chain variable region comprises the amino acid sequence as set forth in SEQ ID NO: 594, or an amino acid sequence having at least 90%, 91%, 92%, 93%, 94%, 95%, 96%, 97%, 98% or 99% identity with the amino acid sequence of SEQ ID NO: 594, or consists of SEQ ID NO: 594;
95) the heavy chain variable region comprises the amino acid sequence as set forth in SEQ ID NO: 597, or an amino acid sequence having at least 90%, 91%, 92%, 93%, 94%, 95%, 96%, 97%, 98% or 99% identity with the amino acid sequence of SEQ ID NO: 597, or consists of SEQ ID NO: 597, and the light chain variable region comprises the amino acid sequence as set forth in SEQ ID NO: 598, or an amino acid sequence having at least 90%, 91%, 92%, 93%, 94%, 95%, 96%, 97%, 98% or 99% identity with the amino acid sequence of SEQ ID NO: 598, or consists of SEQ ID NO: 598;
96) the heavy chain variable region comprises the amino acid sequence as set forth in SEQ ID NO: 597, or an amino acid sequence having at least 90%, 91%, 92%, 93%, 94%, 95%, 96%, 97%, 98% or 99% identity with the amino acid sequence of SEQ ID NO: 597, or consists of SEQ ID NO: 597, and the light chain variable region comprises the amino acid sequence as set forth in SEQ ID NO: 594, or an amino acid sequence having at least 90%, 91%, 92%, 93%, 94%, 95%, 96%, 97%, 98% or 99% identity with the amino acid sequence of SEQ ID NO: 594, or consists of SEQ ID NO: 594;
97) the heavy chain variable region comprises the amino acid sequence as set forth in SEQ ID NO: 599, or an amino acid sequence having at least 90%, 91%, 92%, 93%, 94%, 95%, 96%, 97%, 98% or 99% identity with the amino acid sequence of SEQ ID NO: 599, or consists of SEQ ID NO: 599, and the light chain variable region comprises the amino acid sequence as set forth in SEQ ID NO: 600, or an amino acid sequence having at least 90%, 91%, 92%, 93%, 94%, 95%, 96%, 97%, 98% or 99% identity with the amino acid sequence of SEQ ID NO: 600, or consists of SEQ ID NO: 600;
98) the heavy chain variable region comprises the amino acid sequence as set forth in SEQ ID NO: 601, or an amino acid sequence having at least 90%, 91%, 92%, 93%, 94%, 95%, 96%, 97%, 98% or 99% identity with the amino acid sequence of SEQ ID NO: 601, or consists of SEQ ID NO: 601, and the light chain variable region comprises the amino acid sequence as set forth in SEQ ID NO: 602, or an amino acid sequence having at least 90%, 91%, 92%, 93%, 94%, 95%, 96%, 97%, 98% or 99% identity with the amino acid sequence of SEQ ID NO: 602, or consists of SEQ ID NO: 602;
99) the heavy chain variable region comprises the amino acid sequence as set forth in SEQ ID NO: 603, or an amino acid sequence having at least 90%, 91%, 92%, 93%, 94%, 95%, 96%, 97%, 98% or 99% identity with the amino acid sequence of SEQ ID NO: 603, or consists of SEQ ID NO: 603, and the light chain variable region comprises the amino acid sequence as set forth in SEQ ID NO: 604, or an amino acid sequence having at least 90%, 91%, 92%, 93%, 94%, 95%, 96%, 97%, 98% or 99% identity with the amino acid sequence of SEQ ID NO: 604, or consists of SEQ ID NO: 604;
100) the heavy chain variable region comprises the amino acid sequence as set forth in SEQ ID NO: 605, or an amino acid sequence having at least 90%, 91%, 92%, 93%, 94%, 95%, 96%, 97%, 98% or 99% identity with the amino acid sequence of SEQ ID NO: 605, or consists of SEQ ID NO: 605, and the light chain variable region comprises the amino acid sequence as set forth in SEQ ID NO: 606, or an amino acid sequence having at least 90%, 91%, 92%, 93%, 94%, 95%, 96%, 97%, 98% or 99% identity with the amino acid sequence of SEQ ID NO: 606, or consists of SEQ ID NO: 606;
101) the heavy chain variable region comprises the amino acid sequence as set forth in SEQ ID NO: 607, or an amino acid sequence having at least 90%, 91%, 92%, 93%, 94%, 95%, 96%, 97%, 98% or 99% identity with the amino acid sequence of SEQ ID NO: 607, or consists of SEQ ID NO: 607, and the light chain variable region comprises the amino acid sequence as set forth in SEQ ID NO: 608, or an amino acid sequence having at least 90%, 91%, 92%, 93%, 94%, 95%, 96%, 97%, 98% or 99% identity with the amino acid sequence of SEQ ID NO: 608, or consists of SEQ ID NO: 608;
102) the heavy chain variable region comprises the amino acid sequence as set forth in SEQ ID NO: 609, or an amino acid sequence having at least 90%, 91%, 92%, 93%, 94%, 95%, 96%, 97%, 98% or 99% identity with the amino acid sequence of SEQ ID NO: 609, or consists of SEQ ID NO: 609, and the light chain variable region comprises the amino acid sequence as set forth in SEQ ID NO: 610, or an amino acid sequence having at least 90%, 91%, 92%, 93%, 94%, 95%, 96%, 97%, 98% or 99% identity with the amino acid sequence of SEQ ID NO: 610, or consists of SEQ ID NO: 610;
103) the heavy chain variable region comprises the amino acid sequence as set forth in SEQ ID NO: 611, or an amino acid sequence having at least 90%, 91%, 92%, 93%, 94%, 95%, 96%, 97%, 98% or 99% identity with the amino acid sequence of SEQ ID NO: 611, or consists of SEQ ID NO: 611, and the light chain variable region comprises the amino acid sequence as set forth in SEQ ID NO: 612, or an amino acid sequence having at least 90%, 91%, 92%, 93%, 94%, 95%, 96%, 97%, 98% or 99% identity with the amino acid sequence of SEQ ID NO: 612, or consists of SEQ ID NO: 612;
104) the heavy chain variable region comprises the amino acid sequence as set forth in SEQ ID NO: 613, or an amino acid sequence having at least 90%, 91%, 92%, 93%, 94%, 95%, 96%, 97%, 98% or 99% identity with the amino acid sequence of SEQ ID NO: 613, or consists of SEQ ID NO: 613, and the light chain variable region comprises the amino acid sequence as set forth in SEQ ID NO: 614, or an amino acid sequence having at least 90%, 91%, 92%, 93%, 94%, 95%, 96%, 97%, 98% or 99% identity with the amino acid sequence of SEQ ID NO: 614, or consists of SEQ ID NO: 614;
105) the heavy chain variable region comprises the amino acid sequence as set forth in SEQ ID NO: 615, or an amino acid sequence having at least 90%, 91%, 92%, 93%, 94%, 95%, 96%, 97%, 98% or 99% identity with the amino acid sequence of SEQ ID NO: 615, or consists of SEQ ID NO: 615, and the light chain variable region comprises the amino acid sequence as set forth in SEQ ID NO: 616, or an amino acid sequence having at least 90%, 91%, 92%, 93%, 94%, 95%, 96%, 97%, 98% or 99% identity with the amino acid sequence of SEQ ID NO: 616, or consists of SEQ ID NO: 616;
106) the heavy chain variable region comprises the amino acid sequence as set forth in SEQ ID NO: 617, or an amino acid sequence having at least 90%, 91%, 92%, 93%, 94%, 95%, 96%, 97%, 98% or 99% identity to the amino acid sequence of SEQ ID NO: 617, or consists of SEQ ID NO: 617, and the light chain variable region comprises the amino acid sequence as set forth in SEQ ID NO: 618, or an amino acid sequence having at least 90%, 91%, 92%, 93%, 94%, 95%, 96%, 97%, 98% or 99% identity to the amino acid sequence of SEQ ID NO: 618, or consists of SEQ ID NO: 618; or
107) The heavy chain variable region comprises the amino acid sequence as set forth in SEQ ID NO: 619, or an amino acid sequence having at least 90%, 91%, 92%, 93%, 94%, 95%, 96%, 97%, 98% or 99% identity to the amino acid sequence of SEQ ID NO: 619, or consists of SEQ ID NO: 619, and the light chain variable region comprises the amino acid sequence as set forth in SEQ ID NO: 620, or an amino acid sequence having at least 90%, 91%, 92%, 93%, 94%, 95%, 96%, 97%, 98% or 99% identity to the amino acid sequence of SEQ ID NO: 620, or consists of SEQ ID NO: 620.
108) the heavy chain variable region comprises the amino acid sequence as set forth in SEQ ID NO: 621, or an amino acid sequence having at least 90%, 91%, 92%, 93%, 94%, 95%, 96%, 97%, 98% or 99% identity with the amino acid sequence of SEQ ID NO: 621, or consists of SEQ ID NO: 621, and the light chain variable region comprises the amino acid sequence as set forth in SEQ ID NO: 622, or an amino acid sequence having at least 90%, 91%, 92%, 93%, 94%, 95%, 96%, 97%, 98% or 99% identity with the amino acid sequence of SEQ ID NO: 622, or consists of SEQ ID NO: 622;
109) the heavy chain variable region comprises the amino acid sequence as set forth in SEQ ID NO: 623, or an amino acid sequence having at least 90%, 91%, 92%, 93%, 94%, 95%, 96%, 97%, 98% or 99% identity with the amino acid sequence of SEQ ID NO: 623, or consists of SEQ ID NO: 623, and the light chain variable region comprises the amino acid sequence as set forth in SEQ ID NO: 624, or an amino acid sequence having at least 90%, 91%, 92%, 93%, 94%, 95%, 96%, 97%, 98% or 99% identity with the amino acid sequence of SEQ ID NO: 624, or consists of SEQ ID NO: 624;
110) the heavy chain variable region comprises the amino acid sequence as set forth in SEQ ID NO: 625, or an amino acid sequence having at least 90%, 91%, 92%, 93%, 94%, 95%, 96%, 97%, 98% or 99% identity with the amino acid sequence of SEQ ID NO: 625, or consists of SEQ ID NO: 625, and the light chain variable region comprises the amino acid sequence as set forth in SEQ ID NO: 626, or an amino acid sequence having at least 90%, 91%, 92%, 93%, 94%, 95%, 96%, 97%, 98% or 99% identity with the amino acid sequence of SEQ ID NO: 626, or consists of SEQ ID NO: 626;
111) the heavy chain variable region comprises the amino acid sequence as set forth in SEQ ID NO: 627, or an amino acid sequence having at least 90%, 91%, 92%, 93%, 94%, 95%, 96%, 97%, 98% or 99% identity with the amino acid sequence of SEQ ID NO: 627, or consists of SEQ ID NO: 627, and the light chain variable region comprises the amino acid sequence as set forth in SEQ ID NO: 628, or an amino acid sequence having at least 90%, 91%, 92%, 93%, 94%, 95%, 96%, 97%, 98% or 99% identity with the amino acid sequence of SEQ ID NO: 628, or consists of SEQ ID NO: 628;
112) the heavy chain variable region comprises the amino acid sequence as set forth in SEQ ID NO: 629, or an amino acid sequence having at least 90%, 91%, 92%, 93%, 94%, 95%, 96%, 97%, 98% or 99% identity with the amino acid sequence of SEQ ID NO: 629, or consists of SEQ ID NO: 629, and the light chain variable region comprises the amino acid sequence as set forth in SEQ ID NO: 630, or an amino acid sequence having at least 90%, 91%, 92%, 93%, 94%, 95%, 96%, 97%, 98% or 99% identity with the amino acid sequence of SEQ ID NO: 630, or consists of SEQ ID NO: 630;
113) the heavy chain variable region comprises the amino acid sequence as set forth in SEQ ID NO: 631, or an amino acid sequence having at least 90%, 91%, 92%, 93%, 94%, 95%, 96%, 97%, 98% or 99% identity with the amino acid sequence of SEQ ID NO: 631, or consists of SEQ ID NO: 631, and the light chain variable region comprises the amino acid sequence as set forth in SEQ ID NO: 632, or an amino acid sequence having at least 90%, 91%, 92%, 93%, 94%, 95%, 96%, 97%, 98% or 99% identity with the amino acid sequence of SEQ ID NO: 632, or consists of SEQ ID NO: 632;
114) the heavy chain variable region comprises the amino acid sequence as set forth in SEQ ID NO: 617, or an amino acid sequence having at least 90%, 91%, 92%, 93%, 94%, 95%, 96%, 97%, 98% or 99% identity with the amino acid sequence of SEQ ID NO: 617, or consists of SEQ ID NO: 617, and the light chain variable region comprises the amino acid sequence as set forth in SEQ ID NO: 633, or an amino acid sequence having at least 90%, 91%, 92%, 93%, 94%, 95%, 96%, 97%, 98% or 99% identity with the amino acid sequence of SEQ ID NO: 633, or consists of SEQ ID NO: 633;
115) the heavy chain variable region comprises the amino acid sequence as set forth in SEQ ID NO: 634, or an amino acid sequence having at least 90%, 91%, 92%, 93%, 94%, 95%, 96%, 97%, 98% or 99% identity with the amino acid sequence of SEQ ID NO: 634, or consists of SEQ ID NO: 634, and the light chain variable region comprises the amino acid sequence as set forth in SEQ ID NO: 635, or an amino acid sequence having at least 90%, 91%, 92%, 93%, 94%, 95%, 96%, 97%, 98% or 99% identity with the amino acid sequence of SEQ ID NO: 635, or consists of SEQ ID NO: 635;
116) the heavy chain variable region comprises the amino acid sequence as set forth in SEQ ID NO: 636, or an amino acid sequence having at least 90%, 91%, 92%, 93%, 94%, 95%, 96%, 97%, 98% or 99% identity with the amino acid sequence of SEQ ID NO: 636, or consists of SEQ ID NO: 636, and the light chain variable region comprises the amino acid sequence as set forth in SEQ ID NO: 637, or an amino acid sequence having at least 90%, 91%, 92%, 93%, 94%, 95%, 96%, 97%, 98% or 99% identity with the amino acid sequence of SEQ ID NO: 637, or consists of SEQ ID NO: 637;
117) the heavy chain variable region comprises the amino acid sequence as set forth in SEQ ID NO: 638, or an amino acid sequence having at least 90%, 91%, 92%, 93%, 94%, 95%, 96%, 97%, 98% or 99% identity with the amino acid sequence of SEQ ID NO: 638, or consists of SEQ ID NO: 638, and the light chain variable region comprises the amino acid sequence as set forth in SEQ ID NO: 639, or an amino acid sequence having at least 90%, 91%, 92%, 93%, 94%, 95%, 96%, 97%, 98% or 99% identity with the amino acid sequence of SEQ ID NO: 639, or consists of SEQ ID NO: 639;
118) the heavy chain variable region comprises the amino acid sequence as set forth in SEQ ID NO: 640, or an amino acid sequence having at least 90%, 91%, 92%, 93%, 94%, 95%, 96%, 97%, 98% or 99% identity with the amino acid sequence of SEQ ID NO: 640, or consists of SEQ ID NO: 640, and the light chain variable region comprises the amino acid sequence as set forth in SEQ ID NO: 641, or an amino acid sequence having at least 90%, 91%, 92%, 93%, 94%, 95%, 96%, 97%, 98% or 99% identity with the amino acid sequence of SEQ ID NO: 641, or consists of SEQ ID NO: 641;
119) the heavy chain variable region comprises the amino acid sequence as set forth in SEQ ID NO: 642, or an amino acid sequence having at least 90%, 91%, 92%, 93%, 94%, 95%, 96%, 97%, 98% or 99% identity with the amino acid sequence of SEQ ID NO: 642, or consists of SEQ ID NO: 642, and the light chain variable region comprises the amino acid sequence as set forth in SEQ ID NO: 643, or an amino acid sequence having at least 90%, 91%, 92%, 93%, 94%, 95%, 96%, 97%, 98% or 99% identity with the amino acid sequence of SEQ ID NO: 643, or consists of SEQ ID NO: 643;
120) the heavy chain variable region comprises the amino acid sequence as set forth in SEQ ID NO: 644, or an amino acid sequence having at least 90%, 91%, 92%, 93%, 94%, 95%, 96%, 97%, 98% or 99% identity with the amino acid sequence of SEQ ID NO: 644, or consists of SEQ ID NO: 644, and the light chain variable region comprises the amino acid sequence as set forth in SEQ ID NO: 645, or an amino acid sequence having at least 90%, 91%, 92%, 93%, 94%, 95%, 96%, 97%, 98% or 99% identity with the amino acid sequence of SEQ ID NO: 645, or consists of SEQ ID NO: 645;
121) the heavy chain variable region comprises the amino acid sequence as set forth in SEQ ID NO: 646, or an amino acid sequence having at least 90%, 91%, 92%, 93%, 94%, 95%, 96%, 97%, 98% or 99% identity with the amino acid sequence of SEQ ID NO: 646, or consists of SEQ ID NO: 646, and the light chain variable region comprises the amino acid sequence as set forth in SEQ ID NO: 647, or an amino acid sequence having at least 90%, 91%, 92%, 93%, 94%, 95%, 96%, 97%, 98% or 99% identity with the amino acid sequence of SEQ ID NO: 647, or consists of SEQ ID NO: 647;
122) the heavy chain variable region comprises the amino acid sequence as set forth in SEQ ID NO: 648, or an amino acid sequence having at least 90%, 91%, 92%, 93%, 94%, 95%, 96%, 97%, 98% or 99% identity with the amino acid sequence of SEQ ID NO: 648, or consists of SEQ ID NO: 648, and the light chain variable region comprises the amino acid sequence as set forth in SEQ ID NO: 649, or an amino acid sequence having at least 90%, 91%, 92%, 93%, 94%, 95%, 96%, 97%, 98% or 99% identity with the amino acid sequence of SEQ ID NO: 649, or consists of SEQ ID NO: 649;
123) the heavy chain variable region comprises the amino acid sequence as set forth in SEQ ID NO: 650, or an amino acid sequence having at least 90%, 91%, 92%, 93%, 94%, 95%, 96%, 97%, 98% or 99% identity with the amino acid sequence of SEQ ID NO: 650, or consists of SEQ ID NO: 650, and the light chain variable region comprises the amino acid sequence as set forth in SEQ ID NO: 651, or an amino acid sequence having at least 90%, 91%, 92%, 93%, 94%, 95%, 96%, 97%, 98% or 99% identity with the amino acid sequence of SEQ ID NO: 651, or consists of SEQ ID NO: 651;
124) the heavy chain variable region comprises the amino acid sequence as set forth in SEQ ID NO: 652, or an amino acid sequence having at least 90%, 91%, 92%, 93%, 94%, 95%, 96%, 97%, 98% or 99% identity with the amino acid sequence of SEQ ID NO: 652, or consists of SEQ ID NO: 652, and the light chain variable region comprises the amino acid sequence as set forth in SEQ ID NO: 653, or an amino acid sequence having at least 90%, 91%, 92%, 93%, 94%, 95%, 96%, 97%, 98% or 99% identity with the amino acid sequence of SEQ ID NO: 653, or consists of SEQ ID NO: 653;
125) the heavy chain variable region comprises the amino acid sequence as set forth in SEQ ID NO: 654, or an amino acid sequence having at least 90%, 91%, 92%, 93%, 94%, 95%, 96%, 97%, 98% or 99% identity with the amino acid sequence of SEQ ID NO: 654, or consists of SEQ ID NO: 654, and the light chain variable region comprises the amino acid sequence as set forth in SEQ ID NO: 655, or an amino acid sequence having at least 90%, 91%, 92%, 93%, 94%, 95%, 96%, 97%, 98% or 99% identity with the amino acid sequence of SEQ ID NO: 655, or consists of SEQ ID NO: 655;
126) The heavy chain variable region comprises the amino acid sequence as set forth in SEQ ID NO: 656, or an amino acid sequence having at least 90%, 91%, 92%, 93%, 94%, 95%, 96%, 97%, 98% or 99% identity to the amino acid sequence of SEQ ID NO: 656, or consists of SEQ ID NO: 656, and the light chain variable region comprises the amino acid sequence as set forth in SEQ ID NO: 653, or an amino acid sequence having at least 90%, 91%, 92%, 93%, 94%, 95%, 96%, 97%, 98% or 99% identity to the amino acid sequence of SEQ ID NO: 653, or consists of SEQ ID NO: 653.

In some embodiments, the antibody or antigen-binding fragment thereof further comprises a heavy chain and/or light chain constant region sequence from a consensus sequence of human antibody germ lines. The light chain constant region is preferably a human source κ or lambda chain constant region. The heavy chain constant region may be γ, µ, α, δ, or epsilon chain, and in some embodiments, the heavy chain constant region is preferably a constant region sequence of human IgG1, IgG2, IgG3, IgG4. In one embodiment, the light chain constant region comprises, or consists of, the sequence as set forth in SEQ ID NO: 660. In another embodiment, the heavy chain constant region comprises the Fc sequence as set forth in SEQ ID NO: 661. In yet another embodiment, the heavy chain constant region comprises or consists of the sequence as set forth in SEQ ID NO: 659.

It should be understood that sequence variants of these constant region domains may also be used, for example comprising one or more amino acid modifications, wherein the amino acid sites are the (1991) EU index system identity of Kabat et al.

In certain embodiments of the antibody of any of the preceding, the antibody is a monoclonal antibody.

In certain embodiments of the antibody of any of the preceding, the antibody is a full-length antibody.

In one embodiment, the anti-CD112R antibody of the invention is an intact antibody, such as IgG1, IgG2, IgG3, IgG4 antibody. In another embodiment, the anti-CD 112R antibody of the invention encompasses only an antigen binding portion thereof, such as Fab, Fab'-SH, Fv, scFv, or (Fab')₂ fragment).

In the second aspect, the invention provides an isolated polynucleotide molecule encoding any antibody or antigen-binding fragment thereof according to the first aspect.

In the third aspect, the invention provides an expression vector comprising the nucleotide molecule according to the second aspect. In one embodiment, the vector is a eukaryotic expression vector.

In the fourth aspect, the invention provides a host cell comprising the vector according to the third aspect or the nucleotide molecule according to the second aspect. In some embodiments, the host cell is prokaryotic, such as E. coli. In other embodiments, the host cell is eukaryotic, such as 293 cells, CHO cells, yeast cells, or plant cells.

In the fifth aspect, the invention provides a pharmaceutical composition comprising (1) the antibody or an antigen-binding fragment thereof according to the first aspect, or the polynucleotide molecule according to the second aspect, or the expression vector according to the third aspect, or the host cell according to the fourth aspect, and (2) a pharmaceutically acceptable carrier or excipient.

In the sixth aspect, the invention provides a method for preparing the anti-CD112R antibody or antigen-binding fragment thereof described herein, comprising expressing the antibody or antigen-binding fragment thereof in a host cell described herein under conditions suitable for expression of the antibody or antigen-binding fragment thereof, and recovering the expressed antibody or antigen-binding fragment thereof from the host cell.

In the seventh aspect, the antibody or antigen-binding fragment thereof or the pharmaceutical composition according to the invention can also be administered in combination with one or more other therapies, e.g., therapeutic modalities and/or other therapeutics, for the uses as described herein, e.g., for the prevention and/or treatment of the associated diseases or conditions mentioned herein.

In the eighth aspect, the invention provides a pharmaceutical combination comprising the antibody or antigen-binding fragment thereof as described herein or the pharmaceutical composition, and one or more additional therapeutics.

In the ninth aspect, the invention provides a method for preventing or treating an CD112R-mediated disease in a subject in need thereof, comprising administering to the subject a prophylactically effective amount or a therapeutically effective amount of the antibody or antigen-binding fragment thereof according to the invention, the polynucleotide molecule as described herein, the expression vector as described herein, the host cell as described herein, the pharmaceutical composition as described herein, or the pharmaceutical combination as described herein, preferably the disease or condition is an CD112R-associated cancer, more preferably the cancer is selected from melanoma, renal cancer, prostate cancer, breast cancer, colon cancer, lung cancer, bone cancer, pancreatic cancer, skin cancer, head and neck cancer, uterine cancer, ovarian cancer and rectal cancer.

In the tenth aspect, the invention provides use of the antibody or antigen-binding fragment thereof described herein, the polynucleotide molecule described herein, the expression vector described herein, the host cell described herein, the pharmaceutical composition described herein, or the pharmaceutical combination described herein in the preparation of a medicament for the treatment and/or prevention of an CD112R-mediated disease or condition, preferably the disease or condition is an CD112R-associated cancer, more preferably the cancer is selected from melanoma, renal cancer, prostate cancer, breast cancer, colon cancer, lung cancer, bone cancer, pancreatic cancer, skin cancer, head and neck cancer, uterine cancer, ovarian cancer, and rectal cancer.

In the eleventh aspect, the invention provides the antibody or antigen-binding fragment thereof as described herein, the polynucleotide molecule as described herein, the expression vector as described herein, the host cell as described herein, the pharmaceutical composition as described herein, or the pharmaceutical combination described herein for the treatment and/or prevention of an CD112R-mediated disease or condition, preferably the disease or condition is cancer, more preferably the cancer is selected from melanoma, renal cancer, prostate cancer, breast cancer, colon cancer, lung cancer, bone cancer, pancreatic cancer, skin cancer, head and neck cancer, uterine cancer, ovarian cancer, and rectal cancer.

In the twelfth aspect, the invention further provides a kit comprising the anti-CD112R antibody or the antigen-binding fragment thereof according to the invention, the polynucleotide molecule according to the invention, the expression vector according to the invention, the host cell according to the invention, the pharmaceutical composition according to the invention, or the pharmaceutical combination according to the invention, or a pharmaceutical composition, and instructions for use. The kit may also comprise a suitable container. In certain embodiments, the kit further comprises a device for administration. Kits generally include a label indicating the intended use and/or method of use of the contents of the kit. The term "label" includes any written or recorded material provided on or with the kit or otherwise provided with the kit.

In another aspect, the invention provides a method for detecting the presence of CD112R in a sample using the antibody or antigen-binding fragment thereof described herein or a detection composition containing the antibody or antigen-binding fragment thereof.

### Detailed description of the invention

Before describing the invention in detail, it should be understood that the invention is not limited to the specific methods and experimental conditions in this specification, as the methods and conditions may be varied. In addition, the terminology used herein is for the purpose of describing particular embodiments only and is not intended to be limiting.

### I. Definitions

Unless defined otherwise, all technical and scientific terms used herein have the same meanings as commonly understood by one of ordinary skill in the art. For the purposes of the invention, the following terms are defined below.

The term "about" when used in conjunction with a numerical value is intended to encompass a numerical value within a range having a lower limit than the specified number value 10% and an upper limit value greater than the specified number value 10%.

The term "and/or" when used to connect two or more alternatives should be understood to mean any two or more of the alternatives.

As used herein, the terms "comprise" or "comprising" are intended to include the recited element, integer or step, but do not exclude any other elements, integers or steps. Herein, when the term "comprise" or "comprising" is used, the case consisting of the recited elements, integers or steps is also covered, unless otherwise specified. For example, when reference is made to an antibody variable region "comprising" a specific sequence, it is also intended to cover an antibody variable region consisting of the specific sequence.

The terms "CD112R", "PVR Related Immunoglobulin Domain", "CD112 receptor", "Poliovirus Receptor Related Immunoglobulin Domain", "Nectin2 receptor", "C7orfl5", "transmembrane protein PVRIG" are all used interchangeably and include variants, subtypes, species homologs of human CD112R or CD112R of other species and analogs having at least one common epitope of CD112R, unless otherwise specified. The term includes untreated full length CD112R and any form of CD112R due to treatment in cells. The term encompasses both "full-length" unprocessed CD112R and any form of CD112R or any fragment thereof produced by intracellular processing, such as splice variants or allelic variants. The variant or homolog maintains the activity of CD112R activity, e.g., at least 50%, 80%, 90%, 95%, 96%, 97%, 98%, 99%, or 100%. The variant or homolog has at least 90%, 95%, 96%, 97%, 98%, 99%, or 100% amino acid sequence identity over full sequence or partial sequence (e.g., 50, 100, 150, or 200 contiguous amino acid portions) as compared to a naturally occurring CD112R protein.

The term "antibody" is used herein in its broadest sense and encompasses numerous antibody structures, including but not limited to monoclonal antibodies, polyclonal antibodies, multispecific antibodies (e.g., bispecific antibodies), and antibody fragments, as long as they exhibit the desired antigen binding activity. Intact antibody typically comprises at least two full-length heavy chains and two full-length light chains, but in some cases may comprise fewer chains, for example a naturally occurring antibody in camelids may comprise only heavy chains.

The term "antibody fragment" refers to a molecule that is different from an intact antibody and comprises a portion of the intact antibody and is capable of binding to an antigen to which the intact antibody binds. Examples of antibody fragments include but are not limited to Fv, Fab, Fab', Fab'-SH, F(ab')₂; diabodies; linear antibodies; single-chain antibodies (e.g. scFv); single-domain antibodies; bivalent or bispecific antibodies or fragments thereof; camelid antibodies (heavy chain antibodies); and multispecific antibodies formed from antibody fragments.

The term "variable region" or "variable domain" refers to a domain of an antibody heavy or light chain involved in binding of an antibody to an antigen. The variable domains of the heavy and light chains of a natural antibody typically have similar structures, with each domain comprising four conserved framework regions (FRs) and three complementarity determining regions (CDRs) (see, e.g., Kindt et al. Kuby Immunology, 6th ed., W. H. Freeman and Co., page 91 (2007)). A single VH or VL domain is sufficient to confer antigen-binding specificity.

A "complementarity determining region" or "CDR region" or "CDR" is a region of the antibody variable domain that is highly variable in sequence and forms structurally determined loops ( "hypervariable loops") and/or contains antigen-contacting residues ("antigen-contacting sites"). The CDRs are primarily responsible for binding to epitopes in antigen. The CDRs in the variable domain are commonly referred to as CDR1, CDR2 and CDR3, numbered sequentially from the N-terminus. In a given variable region amino acid sequence, the exact amino acid sequence boundaries of each CDR can be determined using any one or a combination of many well-known antibody CDR designation systems, including, for example: Chothia (Chothia et al. (1989) Nature 342:877-883, Al-L azikani et al., "Standard formulations for the canonical structures of immunoglobulins", Journal of Molecular Biology, 273, 927-948 (1997)) based on three-dimensional structure of antibody and topology of CDR loops, Kabat (Kabat et al., Sequences of Proteins of Immunological Interest, 4th ed., U.S. Department of Health and Human Services, National Institutes of Health (1987)), AbM (University of Bath), Contact (University College London), International ImMunoGeneTics database (IMGT) based on antibody sequence variability, and North CDR definitions based on affinity propagation clustering with a large number of crystal structures.

Unless otherwise specified, in the invention, the term "CDR" or "CDR sequence" encompasses a CDR sequence determined in any of the methods described above.

The CDR may also be determined based on having the same AbM numbering locations as the reference CDR sequence (e.g., any of the CDRs of the present examples). In one embodiment, the positions of CDRs of the antibody of the invention are determined according to AbM numbering scheme.

Unless otherwise specified, in the invention, when reference is made to the position of residues in an antibody variable region and a CDR, including heavy chain variable region residues, it refers to the numbering position according to the AbM numbering system.

By "humanized antibody" is meant a chimeric antibody comprising amino acid residues from non-human CDRs and amino acid residues from human FRs. In some embodiments, all or substantially all of the CDRs (e.g., CDRs) in a humanized antibody correspond to those of a non-human antibody, and all or substantially all of the FRs correspond to those of a human antibody. The humanized antibody optionally may comprise at least a portion of an antibody constant region derived from a human antibody. "Humanized form" of an antibody (e.g., a non-human antibody) refers to an antibody that has been humanized.

As used herein, the term "bind" or "specifically binds" means that the binding effect is selective to the antigen and may be distinguished from unwanted or nonspecific interactions. The ability of the antigen-binding site to bind to a specific antigen can be determined by methods such as enzyme-linked immunosorbent assay (ELISA).

The term "half effective concentration (EC₅₀)" refers to the concentration of a drug, antibody or toxin that induces a response at 50% between baseline and maximum after a specified exposure time.

The term "therapeutic" as used herein encompasses any substance that is effective in prevention or treatment of a tumor, such as cancer, including chemotherapeutic agents, cytokines, angiogenesis inhibitors, cytotoxic agents, other antibodies, small molecule drugs, or immunomodulators (e.g., immunosuppressive agents).

The term "treatment" refers to slowing, interrupting, retarding, alleviating, stopping, reducing, or reversing the progression or severity of a pre-existing symptom, disorder, condition, or disease. Desirable therapeutic effects include, but not limited to, preventing the occurrence or recurrence of a disease, reducing symptoms, reducing any direct or indirect pathological consequences of a disease, preventing metastasis, reducing the rate of disease progression, ameliorating or alleviating a disease state, and alleviating or improving prognosis. In some embodiments, the antibody of the invention is used to delay disease development or to slow the progression of a disease.

The term "prevention" includes inhibition of the occurrence or development of a disease or disorder or a symptom of a particular disease or disorder. In some embodiments, a subject having a history of cancer families is a candidate for a prophylactic regimen. Generally, in the context of cancer, the term "prevention" refers to the administration of a drug prior to the onset of signs or symptoms of cancer, particularly in subjects at a risk of having a cancer.

The term "effective amount" refers to the amount or dose of an antibody or conjugate or composition of the invention that produces a desired effect in a patient in need of treatment or prevention following administration to the patient in single or multiple doses. Effective amounts can be readily determined by attending physicians as those skilled in the art by considering a variety of factors such as species of mammals; weight, age and general health; specific diseases involved; degree or severity of disease; response of individual patients; specific antibody administered; mode of administration; bioavailability characteristics of administered formulations; selected dosing regimen; and use of any concomitant therapy.

The term "therapeutically effective amount" refers to an amount effective to achieve a desired therapeutic result at a desired dose and for a desired period of time. The therapeutically effective amount of an antibody or antibody fragment or conjugate or composition thereof can vary depending on a variety of factors, such as disease state, age, sex, and weight of the subject, and the ability of the antibody or antibody portion to elicit a desired response in the subject. A therapeutically effective amount is also an amount in which any toxic or detrimental effects of an antibody or antibody fragment or conjugate or composition thereof are less than the therapeutically beneficial effects. "Therapeutically effective amount" preferably inhibits measurable parameters (e.g., tumor growth rate, tumor volume, etc.) by at least about 20%, more preferably at least about 40%, even more preferably at least about 50%, 60%, or 70% and still more preferably at least about 80% or 90% relative to untreated subjects. The ability of a compound to inhibit measurable parameters (e.g., cancer) can be evaluated in an animal model system that predicts efficacy in human tumors.

The term "prophylactically effective amount" refers to an amount effective to achieve a desired prophylactic result at a desired dose and for a desired period of time. Typically, the prophylactically effective amount will be less than the therapeutically effective amount, since the prophylactic dose is used in a subject prior to or at an earlier stage of the disease.

The term "pharmaceutical composition" refers to a composition that allows an active ingredient contained therein to be present in a biological active form and does not contain an additional ingredient that has unacceptable toxicity to a subject in which the composition is administered.

### II. Compositions of the Invention

In some embodiments, the invention provides a composition comprising any of the anti-CD112R antibodies described herein and antigen-binding fragments thereof, preferably the composition is a pharmaceutical composition. In one embodiment, the composition further comprises a pharmaceutic adjuvant, such as a pharmaceutically acceptable carrier, a pharmaceutically acceptable excipient, including a buffer, as is known in the art. In one embodiment, the composition (e.g., a pharmaceutical composition) comprises the anti-CD112R antibody and antigen-binding fragment thereof of the invention, as well as a combination of one or more other therapeutics.

As used herein, "pharmaceutically acceptable carrier" includes any and all solvents, dispersion media, isotonic agents and absorption delaying agents and the like that are physiologically compatible.

For the use and use of pharmaceutical adjuvant, see also "Handbook of Pharmaceutical Excipients," Eighth Edition, R. C. Rowe, P. J. Seskey and S. C. Owen, Pharmaceutical Press, London, Chicago.

The compositions of the invention can be in a variety of forms. These forms include, for example, liquid, semi-solid, and solid dosage forms such as liquid solutions (e.g., injectable solutions and infusion solutions), powders or suspensions, liposome formulations, and suppositories. Preferred forms depend on the intended mode of administration and therapeutic use.

The route of administration of the composition of the invention is via known methods, for example, orally, intravenous injection, intraperitoneal, intracerebral (parenchyma), intracerebroventricular, intramuscular, intraocular, intraarterial, portal or intralesional routes; by sustained release systems or by implanted devices. In certain embodiments, the composition may be administered by bolus injection or by continuous infusion or by an implant device.

The subject may be a mammal, e.g., a primate, preferably a higher primate, e.g., a human (e.g., a subject having a disease described herein or having a risk of having a disease described herein). In one embodiment, a subject has or has a risk of having a disease described herein (e.g., cancer). In certain embodiments, the subject receives or has received other treatments, such as chemotherapy treatment and/or radiotherapy. In some embodiments, the subject has previously received or is receiving immunotherapy.

Drug comprising the antibody described herein can be prepared by mixing the anti-CD112R antibody and antigen-binding fragment thereof of the invention having a desired purity with one or more optional pharmaceutical adjuvants, preferably in the form of lyophilized preparation or aqueous solution.

The pharmaceutical composition or formulation of the invention may further comprise more than one active ingredients required for the particular indication being treated, preferably those having complementary activities that do not adversely affect each other. For example, it may be desirable to also provide other therapeutics, including chemotherapeutic agents, angiogenesis inhibitors, cytokines, cytotoxic agents, other antibodies, small molecule drugs or immunomodulators (e.g., immune checkpoint inhibitors or agonists), and the like. The active ingredient is suitably combined in an amount effective for the intended use.

### III. Preparation of Antibodies of the Invention

In one embodiment, the invention provides a method of preparing an anti-CD112R antibody, comprising culturing a host cell comprising a nucleic acid encoding the anti-CD112R antibody or an expression vector comprising the nucleic acid under conditions suitable for expression of the nucleic acid encoding the anti-CD112R antibody, and optionally isolating the anti-CD112R antibody. In an embodiment, the method further comprises recovering the anti-CD112R antibody from the host cell (or host cell culture medium).

To recombinantly produce the anti-CD112R antibody of the invention, the nucleic acid encoding the anti-CD112R antibody of the invention is firstly isolated and is inserted into a vector for further cloning and/or expression in a host cell. Such nucleic acids are readily isolated and sequenced using conventional protocols, such as by using oligonucleotide probes capable of specifically binding to nucleic acids encoding the anti-CD112R antibody of the invention.

The anti-CD112R antibody of the invention prepared as described herein may be purified by known prior art techniques such as high performance liquid chromatography, ion exchange chromatography, gel electrophoresis, affinity chromatography, size exclusion chromatography, and the like. The actual conditions used to purify a particular protein also depend on factors such as net charge, hydrophobicity, hydrophilicity, etc., and are apparent to those skilled in the art.

### Examples

The invention is further illustrated by the following examples, however, it is to be understood that the examples are described by way of illustration instead of limitation, and that various modifications may be made by those skilled in the art. The present disclosure will be further described in detail below with reference to specific examples, but these examples are not intended to limit the scope of the invention. The experimental methods for which specific conditions are not indicated in the examples of the present disclosure are generally carried out under conventional conditions, or according to the conditions recommended by the raw materials or commercial manufacturers; and the unspecified reagents are generally conventional reagents commercially available.

Unless expressly indicated to the contrary, examples of the invention will employ methods of conventional chemistry, biochemistry, organic chemistry, molecular biology, microbiology, recombinant DNA technology, genetics, immunology, and cell biology within the skill of the art.

### Example 1: Preparation and Identification of Raw Materials

### 1.1 CD112R Control Antibody Preparation and Identification

CD112R control antibody preparation: the present application used anti-CD112R antibody CHA.7.518 (hereinafter referred to as Tab-1) as a positive control antibody, and gene synthesis by GenScript Biotech Corporation was according to the sequence disclosed in WO/2016/134333, and an expression plasmid was constructed, where the specific VH and VL sequences of Tab-1 were shown in SEQ ID NOs: 657 and 658. Expi293F cells were transiently transfected and cultured for 6 days, the supernatant was collected, and antibody affinity purification was performed using Protein A Magnetic Beads (GenScript Biotech Corporation). Protein A Magnetic Beads were added to the supernatant and incubated at room temperature for 2 hours. After incubation, Protein A Magnetic Beads were equilibrated with Binding Buffer (PBS, pH 7.4) for 10 column volumes. The Protein A Magnetic Beads were added to an elution buffer (0.1M citric acid buffer, pH 3.0) to elute, the eluate was collected, a Neutralizing buffer (0.1M Tris pH 9.0) of 1/10 volume of the eluate were added to adjust pH to be neutral. After elution, Protein A Magnetic Beads were equilibrated with Binding buffer for 10 column volumes. The antibody was eluted and neutralized, placed in a dialysis bag for dialyzing against Dialysis buffer (PBS, pH 7.4) for 2 hours at room temperature, and dialysis buffer was replaced at 2-8°C for 16 hours. The antibody was transferred from the dialysis bag to a centrifuge tube and the volume was recorded.

CD112R control antibody identification: the activity of the prepared positive control antibody Tab-1 was detected by using the purchased CD112R-His antigen protein (Acro Biosysterm, PVG-H52H4), and the specific method was as follows: CD112R-His (0.5 µg/mL, 100 µL/well) was coated on a 96-well ELISA plate at 37°C for 1 hour; after washing once, the plate was blocked with 1% BSA prepared in PBS for 1 hour; after washing for 3 times, the control antibody Tab-1 diluted gradiently with PBS was added and incubated at room temperature for 1 hour; after washing the plate, a secondary antibody (Peroxidase-AffiniPure Goat Anti-Mouse IgG, Fcy Fragment Specific) diluted with PBS (1:6000) was added and incubated at 37°C for 0.5 hours, the plate was washed for 3 times, and the TMB was added for development for 5-20 min; after stopping development, the data was read at OD450 with a reader. The results showed that the expressed control antibody Tab-1 can bind the CD112R protein, and as the antibody concentration decreased, the OD450 reads decreased, indicating that the control antibody had normal anti-CD112R activity.

### Example 2: Construction and Identification of Cell Lines Expressing Human and Monkey CD112R

Construction of CHOK1 cell lines overexpressing human and monkey CD112R (hereinafter referred to as huCD112R-CHO K1; cynoCD112R-CHO K1): the nucleic acid sequences encoding full-length human (Uniprot ID: Q6DKI7) and monkey CD112R (Uniprot ID: A0A2K5WVV8) were constructed onto lentiviral vectors. Then, the lentiviral plasmid was transfected into HEK293T cells for virus package, cultured for 4 days, and the cell culture supernatant was collected. CHO-K1 cells were transfected with the harvested lentivirus supernatant, and Puromycin was used as screening pressure. After screening for 3 days, the antigen expression in cell pool was screened by flow cytometry. After obtaining the positive cell pool, the positive cell pool was selected by using limited dilution method or flow sorting. After monoclone selection, the culture was continued for about 1 week, and the antigen expression in cell strains was identified.

Flow identification of antigen expression in cell strain: the cell strains in logarithmic growth phase were digested and plated into a 96-well plate, washed with FACS buffer (1 × PBS buffer containing 2% of FBS v/v), and incubated with a PBS gradient diluted primary Mouse anti-human PVRIG antibody (GenScript Probio, Cat. No.: C523UGG270-2D) for 30 min; after washing, the prepared fluorescent secondary PE Goat anti-mouse IgG antibody (BioLegend, Cat. No. 405307) was added, and incubated at 4°C for 30 min; and finally detected by flow cytometry (Beckman, CytoFLEXAOO-1-1102). The results showed that huCD112R-CHO K1 and cynoCD112R-CHO K1 cell lines with high surface expression human and monkey CD112R were obtained.

### Example 3: Animal Immunization and Immune Library Construction

### 3.1 Immune Protocol

Different species of mice (Balb/c, SJL, A/J) were immunized with various immunogens, CD112R-His or CD112R-mFc protein antigen (Acro Biosysterm, PVG-H5259); CD112R expression plasmid-DNA antigen; CD112R overexpressing CHO cells, alone or alternatively. Mice were immunized by subcutaneous/intraperitoneal injection or gene-gun depending on the type of antigen selected, once every two weeks, for a total of 3-5 immunizations. Mouse blood was taken one week after the last immunization for immune titer detection, and finally, the mice was boosted once again with the above antigen.

**Table 1. Summary of immunization in this example**

| | Immunization Summary |
|---|---|
| 1 | CD112R-His protein antigen (Acro Biosysterm, PVG-H52H4) |
| 2 | CD112R-mFc protein antigen (Acro Biosysterm, PVG-H5259) |
| 3 | DNA antigen (SEQ ID NO: 662)+cell antigen (human protein overexpressing cell line constructed in Example 2)+CD112R-His protein antigen (Acro Biosysterm, PVG-H52H4) |
| 4 | CD112R-His protein antigen (Acro Biosysterm, PVG-H52H4)+cell antigen (human protein overexpressing cell line constructed in Example 2)+CD112R-His protein antigen (Acro Biosysterm, PVG-H52H4) |

### 3.2 Detection of antibody titer in mice serum after immunization

The antigen was diluted to 0.5 µg/mL with coating solution (PBS, pH 7.4), mixed well and added into an ELISA plate, 100 µL/well, and placed in a refrigerator at 4°C overnight for 16 h. The coating solution was discarded, the plate was washed once, and the plate was dried. 150 µL of blocking solution (3% BSA solution) was added to each well and placed in a 37°C incubator for 1 h. The plate was taken out and the blocking solution was discarded. After gradient dilution of mice serum, 100 µL/well was added to the wells of the plate. Incubation for 1 h at 37°C incubator. The plate was taken out, the liquid in the plate was discarded, and the plate was washed 3 times. 100 µL of the diluted enzyme-labeled secondary antibody Peroxidase-AffiniPure Goat Anti-Mouse IgG, Fcy Fragment Specific (min X Hu, Bov, Hrs Sr Prot) was added to each well and incubated at 37°C for 0.5 hours. The ELISA plate was taken out, the liquid in the ELISA plate was discarded, the plate was washed for 3 times, and 100 µl of color development solution was added after the plate was dried, and reacted at 25°C for 15 min. 50 µL of 1M HCl was added to terminate the reaction, and the reading at OD450 was performed with a microplate reader. Mice with a titer greater than 512000 (dilution fold) were selected for hybridoma fusion.

### 3.3 Hybridoma Cell Fusion

A suspension of myeloma cells (SP2/0) was prepared and the total number of SP2/0 cells was calculated with a hemocytometer. The spleen cell suspension was prepared, and the total number of cells was counted using the blood cell counting plate. Myeloma cells and splenocytes were mixed at a ratio of 1:2 to perform cell electrofusion. After cell fusion, HAT selective medium was used to mix the cells after fusion into a cell suspension, and then the cells were plated into 96-well cell culture plate by using a multichannel pipette. The fused 96-well cell culture plate was placed in a 5.5% CO₂ incubator, and cultured at a constant temperature of 37°C. After the hybridoma cell culture, the supernatant was taken for mother clone screening, and the cell culture supernatant was screened for antigen binding ability at protein and cell levels to obtain a mother clone cell line capable of binding to an antigen.

### 3.4 Hybridoma Subclone Screening

The mother clone cell line obtained by screening in step 3.3 was picked out, and the cells to be subcloned were pipetted gently and mixed well. 20 µl of cell suspension was taken and added to 20 µl of trypan blue solution for uniform mixing. 20 µl of the solution was taken for counting by using blood cell count plate. Using a limited dilution method, cells were plated in a 96-well cell culture plate with 1 × HT replacement feeder cell culture medium, marked on the 96-well plate, and placed in CO₂ incubator for culture. The subcloned 96 well plate was incubated in a 37°C, 5.5% CO₂ incubator. After 7 days of culture, the supernatant was taken for monoclonal screening.

Through subclone screening, the obtained antibodies were selected for sequencing, and the CDR sequences of the antibodies were shown in Table 2 and Table 3 below. CDR sequences are determined by using AbM to define CDRs.

**Table 2: Heavy Chain CDR Sequences of the Anti-CD112R Antibodies**

| Antibody No. | SEQ ID No: | HCDR1 | SEQ ID No: | HCDR2 | SEQ ID No: | HCDR3 |
|---|---|---|---|---|---|---|
| C1 | 1 | DYYLN | 64 | WIFPGSESTFYNEKFKG | 162 | DGGLRGPFDY |
| C3 | 2 | DYYIN | 65 | WIFPGSDYTYYNEKFKG | 162 | DGGLRGPFDY |
| C4 | 3 | DYFMN | 66 | DIIPNNDSPNYNQKFKG | 163 | GYFDV |
| C6 | 4 | AYYIN | 67 | WIFPGSDSTYYNEKFKG | 164 | DGELRGPFDY |
| C7, C7-2 | 5 | DYYMN | 68 | DINPNNGGTNFNQKFKG | 165 | LFFDY |
| C8 | 6 | DYYMF | 69 | TISDGGGYIYYPDSVKG | 166 | EDF |
| C9 | 7 | DIYMH | 70 | RIDPTNGNSEYDPRFRG | 167 | FGIYPYFDY |
| C10 | 8 | DSYMC | 71 | TISDGGGLTYYPDSVKG | 168 | EDL |
| C11 | 3 | DYFMN | 72 | DIHPNNGSPNYNQKFKG | 163 | GYFDV |
| C12 | 9 | DYYMY | 73 | TISDGGGYTYYPDSVKG | 168 | EDL |
| C13 | 3 | DYFMN | 74 | DINPNNGSPNYNQKFKG | 163 | GYFDV |
| C14 | 10 | SYWMH | 75 | VIDASDSNTKYNQKFKG | 169 | YYSGSSYGLDY |
| C16 | 10 | SYWMH | 75 | VIDASDSNTKYNQKFKG | 170 | YFSGSSYGLDY |
| C18 | 9 | DYYMY | 76 | TISDGGGFIYYPDSVKG | 168 | EDL |
| C19 | 9 | DYYMY | 77 | TISDAGGYTYYPDSVKG | 168 | EDL |
| C20 | 8 | DSYMC | 77 | TISDAGGYTYYPDSVKG | 168 | EDL |
| C21 | 7 | DIYMH | 78 | RVDPTNGNSEYDPRFQG | 167 | FGIYPYFDY |
| C22 | 11 | DYNIH | 79 | YIYPYNGGAGYNQKFKS | 171 | EELRRDYGMDY |
| C23 | 2 | DYYIN | 80 | WIFPGSGNTYYNEKFKG | 162 | DGGLRGPFDY |
| C24 | 12 | GYGIN | 81 | VIWSGGNTEYNAAFRS | 172 | DSTGLYAMDY |
| C25 | 10 | SYWMH | 82 | VIDASDSKTKYNQKFKG | 169 | YYSGSSYGLDY |
| C28 | 2 | DYYIN | 83 | WIYPGSGYTKYNEKLKG | 173 | DSFYAMDY |
| C31 | 13 | DYYIA | 84 | WIYLGSGHTMYNENFKG | 173 | DSFYAMDY |
| C33 | 2 | DYYIN | 85 | WIFPGSGSTYYNEKFKG | 174 | EDYFDRGDC |
| C36 | 14 | TYGMS | 86 | WINTYSGVPTYADDFKG | 175 | ELPFTTIIGTKDY |
| C38 | 15 | GYWIE | 87 | EILPESGRTNYNEKFKG | 176 | LLFFDY |
| C39 | 16 | SYNIH | 88 | AIYPGNADISYNQKFRD | 177 | PLRIYAMDC |
| C41 | 2 | DYYIN | 89 | DIEPNNGGTNYNQKFKG | 178 | LYFDY |
| C43 | 17 | TYWMH | 75 | VIDASDSNTKYNQKFKG | 169 | YYSGSSYGLDY |
| C44 | 18 | DSYIN | 85 | WIFPGSGSTYYNEKFKG | 162 | DGGLRGPFDY |
| C45 | 2 | DYYIN | 90 | WIFPGSGTTYYNEKFKG | 162 | DGGLRGPFDY |
| C47-2 | 19 | SYWMQ | 91 | EIDPFDSYTNYNQKFKG | 179 | RVYYGYDRYFFDY |
| C48, C48-2 | 2 | DYYIN | 85 | WIFPGSGSTYYNEKFKG | 180 | DGSYDPPFAY |
| C51 | 20 | DYEMH | 92 | AIDPETGGTAYNQKFKG | 181 | WITTVVATLYYFDY |
| C52 | 20 | DYEMH | 92 | AIDPETGGTAYNQKFKG | 181 | WITTVVATLYYFDY |
| C55 | 21 | DYYMS | 93 | DVNPNNGDTSYNQKFKG | 182 | SGLPYVLMDY |
| C56 | 22 | GYALS | 94 | TIRDGGSYTYYSDNEKG | 183 | DQVNRGWFTY |
| C56-2 | 23 | DYALS | 94 | TIRDGGSYTYYSDNEKG | 183 | DQVNRGWFTY |
| C57 | 24 | DYYMH | 95 | IINPYNGDTSYNQKFKD | 184 | DSSNVGNAMDY |
| C58 | 21 | DYYMS | 93 | DVNPNNGDTSYNQKFKG | 182 | SGLPYVLMDY |
| C59 | 22 | GYALS | 94 | TIRDGGSYTYYSDNEKG | 183 | DQVNRGWFTY |
| C61 | 25 | SNYYWN | 96 | YISSDGTNNYNPSLRN | 185 | GLRYGP |
| C62 | 26 | SYSMS | 97 | TIGGVGGNTYYPDSVKG | 186 | HEDYYYYAMDY |
| C63 | 27 | SYIMS | 98 | TIRGGGGNTYYLDSVKG | 187 | HEDYEWYAMDN |
| C64 | 28 | SYFMS | 99 | TISGGGDKTYYLDILKG | 188 | HEDYAWYAMDY |
| C66 | 22 | GYALS | 94 | TIRDGGSYTYYSDNEKG | 183 | DQVNRGWFTY |
| C67 | 5 | DYYMN | 100 | DINPDNGGSTYNQKFKG | 189 | SGPPYVLLDY |
| C69 | 24 | DYYMH | 101 | IINPYNGGTSYNQKFKG | 190 | DSSNVGNDMDY |
| C71 | 28 | SYFMS | 102 | TISGGGDNTYYPDSVKG | 191 | HEDYEWYAMDY |
| C72 | 28 | SYFMS | 103 | TIRGGGSYTYYLDSVKG | 192 | HEDYDYYAMDY |
| C75 | 29 | SYWIT | 104 | DIYPGNFSPNYNEKFKN | 163 | GYFDV |
| C76 | 30 | HYYKN | 105 | AINPNIGGPSYNQKFKG | 193 | SDDYGGYYFDY |
| C77 | 5 | DYYMN | 106 | DIYPNNGDTNYNQKFKG | 194 | GVGLGFPY |
| C78 | 31 | HYYVN | 107 | VIKPYNGAPSYNQKFKG | 195 | SDDFDGYAMDY |
| C79-2 | 31 | HYYVN | 107 | VIKPYNGAPSYNQKFKG | 195 | SDDFDGYAMDY |
| C79-3, C79-4 | 32 | NYGMS | 108 | TISRGGSFTYYPDSVKG | 196 | PIYYEYDGFAY |
| C80 | 29 | SYWIT | 109 | DIYPGNDSPNYNEKFKN | 197 | GYYDY |
| C81 | 33 | GYYVN | 110 | LIDPSNGGTNYNQKFKD | 198 | GTVY |
| C82 | 5 | DYYMN | 111 | TFNPNRGGPSYNLKFKG | 199 | ADDYDGYYFDY |
| C84, C84-2 | 34 | DYFIS | 112 | WIFPGSGSTYFNENFKG | 200 | YYGSNY |
| C85 | 35 | DYSMN | 113 | DINPNNGGTSYNQKFKG | 201 | VWFAY |
| C87 | 36 | ASAMH | 114 | SFTMYSDATEYSENFKG | 202 | GYSGNGFTY |
| C88 | 29 | SYWIT | 115 | DIHPGNTSPNYNEKFRT | 203 | GWGDY |
| C89 | 37 | DYGMY | 116 | YIRSDSSTIYYTDTVKG | 204 | RSPVVVNYAMDY |
| C90 | 5 | DYYMN | 117 | EINPKNGDTNYNQNFKG | 205 | EGVSSYYAMDY |
| C91 | 38 | GYYIN | 110 | LIDPSNGGTNYNQKFKD | 198 | GTVY |
| C92 | 39 | DYGMH | 118 | YIRSDSSTIYYADTVKG | 204 | RSPVVVNYAMDY |
| C93 | 5 | DYYMN | 119 | EINPKNGDTNYSQKFRD | 206 | EGVGSYYAMDY |
| C94 | 29 | SYWIT | 109 | DIYPGNDSPNYNEKFKN | 197 | GYYDY |
| C95 | 38 | GYYIN | 110 | LIDPSNGGTNYNQKFKD | 198 | GTVY |
| C96 | 5 | DYYMN | 120 | EINPNNGDTNYSQKFRD | 205 | EGVSSYYAMDY |
| C97 | 5 | DYYMN | 121 | EINPKNGDTNYNQNFKA | 207 | EGVSSYYGMDS |
| C98 | 35 | DYSMN | 122 | DINPNNGGTNYNQKFKG | 201 | VWFAY |
| C99 | 35 | DYSMN | 123 | DINPDNGGTSYNQKFKG | 208 | IWFAY |
| C100 | 5 | DYYMN | 124 | EINPNNGDTNFNQKFKG | 209 | EGGSSYYAMDY |
| C101 | 40 | DYNVD | 125 | AINPNDGGTSYNQKFEG | 210 | WVNNDWYFDV |
| C102 | 41 | NYGIS | 126 | EIYPGTINIYYSERFKG | 211 | DDGYYDAMDY |
| C103 | 10 | SYWMH | 127 | EIDPSDSYTNYNQKFKG | 212 | SIYYSNYVRYYFDY |
| C103-2 | 40 | DYNVD | 125 | AINPNDGGTSYNQKFEG | 213 | WVINDWYFDV |
| C104 | 42 | DYNMD | 128 | ANDPKNGGFSYNQRFKG | 214 | WDSNYWYFDV |
| C105 | 43 | DYSMD | 129 | AINPNNGGTSYNQKFKD | 215 | WDSDYWYFDV |
| C106 | 42 | DYNMD | 130 | AVNPNTGDISYTQKFKD | 216 | WSESWYFDV |
| C107 | 44 | DYNID | 131 | AIDPNNGGTNYNQKFKD | 217 | WDSNDWYFDV |
| C108, C108-2 | 44 | DYNID | 132 | AIDPNNDSTNYNQKFKG | 218 | WDSDDWYFDV |
| C110 | 45 | DTYIH | 133 | GIDPAIGNTVSASKFQD | 219 | ERVHSNTLYYAMD Y |
| C111, C111-2 | 46 | SHGIS | 134 | EIYPGSESPYYSEKFKG | 220 | SDYSDYWAMDY |
| C112, C112-2 | 47 | SSWMH | 135 | EINPSDGRTNYNEKFKS | 221 | NADRAYYTMDY |
| C113, C113-2 | 48 | DYNLD | 136 | AMSPNNGATSYNQKFKG | 222 | WDSNSWYFDV |
| C114 | 49 | NSWIN | 137 | DIYPGNTFPNYNEKFKG | 163 | GYFDV |
| C115 | 45 | DTYIH | 138 | GIDPAIGNTLYASKFQD | 223 | ERVYSNTLYYAMDY |
| C116 | 50 | SYTMS | 139 | KISNSGGSTNYPDTVKG | 224 | YSWDVYAMDY |
| C117 | 51 | DYWMQ | 140 | AIFPGNGETRDTQKFKG | 225 | FNNYLDY |
| C118 | 47 | SSWMH | 141 | EINPSDGRTNYNEKFKN | 221 | NADRAYYTMDY |
| C119 | 52 | TYIMS | 142 | DISNSGGNTYYSDTVKG | 226 | PDNWDPFVY |
| C120 | 53 | DYFIN | 143 | DINPDNGAGNYNQKFKG | 178 | LYFDY |
| C121 | 53 | DYFIN | 144 | DINPDNGGTNYNQKFKG | 178 | LYFDY |
| C122 | 53 | DYFIN | 145 | DVNPDNGGTNYNQKFKG | 227 | LFFDS |
| C123 | 54 | NFAMS | 146 | TIVNGGINTYYPDTVKG | 228 | HDDFLYYAMDY |
| C124 | 55 | SFAMS | 147 | TINNGGINTYYPDTVKG | 228 | HDDFLYYAMDY |
| C125 | 50 | SYTMS | 148 | ELSNSGGSFHYPDNVKG | 229 | HNWDVYAMDY |
| C129 | 56 | DYWIE | 149 | EILPGYISTNYNEKFKD | 230 | IVTTYYYFMDY |
| C132 | 57 | DYWIN | 150 | EILPGSVTTNYNEKFRG | 231 | GSDYSYYAMDY |
| C136 | 58 | ENYMY | 151 | TISDGASYTYYPDNVKG | 232 | DELIAPFAY |
| C138 | 59 | SYAMS | 152 | TITSGDIRTYYPDTVKG | 233 | HDDFHFYAMDY |
| C140 | 60 | NYWMN | 153 | AINPSNGYIGYNQKFKD | 234 | GEDFDGYAMDY |
| C143 | 54 | NFAMS | 146 | TIVNGGINTYYPDTVKG | 228 | HDDFLYYAMDY |
| C145 | 61 | DFFIN | 154 | DVNPDSNGANYNQKFK G | 235 | LFFDN |
| C146 | 40 | DYNVD | 125 | AINPNDGGTSYNQKFEG | 210 | WVNNDWYFDV |
| C147 | 44 | DYNID | 155 | AINPNNGGTFYNQKFKG | 236 | WDSDSWYFDV |
| C148 | 44 | DYNID | 156 | AIDPNNGGTNYNQKFKG | 217 | WDSNDWYFDV |
| C149 | 62 | TFGLGIG | 157 | HIWWDDDKYYNPALKS | 237 | GTYWFTY |
| C150 | 24 | DYYMH | 158 | IINPYNGDTSYNQKFKG | 184 | DSSNVGNAMDY |
| C151 | 3 | DYFMN | 159 | DINPNNGDTTYSQKFMG | 189 | SGPPYVLLDY |
| C152 | 63 | DYWIQ | 160 | AIFPENGDTRYTQKFKG | 238 | FINYVDY |
| C153 | 42 | DYNMD | 161 | AIDPKNGATSYNQKFKG | 214 | WDSNYWYFDV |

**Table 3: Light Chain CDR Sequences of the Anti-CD112R Antibodies**

| Antibody No. | SEQ ID No: | LCDR1 | SEQ ID No: | LCDR2 | SEQ ID No: | LCDR3 |
|---|---|---|---|---|---|---|
| C1 | 239 | KASQDINKYIA | 307 | YTSTLHP | 357 | LQYDYLLT |
| C3 | 239 | KASQDINKYIA | 308 | YTSTLQP | 358 | LQYDNLHT |
| C4 | 240 | KSSQSLLNSGNQKNFLA | 309 | GTSTRES | 359 | QNDHSYPWT |
| C6 | 239 | KASQDINKYIA | 310 | YTSTLQA | 360 | LQYDNLLT |
| C7 | 241 | KSSQSLLNSGNQKNYLA | 311 | GASTRES | 361 | QNAHSSPYT |
| C7-2 | 242 | KASQNVGTAVA | 312 | SASNRYT | 362 | QQYSSYRT |
| C8 | 243 | RASQDIGRSLN | 313 | ATSRLDS | 363 | LQYASFPYT |
| C9 | 244 | KASQSVNKAVV | 314 | YASNRYT | 364 | QQDYSSPWT |
| C10 | 245 | RASQDIGSSLN | 313 | ATSRLDS | 365 | LQYASSPYT |
| C11 | 241 | KSSQSLLNSGNQKNYLA | 311 | GASTRES | 359 | QNDHSYPWT |
| C12 | 245 | RASQDIGSSLN | 313 | ATSRLDS | 363 | LQYASFPYT |
| C13 | 246 | KSSQSLLNSGTQKNYLA | 311 | GASTRES | 359 | QNDHSYPWT |
| C14 | 247 | RASQDISNYLN | 315 | YTSRLHS | 366 | QQGNMFPPT |
| C16 | 247 | RASQDISNYLN | 315 | YTSRLHS | 366 | QQGNMFPPT |
| C18 | 243 | RASQDIGRSLN | 313 | ATSRLDS | 363 | LQYASFPYT |
| C19 | 248 | RASQDIGYSLN | 313 | ATSRLDS | 363 | LQYASFPYT |
| C20 | 243 | RASQDIGRSLN | 313 | ATSRLDS | 365 | LQYASSPYT |
| C21 | 244 | KASQSVNKAVV | 314 | YASNRYT | 364 | QQDYSSPWT |
| C22 | 249 | RASENIYSYLA | 316 | NAKTLAE | 367 | QHHYGTPYT |
| C23 | 239 | KASQDINKYIA | 317 | FTSTLQS | 368 | LQYDYLYT |
| C24 | 250 | RTSQDISNFLN | 315 | YTSRLHS | 369 | QQGYMLPPT |
| C25 | 247 | RASQDISNYLN | 315 | YTSRLHS | 370 | QQGNTLPPT |
| C28 | 242 | KASQNVGTAVA | 312 | SASNRYT | 371 | LQYSSYLT |
| C31 | 242 | KASQNVGTAVA | 312 | SASNRYT | 372 | QQYSTYLT |
| C33 | 251 | SASSSINYMH | 318 | DTSKLAS | 373 | HQRSSYPFT |
| C36 | 252 | RASQFISNNLH | 319 | YASQSIS | 374 | QQSNSWPHT |
| C38 | 253 | KSSQSLLDSDGKTYLN | 320 | LVSKLDS | 375 | WQGTHFPLT |
| C39 | 254 | KASQNVGTNVA | 321 | SASYRYS | 376 | QQYNSRPLT |
| C41 | 241 | KSSQSLLNSGNQKNYLA | 311 | GASTRES | 377 | QNDHSYPFT |
| C43 | 247 | RASQDISNYLN | 315 | YTSRLHS | 366 | QQGNMFPPT |
| C44 | 239 | KASQDINKYIA | 322 | YTSTLQS | 378 | LQYDHLLT |
| C45 | 255 | KANQDINKYIA | 308 | YTSTLQP | 368 | LQYDYLYT |
| C47-2 | 239 | KASQDINKYIA | 322 | YTSTLQS | 378 | LQYDHLLT |
| C48 | 256 | SASSSVSYMH | 323 | STSNLAS | 379 | QQRSSYPPT |
| C48-2 | 239 | KASQDINKYIA | 308 | YTSTLQP | 380 | LQYDNLYT |
| C51 | 257 | KASQNVRTAVA | 324 | LASNRHT | 381 | LQHWNYPLT |
| C52 | 257 | KASQNVRTAVA | 324 | LASNRHT | 381 | LQHWNYPLT |
| C55 | 258 | RASENINSYLA | 325 | NTKTLTE | 382 | QHHYGTPLT |
| C56, C56-2 | 259 | SASSSVSSSYFH | 323 | STSNLAS | 383 | HQHHRSPLT |
| C57 | 247 | RASQDISNYLN | 326 | YSSRLHS | 384 | QQGYTLPRT |
| C58 | 247 | RASQDISNYLN | 326 | YSSRLHS | 385 | QQGNTLPRT |
| C59 | 259 | SASSSVSSSYFH | 323 | STSNLAS | 383 | HQHHRSPLT |
| C61 | 260 | TLSSQHSTYTIE | 327 | GSHRTGD | 386 | GVGDINNYI |
| C62 | 261 | SANSTVSYIY | 328 | DTSNLAS | 387 | QQWSSFPFT |
| C63 | 262 | SARSSVSYMY | 328 | DTSNLAS | 388 | QQWSSYPLT |
| C64 | 263 | SASSSVNYIY | 328 | DTSNLAS | 388 | QQWSSYPLT |
| C66 | 239 | KASQDINKYIA | 308 | YTSTLQP | 389 | LQYDNLYM |
| C67 | 264 | QTSENIYSYLA | 329 | NTQTLAE | 390 | QHHYGVPLT |
| C69 | 247 | RASQDISNYLN | 326 | YSSRLHS | 385 | QQGNTLPRT |
| C71 | 265 | SASSSVSYIY | 328 | DTSNLAS | 391 | QQWTSYPLT |
| C72 | 266 | SASSSVSYMY | 328 | DTSNLAS | 388 | QQWSSYPLT |
| C75 | 241 | KSSQSLLNSGNQKNYLA | 311 | GASTRES | 377 | QNDHSYPFT |
| C76 | 267 | RANSSVNYMH | 330 | ATSNLAS | 392 | QQWTSNPLT |
| C77 | 268 | QASQVVSTAVA | 331 | SASFRYS | 393 | QQHYRTPYT |
| C78 | 256 | SASSSVSYMH | 318 | DTSKLAS | 392 | QQWTSNPLT |
| C79-3 | 256 | SASSSVSYMH | 318 | DTSKLAS | 392 | QQWTSNPLT |
| C79-2, C79-4 | 269 | RSSQSIVHSNGNTYLE | 332 | KVSNRFS | 394 | FQGSHVPRT |
| C80 | 241 | KSSQSLLNSGNQKNYLA | 311 | GASTRES | 395 | QNDHSYPHT |
| C81 | 270 | RASQDIGNNLN | 333 | ATSSLDS | 396 | LQYASSPVT |
| C82 | 256 | SASSSVSYMH | 318 | DTSKLAS | 397 | QQWTNNPLT |
| C84 | 269 | RSSQSIVHSNGNTYLE | 334 | TVSNRLS | 398 | FQTSHVPPT |
| C84-2 | 271 | RASQSISSWLA | 335 | KASTLES | 399 | QQYNTYWT |
| C85 | 241 | KSSQSLLNSGNQKNYLA | 311 | GASTRES | 400 | QNAHSYPFT |
| C87 | 272 | RASESVDNYGISFMH | 336 | RASNLES | 401 | QQSNKDPMYT |
| C88 | 241 | KSSQSLLNSGNQKNYLA | 311 | GASTRES | 402 | QNDHGYPLT |
| C89 | 273 | RASENIYSHLV | 337 | NGKILAE | 403 | QHHYGSPIT |
| C90 | 274 | KSSQSLLNSFNQKNFLA | 338 | FASTRES | 404 | QQHHSTPPT |
| C91 | 270 | RASQDIGNNLN | 333 | ATSSLDS | 405 | LQYASSPPT |
| C92 | 275 | RASENIYSYLV | 337 | NGKILAE | 406 | QHHYGSPLT |
| C93 | 276 | KSSQSLLSSNNQKSFLA | 338 | FASTRES | 407 | QQHYSTPPT |
| C94 | 241 | KSSQSLLNSGNQKNYLA | 311 | GASTRES | 395 | QNDHSYPHT |
| C95 | 270 | RASQDIGNNLN | 333 | ATSSLDS | 405 | LQYASSPPT |
| C96 | 277 | KSSQSLLNSNNQKNFLA | 338 | FASTRES | 407 | QQHYSTPPT |
| C97 | 278 | KSSQSLLNGFNQKNFLA | 338 | FASTRES | 404 | QQHHSTPPT |
| C98 | 279 | KSSQSLLSSGNQKNFLA | 311 | GASTRES | 408 | QNAHSSPFT |
| C99 | 280 | KSSQSLLNSGIQKNFLA | 311 | GASTRES | 400 | QNAHSYPFT |
| C100 | 281 | KSSQSLLSNNNQKNFLA | 338 | FASTRES | 407 | QQHYSTPPT |
| C101 | 282 | KASQNVRSAVD | 339 | SASNRHT | 409 | LQHWNYPYT |
| C102 | 245 | RASQDIGSSLN | 333 | ATSSLDS | 410 | LQYTSSPWT |
| C103, C103-2 | 239 | KASQDINKYIA | 308 | YTSTLQP | 411 | LQYDNLWT |
| C104 | 283 | KASQDVRSAVA | 324 | LASNRHT | 412 | LQHWTYPYT |
| C105 | 284 | KASQNVRTAVD | 339 | SASNRHT | 409 | LQHWNYPYT |
| C106 | 285 | KASQNVRAAVD | 340 | LASSRHT | 409 | LQHWNYPYT |
| C107 | 286 | KASQNVRIAVD | 341 | SSSNRHT | 413 | LQHWISPYT |
| C108 | 282 | KASQNVRSAVD | 341 | SSSNRHT | 414 | LQHWNSPYT |
| C108-2 | 287 | KASEDIYNRLA | 342 | GTTSLEI | 415 | QQYWSTPWT |
| C110 | 288 | RASKSVSISGYSYMH | 343 | LASNLES | 416 | QHSRELPPT |
| C111 | 289 | KASQNVRSAVA | 324 | LASNRHT | 417 | LQHWNYPWT |
| C111-2 | 290 | RASQSVSSNLA | 344 | GASTRAT | 418 | HQYNNWPRT |
| C112 | 291 | KSSQSLLNSFNQRNFLA | 345 | FASTRDS | 407 | QQHYSTPPT |
| C112-2 | 290 | RASQSVSSNLA | 344 | GASTRAT | 418 | HQYNNWPRT |
| C113 | 292 | KASQIVRTAVD | 346 | SASTRHT | 409 | LQHWNYPYT |
| C113-2 | 290 | RASQSVSSNLA | 344 | GASTRAT | 418 | HQYNNWPRT |
| C114 | 240 | KSSQSLLNSGNQKNFLA | 347 | GASTRQS | 419 | QNDHSYPYT |
| C115 | 288 | RASKSVSISGYSYMH | 343 | LASNLES | 416 | QHSRELPPT |
| C116 | 249 | RASENIYSYLA | 348 | NAETLVE | 420 | QHHYGDPT |
| C117 | 256 | SASSSVSYMH | 349 | DTSKLTS | 421 | QQWISNPYT |
| C118 | 293 | KSSQSLLNGFNQRNFLA | 345 | FASTRDS | 407 | QQHYSTPPT |
| C119 | 294 | KASEDIYARLA | 350 | GATSLET | 422 | QQHWSTPWT |
| C120 | 295 | RSSQSLLNSGNQKNYLA | 311 | GASTRES | 423 | QNAHTSPYT |
| C121 | 241 | KSSQSLLNSGNQKNYLA | 311 | GASTRES | 361 | QNAHSSPYT |
| C122 | 241 | KSSQSLLNSGNQKNYLA | 311 | GASTRES | 361 | QNAHSSPYT |
| C123 | 296 | SASSSVSYMF | 328 | DTSNLAS | 391 | QQWTSYPLT |
| C124 | 266 | SASSSVSYMY | 328 | DTSNLAS | 391 | QQWTSYPLT |
| C125 | 297 | RASENVNSYLA | 351 | NAQTLAE | 424 | QHHYGNPT |
| C129 | 298 | SASSSVTYMH | 323 | STSNLAS | 425 | QQRSSYPWT |
| C132 | 299 | KSSQSLLNIFNQKNFLA | 338 | FASTRES | 407 | QQHYSTPPT |
| C136 | 300 | KASQDVGTAVA | 352 | WASTRHT | 426 | QQYNSYPWT |
| C138 | 301 | SASSSVSYLY | 328 | DTSNLAS | 388 | QQWSSYPLT |
| C140 | 239 | KASQDINKYIA | 308 | YTSTLQP | 360 | LQYDNLLT |
| C143 | 302 | SASSSVNYMF | 328 | DTSNLAS | 391 | QQWTSYPLT |
| C145 | 241 | KSSQSLLNSGNQKNYLA | 311 | GASTRES | 361 | QNAHSSPYT |
| C146 | 282 | KASQNVRSAVD | 353 | SASSRHT | 409 | LQHWNYPYT |
| C147 | 303 | KASENVRAAVD | 324 | LASNRHT | 409 | LQHWNYPYT |
| C148 | 282 | KASQNVRSAVD | 341 | SSSNRHT | 414 | LQHWNSPYT |
| C149 | 304 | RSSQSIEHSNGNTYLQ | 332 | KVSNRFS | 427 | FQGSHVPWT |
| C150 | 247 | RASQDISNYLN | 326 | YSSRLHS | 428 | QQGHALPRT |
| C151 | 249 | RASENIYSYLA | 354 | NAKTLTE | 382 | QHHYGTPLT |
| C152 | 305 | SASSSVNNIH | 355 | DTTKLTS | 429 | QQWNINPYT |
| C153 | 306 | KASQNIRSAVA | 356 | LTSNRHT | 409 | LQHWNYPYT |

### Example 4: Antibody Construction, Expression and Purification

### 4.1 Plasmid Construction

The coding sequences for VHs in the Fab sequence of the screened monoclonal were linked to the coding sequences for the heavy chain constant region (SEQ ID NO: 659) of the human IgG1 to obtain the heavy chain coding sequence of human murine chimeric antibodies, and the coding sequences for VL in the Fab sequence were linked to the coding sequence of the Kappa type (SEQ ID NO: 660) of the human light chain constant region (CL) to obtain the light chain coding sequence of human murine chimeric antibodies. The coding sequences of the heavy and light chains of the antibody were respectively inserted into eukaryotic expression vector plasmids, transformed into E. coli DH5α (Shanghai Sangon), and cultured overnight at 37°C. Plasmid extraction was performed by using an endotoxin-free plasmid extraction kit (Qiagen) to obtain an antibody plasmid without endotoxin for eukaryotic expression.

### 4.2 Expression and Purification of Antibodies

The full-length sequences of the antibodies obtained were expressed by the Expi293F transient expression system (Thermo Fisher, A14635) or the ExpiCHO transient expression system (Thermo Fisher, A29133), and the specific methods can be referred to the operation guidelines of the respective kits by the manufacturers. The isolation and purification of the antibodies were referenced to the purification of the antibodies in Example 1.

### Example 5: Testing Physical and Chemical Properties of the Antibodies

### 5.1 SDS-PAGE characterization of the antibodies

Preparation of a non-reducing solution: 3 µg of each obtained antibody were added into 2 × SDS loading buffer and 40 mM iodoacetamide, respectively, heated at 75°C for 10 min, cooled to room temperature, and centrifuged at 12000 rpm for 5 min to obtain the supernatant.

Preparation of a reducing solution: 3 µg of each obtained antibody were added into 2 × SDS loading buffer and 5 mM DTT, respectively, heated at 99°C for 5 min, cooled to room temperature, and centrifuged immediately. The supernatant was added to Bis-tris 4-15% gradient gel (GenScript Biotech Corporation) for gel electrophoresis and the protein bands were developed by Coomassie brilliant blue staining.

The protein gel (decolorized with decolorization liquid to gel background) with the developed protein bands was photographed using an Odyssey 9140 two-color infrared laser imaging system, and the purity of the reduced and non-reduced bands was calculated by its own software.

The test results showed that the bands of respective antibodies of the non-reductive gel were at about 150 kD, and the bands of the reductive gel were at about 55 kD and about 25 kD, with expected sizes.

### Example 6: Detection of Antigen Binding Activity of the Antibodies

In this example, the binding of the expressed antibodies to the human CD112R antigen protein huCD112R-His was detected based on ELISA method, and the binding of the expressed antibodies to the human CD112R overexpressing cell huCD112R-CHO K1 was also detected based on FACS method.

### 6.1 Detection of the binding capacity of the antibodies to antigen protein huCD112R-His based on ELISA

A 96-well ELISA plate (30 µL/well) was coated with 0.5 µg/mL human CD112R antigen protein huCD112R-His (Acro Biosysterm, PVG-H52H4) overnight at 4°C. On the next day, the plate was washed with PBST for 3 times and then blocked with 5% skim milk for 2 h; after washing with PBST for 3 times, respective antibodies and positive control antibody Tab-1 with gradient dilution (1 µg/ml, 3 times gradient dilution, 9 concentration points) were added and incubated for 1 h. Then, after washing 3 times with PBST, secondary antibody goat-anti-human Fc-HRP (Abcam, ab97225) was added and incubated for 30 min. After the incubation, the plate was washed 6 times with PBST, dried and developed with TMB for 15 min at 25°C, 1M HCl was added to terminate the reaction, and the absorbance was read by a microplate reader at OD450.

The results showed (Table 4) that both the antibodies obtained in the present application and the antigen protein huCD112R had better binding ability, and some antibodies had better binding ability with the antigen protein than the positive control antibody Tab-1.

**Table 4: EC50 values of the antibodies to the human antigen protein**

| Antibody | EC50(nM) | Antibody | EC50(nM) | Antibody | EC50(nM) |
|---|---|---|---|---|---|
| C1 | 0.075 | C38 | 0.064 | C113 | 0.038 |
| C3 | 0.046 | C39 | 0.691 | C114 | 0.042 |
| C4 | 0.032 | C41 | 0.033 | C115 | 0.029 |
| C6 | 0.055 | C42 | 0.056 | C116 | 0.025 |
| C7 | 0.034 | C43 | 0.051 | C117 | 0.041 |
| C8 | 0.046 | C44 | 0.053 | C118 | 0.029 |
| C9 | 0.052 | C45 | 0.059 | C119 | 0.025 |
| C11 | 0.056 | C51 | 0.228 | C120 | 0.035 |
| C12 | 0.155 | C55 | 0.056 | C121 | 0.037 |
| C13 | 0.065 | C57 | 0.034 | C122 | 0.030 |
| C14 | 0.052 | C61 | 0.089 | C123 | 0.035 |
| C18 | 0.069 | C62 | 0.032 | C124 | 0.033 |
| C19 | 0.043 | C63 | 0.054 | C125 | 0.049 |
| C20 | 2.268 | C64 | 0.034 | C129 | 0.034 |
| C21 | 0.066 | C67 | 0.060 | C132 | 0.038 |
| C22 | 0.029 | C56-2 | 0.032 | C136 | 0.039 |
| C23 | 0.060 | C69 | 0.023 | C138 | 0.039 |
| C24 | 0.048 | C59 | 0.044 | C143 | 0.025 |
| C25 | 0.052 | C71 | 0.037 | C145 | 0.038 |
| C28 | 0.049 | C101 | 0.043 | C146 | 0.036 |
| C31 | 0.038 | C105 | 0.021 | C152 | 0.082 |
| C33 | 1.296 | C110 | 0.032 | Tab-1 | 0.034 |
| C36 | 0.219 | C111 | 0.036 | C112 | 0.028 |

### 6.2 Detection of the binding capacity of the antibodies to huCD112R-CHO K1 Cells Based on FACS

In this example, the binding activity of human CD112R overexpressing cell huCD112R-CHO K1 to the antibody was evaluated.

The specific method was as follows: the logarithmic growth phase huCD112R-CHO K1 cells were prepared into a single cell suspension, the density was adjusted to 2×10⁶ cells/mL, 50 µL per well was added to the 96-well round bottom plate, centrifuged at 4°C and 300g, and the supernatant was removed. The detection antibody and the positive control antibody Tab-1 were added to respective wells, and mixed well and incubated at 4°C for 40 min. The incubated cell mixture was washed three times, 100 µL of diluted secondary Goat F(ab')₂ Anti-Human IgG-Fc (Abcam, ab98596) was added, incubated at 4°C in the dark for 30 min, washed three times and then detected by flow cytometry, and the fluorescence intensity value (MFI) was read.

Conclusion: on huCD112R-CHO K1 cells, the antibodies of the invention had binding capacity with CD112R overexpression cells.

### Example 7: Detection of Cross-Activity of Antibody Species and Cognates (Elisa method)

In this example, the cross-reactivity of individual antibodies obtained in the present application on human and monkey species was detected. The binding capacity assay was performed with the antibody prepared in Example 4 and the monkey antigen protein (Uniprot ID: A0A2K5WVV8) and the monkey CD112R expressing cell strain cynoCD112R-CHO K1. For specific operations, refer to Example 6.1.

The results (Table 5) showed that all antibodies obtained in the present application can bind to the monkey antigen protein and the cell strain overexpressing the monkey protein, showing good cross-activity of human monkeys, so the antibodies obtained in the present application had a wide application in experiments and tests based on a monkey model.

The antibodies of the invention all had a better human-monkey species cross-activity.

**Table 5: EC50 values of the antibodies to the monkey antigen protein**

| Antibody | EC50(nM) | Antibody | EC50(nM) | Antibody | EC50(nM) |
|---|---|---|---|---|---|
| C1 | 0.116 | C41 | 0.052 | C115 | 0.034 |
| C3 | 0.118 | C42 | 0.078 | C116 | 0.061 |
| C4 | 0.059 | C43 | 0.044 | C117 | 0.045 |
| C6 | 0.058 | C44 | 0.076 | C118 | 0.057 |
| C7 | 0.080 | C45 | 0.114 | C119 | 0.080 |
| C8 | 0.047 | C51 | 0.224 | C120 | 0.062 |
| C9 | 0.070 | C55 | 1.291 | C121 | 0.055 |
| C11 | 0.079 | C57 | 0.077 | C122 | 0.031 |
| C12 | 0.768 | C62 | 0.043 | C123 | 0.056 |
| C13 | 0.059 | C63 | 0.090 | C124 | 0.059 |
| C14 | 0.067 | C64 | 0.054 | C125 | 0.079 |
| C18 | 0.069 | C67 | 0.080 | C132 | 0.094 |
| C19 | 0.034 | C56-2 | 0.044 | C136 | 0.060 |
| C20 | 8.363 | C69 | 0.051 | C138 | 0.052 |
| C21 | 0.068 | C59 | 0.049 | C143 | 0.040 |
| C22 | 0.125 | C71 | 0.050 | C145 | 0.042 |
| C23 | 0.125 | C101 | 0.085 | C146 | 0.074 |
| C24 | 0.169 | C105 | 0.065 | C152 | 0.074 |
| C25 | 3.803 | C110 | 0.034 | | |
| C28 | 0.054 | C111 | 0.074 | | |
| C31 | 0.120 | C112 | 0.055 | | |
| C36 | 0.101 | C113 | 0.104 | | |
| C39 | 0.141 | C114 | 0.077 | | |

### Example 8: Antibody affinity kinetics (Biacore) analysis

In this example, the affinity between the obtained antibody or the positive control antibody Tab-1 and the antigen protein huCD112R-His was detected based on the Biacore device. The anti-CD112R antibody sample was diluted to 10 µg/mL using running buffer HBS-EP+(10 mM HEPES, 150 mM NaCl, 3 mM EDTA and 0.05% P20, pH 7.4) (BR100826, GE), respectively, and the human CD112R antigen protein huCD112R-His (Acro Biosysterm, PVG-H52H4) was diluted with HBS-EP+ buffer at 2× gradient to be between 0.03125 nM and 2 nM. The anti-CD112R antibody was captured with Protein A biosensor chip (BR100354, GE), with a capture time of 60s and a flow rate of 30 µL/min. Then, human CD112R samples diluted to different concentrations were loaded sequentially from zero to the highest concentration, with a binding time of 120s, a dissociation time of 360s, and a flow rate of 30 µL/min. Biacore X100 instrument was used to detect the reaction signal in real time to obtain binding and dissociation curves. After the dissociation of each experimental cycle was completed, the Protein A biosensor chip was cleaned and regenerated with 10 mM Glycine-HCl (pH 1.5) (29127558, GE), the regeneration time was set to 30 s, and the flow rate was 30 µL/min. Parameter fitting: the experiment was run in a single-cycle mode to analyze the time as the abscissa and the response value as the ordinate. After double reference deduction was performed on the obtained data, fitting was performed by using BIAcore X100 (GE) analysis software, and the fitting model used was a 1:1 Langmuir binding model, and affinity indicators such as binding dissociation constants were determined. The results showed that the affinity of the antibodies of the invention was superior or comparable to that of the positive control Tab-1.

**Table 6: KD values of binding of the antibodies to antigen protein huCD112R-His**

| Antibody | KD(M) | Antibody | KD(M) |
|---|---|---|---|
| Tab-1 | 3.46E-10 | C57 | 1.30E-10 |
| C1 | 9.05E-10 | C62 | 7.04E-10 |
| C3 | 1.81E-10 | C63 | 4.88E-10 |
| C4 | 1.41E-11 | C64 | 8.00E-10 |
| C7 | 5.76E-11 | C67 | 2.00E-11 |
| C8 | 3.97E-10 | C71 | 1.58E-10 |
| C11 | 6.22E-11 | C101 | 3.92E-11 |
| C12 | 2.90E-09 | C105 | 7.16E-10 |
| C13 | 2.18E-11 | C114 | 3.56E-11 |
| C14 | 6.87E-11 | C115 | 2.22E-10 |
| C18 | 2.06E-10 | C120 | 7.69E-11 |
| C19 | 5.67E-10 | C124 | 2.43E-10 |
| C20 | 1.14E-10 | C129 | 3.75E-10 |
| C24 | 4.67E-11 | C132 | 1.56E-10 |
| C41 | 2.20E-11 | C138 | 5.50E-10 |
| C55 | 5.06E-10 | C152 | 2.74E-10 |
| C57 | 2.19E-10 | C154 | 7.24E-10 |
| C62 | 5.71E-10 | C155 | 6.03E-10 |
| C55 | 3.34E-10 | C156 | 9.46E-10 |

### Example 9: Detection of Blocking the Signaling Pathway of Binding of CD112R to CD112 by the Antibodies (Reporter Gene method)

The reporter gene method consists of two genetically engineered cell lines, one is GS-J2C/CD112R effector cell line expressing human CD112R and luciferase reporter gene driven by NFAT-RE, the other is GS-C2/CD112 target cell line expressing human CD112 in an engineered cell surface protein, intending to activate homologous TCR in an antigen-independent manner. When the GS-J2C/CD112R effector cell line was co-cultured with the GS-C2/CD112 target cell line, the activation of the TCR signaling pathway was inhibited due to the CD112R/CD112 interaction, thereby inhibiting the expression of the luciferase driven by the NFAT, so the corresponding fluorescence signal was relatively low; the addition of the CD112R therapeutic antibody blocked the CD112R/CD112 interaction, thereby restoring the TCR signaling pathway and increasing the fluorescence signal value.

First, GS-2/CD112 target cells were constructed. Human CD112 full-length DNA fragment (NP_001036189.1) was inserted onto a lentiviral expression vector (Blasticidin resistance), mixed with a helper plasmid and transfected into 293T cells, packaged into CD112-Blastcidin recombinant lentivirus. CHO-K1/APC cells were infected with the above recombinant lentivirus (designated as GS-C2), and a cell pool of successfully infected with CD112 was obtained by screening with 10 µg/ml of Blasticidin. GS-C2/CD112 monoclonal cells were selected by a limited dilution method.

GS-J2C/CD112R effector cells construction. Human CD112R full-length DNA fragment (XP_011514877.1) was inserted onto a Puromycin resistant lentiviral expression vector, mixed with a helper plasmid and transfected into 293T cells, packaged into CD112-Puromycin recombinant lentivirus. Jurkat-Luc Reporter cell was infected with the above lentivirus (designated as GS-J2C), and a cell pool of successfully infected with CD112R was obtained by screening with 1 µg/ml of Puromycin. GS-J2C/PVRIG monoclonal cells were selected by a limited dilution method.

The target cells were collected by centrifugation and resuspended using respective complete media (10% FBS F-12K). The target cell density was adjusted, inoculated into the experimental plate, and the experimental plate was transferred to a cell incubator (37°C/5% CO₂) for incubation for about 16 hours). The effector cells were collected by centrifugation and then were resuspended using an assay buffer (1.25% FBS RPMI 1640) and cell density was adjusted. The plate incubated with the target cells was taken out from the cell incubator and the medium was removed. The control and the test sample working solution and the effector cell suspension were sequentially transferred to the experiment board. The experimental plate was transferred to a cell incubator (37°C/5% CO₂) for incubation for 6 hours). The incubated experiment plate was taken out of the cell incubator, and the luciferase detection reagent working solution was transferred to the corresponding wells of the experiment plate, and incubated at room temperature for 3-5 minutes. The chemiluminescent values were read using PHERA star, and data was recorded.

The results showed that the antibodies of the invention effectively blocked the binding of CD112R to CD112.

**Table 7: Blokcing effects of the antibodies**

| Antibody | EC50(µg/ml) | Antibody | EC50(µg/ml) |
|---|---|---|---|
| C1 | 0.045 | C57 | 0.061 |
| C3 | 0.040 | C62 | 0.032 |
| C4 | 0.024 | C63 | 0.023 |
| C7 | 0.031 | C64 | 0.039 |
| C8 | 0.026 | C67 | 0.030 |
| C11 | 0.021 | C71 | 0.029 |
| C12 | 0.024 | C124 | 0.015 |
| C13 | 0.019 | C129 | 0.045 |
| C14 | 0.052 | C132 | 0.024 |
| C18 | 0.034 | C138 | 0.029 |
| C19 | 0.033 | C152 | 0.082 |
| C20 | 0.025 | Tab-1 | 0.065 |
| C24 | 0.050 | C154 | 0.071 |
| C41 | 0.024 | C155 | 0.082 |
| C55 | 0.052 | C156 | 0.096 |

### Example 10: Blocking the Binding of CD112R to CD112 by the Antibodies Enhancing Antigen-Specific CD8 T cell activation (primary immune cell method)

Assaying the activation of the antibodies on antigen-specific CD8⁺ T cells. PBMC cells of specific genotype (HLA-A*0201) were collected by centrifugation, resuspended using RPMI 1640 culture medium (Gibco) supplemented with 10% FBS (Gibco) and added with a volume of pp65(495-503) polypeptide (CEF20, Cytomegalovirus, CMV pp65(495-503), GenScript) and cytokines (IL-2 Human and IL-7 Human, GenScript), to induce the generation of antigen-specific CD8⁺ T cells. After 11 days of culture, the PBMC cells were collected by centrifugation and CD8⁺ T cells were obtained by using human CD8⁺ T cell isolation kit (17953, STEMCELL). 5×10⁴ targets cells (Ksaumi-1 and Colo-205) were added into a 96-well bottom plate, and the pp65(495-503) polypeptide working solution was added into respective wells, and then placing in a cell culture incubator for 1 hour. 25×10⁴ CD8⁺ T cells were added into respective 96-well plate. Then, the antibodies to be assayed were added into corresponding wells according to detection concentrations. The 96-well plate was placed in a cell culture incubator (37 °C/5% CO₂) for 18-22 hours. After incubation, the 96-well plate was taken out and centrifuged at 2000 rpm for 5 min, the supernatant was transferred into a new 96-well plate. The cytokine interferon γ (IFN-γ) was assayed according to the instruction of the cytokine detection kit (62HIFNGPEH, Cisbio).

**Table 8**

| Antibody | | IFN-γ(pg/ml) | |
|---|---|---|---|
| Concentration | 0.5µg/ml | 5µg/ml | 50µg/ml |
| PMA + Iono | | | 768.1802 |
| Human IgG1 | 623.9928 | 669.4832 | 680.8562 |
| C12 | 607.8522 | 677.7104 | 823.5244 |
| C13 | 623.9640 | 658.3758 | 767.2709 |
| C14 | 619.4987 | 674.0936 | 759.3625 |
| C19 | 570.8819 | 744.4827 | 828.833 |
| C24 | 610.7736 | 656.0283 | 733.1607 |
| C41 | 573.3831 | 750.7923 | 659.2455 |
| C1 | 542.6812 | 640.8722 | 755.0672 |
| C3 | 534.5355 | 633.3608 | 755.4967 |
| Tab-1 | 695.9589 | 717.9702 | 763.2948 |
| C4 | 662.69 | 740.1729 | 874.9431 |
| C8 | 722.1538 | 783.3482 | 818.8240 |
| C11 | 675.5745 | 747.3697 | 617.3214 |
| Antibody | IFN-γ(pg/ml) | Antibody | IFN-γ(pg/ml) |

| Concentration | 0.5µg/ml | 5µg/ml | Highest concentration |
|---|---|---|---|
| | | | Recorded individually |
| C7 | 817.85 | 837.39 | 1108.80 (concentration 38.5µg/ml ) |
| C18 | 748.21 | 801.89 | 1082.83 (concentration 22.75µg/ml ) |
| C20 | 707.57 | 834.8126 | 1095.479 (concentration 31.5µg/ml) |

The results showed that the antibodies of the invention effectively blocked the binding of CD112R and CD112, resulting in enhanced activation of antigen-specific CD8 T cells and secretion of cytokine interferon γ (IFN- γ).

In addition to those described herein, modifications to the invention are intended to fall within the scope of the following claims in light of the foregoing description. Each reference cited in this application, including all patents, patent applications, journal articles, books, and any other publications, are incorporated herein by reference in entirety.

### Sequence listing

| SEQ ID NO: | Sequence Description | Sequence information |
|---|---|---|
| 657 | Tab 1 VH | QVQLVQSGAEVKKPGASVKVSCKASGYTFTDYNINWVRQAPGQGLEWMGYIYPYIGG SGYAQKFQGRVTMTRDTSTSTVYMELSSLRSEDTAVYYCAREDKTARNAMDYWGQGT LVTVSSASTKGPSVFPLAPCSRSTSESTAALGCLVKDYFPEPVTVSWNSGALTSGVHTFP AVLQSSGLYSLSSVVTVPSSSLGTKTYTCNVDHKPSNTKVDKRVESKYGPPCPPCPAPEF LGGPSVFLFPPKPKDTLMISRTPEVTCVVVDVSQEDPEVQFNWYVDGVEVHNAKTKPRE EQFNSTYRVVSVLTVLHQDWLNGKEYKCKVSNKGLPSSIEKTISKAKGQPREPQVYTLP PSQEEMTKNQVSLTCLVKGFYPSDIAVEWESNGQPENNYKTTPPVLDSDGSFFLYSRLT VDKSRWQEGNVFSCSVMHEALHNHYTQKSLSLSPGK |
| 658 | Tab 1 VL | DIQMTQSPSSLSASVGDRVTITCRVSENIYSNLAWYQQKPGKAPKLLIYEATNLAEGVPSR FSGSGSGTDFTLTISSLQPEDFATYYCQHFWGTPYTFGQGTKLEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC |
| 659 | Human IgG1 heavy chain constant region | |
| 660 | Human light chain constant region (CL) Kappa | |
| 661 | Human IgG1 Fc | EPKSCDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGK |
| 662 | PVRIG DNA | atgagaacagaggcccaagtgcctgctctgcagcctccggagcctggcctggaaggcgccatgggccaccggaccctggtgctgccttg ggtcctgctgaccctgtgtgtgaccgccggcacacctgaggtgtgggtgcaggttagaatggaggccacagagctgagctcttttaccatc cggtgcggattcctgggatccggcagcatttctctggtcaccgtgtcttggggcggacctaatggcgctggcggcaccacactggccgtgc tgcaccctgagcggggcatcagacagtgggcccctgccagacaggccagatgggaaacccagagcagcatctctctgatcctggaagg aagcggcgccagcagcccctgcgccaacacaacattctgctgcaagttcgcctccttcccagagggaagctgggaggcctgcggcagcc tgcctcctagcagtgatcccggcctcagcgcccctcctacccctgctccaatcctgagagccgacctggccggaatcctgggcgtgtccgg cgtgctgcttttcggctgcgtgtacctgctgcatctgctgaggcggcacaagcacagaccagcccctagactgcaaccttctagaaccagcc ctcaggcccctcgcgcccgggcttgggcccctagccaggcctctcaggctgctctgcatgtgccctacgcaaccatcaacaccagctgta gacctgccaccctggacaccgcccacccccacggaggcccaagctggtgggccagcctgcccacccacgccgcccacagaccccag ggccccgccgcttgggctagcacccctatccccgcaagaggcagctttgtgtccgtggaaaacggcctgtacgcccaggccggcgagag acctccacacacaggccctggcctgacactgttccccgacccccggggccccagagccatggaaggccctctgggcgtgcgg |
| 430 | C1 VH | QVHLQQSGPELVKPGASLKISCKASGYTFTDYYLNWVKQRPGQGLEWIGWIFPGSESTF YNEKFKGKATLTVDKSSSTAYMLLNSLTSEDSAVYFCTRDGGLRGPFDYWGQGTTLTV SS |
| 431 | C1 VL | DIQLTQSPSSLSASLGGKVTITCKASQDINKYIAWCQHKPGKGPRLLIHYTSTLHPGIPSRF SGSGSGRDYSFSISNLEPEDSATYYCLQYDYLLTFGGGTKLEIK |
| 432 | C3 VH | QVQLQQSGPELVKPGASVKISCRSSGYTFTDYYINWVKQRPGQGLEWIGWIFPGSDYTY YNEKFKGKATLTVDKSSTTAYMLLSSLTSEDSAVYFCARDGGLRGPFDYWGQGTTLTV SS |
| 433 | C3 VL | DIQMTQSPSSLSASLGGKVTITCKASQDINKYIAWYQQKPGKGPRLLIHYTSTLQPGIPSR FSGSGSGRNSSFSISNLEPEDIATYYCLQYDNLHTFGGGTKLEIK |
| 434 | C4 VH | EVQLQQSGPELVKPGASVKISCKASGYTFTDYFMNWVKQSHGKSLEWIGDIIPNNDSPN YNQKFKGKATLTVDKSSNTAYMELRSLTSEDSAVYYCASGYFDVWGTGTTVTVSS |
| 435 | C4 VL | DIVMTQSPSSLSVSAGEKVTMSCKSSQSLLNSGNQKNFLAWYQQKPGQPPKLLIYGTST RESGVPDRFTGSGSGTDFTLTISSVQAEDLAVYYCQNDHSYPWTFGGGTKLEIK |
| 436 | C6 VH | QVHLHQSGPELVKPGASVKISCKASGYTFTAYYINWVKQRPGQGLEWIGWIFPGSDSTY YNEKFKGRATLTVDKSSSTAYMLLNSLTSEDSAVYFCARDGELRGPFDYWGQGTTLTV SS |
| 437 | C6 VL | DIQMTQSPSSLSASLGGKVTITCKASQDINKYIAWYQHKPGKSPRLLIHYTSTLQAGIPSR FSGSGSGRDYSFSISNLEPEDSATYYCLQYDNLLTFGGGTKLEIK |
| 438 | C7 VH and C7-2 VH | EVQLQQSGPELVKPGASVKISCKASGYTLTDYYMNWVKQSHGKSLEWIGDINPNNGGT NFNQKFKGKATLTIDKSSSTTYMELRSLTSEDSAVYYCASLFFDYWGQGTTLTVSS |
| 439 | C7 VL | DIVMTQSPSSLSVSAGEKVTMSCKSSQSLLNSGNQKNYLAWYQQKPGQPPKLLIYGAST RESGVPDRFTGSGSGTDFTLTISSVQAEDLALYYCQNAHSSPYTFGGGTKLEIK |
| 440 | C7-2 VL | DIVMTQSQKFMSTTVGDRVSITCKASQNVGTAVAWYQQKPGQSPKLLIYSASNRYTGV PDRFTGSGSGTDFTLTISNMQSEDLADYFCQQYSSYRTFGGGTKLEIK |
| 441 | C8 VH | EVQLVASGGGLVKPGGSLKLSCAASGFTFSDYYMFWVRQTPEKRLEWVATISDGGGYI YYPDSVKGRFTISRDNDRNSLYLQMSSLKSEDTAIYYCGREDFWGQGTSVTVSS |
| 442 | C8 VL | DIQMTQSPSSLSASLGERVSLTCRASQDIGRSLNWLQQEPDGSVRRLIYATSRLDSGVPK RFSGSRSGSDYSLTISSLESEDFVDYYCLQYASFPYTFGGGTKLEIK |
| 443 | C9 VH | EVQLQQSGAELVKPGASVKLSCTASGFNIKDIYMHWLKQRPEQGLEWIGRIDPTNGNSE YDPRFRGKATLTADTSSNIAYLQLSSLTSEDTAVYYCARFGIYPYFDYWGQGTTLTVSS |
| 444 | C9 VL | GIVMTQTPKFLLVSAGDRVTITCKASQSVNKAVVWYQQKPGQSPKLLISYASNRYTGVP DRFTGSGYGTDFTFSISTVQAEDLAVYFCQQDYSSPWTFGGGTKLEIK |
| 445 | C10 VH | EVELVESGGGLVKPGGSLKLSCAASGFTFSDSYMCWVRQTPEKRLEWVATISDGGGLT YYPDSVKGRFTISRDNDRNNLYLQMDSLKSEDTAVYYCGREDLWGQGTSVIVSS |
| 446 | C10 VL | DIQMTQSPSSLSASLGERVSLTCRASQDIGSSLNWLQQEPDGTIKRLIYATSRLDSGVPKR FSGSRSGSDYSLTISSLESEDFVDYYCLQYASSPYTFGGGTKLEIK |
| 447 | C11 VH | EVQLQQSGPELVKPGASVKISCKASGYTFTDYFMNWVRQSHGKSLEWIGDIHPNNGSPN YNQKFKGKATLTVGKSSSTAYMELRSLTSEDSAVYYCATGYFDVWGTGTTVTVSS |
| 448 | C11 VL | DIVMTQSPSSLSVSAGEKVTMSCKSSQSLLNSGNQKNYLAWYQQKPGQPPKLLIYGAST RESGVPDRFTGSGSGTDFTLTISSVQAEDLAVYYCQNDHSYPWTFGGGTKLEIK |
| 449 | C12 VH | EVQLVASGGGLVKPGGSLKLSCAASGFTFSDYYMYWVRQTPEKRLEWVATISDGGGYT YYPDSVKGRFTISRDNDRNSLYLQMSSLKSEDTAMYYCGREDLWGQGTSVTVSS |
| 450 | C12 VL | DIQMTQSPSSLSASLGERVSLTCRASQDIGSSLNWLQQEPDGTVKRLIYATSRLDSGVPK RFSGSRSGSDYSLTISSLESEDFVDYYCLQYASFPYTFGGGTKLEIK |
| 451 | C13 VH | EVQLQQSGPELVKPGASVKISCKASGYTFTDYFMNWVKQSHGKSLEWIGDINPNNGSPN YNQKFKGRATLTVDKSSSTAYMELRSLTSEDSAVYYCASGYFDVWGTGTTVTVSS |
| 452 | C13 VL | DIVMTQSPSSLSVSAGEKVTMSCKSSQSLLNSGTQKNYLAWYQQKPGQPPKLLIYGAST RESGVPDRFTGSGSGTDFTLTISSVQAEDLAVYYCQNDHSYPWTFGGGTKLEIK |
| 453 | C14 VH | QVQLQQPGAELVRPGTSVKLSCKASGNTFTSYWMHWVKQRPGQGHEWIGVIDASDSN TKYNQKFKGKATLTVDTSSSTAYMQLSSLTSEDSAVYYCARYYSGSSYGLDYWGQGTT LTVSS |
| 454 | C14 VL | DIQMTQTASSLSASLGDRVTISCRASQDISNYLNWYQQKPDGTVKLLIYYTSRLHSGVPS RFSGSGSGTDFSLTITHLEQEDIASYFCQQGNMFPPTFGGGTKLEIK |
| 455 | C16 VH | QVQLQQPGAELVRPGTSVKLSCKASGNTFTSYWMHWVKQRPGQGHEWIGVIDASDSN TKYNQKFKGKATLTVDTSSSTAYMQLSSLTSEDSAVYYCARYFSGSSYGLDYWGQGTT LTVSS |
| 456 | C16 VL | DIQMTQTASSLSASLGDRVTISCRASQDISNYLNWYQQKPDGTVKLLIYYTSRLHSGVPS RFSGSGSGTDYSLTITNLEQEDIASYFCQQGNMFPPTFGGGTKLEIK |
| 457 | C18 VH | EVQLVESGGGLVKPGGSLKLSCAASGFTFSDYYMYWVRQTPEKRLEWVATISDGGGFI YYPDSVKGRFTISRDNDRNSLYLLMSSLKSEDTAMYFCGREDLWGQGTSVTVSS |
| 458 | C18 VL | DIQMTQSPSSLSASLGERVSLTCRASQDIGRSLNWLQQGPDGTVKRLIYATSRLDSGVPT RFSGSRSGSDYSLTISSLESEDFVDYYCLQYASFPYTFGGGTKLEIK |
| 459 | C19 VH | EVQLVESGGGLVKPGGSLKLSCAASGFTFSDYYMYWVRQTPEKRLEWVATISDAGGYT YYPDSVKGRFTISRDNARNSLYLQMSSLKSEDTAMYFCGREDLWGQGTSVTVSS |
| 460 | C19 VL | DIQMTQSPSSLSASLGERVSLTCRASQDIGYSLNWLQQEPDGSVRRLIYATSRLDSGVPK RFSGSRSGSDYSLTISSLESEDFVDYYCLQYASFPYTFGGGTKLEIK |
| 461 | C20 VH | EVQLVESGGGLVKPGGSLKLSCAASGFTFSDSYMCWVRQTPEKRLEWVATISDAGGYT YYPDSVKGRFTISRDNDRNNLYLQMSGLKSEDTAVYYCGREDLWGQGTSVTVSS |
| 462 | C20 VL | DIQMTQSPSSLSASLGERVSLTCRASQDIGRSLNWLQQEPDGTIKRLIYATSRLDSGVPKR FSGSRSGSDYSLTISSLESEDFVDYYCLQYASSPYTFGGGTKLEIK |
| 463 | C21 VH | EVQLQQSGAELVKPGASVKLSCTASGFNIKDIYMHWLKQRPEQGLEWIGRVDPTNGNS EYDPRFQGKATLTADTSSNIAYLQLSSLTSEDTAVYYCARFGIYPYFDYWGQGTTLTVSS |
| 464 | C21 VL | SIVMTQTPKFLLVSAGDRVTITCKASQSVNKAVVWYQQKPGQSPKLLINYASNRYTGVP DRFTGSGYGTDFTFSISTVQAEDLAVYFCQQDYSSPWTFGGGTKLEIK |
| 465 | C22 VH | EVQLQQSGPEPVKPGASVKISCKASGYTFTDYNIHWVRQSHGKSLEWIGYIYPYNGGAG YNQKFKSKATLTVDSSSSTAYMELRSLTSEDSAVYYCAREELRRDYGMDYWGQGTSVT VSS |
| 466 | C22 VL | DIQMTQSPASLSASVGETVTITCRASENIYSYLAWYQQKQGKSPQLLVYNAKTLAEGVP SRFSGSGSGTQFSLKINSLQPEDFGSYYCQHHYGTPYTFGGGTKLETK |
| 467 | C23 VH | QVQLQQSGPELVKPGTSVKISCKASGYTFTDYYINWVKQRPGQGLEWIGWIFPGSGNTY YNEKFKGRATLTVDKSSSTAYMLLSSLTSEDSAVYFCARDGGLRGPFDYWGQGTTLTV SS |
| 468 | C23 VL | DIQMTQSPSSLSASLGGKVTITCKASQDINKYIAWFQHKPGKGPRLLIHFTSTLQSGIPSRF SGSGSGGDYSFSISNLEPEDIATYYCLQYDYLYTFGGGTTLEIK |
| 469 | C24 VH | QVQLKQSGPGLVQPSQNLSITCTVSGFSLTGYGINWVRQSPGKGLEWLGVIWSGGNTEY NAAFRSRLSISKDTSKSQVFLKMNSLQADDTAIYSCARDSTGLYAMDYWGPGTSVTVSS |
| 470 | C24 VL | DLQMTQTTSSLSASLGDRVTISCRTSQDISNFLNWYQQKPDGPLKLLIYYTSRLHSGVPS RFSGSGSGTDYSLTISNLEQEDIATYFCQQGYMLPPTFGGGTKLEIK |
| 471 | C25 VH | QVQLQQPGAELVRPGTSVKLSCKASGNTFTSYWMHWVKQRPGQGHEWIGVIDASDSK TKYNQKFKGKATLTVDTSSSTAYMQLSSLTSEDSAVYYCARYYSGSSYGLDYWGQGTT LTVSS |
| 472 | C25 VL | DIQMTQTASSLSASLGDRVTISCRASQDISNYLNWYQQKPDGTVKLLIYYTSRLHSGVPS RFSGSGSGTDYSLTISNLEQEDIATYFCQQGNTLPPTFGGGTKLEIK |
| 473 | C28 VH | QIQLQQSGPELVKPGPSVKISCKASGYTFTDYYINWVKQRPGQGLEWIGWIYPGSGYTK YNEKLKGRATLTVDTSSSTAYMQLSSLTSEDSAVYFCARDSFYAMDYWGQGTSVTVSS |
| 474 | C28 VL | DIVMTQSQKFMSTTVGDRVSITCKASQNVGTAVAWYQQKPGQSPKLLIYSASNRYTGV PDRFTGSGSGTDFTLTITNMQSEDLADYFCLQYSSYLTFGAGTKLELK |
| 475 | C31 VH | QIQLQQSGPELVKPGASVKISCKASGYTFTDYYIAWVKQRPGQGLEWIGWIYLGSGHTM YNENFKGKATLTVDTSSSTAYMQLSSLTSEDSAVYFCARDSFYAMDYWGQGTSVTVSS |
| 476 | C31 VL | DIVMTQSQKFMSTTVGDRVSITCKASQNVGTAVAWYQQKPGQSPKLLIYSASNRYTGV PDRFTGSGSGTDFTLTISNMQSEDLADYFCQQYSTYLTFGAGTKLELK |
| 477 | C33 VH | QVQLQQSGPELVKPGASVKISCKASGYTFTDYYINWVKQRPGQGLEWIGWIFPGSGSTY YNEKFKGKATPTVDKYSSTAYMLLSSLTSEDSAVYFCAREDYFDRGDCWGQGTSVTVS S |
| 478 | C33 VL | QIVLTQSPAIMSASPGEKVTMTCSASSSINYMHWYQQKPGTSPKRWIYDTSKLASGVPA RFSGSGSGTSYSLTISSMEAEDAATYYCHQRSSYPFTFGSGTKLEIK |
| 479 | C36 VH | QIQLVQSGPELKKPGETVKISCKASGYTFTTYGMSWVKQAPGKGLKWMGWINTYSGVP TYADDFKGRFAFSLETSASNAYLQINNLKNEDTATYFCARELPFTTIIGTKDYWGQGTTL TVSS |
| 480 | C36 VL | DIVLIQSPATLSVTPGDSVSLSCRASQFISNNLHWYQQKSHESPRLLIKYASQSISGIPSRFS GGGSGTDFTLTVNSVETEDFGMYFCQQSNSWPHTFGGGTKLEIK |
| 481 | C38 VH | QVQLQQSGAELMKPGASVKLSCKATGYTFTGYWIEWVKQRPGHGLEWIGEILPESGRT NYNEKFKGKATFTADTSSNTAYMQLSSLTTEDSAIYYCASLLFFDYWGQGTTLTVSS |
| 482 | C38 VL | DVVLTQTPLTLTVTIGQPASISCKSSQSLLDSDGKTYLNWLLQRPGQSPKRLIYLVSKLDS GVPDRFTGSGSGTDFTLKISRVEAEDLGVYYCWQGTHFPLTFGAGTKLELK |
| 483 | C39 VH | QAYLQQSGAELVRPGASVKMSCKASGYTFTSYNIHWVKQTPRQGLEWIGAIYPGNADIS YNQKFRDKATLTVDKSSSTAYMQLSSLTSEDSAVYFCAKPLRIYAMDCWGQGTSVTVS S |
| 484 | C39 VL | DIVMTQSQKFMSTSVGDRVSVTCKASQNVGTNVAWYQQKPGQSPKALIYSASYRYSGV PDRFTGSGSGTDFTLTISNVQSEDLADYFCQQYNSRPLTFGGGTKLEIK |
| 485 | C41 VH | EVQLQQSGPELVKPGASVKISCKASGNTFTDYYINWVKQSHGKSLEWIGDIEPNNGGTN YNQKFKGKATLTVDKSSSTAYMELRSLTSEDSAIYYCATLYFDYWGQGTTLTVSS |
| 486 | C41 VL | DIVMTQSPSSLSVSAGEKVTMSCKSSQSLLNSGNQKNYLAWYQQKPGQPPKLLIYGAST RESGVPDRFTGSGSGTDFTLTISSVQAEDLAVYYCQNDHSYPFTFGSGTKLEIK |
| 487 | C43 VH | QVQLQQPGAELVRPGTSVKLSCKASGNTFTTYWMHWVKQRPGQGHEWIGVIDASDSN TKYNQKFKGKATLTVDTSSSTAYMQLSSLTSEDSAVYYCARYYSGSSYGLDYWGQGTT LTVSS |
| 456 | C43 VL | DIQMTQTASSLSASLGDRVTISCRASQDISNYLNWYQQKPDGTVKLLIYYTSRLHSGVPS RFSGSGSGTDYSLTITNLEQEDIASYFCQQGNMFPPTFGGGTKLEIK |
| 488 | C44 VH | QVQLQQSGPELMKPGASLKISCKASGYTFTDSYINWVKQRPGQGLEWIGWIFPGSGSTY YNEKFKGTATLTVDKSSSTAYMLLSSLTSEDSAAYFCARDGGLRGPFDYWGQGTTLTV SS |
| 489 | C44 VL | DIQMTQSPSSLSASLGGKVTITCKASQDINKYIAWYQHKPGKGPRLLIHYTSTLQSGIPSR FSGSGSGRDYFFSISNLESEDIATYYCLQYDHLLTFGGGTKLEIK |
| 490 | C45 VH | QVHLQQSGPDLVKPGVSVKISCKASGYTFTDYYINWVKQRPGQGLEWIGWIFPGSGTTY YNEKFKGKATLTVDKSSSTAYMLLSSLTSEDSAVYFCARDGGLRGPFDYWGQGTTLTV SS |
| 491 | C45 VL | DIQMTQSPSSLSASLGGKVTITCKANQDINKYIAWYQYKPGKGPRLLIHYTSTLQPDIPSR FSGSGSGRDFSFSISNLEPEDIATYYCLQYDYLYTFGGGTKLEIK |
| 492 | C47-2 VH | QVQLQQPGAELVKPGASVKLSCKASGYSFTSYWMQWIKQRPGQGLEWIGEIDPFDSYT NYNQKFKGKATLTVDTSSSTAYMQLSSLTSEDSAVYYCASRVYYGYDRYFFDYWGQG TTLTVSS |
| 489 | C47-2 VL | DIQMTQSPSSLSASLGGKVTITCKASQDINKYIAWYQHKPGKGPRLLIHYTSTLQSGIPSR FSGSGSGRDYFFSISNLESEDIATYYCLQYDHLLTFGGGTKLEIK |
| 493 | C48 VH and C48-2 VH | QVQLQQSGPELVKPGASVKISCKASGYTFTDYYINWVKQRPGQGLEWIGWIFPGSGSTY YNEKFKGKATLTVDKSSSTAYMLLSSLTSEDSAVYFCAPDGSYDPPFAYWGLGTLVTVS A |
| 494 | C48 VL | QIVLTQSPAIMSASPGEKVTITCSASSSVSYMHWFQQKPGTSPKLWIYSTSNLASGVPAR FSGSGSGTSYSLTISRMEAEDAATYYCQQRSSYPPTFGAGTKLELK |
| 495 | C48-2 VL | DIQMTQSPSSLSASLGGKVTITCKASQDINKYIAWYQHKPGKGPRLLIHYTSTLQPGIPSR FSGSGSGRDYSFSISNLEPEDIATYYCLQYDNLYTFGGGTKLEIK |
| 496 | C51 VH | QVQLQQSGAELVRPGASVTLSCKASGYTFTDYEMHWVKQTPVHGLEWIGAIDPETGGT AYNQKFKGKAILTADKSSSTAYMELRSLTSEDSAVYYCTRWITTVVATLYYFDYWGQG TTLTVSS |
| 497 | C51 VL | DIVMTQSQKFMSTSVGDRVSITCKASQNVRTAVAWYQQKPGQSPKALIYLASNRHTGV PDRFTGSGSGTDFTLTISNVQSEDLADYFCLQHWNYPLTFGAGTKLELK |
| 498 | C52 VH | RVQLQQSGAELVRPGASVTLSCKASGYTFTDYEMHWVKQTPVHGLEWIGAIDPETGGT AYNQKFKGKAILTADKSSSTAYMELRSLTSEDSAVYYCTRWITTVVATLYYFDYWGQG TTLTVSS |
| 497 | C52 VL | DIVMTQSQKFMSTSVGDRVSITCKASQNVRTAVAWYQQKPGQSPKALIYLASNRHTGV PDRFTGSGSGTDFTLTISNVQSEDLADYFCLQHWNYPLTFGAGTKLELK |
| 499 | C55 VH | EVQLQQSGPELVKPGASVKISCKASGYTFTDYYMSWVKQSHGKSLEWIGDVNPNNGDT SYNQKFKGKATLTVDKSSSTAYMELRSLTSEDSAVYYCAASGLPYVLMDYWGQGTSV TVSS |
| 500 | C55 VL | DIQMTQSPASLSASVGETVTITCRASENINSYLAWYQQKQGKSPQLLVYNTKTLTEDVPS RFSGSGSGTQFSLKINSLQPEDFGSYYCQHHYGTPLTFGGGTKLELK |
| 501 | C56 VH | EVQLVESGGGLVKPGGSLKLSCAASGFTFSGYALSWVRQTPEKRLEWVATIRDGGSYT YYSDNEKGRFTISRDNAKNNLYLQMSHLKSEDTAIYYCTRDQVNRGWFTYWGQGTLV TVSA |
| 502 | C56-2 VH | EVQLVESGGGLVKPGGSLKLSCAASGFTFSDYALSWVRQTPEKRLEWVATIRDGGSYT YYSDNEKGRFTISRDNAKNNLYLQMSHLRSEDTAMYYCTRDQVNRGWFTYWGQGTLV TVSA |
| 503 | C56 VL and C56-2 VL | QVILTQSPAIMSASLGERVTMTCSASSSVSSSYFHWFQQKPGSSPKLWIYSTSNLASGVPP RFSGSGSGTSYSLTISSMEAEDAATYYCHQHHRSPLTFGAGTKLELK |
| 504 | C57 VH | EVQLQQSGPVLVKPGASVKMSCKASGYIFTDYYMHWVKQSHGKSLEWIGIINPYNGDT SYNQKFKDKATLTVDKSSSTAYMELNSLTSEDSAVYYCARDSSNVGNAMDYWGQGTS VTVSS |
| 505 | C57 VL | DIQMTQTTSSLSASLGDRVTISCRASQDISNYLNWYLQKPDGTIKLLIYYSSRLHSGVPSR FSGSGSGTDYSLTISNLEQEDIATYFCQQGYTLPRTFGGGTKLEII |
| 499 | C58 VH | EVQLQQSGPELVKPGASVKISCKASGYTFTDYYMSWVKQSHGKSLEWIGDVNPNNGDT SYNQKFKGKATLTVDKSSSTAYMELRSLTSEDSAVYYCAASGLPYVLMDYWGQGTSV TVSS |
| 506 | C58 VL | DIQMTQTTSSLSASLGDRVTISCRASQDISNYLNWYLRKPDGTVKLLIYYSSRLHSGVPS RFSGSGSGTDYSLTISNLEQEDIATYFCQQGNTLPRTFGGGTKLEIK |
| 507 | C59 VH | EVQLVESGGGLVKPGGSLKLSCAASGFTFSGYALSWVRQTPEKRLEWVATIRDGGSYT YYSDNEKGRFTISRDNAKNNLYLQMSHLKSEDTAMYYCTRDQVNRGWFTYWGQGTL VTVSA |
| 503 | C59 VL | QVILTQSPAIMSASLGERVTMTCSASSSVSSSYFHWFQQKPGSSPKLWIYSTSNLASGVPP RFSGSGSGTSYSLTISSMEAEDAATYYCHQHHRSPLTFGAGTKLELK |
| 508 | C61 VH | DVQLQESGPGLVKPSQSLSLTCSVTGYSITSNYYWNWIRQFPGNNLEWMGYISSDGTNN YNPSLRNRISITRDTSKNQFFLKLNSVTTEDTATYYCARGLRYGPWGQGTTLTVSS |
| 509 | C61 VL | QLILTQSSSASFSLGASAKLTCTLSSQHSTYTIEWYQQQPLKAPKYVMEVKNDGSHRTG DGIPDRFSGSSSGADRYLSISNIQPEDEAIYICGVGDINNYIFGGGTKVTVL |
| 510 | C62 VH | EVMLVESGGGLVKPGGSLKLSCAASGFSFSSYSMSWVRQTPEKRLECVATIGGVGGNT YYPDSVKGRFSISRDNAKNTLYLQMSSLRSEDTALYYCARHEDYYYYAMDYWGQGTS VTVSS |
| 511 | C62 VL | QIVLTQSPAIMSASPGEKVTMTCSANSTVSYIYWYQQKPGSSPRLLIYDTSNLASGVPVR FSGSGSGTSYYLTISRMEAEDAATYYCQQWSSFPFTFGSGTKLEIK |
| 512 | C63 VH | EVMLVESGGGLVKPGGSLKLSCAASGFIFSSYIMSWVRQTPEKRLEWVATIRGGGGNTY YLDSVKGRFIISRDNAKNNLYLQMSSLRSEDTALYYCARHEDYEWYAMDNWGQGTSV TVSS |
| 513 | C63 VL | QIVLTQSPAIMSASPGEKVTLTCSARSSVSYMYWYQQKPGSSPKLLIYDTSNLASGVPVR FSGSGSGTSYSLTISRMEAEDAATYYCQQWSSYPLTFGAGTKLELK |
| 514 | C64 VH | EVMLVESGGDLVKPGGSLKLSCAASGFTFSSYFMSWVRQTPEKRLEWVATISGGGDKT YYLDILKGRFTISRDNAKNTLYLQMSSLRSEDTALYYCARHEDYAWYAMDYWGQGTS VTVSS |
| 515 | C64 VL | QIVLTQSPAIMSASPGEKVTMTCSASSSVNYIYWYQQKPGSSPRLLIYDTSNLASGVPVR FSGSGSGTSYSLTISRMEAEDAATYYCQQWSSYPLTFGAGTKLELK |
| 501 | C66 VH | EVQLVESGGGLVKPGGSLKLSCAASGFTFSGYALSWVRQTPEKRLEWVATIRDGGSYT YYSDNEKGRFTISRDNAKNNLYLQMSHLKSEDTAIYYCTRDQVNRGWFTYWGQGTLV TVSA |
| 516 | C66 VL | DIQMTQSPSSLSASLGGKVTITCKASQDINKYIAWYQHKPGKGPRLLIHYTSTLQPGIPSR FSGSGSGRDYSFNISNLEPEDIATYYCLQYDNLYMFGGGTKLEIK |
| 517 | C67 VH | EAQLQQSGPELVKPGASVKISCKASGYTFTDYYMNWVKQSHGKSLDWIGDINPDNGGS TYNQKFKGKATLTVDKSSSTAYMELRSLTSEDSAVYYCSASGPPYVLLDYWGQGTSVI VSS |
| 518 | C67 VL | DIQMTQSPASLSASVGETVTITCQTSENIYSYLAWYQQKQGKSPQLLVYNTQTLAEGVPS RFSGSRSGAQFSLKINSLQPEDFGSYYCQHHYGVPLTFGAGTKLELK |
| 519 | C69 VH | EVQLQQSGPVLVKPGASVKMSCKASGYRLTDYYMHWVKQSHGKSLEWIGIINPYNGGT SYNQKFKGKATLTVDKSSSTAYMELNSLTSDDSAVYYCARDSSNVGNDMDYWGQGTS VSVSS |
| 520 | C69 VL | DIQMTQTTSSLSASLGDRVTISCRASQDISNYLNWYLQKPDGTVKLLIYYSSRLHSGVPS RFSGSGSGTDYSLTINNLEQEDIATYFCQQGNTLPRTFGGGTKLEIR |
| 521 | C71 VH | EVILVESGGGLVKPGGSRKLSCAASGFTFSSYFMSWVRQTPEKRLEWVATISGGGDNTY YPDSVKGRFTISRDNAKNTLYLQMSSLRSEDTALYYCARHEDYEWYAMDYWGQGTSV TVSS |
| 522 | C71 VL | QIVLTQSPAIMSTSPGEKVTMTCSASSSVSYIYWYQQKPGSSPRLLIYDTSNLASGVPVRF SGSGSGTSYSLTISRMEAEDAATYYCQQWTSYPLTFGAGTKLELK |
| 523 | C72 VH | EVQLVESGGDLVKPGGSLKLSCAASGFTFSSYFMSWVRQTPDKRLEWVATIRGGGSYT YYLDSVKGRFTVSRDNAKNTLYLQMSSLKSEDTAMYYCARHEDYDYYAMDYWGQGT SVTVSS |
| 524 | C72 VL | QIVLTQSPAIMSASPGEKVTMTCSASSSVSYMYWYQQKPGSSPRLLIYDTSNLASGVPVR FSGSGSGTSYSLTISRMEAEDAATYYCQQWSSYPLTFGAGTKLELK |
| 525 | C75 VH | QAQLQQPGAELVKPGASVKMSCKASGYTFTSYWITWVKQRPGQGLEWIGDIYPGNFSP NYNEKFKNKATLTVDTSSSTAYMQLSSLTSEDSAVYYCAKGYFDVWGTGTTVTVSS |
| 486 | C75 VL | DIVMTQSPSSLSVSAGEKVTMSCKSSQSLLNSGNQKNYLAWYQQKPGQPPKLLIYGAST RESGVPDRFTGSGSGTDFTLTISSVQAEDLAVYYCQNDHSYPFTFGSGTKLEIK |
| 526 | C76 VH | EVQLQQSGPELVKPGASVKISCKASGYTFTHYYKNWVKQSHGKSLEWIGAINPNIGGPS YNQKFKGKATLTVDKSSSTVYMELRSLTSEDSAVYYCARSDDYGGYYFDYWGQGTTL TVSS |
| 527 | C76 VL | QIVLSQSPALLSASPGEKVTMTCRANSSVNYMHWYQQKPGSSPKPWIYATSNLASGVPA RFSGSGSGTSFSLTISRVEAEDAATYYCQQWTSNPLTFGAGTKLELK |
| 528 | C77 VH | EVQLQQSGPELVKPGASVKISCKASGYTFTDYYMNWVRQSHGKSLEWIGDIYPNNGDT NYNQKFKGKATLTVDKSSKTADMELRSLTSEDSAVYYCIRGVGLGFPYWGQGTLVTVS A |
| 529 | C77 VL | DIVMTQSHKFISTSVGDGVSITCQASQVVSTAVAWYQQKPGQSPRLLLHSASFRYSGVP DRFTGSGSGTDFTFTISTVQAEDLAVYYCQQHYRTPYTFGGGTKLEIK |
| 530 | C78 VH | EVQLQQSGPVLVKPGASVKMSCEPSGYTFTHYYVNWMRQSHGKSLEWIGVIKPYNGAP SYNQKFKGKATLTVDKSSSKINMELNSLTSEDSAVYYCARSDDFDGYAMDYWGQGTS VTVSS |
| 531 | C78 VL | QIVLTQSPAIMFASPGEKVTMTCSASSSVSYMHWYQQKSGTSPKRCISDTSKLASGVPAR FSGSGSGTSYSLTISSMEAEDAATYYCQQWTSNPLTFGSGTKLELK |
| 530 | C79-2 VH | EVQLQQSGPVLVKPGASVKMSCEPSGYTFTHYYVNWMRQSHGKSLEWIGVIKPYNGAP SYNQKFKGKATLTVDKSSSKINMELNSLTSEDSAVYYCARSDDFDGYAMDYWGQGTS VTVSS |
| 532 | C79-3 VH and C79-4 VH | EVQLVESGGDLVKPGGSLKLSCAASGFTFNNYGMSWVRQTPDKRLEWVATISRGGSFT YYPDSVKGRFTISRDNAKNTLYLQMSSLKSEDTAMYYCARPIYYEYDGFAYWGQGTLV TVSA |
| 531 | C79-3 VL | QIVLTQSPAIMFASPGEKVTMTCSASSSVSYMHWYQQKSGTSPKRCISDTSKLASGVPAR FSGSGSGTSYSLTISSMEAEDAATYYCQQWTSNPLTFGSGTKLELK |
| 533 | C79-2 VL and C79-4 VL | DVLMTQTPLSLPVSLGDQASISCRSSQSIVHSNGNTYLEWYLQKPGQSPKLLIYKVSNRF SGVPDRFSGSGSGTDFTLKINRVEAEDLGVYSCFQGSHVPRTFGGGTKLEIK |
| 534 | C80 VH | QVQLQQPGAELVKAGASVKMSCKASGYTFTSYWITWVKQRPGQGLEWIGDIYPGNDSP NYNEKFKNKATLTVDTSSSTAYMQLSSLTSEDSALYYCARGYYDYWGQGTTLTVSS |
| 535 | C80 VL | DIVMTQSPSSLSVSAGEKVTMSCKSSQSLLNSGNQKNYLAWYQQKTGQPPKLLIYGAST RESGVPDRFTGSGSGTDFTLTISSVQAEDLAVYYCQNDHSYPHTFGAGTKLELK |
| 536 | C81 VH | EVQLQQSGPVLVKPGASVKMSCKASGNTFNGYYVNWVKQSHGKSLEWIGLIDPSNGGT NYNQKFKDKATLTVDKSSTTAYMEFNSLTSEDSSVYYCAGGTVYWGQGTTLTVSS |
| 537 | C81 VL | DIQMTQSPSSLSASLGERVSLTCRASQDIGNNLNWLQQEPDGTIKRLIYATSSLDSGVPKR FSGSRSGSDYSLTISSLESEDFVDYYCLQYASSPVTFGGGTKLEIK |
| 538 | C82 VH | EVQLQQSGPELVKPGASVKISCKASGYMFTDYYMNWVKQSHGGSLEWIGTFNPNRGGP SYNLKFKGKATLTVDKSSITAYMDLRSLTSEDSAVYYCARADDYDGYYFDYWGQGTT LTVSS |
| 539 | C82 VL | QIVLTQSPAIMSASPGEKVTMTCSASSSVSYMHWYQQKSGTSPKRWIYDTSKLASGVPA RFSGSGSGTSYSLTISSMEAEDAATYYCQQWTNNPLTFGAGTKLELK |
| 540 | C84 VH and C84-2 VH | QVQLQQSGPELVKPGASVRISCKASGYSFTDYFISWVKQRPGQGLEWIGWIFPGSGSTYF NENFKGEATLTADKSSSTAYLLLSSLTSEDSAVYFCTTYYGSNYWGQGTTLTVSS |
| 541 | C84 VL | DVLMTQAPLSLPVSLGDQASISCRSSQSIVHSNGNTYLEWYLQKPGQSPKLLIYTVSNRL SGVPDRFSGSGSGTDFTLKISRVEAEDLGIYYCFQTSHVPPTFGGGTKLEIK |
| 542 | C84-2 VL | DIQMTQSPSTLSASVGDRVTITCRASQSISSWLAWYQQKPGKAPKVLIYKASTLESGVPS RFSGSGSGTDFTLTISSLQPEDFATYNCQQYNTYWTFGQGTKVEIK |
| 543 | C85 VH | EVQLQQSGPELVKPGASVKISCKASGYTFTDYSMNWVKQSHGKSLEWIGDINPNNGGTS YNQKFKGKATLTVDKSSSTAYMELRSLTSEDSAVYYCARVWFAYWGQGTLVTVSA |
| 544 | C85 VL | DIVMTQSPSSLSVSAGEKVTLSCKSSQSLLNSGNQKNYLAWYQQKPGQPPKLLIYGAST RESGVPDRFTGSGSGTDFTLTISSVQAEDLALYYCQNAHSYPFTFGSGTKLDIK |
| 545 | C87 VH | QHELQQSGAELVRPGSSVKLSCKDSYFAFMASAMHWVKQRPGHGLEWIGSFTMYSDA TEYSENFKGKATLTANTSSNTAYMELSSLISEDSAVYYCASGYSGNGFTYWGQGTLVTV SA |
| 546 | C87 VL | DIVLTQSPASLAMSLGQRATISCRASESVDNYGISFMHWYQQKPGQPPKLLIYRASNLES GIPARFSGSGSRTDFTLTINPVETDDVATYYCQQSNKDPMYTFGGGTKLEIR |
| 547 | C88 VH | QVQLQQPGAELVKPGASVNMSCKASGYTFSSYWITWVKQRPGQGLEWIGDIHPGNTSP NYNEKFRTKATLTVDTSSSTAYMQLSSLTSEDSAVYYCARGWGDYWGQGTTLTVSS |
| 548 | C88 VL | DIVMTQSPSSLSVAAGEKVTMSCKSSQSLLNSGNQKNYLAWYQQKPGQPPKLLIYGAST RESGVPDRFTGSGSGTDFTLTISSVQAEDLAVYYCQNDHGYPLTFGTGTKLELK |
| 549 | C89 VH | EVQLVESGGDLVKPGGSLKLSCVASGLTFSDYGMYWIRQAPEKGLEWVAYIRSDSSTIY YTDTVKGRFTISRDNAKNTLFLQMTSLRSEDTAIYYCARRSPVVVNYAMDYWGQGTSV TVSS |
| 550 | C89 VL | DIQMTQSPASLSASVGETVTITCRASENIYSHLVWYQQKQGKSPHLLVYNGKILAEGVPS RFSGSGSGTQFSLKINSLQPEDFGSYYCQHHYGSPITFGAGTKLELN |
| 551 | C90 VH | EVQLQQSGPELVKPGTSVKISCKASGYTFTDYYMNWVRQSHGKSLEWIGEINPKNGDT NYNQNFKGKATLTVDKSSNTAYMELRSLTSEDSAVFYCAREGVSSYYAMDYWGQGTS VTVSS |
| 552 | C90 VL | DIVMTQSPSSLAMSVGQKVTMSCKSSQSLLNSFNQKNFLAWYQQKPGQSPKLLVYFAS TRESGVPDRFIGSGSGTDFTLTITSVQAEDLADYFCQQHHSTPPTFGGGTKLEIN |
| 553 | C91 VH | EVQLQQSGPVLVKPGASVKMSCKASGNTFNGYYINWVKQSHGKSLEWIGLIDPSNGGT NYNQKFKDKATLTVDKSSTTAYMEFNTLTSEDSSVYYCAGGTVYWGQGTTLTVSS |
| 554 | C91 VL | DIQMTQSPSSLSASLGERVSLTCRASQDIGNNLNWLQQEPDGTIKRLIYATSSLDSGVPKR FSGSRSGSDYSLTISSLESEDFVDYYCLQYASSPPTFGGGTKLEIK |
| 555 | C92 VH | EVQLVESGGGLVKPGGSLKLSCAASGITFSDYGMHWVRQAPEKGLEWVAYIRSDSSTIY YADTVKGRFTISRDNAKNTLFLQMTSLRSDDTAMYYCARRSPVVVNYAMDYWGQGTS VTVSS |
| 556 | C92 VL | DIQMTQSPASLSASVGETVTITCRASENIYSYLVWYQQKQGKSPQLLVYNGKILAEGVPS RFSGSGSGTQFSLKINSLQPEDFGSYYCQHHYGSPLTFGAGTKLELK |
| 557 | C93 VH | EVHLQQSGPELVKPGASVKISCKASGYTFTDYYMNWMKQSHGKGLEWIGEINPKNGDT NYSQKFRDKATLTVDKSSNTAFMKLRSLTSEDSAVYFCAKEGVGSYYAMDYWGQGTS VTVSS |
| 558 | C93 VL | DIVMTQSPSSLAMSVGQKVTMSCKSSQSLLSSNNQKSFLAWYQQKPGQSPKLLVSFAST RESGVPDRFIGGGSGTDFTLTISSVQPEDLADYFCQQHYSTPPTFGEGTKLEIK |
| 559 | C94 VH | QVQLQQPGAELVKAGASVKMSCKASGYTFTSYWITWVKQRPGQGLEWIGDIYPGNDSP NYNEKFKNKATLTVDTSSSTAYMQLSSLTSEDSAVYYCTRGYYDYWGQGTTLTVSS |
| 560 | C94 VL | DIVMTQSPSSLSVSAGEKVTMSCKSSQSLLNSGNQKNYLAWYQQKPGQPPKLLIYGAST RESGVPDRFTGSGSGTDFTLTISSVQAEDLAVYYCQNDHSYPHTFGAGTKLELR |
| 561 | C95 VH | EVQLQQSGPVLVKPGASVKMSCKASGNTFNGYYINWVKQRHGKSLEWIGLIDPSNGGT NYNQKFKDKATLTVDKSSTTAYMEFNSLTSDDSSVYYCAGGTVYWGQGTTLTVSS |
| 554 | C95 VL | DIQMTQSPSSLSASLGERVSLTCRASQDIGNNLNWLQQEPDGTIKRLIYATSSLDSGVPKR FSGSRSGSDYSLTISSLESEDFVDYYCLQYASSPPTFGGGTKLEIK |
| 562 | C96 VH | EVQLQQSGPELVKPGASVKISCKASGYMFTDYYMNWVKQSHGKSLEWIGEINPNNGDT NYSQKFRDKATLTVDKSSNIAFMELRSLTSEDSAVYFCAKEGVSSYYAMDYWGQGTSV TVSS |
| 563 | C96 VL | DIVMTQSPSSLAMSVGQKVTMSCKSSQSLLNSNNQKNFLAWYQQKPGQSPKLLVYFAS TRESGVPDRFIGGGSGTDFTLTISNVQPEDLADYFCQQHYSTPPTFGGGTKLEIK |
| 564 | C97 VH | EVQLQQSGPELVKPGTSVKISCKASGYTFTDYYMNWVRQSHEKSLEWIGEINPKNGDTN YNQNFKAKATLTVDKSSNTAYMELRSLTSEDSAVFYCAREGVSSYYGMDSWGQGTSV TVSS |
| 565 | C97 VL | DIVMTQSPSSLAVSVGQKVTMSCKSSQSLLNGFNQKNFLAWYQQKPGQSPKLLVYFAS TRESGVPDRFIGSGSGTDFTLTITSVQAEDLADYFCQQHHSTPPTFGGGTKLEIN |
| 566 | C98 VH | EVQLQQSGPELVKPGASVKMSCKASGYTFTDYSMNWVKQSHGKSLEWIGDINPNNGGT NYNQKFKGKATLTVDRSSSTAYMELRSLTSEDSAVYYCARVWFAYWGQGTLVTVSA |
| 567 | C98 VL | DIVMTQSPSSLSVSEGEKVTMSCKSSQSLLSGNQKNFLAWYQQKAGQPPKLLIYGAST RESGVPDRFTGSGSGTDFTLTISSVQAEDLAVYYCQNAHSSPFTFGSGTKLEIK |
| 568 | C99 VH | EVQLQQSGPELVKPGASVKISCKASGYTFTDYSMNWVKQSHGKSLEWIGDINPDNGGTS YNQKFKGKATLTVDQSSSTAYMELRSLTSEDSAVYYCARIWFAYWGQGTLVTVSA |
| 569 | C99 VL | DIVMTQSPSSLSVSAGEKVTMSCKSSQSLLNSGIQKNFLAWYQQKPGQPPKLLIYGASTR ESGVPDRFTGSGSGTDFTLTISSVQAEDLAVYYCQNAHSYPFTFGSGTKLEIK |
| 570 | C100 VH | EVQLQQSGPELVKPGASVKISCKASGYTFTDYYMNWVRQSHEKSLEWIGEINPNNGDT NFNQKFKGKATLTVDKSSSTAFMDLRSLTSKDSAVYYCAREGGSSYYAMDYWGQGTS VTVSS |
| 571 | C100 VL | DIVMTQSPSSLAMSVGQKVTMSCKSSQSLLSNNNQKNFLAWYQQKPGQSPKLLVYFAS TRESGVPDRFIGSGSGTDFTLTISSVQAEDLADYFCQQHYSTPPTFGGGTKVEIK |
| 572 | C101 VH | EVQLQQSGPELVKPGSSVKISCEASGFTFADYNVDWVKQSHGKSLEWIGAINPNDGGTS YNQKFEGKATLTVDKSSTTAYMELRSLTSEDSAVYYCARWVNNDWYFDVWGTGTTVT VSS |
| 573 | C101 VL | DIVMTQSQKFMSTSVGDRVSITCKASQNVRSAVDWYQQKPGQSPKTLIHSASNRHTGVP DRFTGSGSGTDFTLTISNIQSEDLADYFCLQHWNYPYTFGGGTKLEVK |
| 574 | C102 VH | QVQLQQSGAELARPGASVKLSCKASGYTFTNYGISWVKQRTGQGLEWIGEIYPGTINIY YSERFKGKATLTADKSSSTAYMQLTSLTSEDSAVYFCARDDGYYDAMDYWGQGTSVT VSS |
| 575 | C102 VL | DIQMTQSPSSLSASLGQKVILTCRASQDIGSSLNWLQQEPDGTIKRLIYATSSLDSGVPKR FSGSRSGSDYSLTISSLESEDFVVYYCLQYTSSPWTFGGGTKLEIK |
| 576 | C103 VH | QVQLQQPGAELVKPGASVKLSCKASGYTFTSYWMHWVKQRPGQGLEWIGEIDPSDSYT NYNQKFKGKATLTVDKSSSTAYMQLSSLTSEDSAVYYCASSIYYSNYVRYYFDYWGQG TTLTVSS |
| 577 | C103-2 VH | EVQLQQSGPELVKPGSSVKISCEASGYTFADYNVDWVKQSHGKSLEWIGAINPNDGGTS YNQKFEGKATLTVDKSSTTAYMELRSLTSEDSAVYYCARWVINDWYFDVWGTGTTVT VSS |
| 578 | C103 VL and C103-2 VL | DIQMTQSPSSLSASLGGKVTITCKASQDINKYIAWYQHKPGKGPRLLIHYTSTLQPGIPSR FSGSGSGRDYSFSISNLEPEDIATYYCLQYDNLWTFGGGTKLEIK |
| 579 | C104 VH | EVQLQQSGPELVRPGSSVKISCKASGYTFTDYNMDWVKQSHGKSLEWIGANDPKNGGF SYNQRFKGKATLTVDKSSSTAYMELRSLTSEDSAVYYCARWDSNYWYFDVWGTGTTV TVSS |
| 580 | C104 VL | DIVMTQSQKFMSTSIGDRVSITYKASQDVRSAVAWYQQKPGQSPKALIYLASNRHTGVP DRFTGSGSGTDFTLTITNVQSEDLADYFCLQHWTYPYTFGGGTKLEIK |
| 581 | C105 VH | EVQLQQSGPELVKPGSSMKISCKASGYTFTDYSMDWVKQSHGKSLEWIGAINPNNGGTS YNQKFKDKATLTVDRSSSTAYMELRSLTSEDSAVYYCARWDSDYWYFDVWATGTTVT VSS |
| 582 | C105 VL | DIVMTQSQKFMSTSVGDRVSITCKASQNVRTAVDWYQQKPGQSPKALIYSASNRHTGV PDRFTGSGSGTDFTLTISNVQSEDLADYFCLQHWNYPYTFGGGTKLEIK |
| 583 | C106 VH | EVQLQQSGPELVKPGSSVKISCKASGYTFTDYNMDWVKQSHGQSLEYIGAVNPNTGDIS YTQKFKDKATLTVDKSSSTAYMELRSLTSEDSAVYYCARWSESWYFDVWGTGTTVTV SS |
| 584 | C106 VL | DIVMTQSQKLMSTSVGDRVSITCKASQNVRAAVDWYQQKPRQSPKALIFLASSRHTGVP DRFTGSGSGTDFTLTITNVQSEDLATYFCLQHWNYPYTFGGGTRVEIK |
| 585 | C107 VH | EVQLQQSGPELVKPGSSVKISCKASGYTFTDYNIDWVKQSHGKSLEWIGAIDPNNGGTN YNQKFKDKATLTVDKSSSTAYMELRSLTSEDSAVYYCTRWDSNDWYFDVWATGTTVT VSS |
| 586 | C107 VL | DIVMTQSQKFMSTSVGDRVSITCKASQNVRIAVDWYQQKPGQSPKALIFSSSNRHTGVP DRFTGSGSGSDFTLTIRNVQSEDLADYFCLQHWISPYTFGGGTKLEIR |
| 587 | C108 VH and C108-2 VH | EVQLQQSGPELAKPGSSVKISCKASGYTFTDYNIDWVKQSHGKSLEWIGAIDPNNDSTN YNQKFKGKATLTVDKSSSTAYMELRSLTSEDSAVYYCTRWDSDDWYFDVWATGTTVT VSS |
| 588 | C108 VL | DIVMTQSQKFMSTSVGDRVSITCKASQNVRSAVDWYQQKPGQSPKALIFSSSNRHTGVP DRFTGSGSGTDFTLTIRNVQSEDLADYFCLQHWNSPYTFGGGTKLEIR |
| 589 | C108-2 VL | DIQMTQSSSSFSVSRGDRVTITCKASEDIYNRLAWYQQKPGNAPRLLISGTTSLEIGFPSRF SGSGSGKDYTLSISSLQTEDVATYYCQQYWSTPWTFGGGTKLEIK |
| 590 | C110 VH | EVQLQQSGAELAKPGASVKLSCTVSGFNIKDTYIHWVKQRPEQGLEWIGGIDPAIGNTVS ASKFQDKATITADTSSSTAYMQLSSLTSGDTAVYYCARERVHSNTLYYAMDYWGQGTS VTVSS |
| 591 | C110 VL | DIVLTQSPASLAVSLGQRATISCRASKSVSISGYSYMHWYQQKPGQPPKLLIYLASNLES GVPARFSGSGSGTDFTLNIHPVEEEDAATYYCQHSRELPPTFGGGTKLEIK |
| 592 | C111 VH and C111-2 VH | QVQLQQSGPELARPGASVKLSCKASGYTLTSHGISWVKQRTGQGLEWIGEIYPGSESPY YSEKFKGKATLTADKSSNIAYMQLSSLTSEDSAVYFCARSDYSDYWAMDYWGQGTSV TVSS |
| 593 | C111 VL | DIVMTQSQKCMSTSVGDRVSITCKASQNVRSAVAWYQQKPGQSPKTLIYLASNRHTGV PDRFTGSGSGTDFTLTITNVQSEDLADYFCLQHWNYPWTFGGGTELEIK |
| 594 | C111-2 VL | EIVMTQSPATLSVSPGERATLSCRASQSVSSNLAWYQQKPGQAPRLLIYGASTRATGIPA RFSGSGSGTEFTLTISSLQSEDFAVYYCHQYNNWPRTFGQGTKVEIK |
| 595 | C112 VH and C112-2 VH | QVQLQQPGAELVKPGASVKLSCKASGYTFTSSWMHWVKQRPGQGLEWIGEINPSDGRT NYNEKFKSKATLTVDHSSSTAYMQLSSLTSEDSAVYYCASNADRAYYTMDYWGQGTS VTVSS |
| 596 | C112 VL | DIVMTQSPSSLAMSVGQKVTMSCKSSQSLLNSFNQRNFLAWYQQKPGQSPKLLVYFAS TRDSGVPDRFIGSGSGTDFTLTISSVQAEDLADYFCQQHYSTPPTFGGGTKLEIK |
| 594 | C112-2 VL | EIVMTQSPATLSVSPGERATLSCRASQSVSSNLAWYQQKPGQAPRLLIYGASTRATGIPA RFSGSGSGTEFTLTISSLQSEDFAVYYCHQYNNWPRTFGQGTKVEIK |
| 597 | C113 VH and C113-2 VH | EVQLQQSGPELVKPGSSVKISCKASGYTFTDYNLDWMKQSHGSSLEWIGAMSPNNGAT SYNQKFKGKATLTVDKSSSTAYMELRSLTSEDSAVYYCARWDSNSWYFDVWGTGTTV TVSS |
| 598 | C113 VL | DIVMTQSQKFMSTSVGDRVSITCKASQIVRTAVDWYQQKSGQSPKALIYSASTRHTGVP DRFTGSGSGTDFTLTIDNVQSEDLAEYFCLQHWNYPYTFGGGTKLEIK |
| 594 | C113-2 VL | EIVMTQSPATLSVSPGERATLSCRASQSVSSNLAWYQQKPGQAPRLLIYGASTRATGIPA RFSGSGSGTEFTLTISSLQSEDFAVYYCHQYNNWPRTFGQGTKVEIK |
| 599 | C114 VH | QVQLQQSGAELVRPGTSVKMSCKAAGYTFTNSWINWVKQRPGHGLEWIGDIYPGNTFP NYNEKFKGKATLTADTSSSTAYMQLSSLTSEDSAVYYCTGGYFDVWGTGTTVTVSS |
| 600 | C114 VL | DIVMTQSPSSLSVSAGEKVTMSCKSSQSLLNSGNQKNFLAWYQQKPGQPPKLLIFGAST RQSGVPDRFTGSGSGTDFTLTISSVQAEDLAVYYCQNDHSYPYTFGGGTKLEMK |
| 601 | C115 VH | EVQLQQSGAELVKPGASVKLSCTASGFNIEDTYIHWVKRRPEQGLEWIGGIDPAIGNTLY ASKFQDMATITADTSSSTAYMQLSSLTSGDTAVYYCARERVYSNTLYYAMDYWGQGT SVTVSS |
| 602 | C115 VL | DIVLTQSPASLVVSLGQRATISCRASKSVSISGYSYMHWYQQKPGQPPKLLIYLASNLES GVPARFSGSGSGTDFTLNIHPVEAEDAATYYCQHSRELPPTFGGGTNLEIK |
| 603 | C116 VH | EVKLVESGGGLVQPGGSLKLSCAVSGFTFSSYTMSWVRQTPEKRLELVAKISNSGGSTN YPDTVKGRFTLSRDNVKNTLYLQMSSLKSEDTAIYYCTRYSWDVYAMDYWGQGTSVT VSS |
| 604 | C116 VL | DIQMTQSPASLSASVGETVIITCRASENIYSYLAWYQQKQGKSPHLLVYNAETLVEGVPS RFSGSGSATQFSLKINSLQPEDFGTYYCQHHYGDPTFGGGTKLEIK |
| 605 | C117 VH | QVQLQQSGAELARPGASVKMSCKASGYTFTDYWMQWVKQRPGQGLEWIGAIFPGNGE TRDTQKFKGRATLTADRSSSTAYIQLSSLTSEDSAVYYCATFNNYLDYWGQGTTLTVAS |
| 606 | C117 VL | QIVLTQSPAIMSASPGEKVTMTCSASSSVSYMHWYQQKSGASPKRWIYDTSKLTSGVPA RFSGSGSGTSYSLTISNMEAEDAATYYCQQWISNPYTFGGGTKLEIK |
| 607 | C118 VH | QVQLQQPGAELVKPGTSVKLSCKAAGYIFTSSWMHWVKQRPGQGLEWIGEINPSDGRT NYNEKFKNKATLTVDHSSSTAYMQLSSLTSEDSAVYYCASNADRAYYTMDYWGQGTS VTVSS |
| 608 | C118 VL | DIVMTQSPSSLAMSVGQKVTMSCKSSQSLLNGFNQRNFLAWYQQKPGQSPKLLVYFAS TRDSGVPDRFIGSGSGTDFTLTISSVQAEDLADYFCQQHYSTPPTFGGGTKLEIK |
| 609 | C119 VH | EVKLVESGGGLVQPGGSLKLSCVASGFTFNTYIMSWVRQTPEKRLELVADISNSGGNTY YSDTVKGRFTISRDNAKNTLYLQMSSLKSEDTAMYYCARPDNWDPFVYWGQGTLVTV SA |
| 610 | C119 VL | DIQMTQSSSSFSVSLGDRVTITCKASEDIYARLAWYQQKPGNAPRLLISGATSLETGVPSR FSGSGSGKNYTLSITSLQTEDVGTYYCQQHWSTPWTFGGGTNLEIK |
| 611 | C120 VH | EVQLQQSGPEVVKPGASVKISCKASGNTLTDYFINWVKQSHGKNLEWIGDINPDNGAG NYNQKFKGKATLTVDKSSGTAFMGLRSLTSEDSAVYYCATLYFDYWGQGTTLTVSS |
| 612 | C120 VL | DIVMTQSPSSLSVSTGEKVTMSCRSSQSLLNSGNQKNYLAWYQQKPGQPPKLLIYGAST RESGVPDRFTGSGSGTDFTLTISSVQAEDLAIYYCQNAHTSPYTFGGGTRLEIK |
| 613 | C121 VH | EVQLQQSGPEVVKPGASVKISCKASGNTFTDYFINWVKQSHGKSLEWIGDINPDNGGTN YNQKFKGKATLTVDKSSGTAFMGLRSLTSEDSAVYYCATLYFDYWGQGTSLTVSS |
| 614 | C121 VL | DIVMTQSPSSLSVSAGEKVTMSCKSSQSLLNSGNQKNYLAWYQQKPGQPPKLLIYGAST RESGVPDRFTGSGSGTDFTLTISSVQAEDLAVYYCQNAHSSPYTFGGGTRLEIK |
| 615 | C122 VH | EVQLQQSGPEVVKPGASVKISCKASGNTFTDYFINWVKQRHGKSLEWIGDVNPDNGGT NYNQKFKGKATLTVDKSSGTAFMGLRSLTSEDSAVYYCATLFFDSWGQGTTLTVSS |
| 616 | C122 VL | DIVMTQSPSSLNVSAGEKVTMSCKSSQSLLNSGNQKNYLAWYQQKPGQPPKLLIYGAST RESGVPDRFTGSGSGTDFTLTISSVQAEDLALYYCQNAHSSPYTFGGGTKLEIK |
| 617 | C123 VH | EVQLVESGGGLVKPGGSLKLSCAASGFTFSNFAMSWVRQTPAMRLESVATIVNGGINTY YPDTVKGRFTVSRDNAKNTLYLQMSSLRSEDTAIYYCARHDDFLYYAMDYWGQGTSV TVSS |
| 618 | C123 VL | QIVLTQSPAIMSASPGEKVTMTCSASSSVSYMFWYQQKPGSSPRLLIYDTSNLASGVPVR FSGSGSGTSYSLTISRLEAEDAATYYCQQWTSYPLTFGAGTKLDLK |
| 619 | C124 VH | EVQLVESGGGLVKPGGSLKLSCAASGFTFSSFAMSWVRQTPAKRLESVATINNGGINTY YPDTVKGRFTVSRDNAKNTLYLQMSSLRSEDTAMYYCARHDDFLYYAMDYWGQGTS VTVSS |
| 620 | C124 VL | QIVLTQSPAIMSASPGEKVTMTCSASSSVSYMYWYQQKPGSSPRLLIYDTSNLASGVPVR FSGSGSGTSYSLTISRMEAEDAATYYCQQWTSYPLTFGAGTKLELK |
| 621 | C125 VH | EVKLVESGGGLVQPGGSLKLSCAASGFTFSSYTMSWVRQTPEKRLELVAELSNSGGSFH YPDNVKGRFTISRDNAKNTLYLQMSSLKSEDTAMYYCARHNWDVYAMDYWGQGTSV TVSS |
| 622 | C125 VL | DIQMSQSPASLSASVGETVTITCRASENVNSYLAWYQQKQGRSPQFLVYNAQTLAEGVP SRFSGGGSGTQFSLKINSLQPEDFGSYYCQHHYGNPTFGGGTKLEIK |
| 623 | C129 VH | QVQLQQSGAELMTPGSSVKLSCKATGYTFTDYWIEWVKKRPGHGLEWIGEILPGYISTN YNEKFKDKATFTADTSSNTTYMQLSSLTSEDSAVYYCVLIVTTYYYFMDYWGQGTSVT VSS |
| 624 | C129 VL | QIVLTQSPAIMSASPGEKVTITCSASSSVTYMHWFQQKPGTSPKLWIYSTSNLASGVPVR FSGSGSGTSYSLTISRMEAEDAATYYCQQRSSYPWTFGGGTKLEIK |
| 625 | C132 VH | QVQLQQSGAELMKPGSSVNLSCKATDYTFTDYWINWVKQRPGHGLEWIGEILPGSVTT NYNEKFRGKATFTADTSSNTAYMQLSSLTSEDSAVYFCARGSDYSYYAMDYWGQGTS VTVSS |
| 626 | C132 VL | DIVMTQSPSSLAMSVGQKVTMSCKSSQSLLNIFNQKNFLAWYQQKPGQSPKLLVYFAST RESGVPDRFIGSGSGTDFTLTISNVQAEDLADYFCQQHYSTPPTFGGGTKLEIK |
| 627 | C136 VH | DVQLVESGGGLVKPGGSLKLSCAASGFSFSENYMYWVRQTPEKRLEWVATISDGASYT YYPDNVKGRFTISRDTAKNNLYLQMSHLKSEDTAMYYCTRDELIAPFAYWGQGTLVTV SA |
| 628 | C136 VL | DIVMTQSHKFMSTSVGDRVSITCKASQDVGTAVAWFQQKPGQSPKLLIYWASTRHTGV PDRFTGSGSGTDFTLTISNVQSEDLADYFCQQYNSYPWTFGGGTKLEIK |
| 629 | C138 VH | EVQLVESGGGLVKPGGSLKLSCAASGFTFSSYAMSWVRQTPAKRLEWVATITSGDIRTY YPDTVKGRFTISRDNAKNTLYLQMRSLRSEDTAMYYCVGHDDFHFYAMDYWGQGTSV TVSS |
| 630 | C138 VL | QIVLTQSPAIMSASPGEKVTMTCSASSSVSYLYWYQQKPGSSPRLLIYDTSNLASGVPVR FSGSGSGTSYSLTLSRMEAEDAATYYCQQWSSYPLTFGAGTKLELK |
| 631 | C140 VH | QVQLQQSGAELARPGTSVKMSCKASGYIFTNYWMNWVQQRPGQGLEWIGAINPSNGYI GYNQKFKDKATLTADKSSNTAYMQLRSLTSEDSAIYYCTRGEDFDGYAMDYWGQGTS VTVSS |
| 632 | C140 VL | DIQMTQSPSSLSASLGGKVTITCKASQDINKYIAWYQHKPGKGPRLLIHYTSTLQPGIPSR FSGSGSGRDYSFSISNLEPEDIATYYCLQYDNLLTFGAGTKLELK |
| 617 | C143 VH | EVQLVESGGGLVKPGGSLKLSCAASGFTFSNFAMSWVRQTPAMRLESVATIVNGGINTY YPDTVKGRFTVSRDNAKNTLYLQMSSLRSEDTAIYYCARHDDFLYYAMDYWGQGTSV TVSS |
| 633 | C143 VL | QIVLTQSPAIMSASPGEKVTMTCSASSSVNYMFWYQQKPGSSPRLLIYDTSNLASGVPVR FSGSGSGTSYSLTISRLEAEDAATYYCQQWTSYPLTFGAGTKLDLK |
| 634 | C145 VH | EVQLQQSGPEVVKPGASVRISCKASGNTFTDFFINWVKQSHGKSLEWIGDVNPDSNGAN YNQKFKGKATLTVDKSSGTAFMGLRSLTSEDSAVYYCATLFFDNWGQGTTLTVSS |
| 635 | C145 VL | DIVMTQSPSSLNVSAGEKVTMSCKSSQSLLNSGNQKNYLAWYQQKPGQPPKLLIYGAST RESGVPDRFTGSGSGTDFILTISSVQAEDLALYYCQNAHSSPYTFGGGTKLEIK |
| 636 | C146 VH | EVQLQQSGPELVKPGSSVKISCEASGYTFADYNVDWVKQSHGKSLEWIGAINPNDGGTS YNQKFEGKATLTVDKSSTTVYMELRSLTSEDSAVYYCARWVNNDWYFDVWGTGTTVT VSS |
| 637 | C146 VL | DIVMTQSQKFMSTSVGDRVSITCKASQNVRSAVDWYQQKPGQSPKTLIHSASSRHTGVP DRFTGSGSGTDFTLTISNIQSEDLADYFCLQHWNYPYTFGGGTKLEVK |
| 638 | C147 VH | EVQLQQSGPELLKPGSSVNISCKASGYTFTDYNIDWVKQSRGKSLEWIGAINPNNGGTFY NQKFKGQATLTVYKSSSTAYMELRSLTSEDSAVYYCVRWDSDSWYFDVWGTGTAVTV SS |
| 639 | C147 VL | DIVMTQSQKFMSTSVGERVSITCKASENVRAAVDWYQQKPGQSPKALIYLASNRHTGIP DRFTGSGSGTDFTLIISNVQSEDLADYFCLQHWNYPYTFGGGTKLEIT |
| 640 | C148 VH | EVQLQQSGPELAKPGSSVKISCKASGYTFTDYNIDWVKESHGKSLEWIGAIDPNNGGTN YNQKFKGKATLTVDKSSSTAYMELRSLTSEDSAVYYCTRWDSNDWYFDVWATGTTVT VSS |
| 641 | C148 VL | DIVMTQSQKFMSTSVGDRVSITCKASQNVRSAVDWYQQKPGQSPKALIFSSSNRHTGVP DRFAGSGSGTDFNLTIRNVQSEDLADYFCLQHWNSPYTFGGGTKLEIR |
| 642 | C149 VH | QVTLKESGPGILQPSQTLSLTCSFSGFSLSTFGLGIGWIRQPSGKGLEWLAHIWWDDDKY YNPALKSRLTISKDTSKNQVFLKIANVDTADTATYYCATGTYWFTYWGQGTLVTVSA |
| 643 | C149 VL | DVLMTQTPLSLPVSLGDQASISCRSSQSIEHSNGNTYLQWYLQKPGQSPKLLIYKVSNRF SGVPDRFSGSGSGTDFTLKISRVEAEDLGVYYCFQGSHVPWTFGGGTKLEIK |
| 644 | C150 VH | EVQLQQSGPVLVKPGASVKMSCKASGYRLTDYYMHWVKQSHGKSLEWIGIINPYNGDT SYNQKFKGKATLTVDKSSSTAYMELDGLTSDDSAVYYCARDSSNVGNAMDYWGQGTS VTVSS |
| 645 | C150 VL | DIQMTQTTSSLSASLGDRITISCRASQDISNYLNWYLQKPDGTIKLLIYYSSRLHSGVPSRF SGSGSGTDYSLTITNLEQEDIATYFCQQGHALPRTFGGGTKLEIK |
| 646 | C151 VH | EVQLQQSGPELVKPGASVKISCKGSGYTFTDYFMNWVKQSHGKSLEWVGDINPNNGDT TYSQKFMGKATLTVDKSSSTAYMELRSLTSEDSAVYHCAASGPPYVLLDYWGQGTSVT VSS |
| 647 | C151 VL | DIQMTQSPASLSASVGETVTITCRASENIYSYLAWYQQKQGKSPQLLVYNAKTLTEGVP TRFSGSGSGTQFSLKIDSLKPEDFGTYYCQHHYGTPLTFGAGTKLELN |
| 648 | C152 VH | QVQLQQSGTILARPGASVKMSCRASDYTFTDYWIQWIKQRPGQGLEWIGAIFPENGDTR YTQKFKGRATLTADKSSSTAYMQVNSLTSEDSAVYYCATFINYVDYWGQGTTLTVSS |
| 649 | C152 VL | QIVLIQSPAIMSASPGEKVTMTCSASSSVNNIHWYQQKSGTSPKRWIYDTTKLTSGVPAR FSGSGSGTSYSLTISSMEAEDAATYYCQQWNINPYTFGGGTKLEIK |
| 650 | C153 VH | EVQLQQSGPELVKPGSLVKISCKTSGYTFTDYNMDWVKQSHGKSLEWIGAIDPKNGATS YNQKFKGKATLTVDKSSSTAYMELRSLTSEDSAVYYCARWDSNYWYFDVWGTGTTVT VSS |
| 651 | C153 VL | DIVMTQSQKIMSTSVGDRVSMTCKASQNIRSAVAWYQQKPGQSPKALIYLTSNRHTGVP DRFTGSGSGTDFTLAISDLQSEDLADYFCLQHWNYPYTFGGGTELEIK |
| 652 | C154VH | EVQLVESGGGLVKPGGSLRLSCAASGFTFSDYYMYWVRQAPGKGLEWVSTISDAGGYT YYPDSVKGRFTISRDNAKNSLYLQMNSLRAEDTAVYYCAREDLWGQGTLVTVSS |
| 653 | C154VL | DIQMTQSPSSLSASVGDRVTITCRASQDIGYSLNWFQQKPGKAPKRLIYATSRLDSGVPK RFSGSGSGTDFTLTISSLQPEDFATYYCLQYASFPYTFGGGTKLEIK |
| 654 | C155VH | EVQLVESGGGLVKPGGSLRLSCAASGFTFSDYYMYWVRQAPGKGLEWVATISDAGGYT YYPDSVKGRFTISRDNAKNSLYLQMNSLRAEDTAVYYCGREDLWGQGTLVTVSS |
| 655 | C155VL | DIQMTQSPSSLSASVGDRVTITCRASQDIGYSLNWFQQKPGKAPRRLIYATSRLDSGVPKR FSGSGSGTDFTLTISSLQPEDFATYYCLQYASFPYTFGGGTKLEIK |
| 656 | C156VH | EVQLVESGGGLVKPGGSLRLSCAASGFTFSDYYMYWVRQAPGKGLEWVATISDAGGYT YYPDSVKGRFTISRDNAKNSLYLQMNSLRAEDTAVYFCGREDLWGQGTLVTVSS |
| 653 | C156VL | DIQMTQSPSSLSASVGDRVTITCRASQDIGYSLNWFQQKPGKAPKRLIYATSRLDSGVPK RFSGSGSGTDFTLTISSLQPEDFATYYCLQYASFPYTFGGGTKLEIK |

## Claims

1. An anti-CD112R antibody or antigen-binding fragment thereof that specifically binds to CD112R, comprising:
1) three heavy chain CDRs (HCDR1, HCDR2, HCDR3) comprised in the heavy chain variable region as set forth in SEQ ID NO: 430 and three light chain CDRs (LCDR1, LCDR2, LCDR3) comprised in the light chain variable region as set forth in SEQ ID NO: 431;
2) three heavy chain CDRs (HCDR1, HCDR2, HCDR3) comprised in the heavy chain variable region as set forth in SEQ ID NO: 432 and three light chain CDRs (LCDR1, LCDR2, LCDR3) comprised in the light chain variable region as set forth in SEQ ID NO: 433;
3) three heavy chain CDRs (HCDR1, HCDR2, HCDR3) comprised in the heavy chain variable region as set forth in SEQ ID NO: 434 and three light chain CDRs (LCDR1, LCDR2, LCDR3) comprised in the light chain variable region as set forth in SEQ ID NO: 435;
4) three heavy chain CDRs (HCDR1, HCDR2, HCDR3) comprised in the heavy chain variable region as set forth in SEQ ID NO: 436 and three light chain CDRs (LCDR1, LCDR2, LCDR3) comprised in the light chain variable region as set forth in SEQ ID NO: 437;
5) three heavy chain CDRs (HCDR1, HCDR2, HCDR3) comprised in the heavy chain variable region as set forth in SEQ ID NO: 438 and three light chain CDRs (LCDR1, LCDR2, LCDR3) comprised in the light chain variable region as set forth in SEQ ID NO: 439;
6) three heavy chain CDRs (HCDR1, HCDR2, HCDR3) comprised in the heavy chain variable region as set forth in SEQ ID NO: 438 and three light chain CDRs (LCDR1, LCDR2, LCDR3) comprised in the light chain variable region as set forth in SEQ ID NO: 440;
7) three heavy chain CDRs (HCDR1, HCDR2, HCDR3) comprised in the heavy chain variable region as set forth in SEQ ID NO: 441 and three light chain CDRs (LCDR1, LCDR2, LCDR3) comprised in the light chain variable region as set forth in SEQ ID NO: 442;
8) three heavy chain CDRs (HCDR1, HCDR2, HCDR3) comprised in the heavy chain variable region as set forth in SEQ ID NO: 443 and three light chain CDRs (LCDR1, LCDR2, LCDR3) comprised in the light chain variable region as set forth in SEQ ID NO: 444;
9) three heavy chain CDRs (HCDR1, HCDR2, HCDR3) comprised in the heavy chain variable region as set forth in SEQ ID NO: 445 and three light chain CDRs (LCDR1, LCDR2, LCDR3) comprised in the light chain variable region as set forth in SEQ ID NO: 446;
10) three heavy chain CDRs (HCDR1, HCDR2, HCDR3) comprised in the heavy chain variable region as set forth in SEQ ID NO: 447 and three light chain CDRs (LCDR1, LCDR2, LCDR3) comprised in the light chain variable region as set forth in SEQ ID NO: 448;
11) three heavy chain CDRs (HCDR1, HCDR2, HCDR3) comprised in the heavy chain variable region as set forth in SEQ ID NO: 449 and three light chain CDRs (LCDR1, LCDR2, LCDR3) comprised in the light chain variable region as set forth in SEQ ID NO: 450;
12) three heavy chain CDRs (HCDR1, HCDR2, HCDR3) comprised in the heavy chain variable region as set forth in SEQ ID NO: 451 and three light chain CDRs (LCDR1, LCDR2, LCDR3) comprised in the light chain variable region as set forth in SEQ ID NO: 452;
13) three heavy chain CDRs (HCDR1, HCDR2, HCDR3) comprised in the heavy chain variable region as set forth in SEQ ID NO: 453 and three light chain CDRs (LCDR1, LCDR2, LCDR3) comprised in the light chain variable region as set forth in SEQ ID NO: 454;
14) three heavy chain CDRs (HCDR1, HCDR2, HCDR3) comprised in the heavy chain variable region as set forth in SEQ ID NO: 455 and three light chain CDRs (LCDR1, LCDR2, LCDR3) comprised in the light chain variable region as set forth in SEQ ID NO: 456;
15) three heavy chain CDRs (HCDR1, HCDR2, HCDR3) comprised in the heavy chain variable region as set forth in SEQ ID NO: 457 and three light chain CDRs (LCDR1, LCDR2, LCDR3) comprised in the light chain variable region as set forth in SEQ ID NO: 458;
16) three heavy chain CDRs (HCDR1, HCDR2, HCDR3) comprised in the heavy chain variable region as set forth in SEQ ID NO: 459 and three light chain CDRs (LCDR1, LCDR2, LCDR3) comprised in the light chain variable region as set forth in SEQ ID NO: 460;
17) three heavy chain CDRs (HCDR1, HCDR2, HCDR3) comprised in the heavy chain variable region as set forth in SEQ ID NO: 461 and three light chain CDRs (LCDR1, LCDR2, LCDR3) comprised in the light chain variable region as set forth in SEQ ID NO: 462;
18) three heavy chain CDRs (HCDR1, HCDR2, HCDR3) comprised in the heavy chain variable region as set forth in SEQ ID NO: 463 and three light chain CDRs (LCDR1, LCDR2, LCDR3) comprised in the light chain variable region as set forth in SEQ ID NO: 464;
19) three heavy chain CDRs (HCDR1, HCDR2, HCDR3) comprised in the heavy chain variable region as set forth in SEQ ID NO: 465 and three light chain CDRs (LCDR1, LCDR2, LCDR3) comprised in the light chain variable region as set forth in SEQ ID NO: 466;
20) three heavy chain CDRs (HCDR1, HCDR2, HCDR3) comprised in the heavy chain variable region as set forth in SEQ ID NO: 467 and three light chain CDRs (LCDR1, LCDR2, LCDR3) comprised in the light chain variable region as set forth in SEQ ID NO: 468;
21) three heavy chain CDRs (HCDR1, HCDR2, HCDR3) comprised in the heavy chain variable region as set forth in SEQ ID NO: 469 and three light chain CDRs (LCDR1, LCDR2, LCDR3) comprised in the light chain variable region as set forth in SEQ ID NO: 470;
22) three heavy chain CDRs (HCDR1, HCDR2, HCDR3) comprised in the heavy chain variable region as set forth in SEQ ID NO: 471 and three light chain CDRs (LCDR1, LCDR2, LCDR3) comprised in the light chain variable region as set forth in SEQ ID NO: 472;
23) three heavy chain CDRs (HCDR1, HCDR2, HCDR3) comprised in the heavy chain variable region as set forth in SEQ ID NO: 473 and three light chain CDRs (LCDR1, LCDR2, LCDR3) comprised in the light chain variable region as set forth in SEQ ID NO: 474;
24) three heavy chain CDRs (HCDR1, HCDR2, HCDR3) comprised in the heavy chain variable region as set forth in SEQ ID NO: 475 and three light chain CDRs (LCDR1, LCDR2, LCDR3) comprised in the light chain variable region as set forth in SEQ ID NO: 476;
25) three heavy chain CDRs (HCDR1, HCDR2, HCDR3) comprised in the heavy chain variable region as set forth in SEQ ID NO: 477 and three light chain CDRs (LCDR1, LCDR2, LCDR3) comprised in the light chain variable region as set forth in SEQ ID NO: 478;
26) three heavy chain CDRs (HCDR1, HCDR2, HCDR3) comprised in the heavy chain variable region as set forth in SEQ ID NO: 479 and three light chain CDRs (LCDR1, LCDR2, LCDR3) comprised in the light chain variable region as set forth in SEQ ID NO: 480;
27) three heavy chain CDRs (HCDR1, HCDR2, HCDR3) comprised in the heavy chain variable region as set forth in SEQ ID NO: 481 and three light chain CDRs (LCDR1, LCDR2, LCDR3) comprised in the light chain variable region as set forth in SEQ ID NO: 482;
28) three heavy chain CDRs (HCDR1, HCDR2, HCDR3) comprised in the heavy chain variable region as set forth in SEQ ID NO: 483 and three light chain CDRs (LCDR1, LCDR2, LCDR3) comprised in the light chain variable region as set forth in SEQ ID NO: 484;
29) three heavy chain CDRs (HCDR1, HCDR2, HCDR3) comprised in the heavy chain variable region as set forth in SEQ ID NO: 485 and three light chain CDRs (LCDR1, LCDR2, LCDR3) comprised in the light chain variable region as set forth in SEQ ID NO: 486;
30) three heavy chain CDRs (HCDR1, HCDR2, HCDR3) comprised in the heavy chain variable region as set forth in SEQ ID NO: 487 and three light chain CDRs (LCDR1, LCDR2, LCDR3) comprised in the light chain variable region as set forth in SEQ ID NO: 456;
31) three heavy chain CDRs (HCDR1, HCDR2, HCDR3) comprised in the heavy chain variable region as set forth in SEQ ID NO: 488 and three light chain CDRs (LCDR1, LCDR2, LCDR3) comprised in the light chain variable region as set forth in SEQ ID NO: 489;
32) three heavy chain CDRs (HCDR1, HCDR2, HCDR3) comprised in the heavy chain variable region as set forth in SEQ ID NO: 490 and three light chain CDRs (LCDR1, LCDR2, LCDR3) comprised in the light chain variable region as set forth in SEQ ID NO: 491;
33) three heavy chain CDRs (HCDR1, HCDR2, HCDR3) comprised in the heavy chain variable region as set forth in SEQ ID NO: 492 and three light chain CDRs (LCDR1, LCDR2, LCDR3) comprised in the light chain variable region as set forth in SEQ ID NO: 489;
34) three heavy chain CDRs (HCDR1, HCDR2, HCDR3) comprised in the heavy chain variable region as set forth in SEQ ID NO: 493 and three light chain CDRs (LCDR1, LCDR2, LCDR3) comprised in the light chain variable region as set forth in SEQ ID NO: 494;
35) three heavy chain CDRs (HCDR1, HCDR2, HCDR3) comprised in the heavy chain variable region as set forth in SEQ ID NO: 493 and three light chain CDRs (LCDR1, LCDR2, LCDR3) comprised in the light chain variable region as set forth in SEQ ID NO: 495;
36) three heavy chain CDRs (HCDR1, HCDR2, HCDR3) comprised in the heavy chain variable region as set forth in SEQ ID NO: 496 and three light chain CDRs (LCDR1, LCDR2, LCDR3) comprised in the light chain variable region as set forth in SEQ ID NO: 497;
37) three heavy chain CDRs (HCDR1, HCDR2, HCDR3) comprised in the heavy chain variable region as set forth in SEQ ID NO: 498 and three light chain CDRs (LCDR1, LCDR2, LCDR3) comprised in the light chain variable region as set forth in SEQ ID NO: 497;
38) three heavy chain CDRs (HCDR1, HCDR2, HCDR3) comprised in the heavy chain variable region as set forth in SEQ ID NO: 499 and three light chain CDRs (LCDR1, LCDR2, LCDR3) comprised in the light chain variable region as set forth in SEQ ID NO: 500;
39) three heavy chain CDRs (HCDR1, HCDR2, HCDR3) comprised in the heavy chain variable region as set forth in SEQ ID NO: 501 and three light chain CDRs (LCDR1, LCDR2, LCDR3) comprised in the light chain variable region as set forth in SEQ ID NO: 503;
40) three heavy chain CDRs (HCDR1, HCDR2, HCDR3) comprised in the heavy chain variable region as set forth in SEQ ID NO: 502 and three light chain CDRs (LCDR1, LCDR2, LCDR3) comprised in the light chain variable region as set forth in SEQ ID NO: 503;
41) three heavy chain CDRs (HCDR1, HCDR2, HCDR3) comprised in the heavy chain variable region as set forth in SEQ ID NO: 504 and three light chain CDRs (LCDR1, LCDR2, LCDR3) comprised in the light chain variable region as set forth in SEQ ID NO: 505;
42) three heavy chain CDRs (HCDR1, HCDR2, HCDR3) comprised in the heavy chain variable region as set forth in SEQ ID NO: 499 and three light chain CDRs (LCDR1, LCDR2, LCDR3) comprised in the light chain variable region as set forth in SEQ ID NO: 506;
43) three heavy chain CDRs (HCDR1, HCDR2, HCDR3) comprised in the heavy chain variable region as set forth in SEQ ID NO: 507 and three light chain CDRs (LCDR1, LCDR2, LCDR3) comprised in the light chain variable region as set forth in SEQ ID NO: 503;
44) three heavy chain CDRs (HCDR1, HCDR2, HCDR3) comprised in the heavy chain variable region as set forth in SEQ ID NO: 508 and three light chain CDRs (LCDR1, LCDR2, LCDR3) comprised in the light chain variable region as set forth in SEQ ID NO: 509;
45) three heavy chain CDRs (HCDR1, HCDR2, HCDR3) comprised in the heavy chain variable region as set forth in SEQ ID NO: 510 and three light chain CDRs (LCDR1, LCDR2, LCDR3) comprised in the light chain variable region as set forth in SEQ ID NO: 511;
46) three heavy chain CDRs (HCDR1, HCDR2, HCDR3) comprised in the heavy chain variable region as set forth in SEQ ID NO: 512 and three light chain CDRs (LCDR1, LCDR2, LCDR3) comprised in the light chain variable region as set forth in SEQ ID NO: 513;
47) three heavy chain CDRs (HCDR1, HCDR2, HCDR3) comprised in the heavy chain variable region as set forth in SEQ ID NO: 514 and three light chain CDRs (LCDR1, LCDR2, LCDR3) comprised in the light chain variable region as set forth in SEQ ID NO: 515;
48) three heavy chain CDRs (HCDR1, HCDR2, HCDR3) comprised in the heavy chain variable region as set forth in SEQ ID NO: 501 and three light chain CDRs (LCDR1, LCDR2, LCDR3) comprised in the light chain variable region as set forth in SEQ ID NO: 516;
49) three heavy chain CDRs (HCDR1, HCDR2, HCDR3) comprised in the heavy chain variable region as set forth in SEQ ID NO: 517 and three light chain CDRs (LCDR1, LCDR2, LCDR3) comprised in the light chain variable region as set forth in SEQ ID NO: 518;
50) three heavy chain CDRs (HCDR1, HCDR2, HCDR3) comprised in the heavy chain variable region as set forth in SEQ ID NO: 519 and three light chain CDRs (LCDR1, LCDR2, LCDR3) comprised in the light chain variable region as set forth in SEQ ID NO: 520;
51) three heavy chain CDRs (HCDR1, HCDR2, HCDR3) comprised in the heavy chain variable region as set forth in SEQ ID NO: 521 and three light chain CDRs (LCDR1, LCDR2, LCDR3) comprised in the light chain variable region as set forth in SEQ ID NO: 522;
52) three heavy chain CDRs (HCDR1, HCDR2, HCDR3) comprised in the heavy chain variable region as set forth in SEQ ID NO: 523 and three light chain CDRs (LCDR1, LCDR2, LCDR3) comprised in the light chain variable region as set forth in SEQ ID NO: 524;
53) three heavy chain CDRs (HCDR1, HCDR2, HCDR3) comprised in the heavy chain variable region as set forth in SEQ ID NO: 525 and three light chain CDRs (LCDR1, LCDR2, LCDR3) comprised in the light chain variable region as set forth in SEQ ID NO: 486;
54) three heavy chain CDRs (HCDR1, HCDR2, HCDR3) comprised in the heavy chain variable region as set forth in SEQ ID NO: 526 and three light chain CDRs (LCDR1, LCDR2, LCDR3) comprised in the light chain variable region as set forth in SEQ ID NO: 527;
55) three heavy chain CDRs (HCDR1, HCDR2, HCDR3) comprised in the heavy chain variable region as set forth in SEQ ID NO: 528 and three light chain CDRs (LCDR1, LCDR2, LCDR3) comprised in the light chain variable region as set forth in SEQ ID NO: 529;
56) three heavy chain CDRs (HCDR1, HCDR2, HCDR3) comprised in the heavy chain variable region as set forth in SEQ ID NO: 530 and three light chain CDRs (LCDR1, LCDR2, LCDR3) comprised in the light chain variable region as set forth in SEQ ID NO: 531;
57) three heavy chain CDRs (HCDR1, HCDR2, HCDR3) comprised in the heavy chain variable region as set forth in SEQ ID NO: 530 and three light chain CDRs (LCDR1, LCDR2, LCDR3) comprised in the light chain variable region as set forth in SEQ ID NO: 533;
58) three heavy chain CDRs (HCDR1, HCDR2, HCDR3) comprised in the heavy chain variable region as set forth in SEQ ID NO: 532 and three light chain CDRs (LCDR1, LCDR2, LCDR3) comprised in the light chain variable region as set forth in SEQ ID NO: 531;
59) three heavy chain CDRs (HCDR1, HCDR2, HCDR3) comprised in the heavy chain variable region as set forth in SEQ ID NO: 532 and three light chain CDRs (LCDR1, LCDR2, LCDR3) comprised in the light chain variable region as set forth in SEQ ID NO: 533;
60) three heavy chain CDRs (HCDR1, HCDR2, HCDR3) comprised in the heavy chain variable region as set forth in SEQ ID NO: 534 and three light chain CDRs (LCDR1, LCDR2, LCDR3) comprised in the light chain variable region as set forth in SEQ ID NO: 535;
61) three heavy chain CDRs (HCDR1, HCDR2, HCDR3) comprised in the heavy chain variable region as set forth in SEQ ID NO: 536 and three light chain CDRs (LCDR1, LCDR2, LCDR3) comprised in the light chain variable region as set forth in SEQ ID NO: 537;
62) three heavy chain CDRs (HCDR1, HCDR2, HCDR3) comprised in the heavy chain variable region as set forth in SEQ ID NO: 538 and three light chain CDRs (LCDR1, LCDR2, LCDR3) comprised in the light chain variable region as set forth in SEQ ID NO: 539;
63) three heavy chain CDRs (HCDR1, HCDR2, HCDR3) comprised in the heavy chain variable region as set forth in SEQ ID NO: 540 and three light chain CDRs (LCDR1, LCDR2, LCDR3) comprised in the light chain variable region as set forth in SEQ ID NO: 541;
64) three heavy chain CDRs (HCDR1, HCDR2, HCDR3) comprised in the heavy chain variable region as set forth in SEQ ID NO: 540 and three light chain CDRs (LCDR1, LCDR2, LCDR3) comprised in the light chain variable region as set forth in SEQ ID NO: 542;
65) three heavy chain CDRs (HCDR1, HCDR2, HCDR3) comprised in the heavy chain variable region as set forth in SEQ ID NO: 543 and three light chain CDRs (LCDR1, LCDR2, LCDR3) comprised in the light chain variable region as set forth in SEQ ID NO: 544;
66) three heavy chain CDRs (HCDR1, HCDR2, HCDR3) comprised in the heavy chain variable region as set forth in SEQ ID NO: 545 and three light chain CDRs (LCDR1, LCDR2, LCDR3) comprised in the light chain variable region as set forth in SEQ ID NO: 546;
67) three heavy chain CDRs (HCDR1, HCDR2, HCDR3) comprised in the heavy chain variable region as set forth in SEQ ID NO: 547 and three light chain CDRs (LCDR1, LCDR2, LCDR3) comprised in the light chain variable region as set forth in SEQ ID NO: 548;
68) three heavy chain CDRs (HCDR1, HCDR2, HCDR3) comprised in the heavy chain variable region as set forth in SEQ ID NO: 549 and three light chain CDRs (LCDR1, LCDR2, LCDR3) comprised in the light chain variable region as set forth in SEQ ID NO: 550;
69) three heavy chain CDRs (HCDR1, HCDR2, HCDR3) comprised in the heavy chain variable region as set forth in SEQ ID NO: 551 and three light chain CDRs (LCDR1, LCDR2, LCDR3) comprised in the light chain variable region as set forth in SEQ ID NO: 552;
70) three heavy chain CDRs (HCDR1, HCDR2, HCDR3) comprised in the heavy chain variable region as set forth in SEQ ID NO: 553 and three light chain CDRs (LCDR1, LCDR2, LCDR3) comprised in the light chain variable region as set forth in SEQ ID NO: 554;
71) three heavy chain CDRs (HCDR1, HCDR2, HCDR3) comprised in the heavy chain variable region as set forth in SEQ ID NO: 555 and three light chain CDRs (LCDR1, LCDR2, LCDR3) comprised in the light chain variable region as set forth in SEQ ID NO: 556;
72) three heavy chain CDRs (HCDR1, HCDR2, HCDR3) comprised in the heavy chain variable region as set forth in SEQ ID NO: 557 and three light chain CDRs (LCDR1, LCDR2, LCDR3) comprised in the light chain variable region as set forth in SEQ ID NO: 558;
73) three heavy chain CDRs (HCDR1, HCDR2, HCDR3) comprised in the heavy chain variable region as set forth in SEQ ID NO: 559 and three light chain CDRs (LCDR1, LCDR2, LCDR3) comprised in the light chain variable region as set forth in SEQ ID NO: 560;
74) three heavy chain CDRs (HCDR1, HCDR2, HCDR3) comprised in the heavy chain variable region as set forth in SEQ ID NO: 561 and three light chain CDRs (LCDR1, LCDR2, LCDR3) comprised in the light chain variable region as set forth in SEQ ID NO: 554;
75) three heavy chain CDRs (HCDR1, HCDR2, HCDR3) comprised in the heavy chain variable region as set forth in SEQ ID NO: 562 and three light chain CDRs (LCDR1, LCDR2, LCDR3) comprised in the light chain variable region as set forth in SEQ ID NO: 563;
76) three heavy chain CDRs (HCDR1, HCDR2, HCDR3) comprised in the heavy chain variable region as set forth in SEQ ID NO: 564 and three light chain CDRs (LCDR1, LCDR2, LCDR3) comprised in the light chain variable region as set forth in SEQ ID NO: 565;
77) three heavy chain CDRs (HCDR1, HCDR2, HCDR3) comprised in the heavy chain variable region as set forth in SEQ ID NO: 566 and three light chain CDRs (LCDR1, LCDR2, LCDR3) comprised in the light chain variable region as set forth in SEQ ID NO: 567;
78) three heavy chain CDRs (HCDR1, HCDR2, HCDR3) comprised in the heavy chain variable region as set forth in SEQ ID NO: 568 and three light chain CDRs (LCDR1, LCDR2, LCDR3) comprised in the light chain variable region as set forth in SEQ ID NO: 569;
79) three heavy chain CDRs (HCDR1, HCDR2, HCDR3) comprised in the heavy chain variable region as set forth in SEQ ID NO: 570 and three light chain CDRs (LCDR1, LCDR2, LCDR3) comprised in the light chain variable region as set forth in SEQ ID NO: 571;
80) three heavy chain CDRs (HCDR1, HCDR2, HCDR3) comprised in the heavy chain variable region as set forth in SEQ ID NO: 572 and three light chain CDRs (LCDR1, LCDR2, LCDR3) comprised in the light chain variable region as set forth in SEQ ID NO: 573;
81) three heavy chain CDRs (HCDR1, HCDR2, HCDR3) comprised in the heavy chain variable region as set forth in SEQ ID NO: 574 and three light chain CDRs (LCDR1, LCDR2, LCDR3) comprised in the light chain variable region as set forth in SEQ ID NO: 575;
82) three heavy chain CDRs (HCDR1, HCDR2, HCDR3) comprised in the heavy chain variable region as set forth in SEQ ID NO: 576 and three light chain CDRs (LCDR1, LCDR2, LCDR3) comprised in the light chain variable region as set forth in SEQ ID NO: 578;
83) three heavy chain CDRs (HCDR1, HCDR2, HCDR3) comprised in the heavy chain variable region as set forth in SEQ ID NO: 577 and three light chain CDRs (LCDR1, LCDR2, LCDR3) comprised in the light chain variable region as set forth in SEQ ID NO: 578;
84) three heavy chain CDRs (HCDR1, HCDR2, HCDR3) comprised in the heavy chain variable region as set forth in SEQ ID NO: 579 and three light chain CDRs (LCDR1, LCDR2, LCDR3) comprised in the light chain variable region as set forth in SEQ ID NO: 580;
85) three heavy chain CDRs (HCDR1, HCDR2, HCDR3) comprised in the heavy chain variable region as set forth in SEQ ID NO: 581 and three light chain CDRs (LCDR1, LCDR2, LCDR3) comprised in the light chain variable region as set forth in SEQ ID NO: 582;
86) three heavy chain CDRs (HCDR1, HCDR2, HCDR3) comprised in the heavy chain variable region as set forth in SEQ ID NO: 583 and three light chain CDRs (LCDR1, LCDR2, LCDR3) comprised in the light chain variable region as set forth in SEQ ID NO: 584;
87) three heavy chain CDRs (HCDR1, HCDR2, HCDR3) comprised in the heavy chain variable region as set forth in SEQ ID NO: 585 and three light chain CDRs (LCDR1, LCDR2, LCDR3) comprised in the light chain variable region as set forth in SEQ ID NO: 586;
88) three heavy chain CDRs (HCDR1, HCDR2, HCDR3) comprised in the heavy chain variable region as set forth in SEQ ID NO: 587 and three light chain CDRs (LCDR1, LCDR2, LCDR3) comprised in the light chain variable region as set forth in SEQ ID NO: 588;
89) three heavy chain CDRs (HCDR1, HCDR2, HCDR3) comprised in the heavy chain variable region as set forth in SEQ ID NO: 587 and three light chain CDRs (LCDR1, LCDR2, LCDR3) comprised in the light chain variable region as set forth in SEQ ID NO: 589;
90) three heavy chain CDRs (HCDR1, HCDR2, HCDR3) comprised in the heavy chain variable region as set forth in SEQ ID NO: 590 and three light chain CDRs (LCDR1, LCDR2, LCDR3) comprised in the light chain variable region as set forth in SEQ ID NO: 591;
91) three heavy chain CDRs (HCDR1, HCDR2, HCDR3) comprised in the heavy chain variable region as set forth in SEQ ID NO: 592 and three light chain CDRs (LCDR1, LCDR2, LCDR3) comprised in the light chain variable region as set forth in SEQ ID NO: 593;
92) three heavy chain CDRs (HCDR1, HCDR2, HCDR3) comprised in the heavy chain variable region as set forth in SEQ ID NO: 592 and three light chain CDRs (LCDR1, LCDR2, LCDR3) comprised in the light chain variable region as set forth in SEQ ID NO: 594;
93) three heavy chain CDRs (HCDR1, HCDR2, HCDR3) comprised in the heavy chain variable region as set forth in SEQ ID NO: 595 and three light chain CDRs (LCDR1, LCDR2, LCDR3) comprised in the light chain variable region as set forth in SEQ ID NO: 596;
94) three heavy chain CDRs (HCDR1, HCDR2, HCDR3) comprised in the heavy chain variable region as set forth in SEQ ID NO: 595 and three light chain CDRs (LCDR1, LCDR2, LCDR3) comprised in the light chain variable region as set forth in SEQ ID NO: 594;
95) three heavy chain CDRs (HCDR1, HCDR2, HCDR3) comprised in the heavy chain variable region as set forth in SEQ ID NO: 597 and three light chain CDRs (LCDR1, LCDR2, LCDR3) comprised in the light chain variable region as set forth in SEQ ID NO: 598;
96) three heavy chain CDRs (HCDR1, HCDR2, HCDR3) comprised in the heavy chain variable region as set forth in SEQ ID NO: 597 and three light chain CDRs (LCDR1, LCDR2, LCDR3) comprised in the light chain variable region as set forth in SEQ ID NO: 594;
97) three heavy chain CDRs (HCDR1, HCDR2, HCDR3) comprised in the heavy chain variable region as set forth in SEQ ID NO: 599 and three light chain CDRs (LCDR1, LCDR2, LCDR3) comprised in the light chain variable region as set forth in SEQ ID NO: 600;
98) three heavy chain CDRs (HCDR1, HCDR2, HCDR3) comprised in the heavy chain variable region as set forth in SEQ ID NO: 601 and three light chain CDRs (LCDR1, LCDR2, LCDR3) comprised in the light chain variable region as set forth in SEQ ID NO: 602;
99) three heavy chain CDRs (HCDR1, HCDR2, HCDR3) comprised in the heavy chain variable region as set forth in SEQ ID NO: 603 and three light chain CDRs (LCDR1, LCDR2, LCDR3) comprised in the light chain variable region as set forth in SEQ ID NO: 604;
100) three heavy chain CDRs (HCDR1, HCDR2, HCDR3) comprised in the heavy chain variable region as set forth in SEQ ID NO: 605 and three light chain CDRs (LCDR1, LCDR2, LCDR3) comprised in the light chain variable region as set forth in SEQ ID NO: 606;
101) three heavy chain CDRs (HCDR1, HCDR2, HCDR3) comprised in the heavy chain variable region as set forth in SEQ ID NO: 607 and three light chain CDRs (LCDR1, LCDR2, LCDR3) comprised in the light chain variable region as set forth in SEQ ID NO: 608;
102) three heavy chain CDRs (HCDR1, HCDR2, HCDR3) comprised in the heavy chain variable region as set forth in SEQ ID NO: 609 and three light chain CDRs (LCDR1, LCDR2, LCDR3) comprised in the light chain variable region as set forth in SEQ ID NO: 610;
103) three heavy chain CDRs (HCDR1, HCDR2, HCDR3) comprised in the heavy chain variable region as set forth in SEQ ID NO: 611 and three light chain CDRs (LCDR1, LCDR2, LCDR3) comprised in the light chain variable region as set forth in SEQ ID NO: 612;
104) three heavy chain CDRs (HCDR1, HCDR2, HCDR3) comprised in the heavy chain variable region as set forth in SEQ ID NO: 613 and three light chain CDRs (LCDR1, LCDR2, LCDR3) comprised in the light chain variable region as set forth in SEQ ID NO: 614;
105) three heavy chain CDRs (HCDR1, HCDR2, HCDR3) comprised in the heavy chain variable region as set forth in SEQ ID NO: 615 and three light chain CDRs (LCDR1, LCDR2, LCDR3) comprised in the light chain variable region as set forth in SEQ ID NO: 616;
106) three heavy chain CDRs (HCDR1, HCDR2, HCDR3) comprised in the heavy chain variable region as set forth in SEQ ID NO: 617 and three light chain CDRs (LCDR1, LCDR2, LCDR3) comprised in the light chain variable region as set forth in SEQ ID NO: 618;
107) three heavy chain CDRs (HCDR1, HCDR2, HCDR3) comprised in the heavy chain variable region as set forth in SEQ ID NO: 619 and three light chain CDRs (LCDR1, LCDR2, LCDR3) comprised in the light chain variable region as set forth in SEQ ID NO: 620;
108) three heavy chain CDRs (HCDR1, HCDR2, HCDR3) comprised in the heavy chain variable region as set forth in SEQ ID NO: 621 and three light chain CDRs (LCDR1, LCDR2, LCDR3) comprised in the light chain variable region as set forth in SEQ ID NO: 622;
109) three heavy chain CDRs (HCDR1, HCDR2, HCDR3) comprised in the heavy chain variable region as set forth in SEQ ID NO: 623 and three light chain CDRs (LCDR1, LCDR2, LCDR3) comprised in the light chain variable region as set forth in SEQ ID NO: 624;
110) three heavy chain CDRs (HCDR1, HCDR2, HCDR3) comprised in the heavy chain variable region as set forth in SEQ ID NO: 625 and three light chain CDRs (LCDR1, LCDR2, LCDR3) comprised in the light chain variable region as set forth in SEQ ID NO: 626;
111) three heavy chain CDRs (HCDR1, HCDR2, HCDR3) comprised in the heavy chain variable region as set forth in SEQ ID NO: 627 and three light chain CDRs (LCDR1, LCDR2, LCDR3) comprised in the light chain variable region as set forth in SEQ ID NO: 628;
112) three heavy chain CDRs (HCDR1, HCDR2, HCDR3) comprised in the heavy chain variable region as set forth in SEQ ID NO: 629 and three light chain CDRs (LCDR1, LCDR2, LCDR3) comprised in the light chain variable region as set forth in SEQ ID NO: 630;
113) three heavy chain CDRs (HCDR1, HCDR2, HCDR3) comprised in the heavy chain variable region as set forth in SEQ ID NO: 631 and three light chain CDRs (LCDR1, LCDR2, LCDR3) comprised in the light chain variable region as set forth in SEQ ID NO: 632;
114) three heavy chain CDRs (HCDR1, HCDR2, HCDR3) comprised in the heavy chain variable region as set forth in SEQ ID NO: 617 and three light chain CDRs (LCDR1, LCDR2, LCDR3) comprised in the light chain variable region as set forth in SEQ ID NO: 633;
115) three heavy chain CDRs (HCDR1, HCDR2, HCDR3) comprised in the heavy chain variable region as set forth in SEQ ID NO: 634 and three light chain CDRs (LCDR1, LCDR2, LCDR3) comprised in the light chain variable region as set forth in SEQ ID NO: 635;
116) three heavy chain CDRs (HCDR1, HCDR2, HCDR3) comprised in the heavy chain variable region as set forth in SEQ ID NO: 636 and three light chain CDRs (LCDR1, LCDR2, LCDR3) comprised in the light chain variable region as set forth in SEQ ID NO: 637;
117) three heavy chain CDRs (HCDR1, HCDR2, HCDR3) comprised in the heavy chain variable region as set forth in SEQ ID NO: 638 and three light chain CDRs (LCDR1, LCDR2, LCDR3) comprised in the light chain variable region as set forth in SEQ ID NO: 639;
118) three heavy chain CDRs (HCDR1, HCDR2, HCDR3) comprised in the heavy chain variable region as set forth in SEQ ID NO: 640 and three light chain CDRs (LCDR1, LCDR2, LCDR3) comprised in the light chain variable region as set forth in SEQ ID NO: 641;
119) three heavy chain CDRs (HCDR1, HCDR2, HCDR3) comprised in the heavy chain variable region as set forth in SEQ ID NO: 642 and three light chain CDRs (LCDR1, LCDR2, LCDR3) comprised in the light chain variable region as set forth in SEQ ID NO: 643;
120) three heavy chain CDRs (HCDR1, HCDR2, HCDR3) comprised in the heavy chain variable region as set forth in SEQ ID NO: 644 and three light chain CDRs (LCDR1, LCDR2, LCDR3) comprised in the light chain variable region as set forth in SEQ ID NO: 645;
121) three heavy chain CDRs (HCDR1, HCDR2, HCDR3) comprised in the heavy chain variable region as set forth in SEQ ID NO: 646 and three light chain CDRs (LCDR1, LCDR2, LCDR3) comprised in the light chain variable region as set forth in SEQ ID NO: 647;
122) three heavy chain CDRs (HCDR1, HCDR2, HCDR3) comprised in the heavy chain variable region as set forth in SEQ ID NO: 648 and three light chain CDRs (LCDR1, LCDR2, LCDR3) comprised in the light chain variable region as set forth in SEQ ID NO: 649;
123) three heavy chain CDRs (HCDR1, HCDR2, HCDR3) comprised in the heavy chain variable region as set forth in SEQ ID NO: 650 and three light chain CDRs (LCDR1, LCDR2, LCDR3) comprised in the light chain variable region as set forth in SEQ ID NO: 651;
124) three heavy chain CDRs (HCDR1, HCDR2, HCDR3) comprised in the heavy chain variable region as set forth in SEQ ID NO: 652 and three light chain CDRs (LCDR1, LCDR2, LCDR3) comprised in the light chain variable region as set forth in SEQ ID NO: 653;
125) three heavy chain CDRs (HCDR1, HCDR2, HCDR3) comprised in the heavy chain variable region as set forth in SEQ ID NO: 654 and three light chain CDRs (LCDR1, LCDR2, LCDR3) comprised in the light chain variable region as set forth in SEQ ID NO: 655; or
126) 3 heavy chain CDRs (HCDR1, HCDR2, HCDR3) included in the heavy chain variable region shown in SEQ ID NO: 656 and three light chain CDRs (LCDR1, LCDR2, LCDR3) included in the light chain variable region shown in SEQ ID NO: 653.

2. An anti-CD112R antibody or antigen-binding fragment thereof that specifically binds to CD112R, comprising:
1) HCDR1, HCDR2 and HCDR3 respectively comprising the sequences as set forth in SEQ ID NOs: 1, 64 and 162, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NOs: 239, 307 and 357, or a sequence containing an amino acid substitution (e.g., conservative substitution), deletion or insertion of one or more and no more than 3 amino acids relative to the sequence;
2) HCDR1, HCDR2 and HCDR3 respectively comprising the sequences as set forth in SEQ ID NOs: 2, 65 and 162, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NOs: 239, 308 and 358, or a sequence containing an amino acid substitution (e.g., conservative substitution), deletion or insertion of one or more and no more than 3 amino acids relative to the sequence;
3) HCDR1, HCDR2 and HCDR3 respectively comprising the sequences as set forth in SEQ ID NO: 3, 66, 163, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 240, 309, 359, or a sequence containing an amino acid substitution (e.g., conservative substitution), deletion or insertion of one or more and no more than 3 amino acids relative to the sequence;
4) HCDR1, HCDR2 and HCDR3 respectively comprising the sequences as set forth in SEQ ID NO: 4, 67, 164, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 239, 310, 360, or a sequence containing an amino acid substitution (e.g., conservative substitution), deletion or insertion of one or more and no more than 3 amino acids relative to the sequence;
5) HCDR1, HCDR2 and HCDR3 respectively comprising the sequences as set forth in SEQ ID NO: 5, 68, 165, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 361, or a sequence containing an amino acid substitution (e.g., conservative substitution), deletion or insertion of one or more and no more than 3 amino acids relative to the sequence;
6) HCDR1, HCDR2 and HCDR3 respectively comprising the sequences as set forth in SEQ ID NO: 5, 68, 165, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 242, 312, 362, or a sequence containing an amino acid substitution (e.g., conservative substitution), deletion or insertion of one or more and no more than 3 amino acids relative to the sequence;
7) HCDR1, HCDR2 and HCDR3 respectively comprising the sequences as set forth in SEQ ID NO: 6, 69, 166, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 243, 313, 363, or a sequence containing an amino acid substitution (e.g., conservative substitution), deletion or insertion of one or more and no more than 3 amino acids relative to the sequence;
8) HCDR1, HCDR2 and HCDR3 respectively comprising the sequences as set forth in SEQ ID NO: 7, 70, 167, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 244, 314, 364, or a sequence containing an amino acid substitution (e.g., conservative substitution), deletion or insertion of one or more and no more than 3 amino acids relative to the sequence;
9) HCDR1, HCDR2 and HCDR3 respectively comprising the sequences as set forth in SEQ ID NO: 8, 71, 168, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 245, 313, 365, or a sequence containing an amino acid substitution (e.g., conservative substitution), deletion or insertion of one or more and no more than 3 amino acids relative to the sequence;
10) HCDR1, HCDR2 and HCDR3 respectively comprising the sequences as set forth in SEQ ID NO: 3, 72, 163, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 359, or a sequence containing an amino acid substitution (e.g., conservative substitution), deletion or insertion of one or more and no more than 3 amino acids relative to the sequence;
11) HCDR1, HCDR2 and HCDR3 respectively comprising the sequences as set forth in SEQ ID NO: 9, 73, 168, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 245, 313, 363, or a sequence containing an amino acid substitution (e.g., conservative substitution), deletion or insertion of one or more and no more than 3 amino acids relative to the sequence;
12) HCDR1, HCDR2 and HCDR3 respectively comprising the sequences as set forth in SEQ ID NO: 3, 74, 163, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 246, 311, 359, or a sequence containing an amino acid substitution (e.g., conservative substitution), deletion or insertion of one or more and no more than 3 amino acids relative to the sequence;
13) HCDR1, HCDR2 and HCDR3 respectively comprising the sequences as set forth in SEQ ID NO: 10, 75, 169, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 247, 315, 366, or a sequence containing an amino acid substitution (e.g., conservative substitution), deletion or insertion of one or more and no more than 3 amino acids relative to the sequence;
14) HCDR1, HCDR2 and HCDR3 respectively comprising the sequences as set forth in SEQ ID NO: 10, 75, 170, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 247, 315, 366, or a sequence containing an amino acid substitution (e.g., conservative substitution), deletion or insertion of one or more and no more than 3 amino acids relative to the sequence;
15) HCDR1, HCDR2 and HCDR3 respectively comprising the sequences as set forth in SEQ ID NO: 9, 76, 168, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 243, 313, 363, or a sequence containing an amino acid substitution (e.g., conservative substitution), deletion or insertion of one or more and no more than 3 amino acids relative to the sequence;
16) HCDR1, HCDR2 and HCDR3 respectively comprising the sequences as set forth in SEQ ID NO: 9, 77, 168, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 248, 313, 363, or a sequence containing an amino acid substitution (e.g., conservative substitution), deletion or insertion of one or more and no more than 3 amino acids relative to the sequence;
17) HCDR1, HCDR2 and HCDR3 respectively comprising the sequences as set forth in SEQ ID NO: 8, 77, 168, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 243, 313, 365, or a sequence containing an amino acid substitution (e.g., conservative substitution), deletion or insertion of one or more and no more than 3 amino acids relative to the sequence;
18) HCDR1, HCDR2 and HCDR3 respectively comprising the sequences as set forth in SEQ ID NO: 7, 78, 167, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 244, 314, 364, or a sequence containing an amino acid substitution (e.g., conservative substitution), deletion or insertion of one or more and no more than 3 amino acids relative to the sequence;
19) HCDR1, HCDR2 and HCDR3 respectively comprising the sequences as set forth in SEQ ID NO: 11, 79, 171, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 249, 316, 367, or a sequence containing an amino acid substitution (e.g., conservative substitution), deletion or insertion of one or more and no more than 3 amino acids relative to the sequence;
20) HCDR1, HCDR2 and HCDR3 respectively comprising the sequences as set forth in SEQ ID NO: 2, 80, 162, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 239, 317, 368, or a sequence containing an amino acid substitution (e.g., conservative substitution), deletion or insertion of one or more and no more than 3 amino acids relative to the sequence;
21) HCDR1, HCDR2 and HCDR3 respectively comprising the sequences as set forth in SEQ ID NO: 12, 81, 172, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 250, 315, 369, or a sequence containing an amino acid substitution (e.g., conservative substitution), deletion or insertion of one or more and no more than 3 amino acids relative to the sequence;
22) HCDR1, HCDR2 and HCDR3 respectively comprising the sequences as set forth in SEQ ID NO: 10, 82, 169, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 247, 315, 370, or a sequence containing an amino acid substitution (e.g., conservative substitution), deletion or insertion of one or more and no more than 3 amino acids relative to the sequence;
23) HCDR1, HCDR2 and HCDR3 respectively comprising the sequences as set forth in SEQ ID NO: 2, 83, 173, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 242, 312, 371, or a sequence containing an amino acid substitution (e.g., conservative substitution), deletion or insertion of one or more and no more than 3 amino acids relative to the sequence;
24) HCDR1, HCDR2 and HCDR3 respectively comprising the sequences as set forth in SEQ ID NO: 13, 84, 173, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 242, 312, 372, or a sequence containing an amino acid substitution (e.g., conservative substitution), deletion or insertion of one or more and no more than 3 amino acids relative to the sequence;
25) HCDR1, HCDR2 and HCDR3 respectively comprising the sequences as set forth in SEQ ID NO: 2, 85, 174, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 251, 318, 373, or a sequence containing an amino acid substitution (e.g., conservative substitution), deletion or insertion of one or more and no more than 3 amino acids relative to the sequence;
26) HCDR1, HCDR2 and HCDR3 respectively comprising the sequences as set forth in SEQ ID NO: 14, 86, 175, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 252, 319, 374, or a sequence containing an amino acid substitution (e.g., conservative substitution), deletion or insertion of one or more and no more than 3 amino acids relative to the sequence;
27) HCDR1, HCDR2 and HCDR3 respectively comprising the sequences as set forth in SEQ ID NO: 15, 87, 176, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 253, 320, 375, or a sequence containing an amino acid substitution (e.g., conservative substitution), deletion or insertion of one or more and no more than 3 amino acids relative to the sequence;
28) HCDR1, HCDR2 and HCDR3 respectively comprising the sequences as set forth in SEQ ID NO: 16, 88, 177, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 254, 321, 376, or a sequence containing an amino acid substitution (e.g., conservative substitution), deletion or insertion of one or more and no more than 3 amino acids relative to the sequence;
29) HCDR1, HCDR2 and HCDR3 respectively comprising the sequences as set forth in SEQ ID NO: 2, 89, 178, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 377, or a sequence containing an amino acid substitution (e.g., conservative substitution), deletion or insertion of one or more and no more than 3 amino acids relative to the sequence;
30) HCDR1, HCDR2 and HCDR3 respectively comprising the sequences as set forth in SEQ ID NO: 17, 75, 169, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 247, 315, 366, or a sequence containing an amino acid substitution (e.g., conservative substitution), deletion or insertion of one or more and no more than 3 amino acids relative to the sequence;
31) HCDR1, HCDR2 and HCDR3 respectively comprising the sequences as set forth in SEQ ID NO: 18, 85, 162, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 239, 322, 378, or a sequence containing an amino acid substitution (e.g., conservative substitution), deletion or insertion of one or more and no more than 3 amino acids relative to the sequence;
32) HCDR1, HCDR2 and HCDR3 respectively comprising the sequences as set forth in SEQ ID NO: 2, 90, 162, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 255, 308, 368, or a sequence containing an amino acid substitution (e.g., conservative substitution), deletion or insertion of one or more and no more than 3 amino acids relative to the sequence;
33) HCDR1, HCDR2 and HCDR3 respectively comprising the sequences as set forth in SEQ ID NO: 19, 91, 179, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 239, 322, 378, or a sequence containing an amino acid substitution (e.g., conservative substitution), deletion or insertion of one or more and no more than 3 amino acids relative to the sequence;
34) HCDR1, HCDR2 and HCDR3 respectively comprising the sequences as set forth in SEQ ID NO: 2, 85, 180, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 256, 323, 379, or a sequence containing an amino acid substitution (e.g., conservative substitution), deletion or insertion of one or more and no more than 3 amino acids relative to the sequence;
35) HCDR1, HCDR2 and HCDR3 respectively comprising the sequences as set forth in SEQ ID NO: 2, 85, 180, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 239, 308, 380, or a sequence containing an amino acid substitution (e.g., conservative substitution), deletion or insertion of one or more and no more than 3 amino acids relative to the sequence;
36) HCDR1, HCDR2 and HCDR3 respectively comprising the sequences as set forth in SEQ ID NO: 20, 92, 181, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 257, 324, 381, or a sequence containing an amino acid substitution (e.g., conservative substitution), deletion or insertion of one or more and no more than 3 amino acids relative to the sequence;
37) HCDR1, HCDR2 and HCDR3 respectively comprising the sequences as set forth in SEQ ID NO: 21, 93, 182, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 258, 325, 382, or a sequence containing an amino acid substitution (e.g., conservative substitution), deletion or insertion of one or more and no more than 3 amino acids relative to the sequence;
38) HCDR1, HCDR2 and HCDR3 respectively comprising the sequences as set forth in SEQ ID NO: 22, 94, 183, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 259, 323, 383, or a sequence containing an amino acid substitution (e.g., conservative substitution), deletion or insertion of one or more and no more than 3 amino acids relative to the sequence;
39) HCDR1, HCDR2 and HCDR3 respectively comprising the sequences as set forth in SEQ ID NO: 23, 94, 183, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 259, 323, 383, or a sequence containing an amino acid substitution (e.g., conservative substitution), deletion or insertion of one or more and no more than 3 amino acids relative to the sequence;
40) HCDR1, HCDR2 and HCDR3 respectively comprising the sequences as set forth in SEQ ID NO: 24, 95, 184, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 247, 326, 384, or a sequence containing an amino acid substitution (e.g., conservative substitution), deletion or insertion of one or more and no more than 3 amino acids relative to the sequence;
41) HCDR1, HCDR2 and HCDR3 respectively comprising the sequences as set forth in SEQ ID NO: 21, 93, 182, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 247, 326, 385, or a sequence containing an amino acid substitution (e.g., conservative substitution), deletion or insertion of one or more and no more than 3 amino acids relative to the sequence;
42) HCDR1, HCDR2 and HCDR3 respectively comprising the sequences as set forth in SEQ ID NO: 22, 94, 183, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 259, 323, 383, or a sequence containing an amino acid substitution (e.g., conservative substitution), deletion or insertion of one or more and no more than 3 amino acids relative to the sequence;
43) HCDR1, HCDR2 and HCDR3 respectively comprising the sequences as set forth in SEQ ID NO: 25, 96, 185, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 260, 327, 386, or a sequence containing an amino acid substitution (e.g., conservative substitution), deletion or insertion of one or more and no more than 3 amino acids relative to the sequence;
44) HCDR1, HCDR2 and HCDR3 respectively comprising the sequences as set forth in SEQ ID NO: 26, 97, 186, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 261, 328, 387, or a sequence containing an amino acid substitution (e.g., conservative substitution), deletion or insertion of one or more and no more than 3 amino acids relative to the sequence;
45) HCDR1, HCDR2 and HCDR3 respectively comprising the sequences as set forth in SEQ ID NO: 27, 98, 187, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 262, 328, 388, or a sequence containing an amino acid substitution (e.g., conservative substitution), deletion or insertion of one or more and no more than 3 amino acids relative to the sequence;
46) HCDR1, HCDR2 and HCDR3 respectively comprising the sequences as set forth in SEQ ID NO: 28, 99, 188, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 263, 328, 388, or a sequence containing an amino acid substitution (e.g., conservative substitution), deletion or insertion of one or more and no more than 3 amino acids relative to the sequence;
47) HCDR1, HCDR2 and HCDR3 respectively comprising the sequences as set forth in SEQ ID NO: 22, 94, 183, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 239, 308, 389, or a sequence containing an amino acid substitution (e.g., conservative substitution), deletion or insertion of one or more and no more than 3 amino acids relative to the sequence;
48) HCDR1, HCDR2 and HCDR3 respectively comprising the sequences as set forth in SEQ ID NO: 5, 100, 189, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 264, 329, 390, or a sequence containing an amino acid substitution (e.g., conservative substitution), deletion or insertion of one or more and no more than 3 amino acids relative to the sequence;
49) HCDR1, HCDR2 and HCDR3 respectively comprising the sequences as set forth in SEQ ID NO: 24, 101, 190, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 247, 326, 385, or a sequence containing an amino acid substitution (e.g., conservative substitution), deletion or insertion of one or more and no more than 3 amino acids relative to the sequence;
50) HCDR1, HCDR2 and HCDR3 respectively comprising the sequences as set forth in SEQ ID NO: 28, 102, 191, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 265, 328, 391, or a sequence containing an amino acid substitution (e.g., conservative substitution), deletion or insertion of one or more and no more than 3 amino acids relative to the sequence;
51) HCDR1, HCDR2 and HCDR3 respectively comprising the sequences as set forth in SEQ ID NO: 28, 103, 192, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 266, 328, 388, or a sequence containing an amino acid substitution (e.g., conservative substitution), deletion or insertion of one or more and no more than 3 amino acids relative to the sequence;
52) HCDR1, HCDR2 and HCDR3 respectively comprising the sequences as set forth in SEQ ID NO: 29, 104, 163, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 377, or a sequence containing an amino acid substitution (e.g., conservative substitution), deletion or insertion of one or more and no more than 3 amino acids relative to the sequence;
53) HCDR1, HCDR2 and HCDR3 respectively comprising the sequences as set forth in SEQ ID NO: 30, 105, 193, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 267, 330, 392, or a sequence containing an amino acid substitution (e.g., conservative substitution), deletion or insertion of one or more and no more than 3 amino acids relative to the sequence;
54) HCDR1, HCDR2 and HCDR3 respectively comprising the sequences as set forth in SEQ ID NO: 5, 106, 194, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 268, 331, 393, or a sequence containing an amino acid substitution (e.g., conservative substitution), deletion or insertion of one or more and no more than 3 amino acids relative to the sequence;
55) HCDR1, HCDR2 and HCDR3 respectively comprising the sequences as set forth in SEQ ID NO: 31, 107, 195, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 256, 318, 392, or a sequence containing an amino acid substitution (e.g., conservative substitution), deletion or insertion of one or more and no more than 3 amino acids relative to the sequence;
56) HCDR1, HCDR2 and HCDR3 respectively comprising the sequences as set forth in SEQ ID NO: 31, 107, 195, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 269, 332, 394, or a sequence containing an amino acid substitution (e.g., conservative substitution), deletion or insertion of one or more and no more than 3 amino acids relative to the sequence;
57) HCDR1, HCDR2 and HCDR3 respectively comprising the sequences as set forth in SEQ ID NO: 32, 108, 196, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 256, 318, 392, or a sequence containing an amino acid substitution (e.g., conservative substitution), deletion or insertion of one or more and no more than 3 amino acids relative to the sequence;
58) HCDR1, HCDR2 and HCDR3 respectively comprising the sequences as set forth in SEQ ID NO: 32, 108, 196, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 269, 332, 394, or a sequence containing an amino acid substitution (e.g., conservative substitution), deletion or insertion of one or more and no more than 3 amino acids relative to the sequence;
59) HCDR1, HCDR2 and HCDR3 respectively comprising the sequences as set forth in SEQ ID NO: 29, 109, 197, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 395, or a sequence containing an amino acid substitution (e.g., conservative substitution), deletion or insertion of one or more and no more than 3 amino acids relative to the sequence;
60) HCDR1, HCDR2 and HCDR3 respectively comprising the sequences as set forth in SEQ ID NO: 33, 110, 198, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 270, 333, 396, or a sequence containing an amino acid substitution (e.g., conservative substitution), deletion or insertion of one or more and no more than 3 amino acids relative to the sequence;
61) HCDR1, HCDR2 and HCDR3 respectively comprising the sequences as set forth in SEQ ID NO: 5, 111, 199, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 256, 318, 397, or a sequence containing an amino acid substitution (e.g., conservative substitution), deletion or insertion of one or more and no more than 3 amino acids relative to the sequence;
62) HCDR1, HCDR2 and HCDR3 respectively comprising the sequences as set forth in SEQ ID NO: 34, 112, 200, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 269, 334, 398, or a sequence containing an amino acid substitution (e.g., conservative substitution), deletion or insertion of one or more and no more than 3 amino acids relative to the sequence;
63) HCDR1, HCDR2 and HCDR3 respectively comprising the sequences as set forth in SEQ ID NO: 34, 112, 200, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 271, 335, 399, or a sequence containing an amino acid substitution (e.g., conservative substitution), deletion or insertion of one or more and no more than 3 amino acids relative to the sequence;
64) HCDR1, HCDR2 and HCDR3 respectively comprising the sequences as set forth in SEQ ID NO: 35, 113, 201, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 400, or a sequence containing an amino acid substitution (e.g., conservative substitution), deletion or insertion of one or more and no more than 3 amino acids relative to the sequence;
65) HCDR1, HCDR2 and HCDR3 respectively comprising the sequences as set forth in SEQ ID NO: 36, 114, 202, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 272, 336, 401, or a sequence containing an amino acid substitution (e.g., conservative substitution), deletion or insertion of one or more and no more than 3 amino acids relative to the sequence;
66) HCDR1, HCDR2 and HCDR3 respectively comprising the sequences as set forth in SEQ ID NO: 29, 115, 203, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 402, or a sequence containing an amino acid substitution (e.g., conservative substitution), deletion or insertion of one or more and no more than 3 amino acids relative to the sequence;
67) HCDR1, HCDR2 and HCDR3 respectively comprising the sequences as set forth in SEQ ID NO: 37, 116, 204, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 273, 337, 403, or a sequence containing an amino acid substitution (e.g., conservative substitution), deletion or insertion of one or more and no more than 3 amino acids relative to the sequence;
68) HCDR1, HCDR2 and HCDR3 respectively comprising the sequences as set forth in SEQ ID NO: 5, 117, 205, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 274, 338, 404, or a sequence containing an amino acid substitution (e.g., conservative substitution), deletion or insertion of one or more and no more than 3 amino acids relative to the sequence;
69) HCDR1, HCDR2 and HCDR3 respectively comprising the sequences as set forth in SEQ ID NO: 38, 110, 198, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 270, 333, 405, or a sequence containing an amino acid substitution (e.g., conservative substitution), deletion or insertion of one or more and no more than 3 amino acids relative to the sequence;
70) HCDR1, HCDR2 and HCDR3 respectively comprising the sequences as set forth in SEQ ID NO: 39, 118, 204, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 275, 337, 406, or a sequence containing an amino acid substitution (e.g., conservative substitution), deletion or insertion of one or more and no more than 3 amino acids relative to the sequence;
71) HCDR1, HCDR2 and HCDR3 respectively comprising the sequences as set forth in SEQ ID NO: 5, 119, 206, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 276, 338, 407, or a sequence containing an amino acid substitution (e.g., conservative substitution), deletion or insertion of one or more and no more than 3 amino acids relative to the sequence;
72) HCDR1, HCDR2 and HCDR3 respectively comprising the sequences as set forth in SEQ ID NO: 29, 109, 197, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 395, or a sequence containing an amino acid substitution (e.g., conservative substitution), deletion or insertion of one or more and no more than 3 amino acids relative to the sequence;
73) HCDR1, HCDR2 and HCDR3 respectively comprising the sequences as set forth in SEQ ID NO: 38, 110, 198, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 270, 333, 405, or a sequence containing an amino acid substitution (e.g., conservative substitution), deletion or insertion of one or more and no more than 3 amino acids relative to the sequence;
74) HCDR1, HCDR2 and HCDR3 respectively comprising the sequences as set forth in SEQ ID NO: 5, 120, 205, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 277, 338, 407, or a sequence containing an amino acid substitution (e.g., conservative substitution), deletion or insertion of one or more and no more than 3 amino acids relative to the sequence;
75) HCDR1, HCDR2 and HCDR3 respectively comprising the sequences as set forth in SEQ ID NO: 5, 121, 207, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 278, 338, 404, or a sequence containing an amino acid substitution (e.g., conservative substitution), deletion or insertion of one or more and no more than 3 amino acids relative to the sequence;
76) HCDR1, HCDR2 and HCDR3 respectively comprising the sequences as set forth in SEQ ID NO: 35, 122, 201, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 279, 311, 408, or a sequence containing an amino acid substitution (e.g., conservative substitution), deletion or insertion of one or more and no more than 3 amino acids relative to the sequence;
77) HCDR1, HCDR2 and HCDR3 respectively comprising the sequences as set forth in SEQ ID NO: 35, 123, 208, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 280, 311, 400, or a sequence containing an amino acid substitution (e.g., conservative substitution), deletion or insertion of one or more and no more than 3 amino acids relative to the sequence;
78) HCDR1, HCDR2 and HCDR3 respectively comprising the sequences as set forth in SEQ ID NO: 5, 124, 209, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 281, 338, 407, or a sequence containing an amino acid substitution (e.g., conservative substitution), deletion or insertion of one or more and no more than 3 amino acids relative to the sequence;
79) HCDR1, HCDR2 and HCDR3 respectively comprising the sequences as set forth in SEQ ID NO: 40, 125, 210, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 282, 339, 409, or a sequence containing an amino acid substitution (e.g., conservative substitution), deletion or insertion of one or more and no more than 3 amino acids relative to the sequence;
80) HCDR1, HCDR2 and HCDR3 respectively comprising the sequences as set forth in SEQ ID NO: 41, 126, 211, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 245, 333, 410, or a sequence containing an amino acid substitution (e.g., conservative substitution), deletion or insertion of one or more and no more than 3 amino acids relative to the sequence;
81) HCDR1, HCDR2 and HCDR3 respectively comprising the sequences as set forth in SEQ ID NO: 10, 127, 212, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 239, 308, 411, or a sequence containing an amino acid substitution (e.g., conservative substitution), deletion or insertion of one or more and no more than 3 amino acids relative to the sequence;
82) HCDR1, HCDR2 and HCDR3 respectively comprising the sequences as set forth in SEQ ID NO: 40, 125, 213, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 239, 308, 411, or a sequence containing an amino acid substitution (e.g., conservative substitution), deletion or insertion of one or more and no more than 3 amino acids relative to the sequence;
83) HCDR1, HCDR2 and HCDR3 respectively comprising the sequences as set forth in SEQ ID NO: 42, 128, 214, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 283, 324, 412, or a sequence containing an amino acid substitution (e.g., conservative substitution), deletion or insertion of one or more and no more than 3 amino acids relative to the sequence;
84) HCDR1, HCDR2 and HCDR3 respectively comprising the sequences as set forth in SEQ ID NO: 43, 129, 215, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 284, 339, 409, or a sequence containing an amino acid substitution (e.g., conservative substitution), deletion or insertion of one or more and no more than 3 amino acids relative to the sequence;
85) HCDR1, HCDR2 and HCDR3 respectively comprising the sequences as set forth in SEQ ID NO: 42, 130, 216, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 285, 340, 409, or a sequence containing an amino acid substitution (e.g., conservative substitution), deletion or insertion of one or more and no more than 3 amino acids relative to the sequence;
86) HCDR1, HCDR2 and HCDR3 respectively comprising the sequences as set forth in SEQ ID NO: 44, 131, 217, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 286, 341, 413, or a sequence containing an amino acid substitution (e.g., conservative substitution), deletion or insertion of one or more and no more than 3 amino acids relative to the sequence;
87) HCDR1, HCDR2 and HCDR3 respectively comprising the sequences as set forth in SEQ ID NO: 44, 132, 218, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 282, 341, 414, or a sequence containing an amino acid substitution (e.g., conservative substitution), deletion or insertion of one or more and no more than 3 amino acids relative to the sequence;
88) HCDR1, HCDR2 and HCDR3 respectively comprising the sequences as set forth in SEQ ID NO: 44, 132, 218, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 287, 342, 415, or a sequence containing an amino acid substitution (e.g., conservative substitution), deletion or insertion of one or more and no more than 3 amino acids relative to the sequence;
89) HCDR1, HCDR2 and HCDR3 respectively comprising the sequences as set forth in SEQ ID NO: 45, 133, 219, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 288, 343, 416, or a sequence containing an amino acid substitution (e.g., conservative substitution), deletion or insertion of one or more and no more than 3 amino acids relative to the sequence;
90) HCDR1, HCDR2 and HCDR3 respectively comprising the sequences as set forth in SEQ ID NO: 46, 134, 220, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 289, 324, 417, or a sequence containing an amino acid substitution (e.g., conservative substitution), deletion or insertion of one or more and no more than 3 amino acids relative to the sequence;
91) HCDR1, HCDR2 and HCDR3 respectively comprising the sequences as set forth in SEQ ID NO: 46, 134, 220, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 290, 344, 418, or a sequence containing an amino acid substitution (e.g., conservative substitution), deletion or insertion of one or more and no more than 3 amino acids relative to the sequence;
92) HCDR1, HCDR2 and HCDR3 respectively comprising the sequences as set forth in SEQ ID NO: 47, 135, 221, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 291, 345, 407, or a sequence containing an amino acid substitution (e.g., conservative substitution), deletion or insertion of one or more and no more than 3 amino acids relative to the sequence;
93) HCDR1, HCDR2 and HCDR3 respectively comprising the sequences as set forth in SEQ ID NO: 47, 135, 221, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 290, 344, 418, or a sequence containing an amino acid substitution (e.g., conservative substitution), deletion or insertion of one or more and no more than 3 amino acids relative to the sequence;
94) HCDR1, HCDR2 and HCDR3 respectively comprising the sequences as set forth in SEQ ID NO: 48, 136, 222, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 292, 346, 409, or a sequence containing an amino acid substitution (e.g., conservative substitution), deletion or insertion of one or more and no more than 3 amino acids relative to the sequence;
95) HCDR1, HCDR2 and HCDR3 respectively comprising the sequences as set forth in SEQ ID NO: 48, 136, 222, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 290, 344, 418, or a sequence containing an amino acid substitution (e.g., conservative substitution), deletion or insertion of one or more and no more than 3 amino acids relative to the sequence;
96) HCDR1, HCDR2 and HCDR3 respectively comprising the sequences as set forth in SEQ ID NO: 49, 137, 163, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 240, 347, 419, or a sequence containing an amino acid substitution (e.g., conservative substitution), deletion or insertion of one or more and no more than 3 amino acids relative to the sequence;
97) HCDR1, HCDR2 and HCDR3 respectively comprising the sequences as set forth in SEQ ID NO: 45, 138, 223, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 288, 343, 416, or a sequence containing an amino acid substitution (e.g., conservative substitution), deletion or insertion of one or more and no more than 3 amino acids relative to the sequence;
98) HCDR1, HCDR2 and HCDR3 respectively comprising the sequences as set forth in SEQ ID NO: 50, 139, 224, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 249, 348, 420, or a sequence containing an amino acid substitution (e.g., conservative substitution), deletion or insertion of one or more and no more than 3 amino acids relative to the sequence;
99) HCDR1, HCDR2 and HCDR3 respectively comprising the sequences as set forth in SEQ ID NO: 51, 140, 225, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 256, 349, 421, or a sequence containing an amino acid substitution (e.g., conservative substitution), deletion or insertion of one or more and no more than 3 amino acids relative to the sequence;
100) HCDR1, HCDR2 and HCDR3 respectively comprising the sequences as set forth in SEQ ID NO: 47, 141, 221, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 293, 345, 407, or a sequence containing an amino acid substitution (e.g., conservative substitution), deletion or insertion of one or more and no more than 3 amino acids relative to the sequence;
101) HCDR1, HCDR2 and HCDR3 respectively comprising the sequences as set forth in SEQ ID NO: 52, 142, 226, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 294, 350, 422, or a sequence containing an amino acid substitution (e.g., conservative substitution), deletion or insertion of one or more and no more than 3 amino acids relative to the sequence;
102) HCDR1, HCDR2 and HCDR3 respectively comprising the sequences as set forth in SEQ ID NO: 53, 143, 178, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 295, 311, 423, or a sequence containing an amino acid substitution (e.g., conservative substitution), deletion or insertion of one or more and no more than 3 amino acids relative to the sequence;
103) HCDR1, HCDR2 and HCDR3 respectively comprising the sequences as set forth in SEQ ID NO: 53, 144, 178, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 361, or a sequence containing an amino acid substitution (e.g., conservative substitution), deletion or insertion of one or more and no more than 3 amino acids relative to the sequence;
104) HCDR1, HCDR2 and HCDR3 respectively comprising the sequences as set forth in SEQ ID NO: 53, 145, 227, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 361, or a sequence containing an amino acid substitution (e.g., conservative substitution), deletion or insertion of one or more and no more than 3 amino acids relative to the sequence;
105) HCDR1, HCDR2 and HCDR3 respectively comprising the sequences as set forth in SEQ ID NO: 54, 146, 228, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 296, 328, 391, or a sequence containing an amino acid substitution (e.g., conservative substitution), deletion or insertion of one or more and no more than 3 amino acids relative to the sequence;
106) HCDR1, HCDR2 and HCDR3 respectively comprising the sequences as set forth in SEQ ID NO: 55, 147, 228, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 266, 328, 391, or a sequence containing an amino acid substitution (e.g., conservative substitution), deletion or insertion of one or more and no more than 3 amino acids relative to the sequence;
107) HCDR1, HCDR2 and HCDR3 respectively comprising the sequences as set forth in SEQ ID NO: 50, 148, 229, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 297, 351, 424, or a sequence containing an amino acid substitution (e.g., conservative substitution), deletion or insertion of one or more and no more than 3 amino acids relative to the sequence;
108) HCDR1, HCDR2 and HCDR3 respectively comprising the sequences as set forth in SEQ ID NO: 56, 149, 230, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 298, 323, 425, or a sequence containing an amino acid substitution (e.g., conservative substitution), deletion or insertion of one or more and no more than 3 amino acids relative to the sequence;
109) HCDR1, HCDR2 and HCDR3 respectively comprising the sequences as set forth in SEQ ID NO: 57, 150, 231, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 299, 338, 407, or a sequence containing an amino acid substitution (e.g., conservative substitution), deletion or insertion of one or more and no more than 3 amino acids relative to the sequence;
110) HCDR1, HCDR2 and HCDR3 respectively comprising the sequences as set forth in SEQ ID NO: 58, 151, 232, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 300, 352, 426, or a sequence containing an amino acid substitution (e.g., conservative substitution), deletion or insertion of one or more and no more than 3 amino acids relative to the sequence;
111) HCDR1, HCDR2 and HCDR3 respectively comprising the sequences as set forth in SEQ ID NO: 59, 152, 233, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 301, 328, 388, or a sequence containing an amino acid substitution (e.g., conservative substitution), deletion or insertion of one or more and no more than 3 amino acids relative to the sequence;
112) HCDR1, HCDR2 and HCDR3 respectively comprising the sequences as set forth in SEQ ID NO: 60, 153, 234, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 239, 308, 360, or a sequence containing an amino acid substitution (e.g., conservative substitution), deletion or insertion of one or more and no more than 3 amino acids relative to the sequence;
113) HCDR1, HCDR2 and HCDR3 respectively comprising the sequences as set forth in SEQ ID NO: 54, 146, 228, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 302, 328, 391, or a sequence containing an amino acid substitution (e.g., conservative substitution), deletion or insertion of one or more and no more than 3 amino acids relative to the sequence;
114) HCDR1, HCDR2 and HCDR3 respectively comprising the sequences as set forth in SEQ ID NO: 61, 154, 235, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 241, 311, 361, or a sequence containing an amino acid substitution (e.g., conservative substitution), deletion or insertion of one or more and no more than 3 amino acids relative to the sequence;
115) HCDR1, HCDR2 and HCDR3 respectively comprising the sequences as set forth in SEQ ID NO: 40, 125, 210, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 282, 353, 409, or a sequence containing an amino acid substitution (e.g., conservative substitution), deletion or insertion of one or more and no more than 3 amino acids relative to the sequence;
116) HCDR1, HCDR2 and HCDR3 respectively comprising the sequences as set forth in SEQ ID NO: 44, 155, 236, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 303, 324, 409, or a sequence containing an amino acid substitution (e.g., conservative substitution), deletion or insertion of one or more and no more than 3 amino acids relative to the sequence;
117) HCDR1, HCDR2 and HCDR3 respectively comprising the sequences as set forth in SEQ ID NO: 44, 156, 217, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 282, 341, 414, or a sequence containing an amino acid substitution (e.g., conservative substitution), deletion or insertion of one or more and no more than 3 amino acids relative to the sequence;
118) HCDR1, HCDR2 and HCDR3 respectively comprising the sequences as set forth in SEQ ID NO: 62, 157, 237, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 304, 332, 427, or a sequence containing an amino acid substitution (e.g., conservative substitution), deletion or insertion of one or more and no more than 3 amino acids relative to the sequence;
119) HCDR1, HCDR2 and HCDR3 respectively comprising the sequences as set forth in SEQ ID NO: 24, 158, 184, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 247, 326, 428, or a sequence containing an amino acid substitution (e.g., conservative substitution), deletion or insertion of one or more and no more than 3 amino acids relative to the sequence;
120) HCDR1, HCDR2 and HCDR3 respectively comprising the sequences as set forth in SEQ ID NO: 3, 159, 189, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 249, 354, 382, or a sequence containing an amino acid substitution (e.g., conservative substitution), deletion or insertion of one or more and no more than 3 amino acids relative to the sequence;
121) HCDR1, HCDR2 and HCDR3 respectively comprising the sequences as set forth in SEQ ID NO: 63, 160, 238, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 305, 355, 429, or a sequence containing an amino acid substitution (e.g., conservative substitution), deletion or insertion of one or more and no more than 3 amino acids relative to the sequence; or
122) HCDR1, HCDR2 and HCDR3 respectively comprising the sequences as set forth in SEQ ID NO: 42, 161, 214, or a sequence containing an amino acid substitution (e.g., conservative substitution), deletion or insertion of one or more and no more than 3 amino acids relative to the sequence; and LCDR1, LCDR2 and LCDR3 respectively comprising the sequences as set forth in SEQ ID NO: 306, 356, 409, or a sequence containing an amino acid substitution (e.g., conservative substitution), deletion or insertion of one or more and no more than 3 amino acids relative to the sequence.

3. The anti-CD112R antibody or antigen-binding fragment thereof of any one of claims 1 to 2, wherein the antigen-binding fragment is selected from Fab, Fab'-SH, Fv (e.g. scFv) or (Fab')2 fragments.

4. The anti-CD112R antibody or antigen-binding fragment thereof of any one of claims 1 to 3, comprising a constant region sequence, wherein at least a portion of the constant region sequence is a human consensus constant region sequence.

5. The antibody or antigen-binding fragment thereof of any one of claims 1 to 4, wherein the heavy chain constant region of the antibody comprises the amino acid sequence as set forth in SEQ ID NO: 659, and the light chain constant region comprises the amino acid sequence as set forth in SEQ ID NO: 660.

6. An isolated polynucleotide molecule encoding the anti-CD112R antibody or antigen-binding fragment thereof of any one of claims 1 to 5.

7. An expression vector comprising the nucleotide molecule of claim 6, preferably, the expression vector is a eukaryotic expression vector.

8. A host cell comprising the expression vector of claim 7 or the polynucleotide molecule of claim 6.

9. A pharmaceutical composition comprising (1) the anti-CD112R antibody or antigen-binding fragment thereof of any one of claims 1 to 5, or the polynucleotide molecule of claim 6, or the expression vector of claim 7, or the host cell of claim 8, and (2) a pharmaceutically acceptable carrier or excipient.

10. A method for preventing or treating a CD112R-mediated disease in a subject in need thereof, comprising administering to the subject a prophylactically effective amount or a therapeutically effective amount of the antibody or antigen-binding fragment thereof of any one of claims 1 to 5, or the polynucleotide molecule of claim 6, or the expression vector of claim 7, or the host cell of claim 8, or the pharmaceutical composition of claim 9.

11. Use of the anti-CD112R antibody or antigen-binding fragment thereof of any one of claims 1 to 5, or the polynucleotide molecule of claim 6, or the expression vector of claim 7, or the host cell of claim 8, or the pharmaceutical composition of claim 9 in the preparation of a medicament for treating and/or preventing a CD112R-mediated disease or disorder.

12. The method of claim 10 or the use of claim 11, wherein the CD112R-mediated disease or disorder is a CD112R-associated cancer, preferably selected from melanoma, renal cancer, prostate cancer, breast cancer, colon cancer, lung cancer, bone cancer, pancreatic cancer, skin cancer, head and neck cancer, uterine cancer, ovarian cancer and rectal cancer.
